# EUROPEAN PATENT APPLICATION

(11) **EP 4 006 162 A1**
(43) Date of publication of application: **01.06.2022**
(21) Application number: 20843995.0
(22) Date of filing: 22.07.2020
(51) Int. Cl.: C12P 13/00, C12N 1/15, C12N 1/19, C12N 1/21, C12N 15/31, C12N 15/53, C12N 15/54

(54) **GENETICALLY MODIFIED MICROORGANISM AND METHOD FOR PRODUCING DIAMINE COMPOUND**

(30) Priority: 22.07.2019 JP 2019134306
(71) Applicant: Asahi Kasei Kabushiki Kaisha, Tokyo 1000006 (JP)
(72) Inventor: YAMADA Yutaro, Tokyo 100-0006 (JP); YONEDA Hisanari, Tokyo 100-0006 (JP)
(74) Representative: D Young & Co LLP
(86) International application number: PCT/JP2020/028456
(87) International publication number: WO 2021/015242

(57) **Abstract**

Provided are a microorganism that produces a diamine compound and a method of producing a diamine compound.

The genetically modified microorganism expresses an enzyme involved in synthesis of a diamine compound, in which the diamine compound is represented by Formula: H₂N-R-NH₂ (wherein, R is a chain or cyclic organic group comprised of one or more atoms selected from the group consisting of C, H, 0, N, and S), and the genetically modified microorganism is modified to reduce an activity of an alcohol dehydrogenase compared to a non-reduced strain.

## Description

### Technical Field

The present invention relates to a genetically modified microorganism that produces a diamine compound and a method of producing a diamine compound.

### Background Art

A diamine compound is widely used as a raw material of a polymer such as a polyamide resin. As the diamine compound that is industrially used, examples of representative compounds include hexamethylenediamine (1,6-diaminohexane), heptamethylenediamine (1,7-diaminoheptane), octamethylenediamine (1,8-diaminooctane), decamethylenediamine (1,10-diaminodecane), and dodecamethylenediamine (1,12-diaminododecane).

For example, hexamethylenediamine is synthesized by obtaining adiponitrile through hydrogenation of butadiene, electrolytic dimerization of acrylonitrile, or nitridation of adipic acid, and further performing hydrogenation using nickel as a catalyst. (Non Patent Literature 1) Hexamethylenediamine is industrially produced by this method, but adiponitrile is once synthesized, and then, a hydrogenation reaction is performed. In addition, as for decanediamine, octanediamine, dodecanediamine, or the like, similarly to the above adipic acid raw material, a method of obtaining a corresponding dinitrile and synthesizing the dinitrile by hydrogenation is known. (Patent Literatures 1 and 2)

In recent years, in a chemical product producing process, it is desired to switch from a fossil fuel-derived raw material which may be depleted and contributes to global warming to a renewable raw material such as a biomass-derived raw material. In order to solve this problem, a method of producing a diamine compound such as 1,3-diaminopropane (Non Patent Literature 2), 1,4-diaminobutane or 1,5-diaminopentane (Non Patent Literature 3), or 4-aminophenylethylamine (Non Patent Literature 4) using a microorganism metabolically modified by a genetic modification is disclosed.

Among them, a method of producing diamine obtained by combining an exogeneous enzyme, for example, a carboxylic acid decarboxylase or aminotransferase from a dicarboxylic acid or an aminocarboxylic acid, a dialdehyde, and the like in cells using a genetically modified microorganism has wide applicability of a compound as a substrate, and for example, hexamethylenediamine (Patent Literatures 3 and 4) and heptamethylenediamine (Patent Literature 5) have been reported.

Patent Literature 3 expects and exemplifies an enzyme gene whose yield is expected to improve due to deletion or disruption in a microbial host modified to have a hexamethylenediamine production pathway based on a metabolic simulation in in silico. However, neither a by-product derived from an intermediate in the hexamethylenediamine production pathway nor a method of suppressing the same is mentioned.

Patent Literature 4 describes a method of producing hexamethylenediamine by an enzymatic reaction pathway via 6-hydroxyhexanoic acid. However, neither production of a by-product derived from an intermediate in a hexamethylenediamine production pathway newly constructed by a genetic modification nor a suppression method thereof is mentioned.

Patent Literature 5 describes a method of producing heptamethylenediamine using an enzymatic reaction pathway via pimelic acid or the like. However, neither a by-product derived from an intermediate in a reaction pathway nor a suppression method thereof is mentioned.

Therefore, the prior arts do not disclose production of a by-product due to a conversion pathway to a diamine compound or a suppression method thereof, at all. In the production of the diamine compound by the genetically modified microorganism, a technique capable of more efficiently suppressing a by-product and efficiently producing a diamine compound is required.

### Citation List

### Patent Literatures

Patent Literature 1: JP 57-70842 A
Patent Literature 2: JP 49-24446 B
Patent Literature 3: JP 2015-146810 A
Patent Literature 4: JP 2017-544854 A
Patent Literature 5: JP 2014-525741 A

### Non Patent Literatures

Non Patent Literature 1: Process Economics Program Report 31B (IHS market)
Non Patent Literature 2: Chae, T. et al., Metabolic engineering of Escherichia coli for the production of 1,3-diaminopropane, a three carbon diamine., Sci Rep. 2015 Aug 11; 5:13040
Non Patent Literature 3: Tsuge,Y. et al., Engineering cell factories for producing building block chemicals for biopolymer synthesis., Microb. Cell Fact., Vol., 15, 19 (2016)
Non Patent Literature 4: Masuo, S., et al, Bacterial fermentation platform for producing artificial aromatic amines., Scientific Reports volume 6, Article number: 25764 (2016)

### Summary of Invention

### Technical Problem

An object of the present invention is to provide a microorganism that produces a diamine compound and a method of producing a diamine compound.

### Solution to Problem

In conducting the studies, the present inventors found that an alcohol form derived from a diamine biosynthetic pathway intermediate is generated as a by-product by an endogenous alcohol dehydrogenase activity in a microorganism having a diamine compound production pathway. As a result of conducting intensive studies, the present inventors achieved the present invention based on the findings that production of an alcohol form that is a by-product can be suppressed and/or a production amount of a diamine compound can be increased by performing a modification so as to reduce an alcohol dehydrogenase activity of a host microorganism.

That is, the present invention provides the following:
[1] A genetically modified microorganism that expresses an enzyme involved in synthesis of a diamine compound, in which
   the diamine compound is represented by Formula: H₂N-R-NH₂
   (wherein, R is a chain or cyclic organic group comprised of one or more atoms selected from the group consisting of C, H, O, N, and S), and
   the genetically modified microorganism is modified to reduce an activity of an alcohol dehydrogenase compared to a non-reduced strain;
[2] The genetically modified microorganism according to [1], in which the modification performed to reduce the activity of the alcohol dehydrogenase compared to the non-reduced strain is
   a modification to suppress expression of a gene encoding an alcohol dehydrogenase or
   a modification to suppress expression of a gene encoding an alcohol dehydrogenase and to suppress an activity of an alcohol dehydrogenase;
[3] The modified microorganism according to [1] or [2], in which the alcohol dehydrogenase is a protein encoded by
   DNA consisting of a base sequence selected from SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, and 100,
   DNA consisting of a base sequence having 85% or more of sequence identity with a base sequence selected from SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, and 100 and encoding a protein having an alcohol dehydrogenase activity,
   DNA consisting of a base sequence encoding a protein consisting of an amino acid sequence obtained by deleting, substituting, inserting, and/or adding 1 to 10 amino acids with respect to an amino acid sequence of a protein encoded by a base sequence selected from SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, and 100 and encoding a protein having an alcohol dehydrogenase activity, or
   DNA consisting of a degenerate isomer of a base sequence selected from SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, and 100;
[4] The modified microorganism according to any one of [1] to [3], in which the alcohol dehydrogenase is a protein containing an amino acid sequence having 80% or more of sequence identity with an amino acid sequence selected from SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, and 99 and having an alcohol dehydrogenase activity;
[5] The genetically modified microorganism according to any one of [1] to [4], in which the alcohol dehydrogenase is a protein encoded by at least one gene selected from the group consisting of yqhD, fucO, adhP, ybbO, eutG, ahr, yahK, adhE, ybdR, dkgA, yiaY, frmA, dkgB, yghA, ydjG, gldA, yohF, yeaE, ADH1, ADH2, ADH3, ADH4, ADH5, ADH6, ADH7, SFA1, AAD3, AAD4, AAD10, AAD14, AAD15, YPR1, NCgl0324, NCgl0313, NCgl0219, NCgl2709, NCgl1112, NCgl2382, NCgl0186, NCgl0099, NCgl2952, NCgl1459, yogA, bdhK, bdhJ, akrN, yqkF, yccK, iolS, and yrpG;
[6] The genetically modified microorganism according to any one of [1] to [5], in which the alcohol dehydrogenase is a protein encoded by at least one gene selected from the group consisting of yqhD, fucO, adhP, eutG, ybbO, ahr, and yahK;
[7] The genetically modified microorganism according to any one of [1] to [6], in which the alcohol dehydrogenase is a protein encoded by at least one gene selected from the group consisting of yqhD and adhP;
[8] The genetically modified microorganism according to [7], in which the alcohol dehydrogenase is a protein encoded by an adhP gene;
[9] The genetically modified microorganism according to any one of [1] to [6], in which the alcohol dehydrogenase is a protein encoded by at least one gene selected from the group consisting of yqhD, fucO, eutG, ybbO, ahr, and yahK;
[10] The genetically modified microorganism according to [9], in which the alcohol dehydrogenase is a protein encoded by at least one gene selected from the group consisting of eutG, ybbO, ahr, and yahK;
[11] The genetically modified microorganism according to any one of [1] to [6], in which the alcohol dehydrogenase is a protein encoded by two or more genes selected from the group consisting of yqhD, fucO, adhP, eutG, ybbO, ahr, and yahK;
[12] The genetically modified microorganism according to any one of [1] to [6], in which the alcohol dehydrogenase is a protein encoded by a gene of one combination selected from the group consisting of:
   - yqhD and fucO,
   - yqhD and adhP,
   - yqhD and eutG,
   - yqhD and ybbO,
   - yqhD and ahr,
   - yqhD and yahK,
   - yqhD, fucO, and adhP,
   - yqhD, fucO, adhP, and eutG,
   - yqhD, fucO, adhP, eutG, and ybbO,
   - yqhD, fucO, adhP, eutG, ybbO, and ahr,
      and
   - yqhD, fucO, adhP, eutG, ybbO, ahr, and yahK;
[13] The modified microorganism according to any one of [1] to [12], in which the modification performed to reduce the activity of the alcohol dehydrogenase compared to the non-reduced strain is performed by one or more selected from the group consisting of
   a reduction in transcription amount and/or translation amount of a gene encoding the alcohol dehydrogenase in the microorganism and
   a disruption of a gene encoding the alcohol dehydrogenase in the microorganism;
[14] The genetically modified microorganism according to any one of [1] to [13], in which the genetically modified microorganism belongs to a genus selected from the group consisting of the genus Escherichia, the genus Corynebacterium, the genus Bacillus, the genus Acinetobacter, the genus Burkholderia, the genus Pseudomonas, the genus Clostridium, the genus Saccharomyces, the genus Schizosaccharomyces, the genus Yarrowia, the genus Candida, the genus Pichia, and the genus Aspergillus;
[15] The genetically modified microorganism according to any one of [1] to [14], in which the genetically modified microorganism is Escherichia coli;
[16] The genetically modified microorganism according to any one of [1] to [15], in which the genetically modified microorganism expresses an aminotransferase as the enzyme involved in the synthesis of the diamine compound;
[17] The genetically modified microorganism according to any one of [1] to [16], in which the genetically modified microorganism expresses a carboxylic acid reductase as the enzyme involved in the synthesis of the diamine compound;
[18] The genetically modified microorganism according to [17], in which the carboxylic acid reductase has an activity of converting a carboxyl group of a carboxylic acid semialdehyde, a dicarboxylic acid, or an aminocarboxylic acid into an aldehyde;
[19] The genetically modified microorganism according to any one of [1] to [18], in which the genetically modified microorganism
   has an ability of producing a dicarboxylic acid, a carboxylic acid semialdehyde, or an aminocarboxylic acid, and
   further expresses an aminotransferase and a carboxylic acid reductase;
[20] The genetically modified microorganism according to any one of [1] to [19], in which the genetically modified microorganism
   has an ability of producing adipic acid, adipic acid semialdehyde, or 6-aminohexanoic acid, and
   further expresses an aminotransferase and a carboxylic acid reductase;
[21] The genetically modified microorganism according to any one of [11] to [20], in which the genetically modified microorganism is further modified to increase an activity of a phosphopantetheinyl transferase;
[22] The genetically modified microorganism according to any one of [16] to [21], in which a gene encoding the aminotransferase is ygjG;
[23] The genetically modified microorganism according to any one of [17] to [22], in which a gene encoding the carboxylic acid reductase is MaCar;
[24] The genetically modified microorganism according to any one of [21] to [23], in which a gene encoding the phosphopantetheinyl transferase is Npt;
[25] The modified microorganism according to any one of [1] to [24], in which the modified microorganism contains
   a base sequence having 85% or more of sequence identity with a base sequence set forth in SEQ ID NO: 115 and encoding a protein having an aminotransferase activity or
   a base sequence having 85% or more of sequence identity with a base sequence encoding an amino acid sequence set forth in any one of SEQ ID NOs: 110 to 114 and encoding a protein having an aminotransferase activity;
[26] The modified microorganism according to any one of [1] to [25], in which the modified microorganism contains
   a base sequence having 85% or more of sequence identity with a base sequence set forth in SEQ ID NO: 105 and encoding a protein having a carboxylic acid reductase activity or
   a base sequence having 85% or more of sequence identity with a base sequence encoding an amino acid sequence set forth in any one of SEQ ID NOs: 101 to 104 and encoding a protein having a carboxylic acid reductase activity;
[27] The modified microorganism according to any one of [21] to [26], in which the modified microorganism contains
   a base sequence having 85% or more of sequence identity with a base sequence set forth in SEQ ID NO: 109 and encoding a protein having a phosphopantetheinyl transferase activity or
   a base sequence having 80% or more of sequence identity with a base sequence encoding an amino acid sequence set forth in any one of SEQ ID NOs: 106 to 108 and encoding a protein having a phosphopantetheinyl transferase activity;
[28] The genetically modified microorganism according to any one of [1] to [27], in which the genetically modified microorganism expresses one or more enzymes selected from the group consisting of
   acyl-(acyl carrier protein (ACP)) reductase (AAR),
   an enzyme that produces an aldehyde from acyl-CoA, and
   an enzyme that produces an aldehyde from acyl phosphate;
[29] A method of producing a diamine compound using the genetically modified microorganism according to any one of [1] to [28];
[30] A method of producing a diamine compound, the method including a culture step of culturing the genetically modified microorganism according to [1] to [28] in a medium containing a carbon source and a nitrogen source to obtain a culture medium containing bacterial cells;
[31] The method of producing a diamine compound according to [30], in which
   the medium further contains a precursor of a diamine compound, or
   in the culture step, the precursor is added to the medium;
[32] The method of producing a diamine compound according to [30] or [31], further including a reaction step of bringing the culture medium and/or the bacterial cells into contact with an aqueous solution containing a precursor of a diamine compound to obtain a reaction solution containing a diamine compound; and
[33] The method of producing a diamine compound according to [31] or [32], in which the precursor is selected from the group consisting of a dicarboxylic acid, a carboxylic acid semialdehyde, an aminocarboxylic acid, an aminoaldehyde, a dialdehyde, acyl-ACP, acyl-CoA, and acyl phosphate.

### Advantageous Effects of Invention

According to the present invention, a diamine compound can be produced.

### Brief Description of Drawings

Fig. 1 schematically illustrates conversion of functional groups from various precursors into an amine as an example of a production pathway for a diamine compound in a genetically modified microorganism of the present invention.
Fig. 2 illustrates a concentration of 1,6-hexanediol in a culture supernatant after culturing an E. coli strain in which an ADH gene is disrupted in a medium containing 1,6-hexanediol for 48 hours.
Fig. 3 is a plasmid map of pDA56, in which "lacI" represents an lacI gene, "T7 Promoter" represents a T7 promotor, "T7 Terminator" represents a T7 terminator, "ygjG" represents a ygjG gene derived from Escherichia coli, "MaCar" represents a carboxylic acid reductase gene derived from Mycobacterium abcessus, "Npt" represents a phosphopantetheinyl transferase gene derived from Nocardia iowensis, "CAT" represents a chloramphenicol acetyltransferase gene, and "P15Aori" represents a replication point.

### Description of Embodiments

Hereinafter, embodiments of the present invention will be described in detail.

A genetically modified microorganism according to the present invention is a genetically modified microorganism that has a diamine compound production pathway and, at the same time, is modified to reduce an alcohol dehydrogenase activity. Here, the modification includes substitution, deletion, insertion, and/or addition. Hereinafter, the "genetically modified microorganism" is simply referred to as a "modified microorganism".

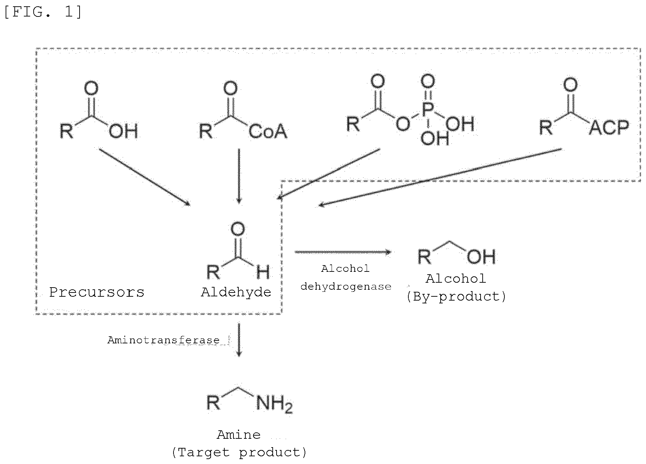

The modified microorganism expresses an enzyme involved in synthesis of a diamine compound or an enzyme group involved in synthesis of a diamine compound. Examples of the enzyme involved in synthesis of a diamine compound include a carboxylic acid reductase and an aminotransferase. As illustrated in Fig. 1, the carboxylic acid reductase has an activity of converting, for example, a carboxyl group of a carboxylic acid semialdehyde, a dicarboxylic acid, or an aminocarboxylic acid into an aldehyde. As illustrated in Fig. 1, the aminotransferase has an activity of converting an aldehyde into an amine. In the present invention, the microorganism "that expresses an enzyme involved in synthesis of a diamine compound or an enzyme group involved in synthesis of a diamine compound" means that a host microorganism itself may have an ability of expressing an enzyme or an enzyme group or a host microorganism may be modified to express an enzyme or an enzyme group.

The "diamine compound" (hereinafter, simply referred to as a "diamine") in the present invention is represented by Formula: H₂N-R-NH₂. In the formula, R is a chain or cyclic divalent organic group comprised of one or more atoms selected from the group consisting of C, H, O, N, and S. A chain organic group includes a linear organic group and a branched organic group. A cyclic organic group includes an alicyclic organic group, a heterocyclic organic group, a polycyclic organic group, and an aromatic organic group.

Examples of the organic group constituting R include, but are not limited to, an aliphatic hydrocarbon group such as a methylene group, an ethylene group, a vinylene group, a trimethylene group, a propylene group, a propenylene group, a tetramethylene group, an isobutylene group, a pentamethylene group, a hexamethylene group, and an octamethylene group; an alicyclic hydrocarbon group such as a cyclobutylene group, a cyclopentylene group, a cyclohexylene group, a cycloheptylene group, a cyclooctylene group, a cyclohexenylene group, and a cyclohexadienylene group; an aromatic hydrocarbon group such as an o-phenylene group, an m-phenylene group, a p-phenylene group, a diphenylene group, a naphthylene group, a 1,2-phenylenedimethylene group, a 1,3-phenylenedimethylene group, a 1,4-phenylenedimethylene group, a 1,4-phenylylenediethylene group, a methylene diphenylene group, and an ethylene diphenylene group; an oxygen-containing characteristic group such as an oxy group and a carbonyl group; an ether group such as a methylenedioxy group and an ethylenedioxy group; an acyl group such as an oxalyl group, a malonyl group, a succinyl group, a glutalyl group, an adipoyl group, a speroyl group, an o-phthaloyl group, an m-phthaloyl group, and a p-phthaloyl group; a sulfur-containing characteristic group such as a thio group and a thiocarbonyl group; a nitrogen-containing characteristic group such as an imino group and an azo group; and a combination thereof.

In addition, one or more substituents may be included in R. Examples of the substituent that can be included in R include, but are not limited to, an amino group, a carboxy group, a cyano group, a nitro group, a hydroxy group, and a thiol group.

In one aspect, R is a chain or cyclic hydrocarbon, and a linear and branched, saturated and unsaturated hydrocarbons are included in the chain hydrocarbon. In a preferred aspect, R is a hydrocarbon group having 3 to 20 carbon atoms. More preferably, R is a linear saturated hydrocarbon group represented by Formula: CH₂(CH₂)ₙCH₂, wherein n is 1, 2, 3, 4, 5, 6, 7, 8, 9, or 10. Still more preferably, n is 2, 3, 4, 5, 6, 7, or 8, and particularly preferably, n is 4, 5, 6, 7, or 8.

Examples of a typical diamine compound include, but are not limited to, 1,3-diaminopropane (trimethylenediamine), 1,4-diaminobutane (tetramethylenediamine (putrescine)), 1,5-diaminopentane (pentamethylenediamine (cadaverine)), 1,6-diaminohexane (hexamethylenediamine), 1,7-diaminoheptane (heptamethylenediamine), 1,8-diaminooctane (octamethylenediamine), 1,9-diaminononane (nonamethylenediamine), 1,10-diaminodecane (decamethylenediamine), 1,11-diaminoundecane (undecamethylenediamine), 1,12-diaminododecane (dodecamethylenediamine), 3-aminobenzylamine, 4-aminobenzylamine, 2-methylpentamethylenediamine, 2-methylpentamethylenediamine, 2,2,4-trimethylhexamethylenediamine, 2,4,4-trimethylhexamethylenediamine, 1,3-bis(aminomethyl)cyclohexane, 1,4-bis(aminomethyl)cyclohexane, m-xylene diamine, p-xylene diamine, 1-amino-3-aminomethyl-3,5,5-trimethylcyclohexane, bis(4-aminocyclohexyl)methane, bis(4-amino-3-methylcyclohexyl)methane, 1,4-bis(aminopropyl)piperazine, 1,3-diaminocyclohexane, 1,4-diaminocyclohexane, 1,3-phenylenediamine, 1,4-phenylenediamine, N-(3-aminopropyl)1,4-butanediamine (spermidine), 3,3'-diaminodipropylamine, N,N-bis(3-aminopropyl)methylamine, N,N'-bis(3-aminopropyl)ethylenediamine, N,N'-bis(2-aminoethyl)-1,3-propanediamine, N,N'-bis(3-aminopropyl)-1,4-butanediamine (spermine), 2,2'-dithiobis(ethylamine), and dipropylenetriamine. It is appreciated by those skilled in the art that the diamine has a neutral or ionized form including an arbitrary salt form and that the form depends on pH.

The "dicarboxylic acid" in the present specification refers to a compound having a structure represented by a chemical formula HOOC-R-COOH (wherein, R is as described above). An aliphatic dicarboxylic acid and an aromatic carboxylic acid are included in the dicarboxylic acid. Examples of a typical dicarboxylic acid include, but are not limited to, oxalic acid, malonic acid, succinic acid, fumaric acid, itaconic acid, glutaric acid, adipic acid, muconic acid, pimelic acid, suberic acid, azelaic acid, sebacic acid, dodecanedioic acid, malic acid, 2,5-furandicarboxylic acid, phthalic acid, isophthalic acid, terephthalic acid, maleic acid, tartaric acid, and muconic acid. It is appreciated by those skilled in the art that the dicarboxylic acid has a neutral or ionized form including an arbitrary salt form and the form depends on pH.

The "carboxylic acid semialdehyde" in the present specification refers to a compound having a structure represented by a chemical formula HOOC-R-CHO (wherein, R is as described above). Examples of a typical carboxylic acid semialdehyde include, but are not limited to, succinic acid semialdehyde, glutaric acid semialdehyde, adipic acid semialdehyde, pimelic acid semialdehyde, suberic acid semialdehyde, azelaic acid semialdehyde, and sebacic acid semialdehyde. It is appreciated by those skilled in the art that the carboxylic acid semialdehyde has a neutral or ionized form including an arbitrary salt form and that the form depends on pH.

The "aminocarboxylic acid" in the present specification refers to a compound having a structure represented by a chemical formula H₂N-R-COOH (wherein, R is as described above). Examples of a typical aminocarboxylic acid include, but are not limited to, glycine, β-alanine, 4-aminobutanoic acid, 5-aminopentanoic acid, 6-aminohexanoic acid, 7-aminoheptanoic acid, 8-aminooctanoic acid, 9-aminononanoic acid, 10-aminodecanoic acid, and 12-aminododecanoic acid. It is appreciated by those skilled in the art that the aminocarboxylic acid has a neutral or ionized form including an arbitrary salt form and that the form depends on pH.

In the present specification, the term "endogenous" or "endogenous property" is used to mean that the host microorganism without a genetic modification has the gene referred to or the protein encoded by the same (typically, an enzyme), regardless of whether the gene or the protein is functionally expressed to the extent that a predominant biochemical reaction can proceed in the host cell.

In the present specification, the term "exogeneous" or "exogeneous property" is used to mean that a gene or a nucleic acid sequence according to the present invention is introduced into a host in a case where a pre-genetically modified host microorganism does not have a gene to be introduced according to the present invention, in a case where the pre-genetically modified host microorganism does not substantially express an enzyme by the gene, or in a case where the pre-genetically modified host microorganism does not express an endogenous enzyme activity corresponding to after the genetic modification although an amino acid sequence of the enzyme is encoded by a different gene. The term "exogeneous property" and "external property" are used interchangeably in the present specification.

Fig. 1 illustrates an example of conversion of functional groups of synthesis pathways for a diamine compound in the present invention. A diamine is synthesized using an aldehyde-inducible compound and/or an aldehyde as a precursor. The aldehyde is converted into an amine by an aminotransferase. The genetically modified microorganism according to the present invention is modified to reduce an activity of an alcohol dehydrogenase, thereby suppressing the conversion of the aldehyde that is an intermediate in the pathway into an alcohol. Here, the alcohol dehydrogenase includes one or more proteins having an alcohol dehydrogenase activity.

The host microorganism used in the present invention is not particularly limited, and may be either a prokaryote or a eukaryote. Any one of a microorganism isolated and preserved in advance, a microorganism newly isolated from nature, a microorganism subjected to a genetic modification, and a microorganism modified so that the compound can be metabolized can be arbitrarily selected. Examples thereof include, but are not limited to, a bacterium, for example, the genus Escherichia such as Escherichia coli (E. coli), the genus Pseudomonas such as Pseudomonas putida, the genus Bacillus such as Bacillus subtilis, the genus Corynebacterium such as Corynebacterium glutamicum, the genus Clostridium such as Clostridium acetobutylicum, the genus Acinetobacter, and the genus Burkholderia; a yeast, for example, the genus Saccharomyces such as Saccharomyces cerevisiae, the genus Schizosaccharomyces such as Schizosaccharomyces pombe, the genus Pichia such as Pichia pastoris, and the genus Yarrowia such as Yarrowia lipolytica; and a filamentous fungus, for example, the genus Aspergillus such as Aspergillus oryzae. In the present invention, E. coli is preferably used as a host microorganism.

The genetically modified microorganism according to the present invention is further modified to reduce an endogenous alcohol dehydrogenase (ADH) activity compared to a non-reduced strain. The present inventors found that in a host microorganism having a diamine compound production pathway, an alcohol form derived from a diamine biosynthetic pathway intermediate is produced as a by-product due to an endogenous alcohol dehydrogenase activity. After conducting further intensive studies, the present inventors found that production of an alcohol form that is a by-product can be suppressed and/or a production amount of a diamine compound is increased by modifying a host microorganism to reduce an activity of an alcohol dehydrogenase compared to a non-reduced strain, , resulting in efficient production of a diamine compound.

The alcohol dehydrogenase is an enzyme having an activity of reducing an aldehyde and a ketone to be converted into an alcohol in the presence of an electron donor. Here, the alcohol dehydrogenase also includes a protein containing an amino acid sequence in which one or more amino acids are deleted, substituted, inserted, and/or added in an amino acid sequence of the enzyme, the protein being functionally equivalent to the enzyme. Here, the "functionally equivalent protein" is a protein having the equivalent activity to the activity of the enzyme. For example, the "functionally equivalent protein" includes a protein having 80%, 85%, 90%, 95%, 97%, 98%, or 99% or more of sequence identity with the amino acid sequence of the enzyme. Specifically, the term "alcohol dehydrogenase" includes a protein containing an amino acid sequence having 80%, 85%, 90%, 95%, 97%, 98%, or 99% or more of sequence identity with the amino acid sequence set forth in the following specific sequence number, and having an alcohol dehydrogenase activity.

The gene encoding an alcohol dehydrogenase contains:
DNA consisting of a base sequence set forth in the following specific sequence number,
DNA hybridizing to DNA containing a base sequence complementary to a base sequence set forth in the following specific sequence number under a stringent condition and encoding a protein having an alcohol dehydrogenase activity,
DNA consisting of a base sequence having 85%, 90%, 95%, 97%, 98%, or 99% or more of sequence identity with a base sequence set forth in the following specific sequence number and encoding a protein having an alcohol dehydrogenase activity,
DNA consisting of a base sequence encoding a protein consisting of an amino acid sequence in which one or more (for example, 1 to 10, preferably 1 to 7, more preferably 1 to 5, still more preferably 1 to 3, and still further preferably 1 or 2) of amino acids are deleted, substituted, inserted, and/or added in an amino acid sequence of a protein encoded by a base sequence set forth in the following sequence number, and encoding a protein having an alcohol dehydrogenase activity,
   and
DNA consisting of a degenerate isomer of a base sequence set forth in the following specific sequence number.

The "stringent condition" is, for example, a condition of about "1xSSC, 0.1%SDS, 60°C", a more severe condition of about "0.1xSSC, 0.1%SDS, 60°C", and a still more severe condition of about "0.1xSSC, 0.1%SDS, 68°C".

In a preferred aspect of the present invention, an enzyme represented by EC 1.1.1.m (wherein, m is an integer of 1 or more) is included in the alcohol dehydrogenase. Examples of the alcohol dehydrogenase include, but are not limited to, enzymes classified as EC 1.1.1.1, EC 1.1.1.2, and EC 1.1.1.71.

In the case of E. coli, examples of the alcohol dehydrogenase include proteins encoded by yqhD, fucO, adhP, ybbO, eutG, ahr, yahK, adhE, ybdR, dkgA, yiaY, frmA, dkgB, yghA, ydjG, gldA, yohF, and yeaE genes.

In the case of Saccharomyces cerevisiae, examples of the alcohol dehydrogenase include proteins encoded by ADH1, ADH2, ADH3, ADH4, ADH5, ADH6, ADH7, SFA1, AAD3, AAD4, AAD10, AAD14, AAD15, and YPR1 genes. In the case of Corynebacterium glutamicum, examples of the alcohol dehydrogenase include proteins encoded by NCgl0324, NCgl0313, NCgl0219, NCgl2709, NCgl1112, NCgl2382, NCgl0186, NCgl0099, NCgl2952, and NCgl1459 genes. In the case of Bachillus subtilis, examples of the alcohol dehydrogenase include proteins encoded by a yogA, bdhK, bdhJ, akrN, yqkF, yccK, iolS, and yrpG genes. However, the alcohol dehydrogenase is not limited thereto as long as it has an alcohol dehydrogenase activity.

The alcohol dehydrogenase is a protein encoded by, for example, at least one gene selected from the group consisting of yqhD, fucO, adhP, eutG, ybbO, ahr, and yahK. Modification of at least one gene selected from the group consisting of the above genes such that the activity of the alcohol dehydrogenase is reduced compared to a non-reduced strain can suppress production of an alcohol form that is a by-product and/or increase a production amount of a diamine compound, resulting in efficient production of a diamine compound.

The alcohol dehydrogenase is preferably encoded by at least one gene selected from the group consisting of yqhD, fucO, adhP, ybbO, eutG, ahr, and yahK genes, more preferably encoded by at least one gene selected from the group consisting of yqhD, ahr, and yahK genes, and still more preferably encoded by at least one gene selected from the group consisting of ahr and yahK genes.

The alcohol dehydrogenase is preferably encoded by at least one gene selected from the group consisting of yqhD and adhP genes, and more preferably encoded by an adhP gene. In the production of the diamine compound, by modifying the microorganism to reduce the activity of at least one of these genes, the production amount of the diamine compound can be increased in the genetically modified microorganism.

The alcohol dehydrogenase is preferably encoded by at least one gene selected from the group consisting of yqhD, fucO, eutG, ybbO, ahr, and yahK, and more preferably encoded by at least one gene selected from the group consisting of eutG, ybbO, ahr, and yahK. In the production of the diamine compound, by modifying the microorganism to reduce the activity of at least one of these genes, the production of an alcohol form that is a by-product can be suppressed in the genetically modified microorganism.

The alcohol dehydrogenase is preferably encoded by two or more genes selected from the group consisting of yqhD, fucO, adhP, eutG, ybbO, ahr, and yahK, and more preferably encoded by a yqhD gene and one or more genes selected from the group consisting of fucO, adhP, eutG, ybbO, ahr, and yahK. In the production of the diamine compound, by modifying the microorganism to reduce the activities of two or more of these genes, the production amount of the diamine compound can be significantly increased, and production of an alcohol form that is a by-product can also be suppressed in the genetically modified microorganism.

The alcohol dehydrogenase is preferably encoded by a gene of one combination selected from the group consisting of
- yqhD and fucO,
- yqhD and adhP,
- yqhD and eutG,
- yqhD and ybbO,
- yqhD and ahr,
- yqhD and yahK,
- yqhD, fucO, and adhP,
- yqhD, fucO, adhP, and eutG,
- yqhD, fucO, adhP, eutG, and ybbO,
- yqhD, fucO, adhP, eutG, ybbO, and ahr,
   and
- yqhD, fucO, adhP, eutG, ybbO, ahr, and yahK. As described above, in the production of the diamine compound, the production amount of the diamine compound can be significantly increased, and production of an alcohol form that is a by-product can also be significantly suppressed, by modifying the microorganism to reduce the activities of two or more genes.

Amino acid sequences of typical proteins encoded by the aforementioned alcohol dehydrogenase genes and base sequences of coding regions are shown in Tables 1-1 to 1-50. In the first row of each table, genes, protein names, accession numbers, and origins are shown.

**[Table 1-1]**

| yqhD/ACT44688.1/Escherichia coli BL21(DE3) | |
|---|---|
| Amino acid sequence | MNNFNLHTPTRILFGKGAIAGLREQIPHDARVLITYGGGSVKKTGVLDQVLDALKGM DVLEFGGIEPNPAYETLMNAVKLVREQKVTFLLAVGGGSVLDGTKFIAAAANYPENID PWHILQTGGKEIKSAIPMGCVLTLPATGSESNAGAVISRKTTGDKQAFHSAHVQPVFA VLDPVYTYTLPPRQVANGVVDAFVHTVEQYVTKPVDAKIQDRFAEGILLTLIEDGPK ALKEPENYDVRANVMWAATQALNGLIGAGVPQDWATHMLGHELTAMHGLDHAQT LAIVLPALWNEKRDTKRAKLLQYAERVWNITEGSDDERIDAAIAATRNFFEQLGVPTH LSDYGLDGSSIPALLKKLEEHGMTQLGENHDITLDVSRRIYEAAR (SEQ ID NO: 1) |
| Base sequence | ATGAACAACTTTAATCTGCACACCCCAACCCGCATTCTGTTTGGTAAAGGCGCAAT CGCTGGTTTACGCGAACAAATTCCTCACGATGCTCGCGTATTGATTACCTACGGCG GCGGCAGCGTGAAAAAAACCGGCGTTCTCGATCAAGTTCTGGATGCCCTGAAAGG CATGGACGTGCTGGAATTTGGCGGTATTGAGCCAAACCCGGCTTATGAAACGCTG ATGAACGCCGTGAAACTGGTTCGCGAACAGAAAGTGACTTTCCTGCTGGCGGTTG |
| | GCGGCGGTTCTGTACTGGACGGCACCAAATTTATCGCCGCAGCGGCTAACTATCCG GAAAATATCGATCCGTGGCACATTCTGCAAACGGGCGGTAAAGAGATTAAAAGCG CCATCCCGATGGGCTGTGTGCTGACGCTGCCAGCAACCGGTTCAGAATCCAACGC AGGCGCGGTGATCTCCCGTAAAACCACAGGCGACAAGCAGGCGTTCCATTCTGCC CATGTTCAGCCGGTATTTGCCGTGCTCGATCCGGTTTATACCTACACCCTGCCGCCG CGTCAGGTGGCTAACGGCGTAGTGGACGCCTTTGTACACACCGTGGAACAGTATG TTACCAAACCGGTTGATGCCAAAATTCAGGACCGTTTCGCAGAAGGCATTTTGCTG ACGCTAATCGAAGATGGTCCGAAAGCCCTGAAAGAGCCAGAAAACTACGATGTGC GCGCCAACGTCATGTGGGCGGCGACTCAGGCGCTGAACGGTTTGATTGGCGCTGG CGTACCGCAGGACTGGGCAACGCATATGCTGGGCCACGAACTGACTGCGATGCAC GGTCTGGATCACGCGCAAACACTGGCTATCGTCCTGCCTGCACTGTGGAATGAAA AACGCGATACCAAGCGCGCTAAGCTGCTGCAATATGCTGAACGCGTCTGGAACAT CACTGAAGGTTCCGATGATGAGCGTATTGACGCCGCGATTGCCGCAACCCGCAATT TCTTTGAGCAATTAGGCGTGCCGACCCACCTCTCCGACTACGGTCTGGACGGCAG CTCCATCCCGGCTTTGCTGAAAAAACTGGAAGAGCACGGCATGACCCAACTGGGC GAAAATCATGACATTACGTTGGATGTCAGCCGCCGTATATACGAAGCCGCCCGCTA A (SEQ ID NO: 2) |

**[Table 1-2]**

| fucO/ACT44461.1/Escherichia coli BL21(DE3) | |
|---|---|
| Amino acid sequence | MMANRMILNETAWFGRGAVGALTDEVKRRGYQKALIVTDKTLVQCGVVAKVTDKM DAAGLAWAIYDGVVPNPTITVVKEGLGVFQNSGADYLIAIGGGSPQDTCKAIGIISNN PEFADVRSLEGLSPTNKPSVPILAIPTTAGTAAEVTINYVITDEEKRRKFVCVDPHDIPQ VAFIDADMMDGMPPALKAATGVDALTHAIEGYITRGAWALTDALHIKAIEIIAGALRG SVAGDKDAGEEIALGQYVAGMGFSNVGLGLVHGMAHPLGAFYNTPHGVANAILLPH VMRYNADFTGEKYRDIARVMGVKVEGMSLEEARNAAVEAVFALNRDVGIPPHLRDV GVRKEDIPALAQAALNDVCTGGNPREATLEDIVELYHTAW (SEQ ID NO: 3) |
| Base sequence | ATGATGGCTAACAGAATGATTCTGAACGAAACGGCATGGTTTGGTCGGGGTGCTG TTGGGGCTTTAACCGATGAGGTGAAACGCCGTGGTTATCAGAAGGCGCTGATCGT CACCGATAAAACGCTGGTGCAATGCGGCGTGGTGGCGAAAGTGACCGATAAGATG GATGCTGCAGGGCTGGCATGGGCGATTTACGACGGCGTAGTGCCCAACCCAACAA TTACTGTCGTCAAAGAAGGGCTCGGTGTATTCCAGAATAGCGGCGCGGATTACCTG ATCGCTATTGGTGGTGGTTCTCCACAGGATACTTGTAAAGCGATTGGCATTATCAGC AACAACCCGGAGTTTGCCGATGTGCGTAGCCTGGAAGGGCTTTCCCCGACCAATA AACCCAGTGTACCGATTCTGGCAATCCCCACCACAGCAGGCACTGCGGCAGAAGT GACCATTAACTACGTGATCACTGACGAAGAAAAACGGCGCAAGTTTGTTTGCGTT GATCCGCATGATATCCCGCAGGTGGCGTTTATTGACGCTGACATGATGGATGGTATG CCTCCAGCGCTGAAAGCTGCGACGGGTGTCGATGCGCTCACTCATGCTATTGAGG GGTATATTACCCGTGGCGCGTGGGCGCTAACCGATGCACTGCACATTAAAGCGATT GAAATCATTGCTGGGGCGCTGCGAGGATCGGTTGCTGGTGATAAGGATGCCGGAG AAGAAATAGCGCTCGGGCAGTATGTTGCGGGTATGGGCTTCTCGAATGTTGGGTTA GGGTTGGTGCATGGTATGGCGCATCCACTGGGCGCGTTTTATAACACTCCACACGG TGTTGCAAACGCCATCCTGCTACCGCATGTCATGCGCTATAACGCTGACTTTACCG |
| | GTGAGAAGTACCGCGATATCGCGCGCGTTATGGGCGTGAAAGTGGAAGGTATGAG CCTGGAAGAGGCGCGTAATGCCGCTGTTGAAGCGGTGTTTGCTCTCAACCGTGAT GTCGGTATTCCGCCACATTTGCGTGATGTTGGGGTACGCAAGGAAGACATTCCGGC ACTGGCGCAGGCGGCACTGAATGATGTTTGTACCGGTGGCAACCCGCGTGAAGCA ACGCTTGAGGATATTGTAGAGCTTTACCATACCGCCTGGTAA (SEQ ID NO: 4) |

**[Table 1-3]**

| adhP/ACT43318.1/Escherichia coli BL21(DE3) | |
|---|---|
| Amino acid sequence | MKAAVVTKDHHVDVTYKTLRSLKHGEALLKMECCGVCHTDLHVKNGDFGDKTGVI LGHEGIGVVAEVGPGVTSLKPGDRASVAWFYEGCGHCEYCNSGNETLCRSVKNAGY SVDGGMAEECIVVADYAVKVPDGLDSAAASSITCAGVTTYKAVKLSKIRPGQWIAIY GLGGLGNLALQYAKNVFNAKVIAIDVNDEQLKLATEMGADLAINSHTEDAAKIVQE KTGGAHAAVVTAVAKAAFNSAVDAVRAGGRVVAVGLPPESMSLDIPRLVLDGIEVVG SLVGTRQDLTEAFQFAAEGKVVPKVALRPLADINTIFTEMEEGKIRGRMVIDFRH (SEQ ID NO: 5) |
| Base sequence | ATGAAGGCTGCAGTTGTTACGAAGGATCATCATGTTGACGTTACGTATAAAACACT GCGCTCACTGAAACATGGCGAAGCCCTGCTGAAAATGGAGTGTTGTGGTGTATGT CATACCGATCTTCATGTTAAGAATGGCGATTTTGGTGACAAAACCGGCGTAATTCT GGGCCATGAAGGCATCGGTGTGGTGGCAGAAGTGGGTCCAGGTGTCACCTCATTA AAACCAGGCGATCGTGCCAGCGTGGCGTGGTTCTACGAAGGATGCGGTCATTGCG AATACTGTAACAGTGGTAACGAAACGCTCTGCCGTTCAGTTAAAAATGCCGGATAC |
| | AGCGTTGATGGCGGGATGGCGGAAGAGTGCATCGTGGTCGCCGATTACGCGGTAA AAGTGCCAGATGGTCTGGACTCGGCGGCCAGCAGCATTACCTGTGCGGGAGT CACCACCTACAAAGCCGTTAAGCTGTCAAAAATTCGTCCAGGGCAGTGGATTGCT ATCTACGGTCTTGGCGGTCTGGGTAACCTCGCCCTGCAATACGCGAAGAATGTCTT TAACGCCAAAGTGATCGCCATTGATGTCAATGATGAGCAGTTAAAACTGGCAACC GAAATGGGCGCAGATTTAGCGATTAACTCACACACCGAAGACGCCGCCAAAATTG TGCAGGAGAAAACTGGTGGCGCTCACGCTGCGGTGGTAACAGCGGTAGCTAAAG CTGCGTTTAACTCGGCAGTTGATGCTGTCCGTGCAGGCGGTCGTGTTGTGGCTGTC GGTCTACCGCCGGAGTCTATGAGCCTGGATATCCCACGTCTTGTGCTGGATGGTATT GAAGTGGTCGGTTCGCTGGTCGGCACGCGCCAGGATTTAACTGAAGCCTTCCAGT TTGCCGCCGAAGGTAAAGTGGTGCCGAAAGTCGCCCTGCGTCCGTTAGCGGACAT CAACACCATCTTTACTGAGATGGAAGAAGGCAAAATCCGTGGCCGCATGGTGATT GATTTCCGTCACTAA (SEQ ID NO: 6) |

**[Table 1-4]**

| ybbO/ACT42343.1/Escherichia coli BL21(DE3) | |
|---|---|
| Amino acid sequence | MTHKATEILTGKVMQKSVLITGCSSGIGLESALELKRQGFHVLAGCRKPDDVERMNN MGFTGVLIDLDSPESVDRAADEVIALTDNCLYGIFNNAGFGMYGPLSTISRAQMEQQF SANFFGAHQLTMRLLPAMLPHGEGRIVMTSSVMGLISTPGRGAYAASKYALEAWSDA LRMELRHSGIKVSLIEPGPIRTRFTDNVNQTQSDKPVENPGIAARFTLGPEAVVDKVR HAFISEKPKMRYPVTLVTWAVMVLKRLLPGRVMDKILQG (SEQ ID NO: 7) |
| Base sequence | ATGACTCATAAAGCAACGGAGATCCTGACAGGTAAAGTTATGCAAAAATCGGTCTT AATTACCGGATGTTCCAGTGGAATTGGCCTGGAAAGCGCGCTCGAATTAAAACGC CAGGGTTTTCATGTGCTGGCAGGTTGCCGAAAACCGGATGATGTTGAGCGTATGA ACAACATGGGATTTACCGGCGTGTTGATCGATCTGGATTCACCAGAAAGTGTTGAT CGCGCAGCAGACGAGGTGATCGCCCTGACCGATAATTGTCTGTATGGGATCTTTAA CAATGCCGGATTCGGCATGTATGGCCCCCTTTCCACCATCAGCCGTGCGCAGATGG AACAGCAGTTTTCCGCCAACTTTTTCGGCGCACACCAGCTCACCATGCGCCTGTTA CCCGCGATGTTACCGCACGGTGAAGGGCGTATTGTGATGACATCATCGGTGATGGG ATTAATCTCCACGCCGGGTCGTGGCGCTTACGCGGCCAGTAAATATGCGCTGGAGG CGTGGTCAGACGCGCTGCGCATGGAGCTACGCCACAGCGGAATTAAAGTCAGCCT GATCGAACCCGGTCCCATTCGTACTCGCTTCACCGACAACGTCAACCAGACGCAA AGTGATAAACCAGTCGAAAATCCCGGCATCGCCGCCCGCTTTACGTTGGGACCGG AAGCGGTGGTGGACAAAGTACGCCATGCTTTTATTAGCGAGAAGCCGAAGATGCG CTATCCGGTAACGCTGGTGACCTGGGCAGTAATGGTGCTTAAGCGCCTGCTGCCGG GGCGCGTGATGGACAAAATATTGCAGGGGTGA (SEQ ID NO: 8) |

**[Table 1-5]**

| eutG/ACT44165.1/Escherichia coli BL21(DE3) | |
|---|---|
| Amino acid sequence | MQNELQTALFQAFDTLNLQRVKTFSVPPVTLCGPGAVSSCGQQAQTRGLKHLFVMA DSFLHQAGMTAGLTRSLAVKGIAMTLWPCPVGEPCITDVCAAVAQLRESGCDGVIAF GGGSVLDAAKAVALLVTNPDSTLAEMSETSVLQPRLPLIAIPTTAGTGSETTNVTVIID AVSGRKQVLAHASLMPDVAILDAALTEGVPSHVTAMTGIDALTHAIEAYSALNATPFT |
| | DSLAIGAIAMIGKSLPKAVGYGHDLAARESMLLASCMAGMAFSSAGLGLCHAMAHQ PGAALHIPHGLANAMLLPTVMEFNRMVCRERFSQIGRALRTKKSDDRDAINAVSELI AEVGIGKRLGDVGATSAHYGAWAQAALEDICLRSNPRTASLEQIVGLYAAAQ (SEQ ID NO: 9) |
| Base sequence | ATGCAAAATGAATTGCAGACCGCGCTCTTTCAGGCGTTCGATACCCTGAATCTGCA ACGGGTAAAAACATTTAGCGTTCCACCGGTGACGCTTTGCGGTCCGGGCGCGGTG AGCAGTTGCGGGCAGCAAGCGCAAACGCGTGGGCTGAAACATCTGTTCGTGATG GCAGACAGCTTTTTGCATCAGGCGGGGATGACCGCCGGGCTGACGCGCAGCCTGG CTGTTAAAGGCATCGCCATGACGCTCTGGCCATGTCCGGTGGGCGAACCGTGCATT ACCGACGTGTGTGCAGCCGTGGCGCAGTTGCGTGAGTCAGGCTGTGATGGGGTGA TCGCATTTGGCGGCGGCTCGGTGCTGGATGCGGCGAAAGCCGTGGCGTTGCTGGT GACGAACCCCGATAGCACGCTGGCAGAGATGTCAGAAACCAGCGTTCTGCAACC GCGCTTGCCGCTGATTGCCATTCCAACGACCGCCGGAACCGGCTCTGAAACCACC AATGTAACGGTGATTATCGACGCGGTGAGCGGGCGCAAGCAGGTGTTAGCCCATG CCTCGCTGATGCCGGATGTGGCGATCCTCGACGCCGCATTGACCGAAGGTGTGCC GTCGCATGTCACGGCGATGACCGGCATTGATGCGTTAACCCATGCCATTGAAGCAT ACAGCGCCCTGAACGCTACACCGTTTACCGACAGCCTGGCGATTGGTGCCATTGC GATGATTGGCAAATCGCTGCCGAAAGCGGTGGGCTACGGTCACGACCTTGCCGCG CGCGAGAGCATGTTACTGGCTTCATGTATGGCGGGAATGGCGTTTTCCAGTGCGGG TCTTGGGTTGTGCCACGCGATGGCGCATCAGCCGGGCGCGGCGCTGCATATTCCGC ACGGTCTCGCGAACGCCATGTTGCTGCCAACGGTGATGGAATTTAACCGGATGGTT TGTCGTGAACGCTTTAGTCAGATTGGTCGGGCACTGCGAACTAAAAAATCCGACG ATCGTGACGCTATTAACGCGGTAAGTGAGCTGATTGCGGAAGTTGGGATTGGTAAA CGACTGGGCGATGTTGGTGCGACATCTGCGCATTACGGCGCATGGGCGCAGGCCG |
| | CGCTGGAAGATATTTGTCTGCGCAGTAACCCGCGTACCGCCAGCCTGGAGCAGATT GTCGGCCTGTACGCAGCGGCGCAATAA (SEQ ID NO: 10) |

**[Table 1-6]**

| ahr/ACT45923.1/Escherichia coli BL21(DE3) | |
|---|---|
| Amino acid sequence | MSMIKSYAAKEAGGELEVYEYDPGELRPQDVEVQVDYCGICHSDLSMIDNEWGFSQ YPLVAGHEVIGRVVALGSAAQDKGLQVGQRVGIGWTARSCGHCDACISGNQINCEQG AVPTIMNRGGFAEKLRADWQWVIPLPENIDIESAGPLLCGGITVFKPLLMHHITATSRV GVIGIGGLGHIAIKLLHAMGCEVTAFSSNPAKEQEVLAMGADKVVNSRDPQALKALA GQFDLIINTVNVSLDWQPYFEALTYGGNFHTVGAVLTPLSVPAFTLIAGDRSVSGSATG TPYELRKLMRFAARSKVAPTTELFPMSKINDAIQHVRDGKARYRVVLKADY (SEQ ID NO: 11) |
| Base sequence | ATGTCGATGATAAAAAGCTATGCCGCAAAAGAAGCGGGCGGAGAACTGGAAGTTT ATGAGTACGATCCCGGTGAGCTGAGGCCACAAGATGTTGAAGTGCAGGTGGATTA CTGCGGGATCTGCCATTCCGATCTGTCGATGATCGATAACGAATGGGGATTTTCAC AATATCCGCTGGTTGCCGGGCATGAGGTGATTGGGCGCGTGGTGGCACTCGGGAG CGCCGCGCAGGATAAAGGTTTGCAGGTCGGTCAGCGTGTCGGGATTGGCTGGACG GCGCGTAGCTGTGGTCACTGCGACGCCTGTATTAGCGGTAATCAGATCAACTGCGA GCAAGGTGCGGTGCCGACGATTATGAATCGCGGTGGCTTTGCCGAGAAGTTGCGT GCGGACTGGCAATGGGTGATTCCACTGCCAGAAAATATTGATATCGAGTCCGCCGG GCCGCTGTTGTGCGGCGGTATCACGGTCTTTAAACCACTGTTGATGCACCATATCA CTGCTACCAGCCGCGTTGGGGTAATTGGTATTGGCGGGCTGGGGCATATCGCTATA AAACTTCTGCACGCAATGGGATGCGAGGTGACAGCCTTTAGTTCTAATCCGGCGA |
| | AAGAGCAGGAAGTGCTGGCGATGGGTGCCGATAAAGTGGTGAATAGCCGCGATCC GCAGGCACTGAAAGCACTGGCGGGGCAGTTTGATCTCATTATCAACACCGTCAAC GTCAGCCTCGACTGGCAGCCCTATTTTGAGGCGCTGACCTATGGCGGTAATTTCCA TACGGTCGGTGCGGTTCTCACGCCGCTGTCTGTTCCGGCCTTTACGTTAATTGCGG GCGATCGCAGCGTCTCTGGTTCTGCTACCGGCACGCCTTATGAGCTGCGTAAGCTG ATGCGTTTTGCCGCCCGCAGCAAGGTTGCGCCGACCACCGAACTGTTCCCGATGT CGAAAATTAACGACGCCATCCAGCATGTGCGCGACGGTAAGGCGCGTTACCGCGT GGTGTTGAAAGCCGATTATTGA (SEQ ID NO: 12) |

**[Table 1-7]**

| yahK/ACT42179.1/Escherichia coli BL21(DE3) | |
|---|---|
| Amino acid sequence | MKIKAVGAYSAKQPLEPMDITRREPGPNDVKIEIAYCGVCHSDLHQVRSEWAGTVYP CVPGHEIVGRVVAVGDQVEKYAPGDLVGVGCIVDSCKHCEECEDGLENYCDHMTGT YNSPTPDEPGHTLGGYSQQIVVHERYVLRIRHPQEQLAAVAPLLCAGITTYSPLRHWQ AGPGKKVGVVGIGGLGHMGIKLAHAMGAHVVAFTTSEAKREAAKALGADEVVNSR NADEMAAHLKSFDFILNTVAAPHNLDDFTTLLKRDGTMTLVGAPATPHKSPEVFNLI MKRRAIAGSMIGGIPETQEMLDFCAEHGIVADIEMIRADQINEAYERMLRGDVKYRF VIDNRTLTD (SEQ ID NO: 13) |
| Base sequence | ATGAAGATCAAAGCTGTTGGTGCATATTCCGCTAAACAACCACTTGAACCGATGGA TATCACCCGGCGTGAACCGGGACCGAATGATGTCAAAATCGAAATCGCTTACTGTG GCGTTTGCCATTCCGATCTCCACCAGGTCCGTTCCGAGTGGGCGGGGACGGTTTAC CCCTGCGTGCCGGGTCATGAAATTGTGGGGCGTGTGGTAGCCGTTGGTGATCAGG |
| | TAGAAAAATATGCGCCGGGCGATCTGGTCGGTGTCGGCTGCATTGTCGACAGTTGT AAACATTGCGAAGAGTGTGAAGACGGGTTGGAAAACTACTGTGATCACATGACCG GCACCTATAACTCGCCGACGCCGGACGAACCGGGCCATACTCTGGGCGGCTACTC ACAACAGATCGTCGTTCATGAGCGATATGTTCTGCGTATTCGTCACCCGCAAGAGC AGCTGGCGGCGGTGGCTCCTTTGTTGTGTGCAGGGATCACCACGTATTCGCCGCTA CGTCACTGGCAGGCCGGGCCGGGTAAAAAAGTGGGCGTGGTCGGCATCGGCGGT CTGGGACATATGGGGATTAAGCTGGCCCACGCGATGGGGGCACATGTGGTGGCATT TACCACTTCTGAGGCAAAACGCGAAGCGGCAAAAGCCCTGGGGGCCGATGAAGT TGTTAACTCACGCAATGCCGATGAGATGGCGGCTCATCTGAAGAGTTTCGATTTCA TTTTGAATACAGTAGCTGCGCCACATAATCTCGACGATTTTACCACCTTGCTGAAG CGTGATGGCACCATGACGCTGGTTGGTGCGCCTGCGACACCGCATAAATCGCCGG AAGTTTTCAACCTGATCATGAAACGCCGTGCGATAGCCGGTTCTATGATTGGCGGC ATTCCAGAAACTCAGGAGATGCTCGATTTTTGCGCCGAACATGGCATCGTGGCTGA TATAGAGATGATTCGGGCCGATCAAATTAATGAAGCCTATGAGCGAATGCTGCGCG GTGATGTGAAATATCGTTTTGTTATCGATAATCGCACACTAACAGACTGA (SEQ ID NO: 14) |

**[Table 1-8]**

| adhE/ACT43105.1/Escherichia coli BL21(DE3) | |
|---|---|
| Amino acid sequence | MAVTNVAELNALVERVKKAQREYASFTQEQVDKIFRAAALAAADARIPLAKMAVAES GMGIVEDKVIKNHFASEYIYNAYKDEKTCGVLSEDDTFGTITIAEPIGIICGIVPTTNPTS TAIFKSLISLKTRNAIIFSPHPRAKDATNKAADIVLQAAIAAGAPKDLIGWIDQPSVELS NALMHHPDINLILATGGPGMVKAAYSSGKPAIGVGAGNTPVVIDETADIKRAVASVL MSKTFDNGVICASEQSVVVVDSVYDAVRERFATHGGYLLQGKELKAVQDVILKNGA |
| | LNAAIVGQPAYKIAELAGFSVPENTKILIGEVTVVDESEPFAHEKLSPTLAMYRAKDFE DAVEKAEKLVAMGGIGHTSCLYTDQDNQPARVSYFGQKMKTARILINTPASQGGIGDL YNFKLAPSLTLGCGSWGGNSISENVGPKHLINKKTVAKRAENMLWHKLPKSIYFRRG SLPIALDEVITDGHKRALIVTDRFLFNNGYADQITSVLKAAGVETEVFFEVEADPTLSI VRKGAELANSFKPDVIIALGGGSPMDAAKIMWVMYEHPETHFEELALRFMDIRKRIY KFPKMGVKAKMIAVTTTSGTGSEVTPFAVVTDDATGQKYPLADYALTPDMAIVDANL VMDMPKSLCAFGGLDAVTHAMEAYVSVLASEFSDGQALQALKLLKEYLPASYHEGS KNPVARERVHSAATIAGIAFANAFLGVCHSMAHKLGSQFHIPHGLANALLICNVIRYN ANDNPTKQTAFSQYDRPQARRRYAEIADHLGLSAPGDRTAAKIEKLLAWLETLKAEL GIPKSIREAGVQEADFLANVDKLSEDAFDDQCTGANPRYPLISELKQILLDTYYGRDY VEGETAAKKEAAPAKAEKKAKKSA (SEQ ID NO: 15) |
| Base sequence | ATGGCTGTTACTAATGTCGCTGAACTTAACGCACTCGTAGAGCGTGTAAAAAAAGC CCAGCGTGAATATGCCAGTTTCACTCAAGAGCAAGTAGACAAAATCTTCCGCGCC GCCGCTCTGGCTGCTGCAGATGCTCGAATCCCACTCGCGAAAATGGCCGTTGCCG AATCCGGCATGGGTATCGTCGAAGATAAAGTGATCAAAAACCACTTTGCTTCTGAA TATATCTACAACGCCTATAAAGATGAAAAAACCTGTGGTGTTCTGTCTGAAGACGA CACTTTTGGTACCATCACTATCGCTGAACCAATCGGTATTATTTGCGGTATCGTTCC GACCACTAACCCGACTTCAACTGCTATCTTCAAATCGCTGATCAGTCTGAAGACCC GTAACGCCATTATCTTCTCCCCGCACCCGCGTGCAAAAGATGCCACCAACAAAGC GGCTGATATCGTTCTGCAGGCTGCTATCGCTGCCGGTGCTCCGAAAGATCTGATCG GCTGGATCGATCAACCTTCTGTTGAACTGTCTAACGCACTGATGCACCACCCAGAC ATCAACCTGATCCTCGCGACTGGTGGTCCGGGCATGGTTAAAGCCGCATACAGCTC CGGTAAACCAGCTATCGGTGTAGGCGCGGGCAACACTCCAGTTGTTATCGATGAA |
| | ACTGCTGATATCAAACGTGCAGTTGCATCTGTACTGATGTCCAAAACCTTCGACAA CGGCGTAATCTGTGCTTCTGAACAGTCTGTTGTTGTTGACTCTGTTTATGACGC TGTACGTGAACGTTTTGCAACCCACGGCGGCTATCTGTTGCAGGGTAAAGAGCTG AAAGCTGTTCAGGATGTTATCCTGAAAAACGGTGCGCTGAACGCGGCTATCGTTG GTCAGCCAGCCTATAAAATTGCTGAACTGGCAGGCTTCTCTGTACCAGAAAACAC CAAGATTCTGATCGGTGAAGTGACCGTTGTTGATGAAAGCGAACCGTTCGCACAT GAAAAACTGTCCCCGACTCTGGCAATGTACCGCGCTAAAGATTTCGAAGACGCGG TAGAAAAAGCAGAGAAACTGGTTGCTATGGGCGGTATCGGTCATACCTCTTGCCTG TACACTGACCAGGATAACCAACCGGCTCGCGTTTCTTACTTCGGTCAGAAAATGA AAACGGCGCGTATCCTGATTAACACCCCAGCGTCTCAGGGTGGTATCGGTGACCTG TATAACTTCAAACTCGCACCTTCCCTGACTCTGGGTTGTGGTTCTTGGGGTGGTAA CTCCATCTCTGAAAACGTTGGTCCGAAACACCTGATCAACAAGAAAACCGTTGCT AAGCGAGCTGAAAACATGTTGTGGCACAAACTTCCGAAATCTATCTACTTCCGCCG TGGCTCCCTGCCAATCGCGCTGGATGAAGTGATTACTGATGGCCACAAACGTGCG CTCATCGTGACTGACCGCTTCCTGTTCAACAATGGTTATGCTGATCAGATCACTTCC GTACTGAAAGCAGCAGGCGTTGAAACTGAAGTCTTCTTCGAAGTAGAAGCGGAC CCGACCCTGAGCATCGTTCGTAAAGGTGCAGAACTGGCAAACTCCTTCAAACCAG ACGTGATTATCGCGCTGGGTGGTGGTTCCCCGATGGACGCCGCGAAGATCATGTGG GTTATGTACGAACATCCGGAAACTCACTTCGAAGAGCTGGCGCTGCGCTTTATGGA TATCCGTAAACGTATCTACAAGTTCCCGAAAATGGGCGTGAAAGCGAAAATGATCG CTGTCACCACCACTTCTGGTACAGGTTCTGAAGTCACTCCGTTTGCGGTTGTAACT GACGACGCTACTGGTCAGAAATATCCGCTGGCAGACTATGCGCTGACTCCGGATAT GGCGATTGTCGACGCCAACCTGGTTATGGACATGCCGAAGTCCCTGTGTGCTTTCG GTGGTCTGGACGCAGTAACTCACGCCATGGAAGCTTATGTTTCTGTACTGGCATCT |
| | GAGTTCTCTGATGGTCAGGCTCTGCAGGCACTGAAACTGCTGAAAGAATATCTGC CAGCGTCCTACCACGAAGGGTCTAAAAATCCGGTAGCGCGTGAACGTGTTCACAG TGCAGCGACTATCGCGGGTATCGCGTTTGCGAACGCCTTCCTGGGTGTATGTCACT CAATGGCGCACAAACTGGGTTCCCAGTTCCATATTCCGCACGGTCTGGCAAACGC CCTGCTGATTTGTAACGTTATTCGCTACAATGCGAACGACAACCCGACCAAGCAGA CTGCATTCAGCCAGTATGACCGTCCGCAGGCTCGCCGTCGTTATGCTGAAATTGCC GACCACTTGGGTCTGAGCGCACCGGGCGACCGTACTGCTGCTAAGATCGAGAAAC TGCTGGCATGGCTGGAAACGCTGAAAGCTGAACTGGGTATTCCGAAATCTATCCGT GAAGCTGGCGTTCAGGAAGCAGACTTCCTGGCGAACGTGGATAAACTGTCTGAA GATGCATTCGATGACCAGTGCACCGGCGCTAACCCGCGTTACCCGCTGATCTCCGA GCTGAAACAGATCCTGCTGGATACCTACTACGGTCGTGATTATGTAGAAGGTGAAA CTGCAGCGAAAAAAGAAGCCGCTCCGGCTAAAGCTGAGAAAAAAGCGAAAAAAT CCGCTTAA (SEQ ID NO: 16) |

**[Table 1-9]**

| ybdR/ACT42455.1/Escherichia coli BL21(DE3) | |
|---|---|
| Amino acid sequence | MKALTYHGPHHVQVENVPDPGIEQADDIILRITATAICGSDLHLYRGKIPQVKHGDIFG HEFMGEVVETGKDVKNLQKGDRVVIPFVIACGDCFFCRLQQYAACENTNAGKGAAL NKKQIPAPAALFGYSHLYGGVPGGQAEYVRVPKGNVGPFKVPPLLSDDKALFLSDILP TAWQAAKNAQIQQGSSVAVYGAGPVGLLTIACARLLGAEQIFVVDHHPYRLHFAADR YGAIPINFDEDSDPAQSIIEQTAGHRGVDAVIDAVGFEAKGSTTETVLTNLKLEGSSGK ALRQCIAAVRRGGIVSVPGVYAGFIHGFLFGDAFDKGLSFKMGQTHVHAWLGELLPL |
| | IEKGLLKPEEIVTHYMPFEEAARGYEIFEKREEECRKVILVPGAQSAEAAQKAVSGLV NAMPGGTI (SEQ ID NO: 17) |
| Base sequence | ATGAAAGCATTGACTTATCACGGCCCACATCACGTTCAGGTAGAAAATGTTCCCGA TCCGGGCATTGAACAGGCAGATGATATTATTCTGCGTATTACGGCAACGGCGATCT GTGGCTCTGACCTCCATCTTTATCGAGGCAAAATACCCCAGGTTAAACATGGCGAT ATTTTTGGTCATGAATTTATGGGGGAAGTCGTTGAAACCGGAAAGGACGTAAAAA ATTTGCAAAAAGGCGACCGGGTGGTAATTCCGTTTGTCATTGCTTGTGGCGACTGT TTTTTCTGTCGATTACAGCAATATGCCGCCTGCGAAAATACCAATGCGGGTAAAGG CGCTGCGCTCAATAAAAAACAGATACCAGCTCCCGCGGCATTGTTTGGTTATAGTC ACCTGTATGGCGGCGTTCCTGGTGGGCAGGCGGAATATGTCCGCGTCCCTAAAGG GAATGTGGGGCCGTTTAAAGTACCGCCTTTGCTTTCAGATGATAAAGCGCTTTTCC TTTCTGATATTCTGCCAACGGCATGGCAGGCAGCAAAAAATGCGCAGATCCAACA AGGTTCAAGCGTTGCAGTCTATGGTGCTGGTCCTGTGGGATTGTTGACAATCGCCT GTGCACGGTTGCTCGGTGCGGAACAGATTTTTGTTGTTGATCATCATCCCTACCGC TTGCATTTCGCCGCCGACCGCTACGGCGCGATCCCGATTAATTTTGATGAAGACAG CGATCCGGCACAGTCAATTATTGAACAAACGGCAGGTCACCGGGGCGTGGATGCA GTAATAGACGCCGTCGGTTTTGAAGCGAAAGGCAGCACCACGGAAACGGTGCTG ACTAACCTGAAACTGGAGGGCAGCAGCGGTAAAGCGTTGCGTCAGTGTATTGCGG CGGTCAGGCGTGGCGGCATTGTTAGCGTACCGGGCGTCTACGCTGGATTTATTCAC GGTTTCCTGTTTGGCGACGCCTTTGATAAAGGGTTGTCGTTTAAAATGGGACAGAC CCACGTTCACGCATGGCTGGGAGAATTATTACCGTTAATTGAGAAAGGATTACTGA AACCAGAAGAAATTGTTACCCACTATATGCCGTTTGAAGAGGCCGCCCGGGGATAT |
| | GAGATTTTCGAAAAACGTGAAGAGGAGTGCCGTAAGGTGATTCTGGTACCCGGTG CACAAAGCGCAGAGGCGGCGCAGAAGGCGGTTTCAGGTCTAGTGAATGCGATGC CGGGGGGAACAATATGA (SEQ ID NO: 18) |

**[Table 1-10]**

| dkgA/ACT44689.1/Escherichia coli BL21(DE3) | |
|---|---|
| Amino acid sequence | MANPTVIKLQDGNVMPQLGLGVWQASNEEVITAIQKALEVGYRSIDTAAAYKNEEG VGKALKNASVNREELFITTKLWNDDHKRPREALLDSLKKLQLDYIDLYLMHWPVPAI DHYVEAWKGMIELQKEGLIKSIGVCNFQIHHLQRLIDETGVTPVINQIELHPLMQQRQ LHAWNATHKIQTESWSPLAQGGKGVFDQKVIRDLADKYGKTPAQIVIRWHLDSGLV VIPKSVTPSRIAENFDVWDFRLDKDELGEIAKLDQGKRLGPDPDQFGG (SEQ ID NO: 19) |
| Base sequence | ATGGCTAATCCAACCGTTATTAAGCTACAGGATGGCAATGTCATGCCCCAGCTGGG ACTGGGCGTCTGGCAAGCAAGTAATGAGGAAGTAATCACCGCCATTCAAAAAGCG TTAGAAGTGGGTTATCGCTCGATTGATACCGCCGCGGCCTACAAGAACGAAGAAG GTGTCGGCAAAGCCCTGAAAAATGCCTCAGTCAACAGAGAAGAACTGTTCATCAC CACTAAGCTGTGGAACGACGACCACAAGCGCCCCCGCGAAGCCCTGCTCGACAG CCTGAAAAAACTCCAGCTTGATTATATCGACCTCTACTTAATGCACTGGCCCGTTCC CGCTATCGACCATTATGTCGAAGCATGGAAAGGCATGATCGAATTGCAAAAAGAG GGATTAATCAAAAGCATCGGCGTGTGCAACTTCCAGATCCATCACCTGCAACGCCT GATTGATGAAACTGGCGTGACGCCTGTGATAAACCAGATCGAACTTCATCCGCTGA TGCAACAACGCCAGCTACACGCCTGGAACGCGACACACAAAATCCAGACCGAAT CCTGGAGCCCATTAGCGCAAGGAGGGAAAGGCGTTTTCGATCAGAAAGTCATTCG |
| | CGATCTGGCAGATAAATACGGCAAAACCCCGGCGCAGATTGTTATCCGCTGGCATC TGGATAGCGGCCTGGTGGTGATCCCGAAATCGGTCACACCTTCACGTATTGCCGAA AACTTTGATGTCTGGGATTTCCGTCTCGACAAAGACGAACTCGGCGAAATTGCAA AACTCGATCAGGGCAAGCGTCTCGGTCCCGATCCTGACCAGTTCGGCGGCTAA (SEQ ID NO: 20) |

**[Table 1-11]**

| yiaY/ACT45243.1/Escherichia coli BL21(DE3) | |
|---|---|
| Amino acid sequence | MASSTFFIPSVNVIGADSLTDAMNMMADYGFTRTLIVTDNMLTKLGMAGDVQKALE ERNIFSVIYDGTQPNPTTENVAAGLKLLKENNCDSVISLGGGSPHDCAKGIALVAANG GDIRDYEGVDRSAKPQLPMIAINTTAGTASEMTRFCIITDEARHIKMAIVDKHVTPLLS VNDSSLMIGMPKSLTAATGMDALTHAIEAYVSIAATPITDACALKAVTMIAENLPLAVE DGSNAKAREAMAYAQFLAGMAFNNASLGYVHAMAHQLGGFYNLPHGVCNAVLLP HVQVFNSKVAAARLRDCAAAMGVNVTGKNDAEGAEACINAIRELAKKVDIPAGLRD LNVKEEDFAVLATNALKDACGFTNPIQATHEEIVAIYRAAM (SEQ ID NO: 21) |
| Base sequence | ATGGCATCTTCAACTTTCTTTATTCCTTCTGTGAATGTCATCGGCGCTGATTCATTGA CTGATGCAATGAATATGATGGCAGATTATGGATTTACCCGTACCTTAATTGTCACTG ACAATATGTTAACGAAATTAGGTATGGCGGGTGATGTGCAAAAAGCACTGGAAGA ACGCAATATTTTTAGCGTTATTTATGATGGCACCCAACCTAACCCAACCACGGAAA ACGTCGCCGCAGGTTTGAAATTACTTAAAGAAAATAATTGCGATAGCGTGATTTCC TTAGGCGGTGGTTCTCCGCATGACTGTGCAAAAGGTATTGCGCTGGTGGCAGCCA ATGGTGGTGATATCCGTGATTATGAAGGCGTTGACCGCTCTGCAAAACCGCAGCTG CCGATGATCGCCATCAATACCACTGCGGGTACAGCATCAGAAATGACTCGTTTCTG |
| | CATCATCACCGACGAAGCGCGTCACATCAAAATGGCGATTGTTGATAAGCACGTG ACTCCGCTGCTTTCTGTCAATGACTCCTCGCTGATGATCGGTATGCCGAAGTCACT GACCGCCGCCACTGGTATGGACGCCTTAACGCACGCTATCGAAGCGTATGTTTCTA TTGCCGCCACGCCGATCACTGACGCTTGTGCACTGAAAGCCGTGACCATGATTGC CGAAAACCTGCCGTTAGCCGTTGAAGATGGCAGTAATGCGAAAGCGCGTGAAGCA ATGGCTTATGCCCAGTTCCTCGCCGGTATGGCGTTCAATAATGCTTCTCTGGGTTAT GTTCATGCGATGGCGCACCAGCTGGGCGGTTTCTACAACCTGCCACACGGTGTATG TAACGCCGTTTTGCTGCCGCATGTTCAGGTATTCAACAGCAAAGTCGCCGCCGCAC GTCTGCGTGACTGTGCCGCTGCAATGGGCGTGAACGTGACAGGTAAAAACGATGC GGAAGGTGCTGAAGCCTGCATTAACGCCATCCGTGAACTGGCGAAGAAAGTGGAT ATCCCGGCAGGCCTACGCGACCTGAACGTGAAAGAAGATTTCGCGGTTCTGG CGACTAATGCCCTGAAAGATGCCTGTGGTTTTACTAACCCGATCCAGGCAACTCAC GAAGAAATTGTGGCGATTTATCGCGCAGCGATGTAA (SEQ ID NO: 22) |

**[Table 1-12]**

| frmA/ACT42209.1/Escherichia coli BL21(DE3) | |
|---|---|
| Amino acid sequence | MKSRAAVAFAPGKPLEIVEIDVAPPKKGEVLIKVTHTGVCHTDAFTLSGDDPEGVFPV VLGHEGAGVVVEVGEGVTSVKPGDHVIPLYTAECGECEFCRSGKTNLCVAVRETQGK GLMPDGTTRFSYNGQPLYHYMGCSTFSEYTVVAEVSLAKINPEANHEHVCLLGCGVT TGIGAVHNTAKVQPGDSVAVFGLGAIGLAVVQGARQAKAGRIIAIDTNPKKFDLARRF GATDCINPNDYDKPIKDVLLDINKWGIDHTFECIGNVNVMRAALESAHRGWGQSVII GVAGAGQEISTRPFQLVTGRVWKGSAFGGVKGRSQLPGMVEDAMKGDIDLEPFVTH TMSLDEINDAFDLMHEGKSIRTVIRY (SEQ ID NO: 23) |
| Base sequence | ATGAAATCACGTGCTGCCGTTGCATTTGCTCCCGGTAAACCGCTGGAAATCGTTGA AATTGACGTTGCACCACCGAAAAAGGTGAAGTGCTGATTAAAGTCACCCATACC GGCGTTTGCCATACCGACGCATTTACCCTCTCCGGGGATGACCCGGAAGGTGTATT CCCGGTGGTTCTCGGTCACGAAGGGGCCGGCGTTGTGGTTGAAGTCGGTGAAGG CGTAACCAGCGTCAAACCTGGCGACCATGTGATCCCGCTTTACACCGCGGAGTGC GGCGAGTGTGAGTTCTGTCGTTCTGGCAAAACTAACCTCTGTGTTGCGGTTCGCG AAACCCAGGGTAAAGGCTTGATGCCAGACGGCACCACCCGTTTTTCTTACAACGG GCAGCCGCTTTATCACTACATGGGATGCTCAACATTCAGTGAATACACCGTGGTCG CGGAAGTGTCTCTGGCCAAAATTAATCCAGAAGCAAACCATGAACACGTCTGCCT GCTGGGCTGTGGCGTGACCACCGGTATTGGCGCGGTGCACAACACAGCTAAAGTC CAGCCAGGTGATTCTGTTGCCGTGTTTGGTCTTGGCGCGATTGGTCTGGCAGTGGT TCAGGGCGCGCGTCAGGCGAAAGCGGGACGGATTATCGCTATCGATACCAACCCG AAGAAATTCGATCTGGCTCGTCGCTTCGGTGCTACCGACTGCATTAACCCGAATGA CTACGACAAACCGATTAAAGATGTCCTGCTGGATATCAACAAATGGGGTATCGACC ATACCTTTGAATGCATCGGTAACGTCAACGTGATGCGTGCGGCGCTGGAAAGTGC GCACCGCGGCTGGGGTCAGTCGGTGATCATCGGGGTAGCAGGTGCCGGTCAGGA AATCTCCACCCGACCATTCCAGTTGGTCACCGGTCGCGTATGGAAAGGTTCCGCGT TTGGCGGCGTGAAAGGTCGTTCCCAGTTACCGGGTATGGTTGAAGATGCGATGAA AGGTGATATCGATCTGGAACCGTTTGTCACGCATACCATGAGCCTTGATGAAATTA ATGACGCCTTCGACCTGATGCATGAAGGCAAATCCATTCGAACCGTAATTCGTTAC TGA (SEQ ID NO: 24) |

**[Table 1-13]**

| dkgB/ACT42101.1/Escherichia coli BL21(DE3) | |
|---|---|
| Amino acid sequence | MAIPAFGLGTFRLKDDVVISSVKTALELGYRAIDTAQIYDNEAAVGQAIAESGVPRHE LYITTKIWIENLSKDKLIPSLKESLQKLRTDYVDLTLIHWPSPNDEVSVEEFMQELLEA KKEGLTREIGISNFTIPLMEKAIAAVGAENIATNQIELSPYLQNRKVVAWAKQHGIHITS YMTLAYGKALKDEVIARIAAKHNATPAQVILAWAMGEGYSVIPSSTKRKNLESNLKA QNLQLDAEDKKAIAALDCNDRLVSPEGLAPEWD (SEQ ID NO: 25) |
| Base sequence | ATGGCTATCCCTGCATTTGGTTTAGGTACTTTCCGTCTGAAAGACGACGTTGTTATT TCATCTGTGAAAACGGCGCTTGAACTTGGTTATCGCGCAATTGATACTGCACAAAT CTATGATAACGAAGCCGCAGTAGGTCAGGCGATTGCAGAAAGTGGCGTGCCACGT CATGAACTCTACATCACCACTAAAATCTGGATTGAAAATCTCAGCAAAGACAAATT GATCCCGAGTCTGAAAGAGAGCCTGCAAAAATTGCGTACTGATTATGTTGATCTGA CTCTAATCCACTGGCCGTCACCAAACGATGAAGTCTCTGTTGAAGAGTTTATGCAG GAGCTGCTGGAAGCCAAAAAAGAAGGGTTGACGCGTGAGATCGGTATTTCCAACT TCACGATCCCATTGATGGAAAAGGCGATTGCTGCTGTTGGCGCTGAAAACATCGCT ACTAACCAGATTGAACTCTCTCCTTATCTGCAAAACCGTAAAGTGGTTGCCTGGGC TAAACAGCACGGCATCCATATTACTTCCTATATGACGCTGGCGTATGGTAAGGCCCT GAAAGATGAGGTTATTGCTCGTATCGCAGCTAAACACAATGCGACTCCGGCACAA GTGATTCTGGCGTGGGCTATGGGGGAAGGTTACTCAGTAATTCCTTCTTCTACTAA ACGTAAAAACCTGGAAAGTAATCTTAAGGCACAAAATTTACAGCTTGATGCCGAA GATAAAAAAGCGATCGCCGCACTGGATTGCAACGACCGCCTGGTTAGCCCGGAAG GTCTGGCTCCTGAATGGGATTAA (SEQ ID NO: 26) |

**[Table 1-14]**

| yghA/ACT44682.1/Escherichia coli BL21(DE3) | |
|---|---|
| Amino acid sequence | MSHLKDPTTQYYTGEYPKQKQPTPGIQAKMTPVPDCGEKTYVGSGRLKDRKALVTG GDSGIGRAAAIAYAREGADVAISYLPVEEEDAQDVKKIIEECGRKAVLLPGDLSDEKF ARSLVHEAHKALGGLDIMALVAGKQVAIPDIADLTSEQFQKTFAINVFALFWLTQEAIP LLPKGASIITTSSIQAYQPSPHLLDYAATKAAILNYSRGLAKQVAEKGIRVNIVAPGPIW TALQISGGQTQDKIPQFGQQTPMKRAGQPAELAPVYVYLASQESSYVTAEVHGVCGG EHLG (SEQ ID NO: 27) |
| Base sequence | ATGTCTCATTTAAAAGACCCGACCACGCAGTATTACACTGGTGAATATCCCAAACA GAAACAACCGACGCCAGGCATCCAGGCGAAGATGACACCGGTACCGGATTGCGG CGAGAAAACCTATGTTGGTAGCGGTCGCCTGAAAGATCGTAAAGCACTGGTGACA GGGGGCGATTCCGGAATAGGTCGCGCTGCCGCCATCGCTTACGCGCGTGAAGGGG CTGACGTGGCGATCAGTTATCTTCCCGTGGAAGAAGACGCTCAGGATGTGAA AAAGATCATTGAAGAATGCGGACGCAAAGCCGTTCTGCTGCCAGGCGATTTAAGC GATGAGAAATTTGCCCGTTCGCTGGTTCACGAAGCGCACAAGGCGTTAGGCGGGC TGGATATTATGGCGCTGGTCGCCGGGAAACAGGTTGCCATTCCGGATATTGCAGAC CTCACCAGCGAACAGTTTCAAAAGACCTTTGCCATTAACGTTTTCGCGCTGTTCTG GCTAACCCAGGAAGCGATCCCCCTGCTACCGAAAGGTGCAAGTATCATCACCACT TCGTCAATCCAGGCATACCAGCCAAGTCCGCATTTACTGGACTATGCGGCTACGAA GGCGGCGATTCTGAACTACAGCCGTGGCTTGGCAAAACAGGTCGCGGAGAAAGG TATTCGGGTGAATATTGTCGCGCCAGGCCCGATCTGGACAGCACTGCAAATTTCCG GCGGACAAACGCAGGATAAGATCCCGCAGTTTGGTCAGCAAACGCCGATGAAAC GTGCGGGGCAACCGGCGGAACTGGCCCCTGTATATGTTTATCTGGCAAGTCAGGA GTCGAGCTACGTCACCGCAGAAGTGCACGGCGTGTGCGGCGGCGAGCATTTAGGT TAA (SEQ ID NO: 28) |

**[Table 1-15]**

| ydjG/ACT43594.1/Escherichia coli BL21(DE3) | |
|---|---|
| Amino acid sequence | MKKIPLGTTDITLSRMGLGTWAIGGGPAWNGDLDRQICIDTILEAHRCGINLIDTAPGY NFGNSEVIVGQALKKLPREQVVVETKCGIVWERKGSLFNKVGDRQLYKNLSPESIRE EVEASLQRLGIDYIDIYMTHWQSVPPFFTPIAETVAVLNELKAEGKIRAIGAANVDAD HIREYLQYGELDIIQAKYSILDRAMENELLPLCRDNGIVVQVYSPLEQGLLTGTITRDY VPGGARANKVWFQRENMLKVIDMLEQWQPLCARYQCTIPTLALAWILKQSDLISILS GATAPEQVRENVAALNINLSDADATLMREMAEALER (SEQ ID NO: 29) |
| Base sequence | ATGAAAAAAATACCTTTAGGCACAACGGATATTACGCTTTCGCGAATGGGGTTGGG GACATGGGCCATTGGCGGCGGTCCTGCATGGAATGGCGATCTCGATCGGCAAATAT GTATTGATACGATTCTTGAAGCCCATCGTTGCGGCATTAATCTGATTGATACTGCAC CAGGATATAACTTTGGCAATAGTGAAGTTATCGTCGGTCAGGCGTTAAAAAAACTG CCCCGTGAACAGGTTGTAGTAGAAACCAAATGCGGCATTGTCTGGGAACGAAAAG GAAGTTTATTCAACAAAGTTGGCGATCGGCAGTTGTATAAAAACCTTTCCCCGGAA TCTATCCGCGAAGAGGTAGAAGCCAGCTTGCAACGTCTGGGTATTGATTACATCGA TATCTACATGACGCACTGGCAGTCGGTGCCGCCATTTTTTACGCCGATAGCTGAAA CTGTCGCAGTGCTTAATGAGTTAAAAGCCGAAGGAAAATTCGCGCGATAGGCGC TGCTAACGTCGATGCTGACCATATCCGCGAGTATCTGCAATATGGTGAACTGGATAT TATTCAGGCGAAATACAGTATCCTCGACCGGGCAATGGAAAACGAACTGCTGCCG CTATGTCGTGATAATGGCATTGTGGTTCAGGTTTATTCCCCGCTAGAGCAGGGATTG TTGACCGGCACCATCACTCGTGATTACGTTCCGGGCGGCGCTCGGGCAAATAAAG TCTGGTTCCAGCGTGAAAACATGCTGAAAGTGATTGATATGCTTGAACAGTGGCA GCCACTTTGTGCTCGTTATCAGTGCACAATTCCCACTCTGGCACTGGCGTGGATATT AAAACAGAGTGATTTAATCTCCATTCTTAGTGGGGCTACTGCACCGGAACAGGTAC GCGAAAATGTCGCGGCACTGAATATCAACTTATCGGATGCAGACGCAACATTGATG |
| | AGGGAAATGGCAGAGGCCCTGGAGCGTTAA (SEQ ID NO: 30) |

**[Table 1-16]**

| gldA/ACT45624.1/Escherichia coli BL21(DE3) | |
|---|---|
| Amino acid sequence | MDRIIQSPGKYIQGADVINRLGEYLKPLAERWLVVGDKFVLGFAQSTVEKSFKDAGL VVEIAPFGGECSQNEIDRLRGIAETAQCGAILGIGGGKTLDTAKALAHFMGVPVAIAPT IASTDAPCSALSVIYTDEGEFDRYLLLPNNPNMVIVDTKIVAGAPARLLAAGIGDALAT WFEARACSRSGATTMAGGKCTQAALALAELCYNTLLEEGEKAMLAAEQHVVTPAL ERVIEANTYLSGVGFESGGLAAAHAVHNGLTAIPDAHHYYHGEKVAFGTLTQLVLEN APVEEIETVAALSHAVGLPITLAQLDIKEDVPAKMRIVAEAACAEGETIHNMPGGATPD QVYAALLVADQYGQRFLQEWE (SEQ ID NO: 31) |
| Base sequence | ATGGACCGCATTATTCAATCACCGGGTAAATACATCCAGGGCGCTGATGTGATTAAT CGTCTGGGCGAATACCTGAAGCCGCTGGCAGAACGCTGGTTAGTGGTGGGTGACA AATTTGTTTTAGGTTTTGCTCAATCCACTGTCGAGAAAAGCTTTAAAGATGCTGGA CTGGTAGTAGAAATTGCGCCGTTTGGCGGTGAATGTTCGCAAAATGAGATCGACC GTCTGCGTGGCATCGCGGAGACTGCGCAGTGTGGCGCAATTCTCGGTATCGGTGG CGGAAAAACCCTCGATACTGCCAAAGCACTGGCACATTTCATGGGTGTTCCGGTA GCGATCGCACCGACTATCGCCTCTACCGATGCACCGTGCAGCGCATTGTCTGTTAT CTACACCGATGAGGGTGAGTTTGACCGCTATCTGCTGTTGCCAAATAACCCGAATA TGGTCATTGTCGACACCAAAATCGTCGCTGGCGCACCTGCACGTCTGTTAGCGGC GGGTATCGGCGATGCGCTGGCAACCTGGTTTGAAGCGCGTGCCTGCTCTCGTAGC GGCGCGACCACCATGGCGGGCGGCAAGTGCACCCAGGCTGCGCTGGCACTGGCT GAACTGTGCTACAACACCCTGCTGGAAGAAGGCGAAAAAGCGATGCTTGCTGCC GAACAGCATGTAGTGACTCCGGCGCTGGAGCGCGTGATTGAAGCGAACACCTATT |
| | TGAGCGGTGTTGGTTTTGAAAGTGGTGGTCTGGCTGCGGCGCACGCAGTGCATAA CGGCCTGACCGCTATCCCGGACGCGCATCACTATTATCACGGTGAAAAAGTGGCAT TCGGTACGCTGACGCAGCTGGTTCTGGAAAACGCGCCGGTGGAGGAAATCGAAA CCGTAGCTGCGCTTAGCCATGCGGTAGGTTTGCCAATAACTCTCGCTCAACTGGAT ATTAAAGAAGATGTCCCGGCGAAAATGCGAATTGTGGCAGAAGCGGCATGTGCAG AAGGTGAAACCATCCACAACATGCCTGGCGGCGCGACGCCAGATCAGGTTTACGC CGCTCTGCTGGTAGCCGACCAGTACGGTCAGCGTTTCCTGCAAGAGTGGGAATAA (SEQ ID NO: 32) |

**[Table 1-17]**

| yohF/ACT43891.1/Escherichia coli BL21(DE3) | |
|---|---|
| Amino acid sequence | MAQVAIITASDSGIGKECALLLAQQGFDIGITWHSDEEGAKDTAREVVSHGVRAEIVQ LDLGNLPEGALALEKLIQRLGRIDVLVNNAGAMTKAPFLDMAFDEWRKIFTVDVDG AFLCSQIAARQMVKQGQGGRIINITSVHEHTPLPDASAYTAAKHALGGLTKAMALEL VRHKILVNAVAPGAIATPMNGMDDSDVKPDAEPSIPLRRFGATHEIASLVVWLCSEGA NYTTGQSLIVDGGFMLANPQFNPE (SEQ ID NO: 33) |
| Base sequence | ATGGCACAGGTTGCGATTATTACCGCCTCCGATTCGGGGATCGGCAAAGAGTGCGC GTTATTACTGGCGCAGCAGGGGTTTGATATTGGTATTACCTGGCACTCAGATGAAG AAGGGGCAAAAGATACCGCGCGTGAGGTAGTTAGCCACGGCGTACGTGCGGAGA TCGTGCAGCTGGATCTCGGCAATCTACCAGAAGGGGCACTGGCGCTGGAGAAACT CATTCAACGGCTGGGGCGCATTGATGTGCTGGTGAATAATGCGGGTGCAATGACCA AAGCGCCGTTTCTTGATATGGCTTTTGATGAGTGGCGCAAGATTTTTACCGTTGATG TCGATGGTGCATTCTTATGCTCGCAAATTGCGGCTCGTCAGATGGTGAAACAAGGG CAGGGCGGTCGCATCATCAACATTACGTCGGTACATGAACATACGCCGCTGCCGGA |
| | TGCCAGCGCCTACACAGCCGCTAAACATGCGCTCGGTGGGTTAACCAAAGCGATG GCGCTGGAGCTGGTCAGGCATAAGATTTTGGTGAACGCAGTCGCGCCTGGGGCGA TCGCCACGCCAATGAATGGCATGGATGACAGCGACGTGAAGCCCGACGCGGAGCC TTCGATTCCCTTGCGGCGTTTTGGCGCAACGCATGAGATTGCCAGCCTGGTGGTGT GGCTTTGTTCGGAGGGCGCAAATTACACCACCGGGCAGTCGTTGATAGTGGATGG CGGCTTTATGTTGGCGAATCCACAGTTCAACCCAGAATAG (SEQ ID NO: 34) |

**[Table 1-18]**

| yeaE/ACT43604.1/Escherichia coli BL21(DE3) | |
|---|---|
| Amino acid sequence | MQQKMIQFSGDVSLPAVGQGTWYMGEDASQRKTEVAALRAGIELGLTLIDTAEMYA DGGAEKVVGEALTGLREKVFLVSKVYPWNAGGQKAINACEASLRRLNTDYLDLYLL HWSGSFAFEETVAAMEKLIAQGKIRRWGVSNLDYADMQELWQLPGGNQCATNQVLY HLGSRGIEYDLLPWCQQQQMPVMAYSPLAQAGRLRNGLLKNAVVNEIAHAHNISAA QVLLAWVISHQGVMAIPKAATIAHVQQNAAVLEVELSSAELAMLDKAYPAPKGKTAL DMV (SEQ ID NO: 35) |
| Base sequence | ATGCAACAAAAAATGATTCAATTTAGTGGCGATGTCTCACTGCCAGCCGTAGGGCA GGGAACATGGTATATGGGCGAAGATGCCAGTCAGCGCAAAACAGAAGTTGCTGCA CTACGCGCGGGCATTGAACTCGGTTTAACCCTCATTGATACCGCCGAAATGTATGC CGATGGCGGTGCCGAAAAGGTGGTTGGGGAAGCATTAACCGGTCTGCGAGAGAA GGTCTTTCTCGTCTCTAAAGTCTATCCGTGGAATGCTGGCGGCAAAAAGCGATAA ATGCATGCGAAGCCAGTTTACGCCGTCTCAATACTGATTATCTCGATCTTTACTTATT ACACTGGTCTGGCAGTTTCGCTTTTGAAGAGACTGTCGCAGCGATGGAAAAATTG ATCGCCCAGGGAAAAATCCGCCGCTGGGGCGTTTCTAACCTTGATTATGCTGATAT GCAGGAACTCTGGCAGCTGCCGGGGGGAAATCAGTGTGCCACTAATCAGGTGCTT |
| | TACCATCTCGGTTCACGAGGAATTGAGTACGATCTACTCCCCTGGTGCCAGCAACA GCAGATGCCGGTGATGGCTTACAGTCCGTTAGCCCAGGCCGGGCGGTTGCGCAAT GGACTGTTAAAAAACGCGGTAGTCAACGAAATTGCACATGCTCACAATATCAGCG CGGCACAAGTATTGTTGGCGTGGGTGATCAGTCATCAGGGTGTGATGGCGATTCCA AAAGCGGCCACGATTGCCCATGTCCAACAAAATGCGGCTGTGCTTGAGGTCGAAC TTTCTTCAGCGGAATTAGCTATGCTGGATAAGGCATATCCGGCACCAAAAGGAAAA ACTGCGCTGGATATGGTGTGA (SEQ ID NO: 36) |

**[Table 1-19]**

| ADH1/NP_014555.1/Saccharomyces cerevisiae S288C | |
|---|---|
| Amino acid sequence | MSIPETQKGVIFYESHGKLEYKDIPVPKPKANELLINVKYSGVCHTDLHAWHGDWPL PVKLPLVGGHEGAGVVVGMGENVKGWKIGDYAGIKWLNGSCMACEYCELGNESNC PHADLSGYTHDGSFQQYATADAVQAAHIPQGTDLAQVAPILCAGITVYKALKSANLM AGHWVAISGAAGGLGSLAVQYAKAMGYRVLGIDGGEGKEELFRSIGGEVFIDFTKEK DIVGAVLKATDGGAHGVINVSVSEAAIEASTRYVRANGTTVLVGMPAGAKCCSDVFN QVVKSISIVGSYVGNRADTREALDFFARGLVKSPIKVVGLSTLPEIYEKMEKGQIVGR YVVDTSK (SEQ ID NO: 37) |
| Base sequence | ATGTCTATCCCAGAAACTCAAAAAGGTGTTATCTTCTACGAATCCCACGGTAAGTT GGAATACAAAGATATTCCAGTTCCAAAGCCAAAGGCCAACGAATTGTTGATCAAC GTTAAATACTCTGGTGTCTGTCACACTGACTTGCACGCTTGGCACGGTGACTGGCC ATTGCCAGTTAAGCTACCATTAGTCGGTGGTCACGAAGGTGCCGGTGTCGTTGTCG GCATGGGTGAAAACGTTAAGGGCTGGAAGATCGGTGACTACGCCGGTATCAAATG GTTGAACGGTTCTTGTATGGCCTGTGAATACTGTGAATTGGGTAACGAATCCAACT GTCCTCACGCTGACTTGTCTGGTTACACCCACGACGGTTCTTTCCAACAATACGCT |
| | ACCGCTGACGCTGTTCAAGCCGCTCACATTCCTCAAGGTACCGACTTGGCCCAAG TCGCCCCCATCTTGTGTGCTGGTATCACCGTCTACAAGGCTTTGAAGTCTGCTAAC TTGATGGCCGGTCACTGGGTTGCTATCTCCGGTGCTGCTGGTGGTCTAGGTTCTTT GGCTGTTCAATACGCCAAGGCTATGGGTTACAGAGTCTTGGGTATTGACGGTGGTG AAGGTAAGGAAGAATTATTCAGATCCATCGGTGGTGAAGTCTTCATTGACTTCACT AAGGAAAAGGACATTGTCGGTGCTGTTCTAAAGGCCACTGACGGTGGTGCTCACG GTGTCATCAACGTTTCCGTTTCCGAAGCCGCTATTGAAGCTTCTACCAGATACGTT AGAGCTAACGGTACCACCGTTTTGGTCGGTATGCCAGCTGGTGCCAAGTGTTGTTC TGATGTCTTCAACCAAGTCGTCAAGTCCATCTCTATTGTTGGTTCTTACGTCGGTAA CAGAGCTGACACCAGAGAAGCTTTGGACTTCTTCGCCAGAGGTTTGGTCAAGTCT CCAATCAAGGTTGTCGGCTTGTCTACCTTGCCAGAAATTTACGAAAAGATGGAAA AGGGTCAAATCGTTGGTAGATACGTTGTTGACACTTCTAAATAA (SEQ ID NO: 38) |

**[Table 1-20]**

| ADH2/NP_014032.1/Saccharomyces cerevisiae S288C | |
|---|---|
| Amino acid sequence | MSIPETQKAIIFYESNGKLEHKDIPVPKPKPNELLINVKYSGVCHTDLHAWHGDWPLP TKLPLVGGHEGAGVVVGMGENVKGWKIGDYAGIKWLNGSCMACEYCELGNESNCP HADLSGYTHDGSFQEYATADAVQAAHIPQGTDLAEVAPILCAGITVYKALKSANLRA GHWAAISGAAGGLGSLAVQYAKAMGYRVLGIDGGPGKEELFTSLGGEVFIDFTKEKD IVSAVVKATNGGAHGIINVSVSEAAIEASTRYCRANGTVVLVGLPAGAKCSSDVFNHV VKSISIVGSYVGNRADTREALDFFARGLVKSPIKVVGLSSLPEIYEKMEKGQIAGRYV VDTSK (SEQ ID NO: 39) |
| Base sequence | ATGTCTATTCCAGAAACTCAAAAAGCCATTATCTTCTACGAATCCAACGGCAAGTT GGAGCATAAGGATATCCCAGTTCCAAAGCCAAAGCCCAACGAATTGTTAATCAAC |
| | GTCAAGTACTCTGGTGTCTGCCACACCGATTTGCACGCTTGGCATGGTGACTGGCC ATTGCCAACTAAGTTACCATTAGTTGGTGGTCACGAAGGTGCCGGTGTCGTTGTCG GCATGGGTGAAAACGTTAAGGGCTGGAAGATCGGTGACTACGCCGGTATCAAATG GTTGAACGGTTCTTGTATGGCCTGTGAATACTGTGAATTGGGTAACGAATCCAACT GTCCTCACGCTGACTTGTCTGGTTACACCCACGACGGTTCTTTCCAAGAATACGCT ACCGCTGACGCTGTTCAAGCCGCTCACATTCCTCAAGGTACTGACTTGGCTGAAG TCGCGCCAATCTTGTGTGCTGGTATCACCGTATACAAGGCTTTGAAGTCTGCCAAC TTGAGAGCAGGCCACTGGGCGGCCATTTCTGGTGCTGCTGGTGGTCTAGGTTCTTT GGCTGTTCAATATGCTAAGGCGATGGGTTACAGAGTCTTAGGTATTGATGGTGGTC CAGGAAAGGAAGAATTGTTTACCTCGCTCGGTGGTGAAGTATTCATCGACTTCACC AAAGAGAAGGACATTGTTAGCGCAGTCGTTAAGGCTACCAACGGCGGTGCCCACG GTATCATCAATGTTTCCGTTTCCGAAGCCGCTATCGAAGCTTCTACCAGATACTGTA GGGCGAACGGTACTGTTGTCTTGGTTGGTTTGCCAGCCGGTGCAAAGTGCTCCTC TGATGTCTTCAACCACGTTGTCAAGTCTATCTCCATTGTCGGCTCTTACGTGGGGA ACAGAGCTGATACCAGAGAAGCCTTAGATTTCTTTGCCAGAGGTCTAGTCAAGTCT CCAATAAAGGTAGTTGGCTTATCCAGTTTACCAGAAATTTACGAAAAGATGGAGAA GGGCCAAATTGCTGGTAGATACGTTGTTGACACTTCTAAATAA (SEQ ID NO: 40) |

**[Table 1-21]**

| ADH3/NP_013800.1/Saccharomyces cerevisiae S288C | |
|---|---|
| Amino acid sequence | |
| |  (SEQ ID NO: 41) |
| Base sequence | ATGTTGAGAACGTCAACATTGTTCACCAGGCGTGTCCAACCAAGCCTATTTTCTAG AAACATTCTTAGATTGCAATCCACAGCTGCAATCCCTAAGACTCAAAAAGGTGTCA TCTTTTATGAGAATAAGGGGAAGCTGCATTACAAAGATATCCCTGTCCCCGAGCCT AAGCCAAATGAAATTTTAATCAACGTTAAATATTCTGGTGTATGTCACACCGATTTA CATGCTTGGCACGGCGATTGGCCATTACCTGTTAAACTACCATTAGTAGGTGGTCAT GAAGGTGCTGGTGTAGTTGTCAAACTAGGTTCCAATGTCAAGGGCTGGAAAGTCG GTGATTTAGCAGGTATCAAATGGCTGAACGGTTCTTGTATGACATGCGAATTCTGT GAATCAGGTCATGAATCAAATTGTCCAGATGCTGATTTATCTGGTTACACTCATGAT GGTTCTTTCCAACAATTTGCGACCGCTGATGCTATTCAAGCCGCCAAAATTCAACA GGGTACCGACTTGGCCGAAGTAGCCCCAATATTATGTGCTGGTGTTACTGTATATAA AGCACTAAAAGAGGCAGACTTGAAAGCTGGTGACTGGGTTGCCATCTCTGGTGCT GCAGGTGGCTTGGGTTCCTTGGCCGTTCAATATGCAACTGCGATGGGTTACAGAGT TCTAGGTATTGATGCAGGTGAGGAAAAGGAAAAACTTTTCAAGAAATTGGGGGGT GAAGTATTCATCGACTTTACTAAAACAAAGAATATGGTTTCTGACATTCAAGAAGC TACCAAAGGTGGCCCTCATGGTGTCATTAACGTTTCCGTTTCTGAAGCCGCTATTTC TCTATCTACGGAATATGTTAGACCATGTGGTACCGTCGTTTTGGTTGGTTTGCCCGC TAACGCCTACGTTAAATCAGAGGTATTCTCTCATGTGGTGAAGTCCATCAATATCAA GGGTTCTTATGTTGGTAACAGAGCTGATACGAGAGAAGCCTTAGACTTCTTTAGCA GAGGTTTGATCAAATCACCAATCAAAATTGTTGGATTATCTGAATTACCAAAGGTTT ATGACTTGATGGAAAAGGGCAAGATTTTGGGTAGATACGTCGTCGATACTAGTAAA |
| | TAA (SEQ ID NO: 42) |

**[Table 1-22]**

| ADH4/NP_011258.2/Saccharomyces cerevisiae S288C | |
|---|---|
| Amino acid sequence | MSSVTGFYIPPISFFGEGALEETADYIKNKDYKKALIVTDPGIAAIGLSGRVQKMLEER DLNVAIYDKTQPNPNIANVTAGLKVLKEQNSEIVVSIGGGSAHDNAKAIALLATNGGE IGDYEGVNQSKKAALPLFAINTTAGTASEMTRFTIISNEEKKIKMAIIDNNVTPAVAVND PSTMFGLPPALTAATGLDALTHCIEAYVSTASNPITDACALKGIDLINESLVAAYKDGK DKKARTDMCYAEYLAGMAFNNASLGYVHALAHQLGGFYHLPHGVCNAVLLPHVQE ANMQCPKAKKRLGEIALHFGASQEDPEETIKALHVLNRTMNIPRNLKELGVKTEDFEI LAEHAMHDACHLTNPVQFTKEQVVAIIKKAYEY (SEQ ID NO: 43) |
| Base sequence | ATGTCTTCCGTTACTGGGTTTTACATTCCACCAATCTCTTTCTTTGGTGAAGGTGCT TTAGAAGAAACCGCTGATTACATCAAAAACAAGGATTACAAAAAGGCTTTGATCG TTACTGATCCTGGTATTGCAGCTATTGGTCTCTCCGGTAGAGTCCAAAAGATGTTG GAAGAACGTGACTTAAACGTTGCTATCTATGACAAAACTCAACCAAACCCAAATAT TGCCAATGTCACAGCTGGTTTGAAGGTTTTGAAGGAACAAAACTCTGAAATTGTT GTTTCCATTGGTGGTGGTTCTGCTCACGACAATGCTAAGGCCATTGCTTTATTGGCT ACTAACGGTGGGGAAATCGGAGACTATGAAGGTGTCAATCAATCTAAGAAGGCTG CTTTACCACTATTTGCCATCAACACTACTGCTGGTACTGCTTCCGAAATGACCAGAT TCACTATTATCTCTAATGAAGAAAAGAAAATCAAGATGGCTATCATTGACAACAAC GTCACTCCAGCTGTTGCTGTCAACGATCCATCTACCATGTTTGGTTTGCCACCTGCT TTGACTGCTGCTACTGGTCTAGATGCTTTGACTCACTGTATCGAAGCTTATGTTTCC ACCGCCTCTAACCCAATCACCGATGCCTGTGCTTTGAAGGGTATTGATTTGATCAAT GAAAGCTTAGTCGCTGCATACAAAGACGGTAAAGACAAGAAGGCCAGAACTGAC |
| | ATGTGTTACGCTGAATACTTGGCAGGTATGGCTTTCAACAATGCTTCTCTAGGTTAT GTTCATGCCCTTGCTCATCAACTTGGTGGTTTCTACCACTTGCCTCATGGTGTTTGT AACGCTGTCTTGTTGCCTCATGTTCAAGAGGCCAACATGCAATGTCCAAAGGCCA AGAAGAGATTAGGTGAAATTGCTTTGCATTTCGGTGCTTCTCAAGAAGATCCAGA AGAAACCATCAAGGCTTTGCACGTTTTAAACAGAACCATGAACATTCCAAGAAAC TTGAAAGAATTAGGTGTTAAAACCGAAGATTTTGAAATTTTGGCTGAACACGCCAT GCATGATGCCTGCCATTTGACTAACCCAGTTCAATTCACCAAAGAACAAGTGGTTG CCATTATCAAGAAAGCCTATGAATATTAA (SEQ ID NO: 44) |

**[Table 1-23]**

| ADH5/NP_009703.3/Saccharomyces cerevisiae S288C | |
|---|---|
| Amino acid sequence | MPSQVIPEKQKAIVFYETDGKLEYKDVTVPEPKPNEILVHVKYSGVCHSDLHAWHGD WPFQLKFPLIGGHEGAGVVVKLGSNVKGWKVGDFAGIKWLNGTCMSCEYCEVGNE SQCPYLDGTGFTHDGTFQEYATADAVQAAHIPPNVNLAEVAPILCAGITVYKALKRAN VIPGQWVTISGACGGLGSLAIQYALAMGYRVIGIDGGNAKRKLFEQLGGEIFIDFTEE KDIVGAIIKATNGGSHGVINVSVSEAAIEASTRYCRPNGTVVLVGMPAHAYCNSDVFN QVVKSISIVGSCVGNRADTREALDFFARGLIKSPIHLAGLSDVPEIFAKMEKGEIVGRY VVETSK (SEQ ID NO: 45) |
| Base sequence | ATGCCTTCGCAAGTCATTCCTGAAAAACAAAAGGCTATTGTCTTTTATGAGACAGA TGGAAAATTGGAATATAAAGACGTCACAGTTCCGGAACCTAAGCCTAACGAAATT TTAGTCCACGTTAAATATTCTGGTGTTTGTCATAGTGACTTGCACGCGTGGCACGGT GATTGGCCATTTCAATTGAAATTTCCATTAATCGGTGGTCACGAAGGTGCTGGTGT TGTTGTTAAGTTGGGATCTAACGTTAAGGGCTGGAAAGTCGGTGATTTTGCAGGTA TAAAATGGTTGAATGGGACTTGCATGTCCTGTGAATATTGTGAAGTAGGTAATGAA |
| | TCTCAATGTCCTTATTTGGATGGTACTGGCTTCACACATGATGGTACTTTTCAAGAA TACGCAACTGCCGATGCCGTTCAAGCTGCCCATATTCCACCAAACGTCAATCTTGC TGAAGTTGCCCCAATCTTGTGTGCAGGTATCACTGTTTATAAGGCGTTGAAAAGAG CCAATGTGATACCAGGCCAATGGGTCACTATATCCGGTGCATGCGGTGGCTTGGGT TCTCTGGCAATCCAATACGCCCTTGCTATGGGTTACAGGGTCATTGGTATCGATGGT GGTAATGCCAAGCGAAAGTTATTTGAACAATTAGGCGGAGAAATATTCATCGATTT CACGGAAGAAAAAGACATTGTTGGTGCTATAATAAAGGCCACTAATGGCGGTTCT CATGGAGTTATTAATGTGTCTGTTTCTGAAGCAGCTATCGAGGCTTCTACGAGGTAT TGTAGGCCCAATGGTACTGTCGTCCTGGTTGGTATGCCAGCTCATGCTTACTGCAAT TCCGATGTTTTCAATCAAGTTGTAAAATCAATCTCCATCGTTGGATCTTGTGTTGGA AATAGAGCTGATACAAGGGAGGCTTTAGATTTCTTCGCCAGAGGTTTGATCAAATC TCCGATCCACTTAGCTGGCCTATCGGATGTTCCTGAAATTTTTGCAAAGATGGAGA AGGGTGAAATTGTTGGTAGATATGTTGTTGAGACTTCTAAATGA (SEQ ID NO: 46) |

**[Table 1-24]**

| ADH6/NP_014051.3/Saccharomyces cerevisiae S288C | |
|---|---|
| Amino acid sequence | MSYPEKFEGIAIQSHEDWKNPKKTKYDPKPFYDHDIDIKIEACGVCGSDIHCAAGHW GNMKMPLVVGHEIVGKVVKLGPKSNSGLKVGQRVGVGAQVFSCLECDRCKNDNEP YCTKFVTTYSQPYEDGYVSQGGYANYVRVHEHFVVPIPENIPSHLAAPLLCGGLTVYS PLVRNGCGPGKKVGIVGLGGIGSMGTLISKAMGAETYVISRSSRKREDAMKMGADH YIATLEEGDWGEKYFDTFDLIVVCASSLTDIDFNIMPKAMKVGGRIVSISIPEQHEMLS LKPYGLKAVSISYSALGSIKELNQLLKLVSEKDIKIWVETLPVGEAGVHEAFERMEKG DVRYRFTLVGYDKEFSD (SEQ ID NO: 47) |
| Base sequence | ATGTCTTATCCTGAGAAATTTGAAGGTATCGCTATTCAATCACACGAAGATTGGAA |
| | AAACCCAAAGAAGACAAAGTATGACCCAAAACCATTTTACGATCATGACATTGAC ATTAAGATCGAAGCATGTGGTGTCTGCGGTAGTGATATTCATTGTGCAGCTGGTCAT TGGGGCAATATGAAGATGCCGCTAGTCGTTGGTCATGAAATCGTTGGTAAAGTTGT CAAGCTAGGGCCCAAGTCAAACAGTGGGTTGAAAGTCGGTCAACGTGTTGGTGTA GGTGCTCAAGTCTTTTCATGCTTGGAATGTGACCGTTGTAAGAATGATAATGAACC ATACTGCACCAAGTTTGTTACCACATACAGTCAGCCTTATGAAGACGGCTATGTGT CGCAGGGTGGCTATGCAAACTACGTCAGAGTTCATGAACATTTTGTGGTGCCTATC CCAGAGAATATTCCATCACATTTGGCTGCTCCACTATTATGTGGTGGTTTGACTGTG TACTCTCCATTGGTTCGTAACGGTTGCGGTCCAGGTAAAAAAGTTGGTATAGTTGG TCTTGGTGGTATCGGCAGTATGGGTACATTGATTTCCAAAGCCATGGGGGCAGAGA CGTATGTTATTTCTCGTTCTTCGAGAAAAAGAGAAGATGCAATGAAGATGGGCGCC GATCACTACATTGCTACATTAGAAGAAGGTGATTGGGGTGAAAAGTACTTTGACAC CTTCGACCTGATTGTAGTCTGTGCTTCCTCCCTTACCGACATTGACTTCAACATTAT GCCAAAGGCTATGAAGGTTGGTGGTAGAATTGTCTCAATCTCTATACCAGAACAAC ACGAAATGTTATCGCTAAAGCCATATGGCTTAAAGGCTGTCTCCATTTCTTACAGTG CTTTAGGTTCCATCAAAGAATTGAACCAACTCTTGAAATTAGTCTCTGAAAAAGAT ATCAAAATTTGGGTGGAAACATTACCTGTTGGTGAAGCCGGCGTCCATGAAGCCTT CGAAAGGATGGAAAAGGGTGACGTTAGATATAGATTTACCTTAGTCGGCTACGACA AAGAATTTTCAGACTAG (SEQ ID NO: 48) |

**[Table 1-25]**

| ADH7/NP_010030.1/Saccharomyces cerevisiae S288C | |
|---|---|
| Amino acid sequence | MLYPEKFQGIGISNAKDWKHPKLVSFDPKPFGDHDVDVEIEACGICGSDFHIAVGNW GPVPENQILGHEIIGRVVKVGSKCHTGVKIGDRVGVGAQALACFECERCKSDNEQYC TNDHVLTMWTPYKDGYISQGGFASHVRLHEHFAIQIPENIPSPLAAPLLCGGITVFSPL LRNGCGPGKRVGIVGIGGIGHMGILLAKAMGAEVYAFSRGHSKREDSMKLGADHYI AMLEDKGWTEQYSNALDLLVVCSSSLSKVNFDSIVKIMKIGGSIVSIAAPEVNEKLVL KPLGLMGVSISSSAIGSRKEIEQLLKLVSEKNVKIWVEKLPISEEGVSHAFTRMESGDV KYRFTLVDYDKKFHK (SEQ ID NO: 49) |
| Base sequence | ATGCTTTACCCAGAAAAATTTCAGGGCATCGGTATTTCCAACGCAAAGGATTGGAA GCATCCTAAATTAGTGAGTTTTGACCCAAAACCCTTTGGCGATCATGACGTTGATG TTGAAATTGAAGCCTGTGGTATCTGCGGATCTGATTTTCATATAGCCGTTGGTAATT GGGGTCCAGTCCCAGAAAATCAAATCCTTGGACATGAAATAATTGGCCGCGTGGT GAAGGTTGGATCCAAGTGCCACACTGGGGTAAAAATCGGTGACCGTGTTGGTGTT GGTGCCCAAGCCTTGGCGTGTTTTGAGTGTGAACGTTGCAAAAGTGACAACGAGC AATACTGTACCAATGACCACGTTTTGACTATGTGGACTCCTTACAAGGACGGCTAC ATTTCACAAGGAGGCTTTGCCTCCCACGTGAGGCTTCATGAACACTTTGCTATTCA AATACCAGAAAATATTCCAAGTCCGCTAGCCGCTCCATTATTGTGTGGTGGTATTAC AGTTTTCTCTCCACTACTAAGAAATGGCTGTGGTCCAGGTAAGAGGGTAGGTATTG TTGGCATCGGTGGTATTGGGCATATGGGGATTCTGTTGGCTAAAGCTATGGGAGCC GAGGTTTATGCGTTTTCGCGAGGCCACTCCAAGCGGGAGGATTCTATGAAACTCG GTGCTGATCACTATATTGCTATGTTGGAGGATAAAGGCTGGACAGAACAATACTCT AACGCTTTGGACCTTCTTGTCGTTTGCTCATCATCTTTGTCGAAAGTTAATTTTGAC AGTATCGTTAAGATTATGAAGATTGGAGGCTCCATCGTTTCAATTGCTGCTCCTGAA GTTAATGAAAAGCTTGTTTTAAAACCGTTGGGCCTAATGGGAGTATCAATCTCAAG CAGTGCTATCGGATCTAGGAAGGAAATCGAACAACTATTGAAATTAGTTTCCGAAA |
| | AGAATGTCAAAATATGGGTGGAAAAACTTCCGATCAGCGAAGAAGGCGTCAGCCA TGCCTTTACAAGGATGGAAAGCGGAGACGTCAAATACAGATTTACTTTGGTCGATT ATGATAAGAAATTCCATAAATAG (SEQ ID NO: 50) |

**[Table 1-26]**

| SFA1/NP_010113.1/Saccharomyces cerevisiae S288C | |
|---|---|
| Amino acid sequence | MSAATVGKPIKCIAAVAYDAKKPLSVEEITVDAPKAHEVRIKIEYTAVCHTDAYTLSGS DPEGLFPCVLGHEGAGIVESVGDDVITVKPGDHVIALYTAECGKCKFCTSGKTNLCG AVRATQGKGVMPDGTTRFHNAKGEDIYHFMGCSTFSEYTVVADVSVVAIDPKAPLDA ACLLGCGVTTGFGAALKTANVQKGDTVAVFGCGTVGLSVIQGAKLRGASKIIAIDINN KKKQYCSQFGATDFVNPKEDLAKDQTIVEKLIEMTDGGLDFTFDCTGNTKIMRDALE ACHKGWGQSIIIGVAAAGEEISTRPFQLVTGRVWKGSAFGGIKGRSEMGGLIKDYQK GALKVEEFITHRRPFKEINQAFEDLHNGDCLRTVLKSDEIK (SEQ ID NO: 51) |
| Base sequence | ATGTCCGCCGCTACTGTTGGTAAACCTATTAAGTGCATTGCTGCTGTTGCGTATGAT GCGAAGAAACCATTAAGTGTTGAAGAAATCACGGTAGACGCCCCAAAAGCGCAC GAAGTACGTATCAAAATTGAATATACTGCTGTATGCCACACTGATGCGTACACTTTA TCAGGCTCTGATCCAGAAGGACTTTTCCCTTGCGTTCTGGGCCACGAAGGAGCCG GTATCGTAGAATCTGTAGGCGATGATGTCATAACAGTTAAGCCTGGTGATCATGTTA TTGCTTTGTACACTGCTGAGTGTGGCAAATGTAAGTTCTGTACTTCCGGTAAAACC AACTTATGTGGTGCTGTTAGAGCTACTCAAGGGAAAGGTGTAATGCCTGATGGGAC CACAAGATTTCATAATGCGAAAGGTGAAGATATATACCATTTCATGGGTTGCTCTAC TTTTTCCGAATATACTGTGGTGGCAGATGTCTCTGTGGTTGCCATCGATCCAAAAGC TCCCTTGGATGCTGCCTGTTTACTGGGTTGTGGTGTTACTACTGGTTTTGGGGCGG CTCTTAAGACAGCTAATGTGCAAAAAGGCGATACCGTTGCAGTATTTGGCTGCGGG |
| | ACTGTAGGACTCTCCGTTATCCAAGGTGCAAAGTTAAGGGGCGCTTCCAAGATCAT TGCCATTGACATTAACAATAAGAAAAAACAATATTGTTCTCAATTTGGTGCCACGG ATTTTGTTAATCCCAAGGAAGATTTGGCCAAAGATCAAACTATCGTTGAAAAGTTA ATTGAAATGACTGATGGGGGTCTGGATTTACTTTTGACTGTACTGGTAATACCAAA ATTATGAGAGATGCTTTGGAAGCCTGTCATAAAGGTTGGGGTCAATCTATTATCATT GGTGTGGCTGCCGCTGGTGAAGAAATTTCTACAAGGCCGTTCCAGCTGGTCACTG GTAGAGTGTGGAAAGGCTCTGCTTTTGGTGGCATCAAAGGTAGATCTGAAATGGG CGGTTTAATTAAAGACTATCAAAAAGGTGCCTTAAAAGTCGAAGAATTTATCACTC ACAGGAGACCATTCAAAGAAATCAATCAAGCCTTTGAAGATTTGCATAACGGTGA TTGCTTAAGAACCGTCTTGAAGTCTGATGAAATAAAATAG (SEQ ID NO: 52) |

**[Table 1-27]**

| AAD3/NP_010032.1/Saccharomyces cerevisiae S288C | |
|---|---|
| Amino acid sequence | MIGSASDSSSKLGRLRFLSETAAIKVSPLILGEVSYDGARSDFLKSMNKNRAFELLDTF YEAGGNFIDAANNCQNEQSEEWIGEWIQSRRLRDQIVIATKFIKSDKKYKAGESNTAN YCGNHKRSLHVSVRDSLRKLQTDWIDILYVHWWDYMSSIEEFMDSLHILVQQGKVL YLGVSDTPAWVVSAANYYATSYGKTPFSIYQGKWNVLNRDFERDIIPMARHFGMAL APWDVMGGGRFQSKKAMEERRKNGEGIRSFVGASEQTDAEIKISEALAKIAEEHGTE SVTAIAIAYVRSKAKNFFPSVEGGKIEDLKENIKALSIDLTPDNIKYLESIVPFDIGFPNN FIVLNSLTQKYGTNNV (SEQ ID NO: 53) |
| Base sequence | ATGATTGGGTCCGCGTCCGACTCATCTAGCAAGTTAGGACGCCTCCGATTTCTTTCT GAAACTGCCGCTATTAAAGTATCCCCGTTAATCCTAGGAGAAGTCTCATACGATGG AGCACGTTCGGATTTTCTCAAATCAATGAACAAGAATCGAGCTTTTGAATTGCTTG ATACTTTTTACGAGGCAGGTGGAAATTTCATTGATGCCGCAAACAACTGCCAAAAC |
| | GAGCAATCAGAAGAATGGATTGGTGAATGGATACAGTCCAGAAGGTTACGTGATC AAATTGTCATTGCAACCAAGTTTATAAAAAGCGATAAAAAGTATAAAGCAGGTGA AAGTAACACTGCCAACTACTGTGGTAATCACAAGCGTAGTTTACATGTGAGTGTGA GGGATTCTCTCCGCAAATTGCAAACTGATTGGATTGATATACTTTACGTTCACTGGT GGGATTATATGAGTTCAATCGAAGAATTTATGGATAGTTTGCATATTCTGGTCCAGC AGGGCAAGGTCCTCTATTTGGGTGTATCTGATACACCTGCTTGGGTTGTTTCTGCG GCAAACTACTACGCTACATCTTATGGTAAAACTCCCTTTAGTATCTACCAAGGTAAA TGGAACGTGTTGAACAGAGATTTTGAGCGTGATATTATTCCAATGGCTAGGCATTT CGGTATGGCCCTCGCCCCATGGGATGTCATGGGAGGTGGAAGATTTCAGAGTAAA AAAGCAATGGAGGAACGGAGGAAGAATGGAGAGGGTATTCGTTCTTTCGTTGGCG CCTCCGAACAAACAGATGCAGAAATCAAGATTAGTGAAGCATTGGCCAAGATTGC TGAGGAACATGGCACTGAGTCTGTTACTGCTATTGCTATTGCCTATGTTCGCTCTAA GGCGAAAAATTTTTTTCCGTCGGTTGAAGGAGGAAAAATTGAGGATCTCAAAGAG AACATTAAGGCTCTCAGTATCGATCTAACGCCAGACAATATAAAATACTTAGAAAG TATAGTTCCTTTTGACATCGGATTTCCTAATAATTTTATCGTGTTAAATTCCTTGACT CAAAAATATGGTACGAATAATGTTTAG (SEQ ID NO: 54) |

**[Table 1-28]**

| AAD4/NP_010038.1/Saccharomyces cerevisiae S288C | |
|---|---|
| Amino acid sequence | MGSMNKEQAFELLDAFYEAGGNCIDTANSYQNEESEIWIGEWMKSRKLRDQIVIATK FTGDYKKYEVGGGKSANYCGNHKHSLHVSVRDSLRKLQTDWIDILYVHWWDYMSS IEEVMDSLHILVQQGKVLYLGVSDTPAWVVSAANYYATSHGKTPFSIYQGKWNVLNR DFERDIIPMARHFGMALAPWDVMGGGRFQSKKAMEERKKNGEGLRTVSGTSKQTD KEVKISEALAKVAEEHGTESVTAIAIAYVRSKAKNVFPLVGGRKIEHLKQNIEALSIKL |
| | TPEQIEYLESIIPFDVGFPTNFIGDDPAVTKKASLLTAMSAQISFD (SEQ ID NO: 55) |
| Base sequence | ATGGGCTCTATGAATAAGGAACAGGCTTTTGAACTTCTTGATGCTTTTTATGAAGC AGGAGGTAATTGCATTGATACTGCAAACAGTTACCAAAATGAAGAGTCAGAGATT TGGATAGGTGAATGGATGAAATCAAGAAAGTTGCGTGACCAAATTGTAATTGCCAC CAAGTTTACCGGAGATTATAAGAAGTATGAAGTAGGTGGCGGTAAAAGTGCCAAC TATTGTGGTAATCACAAGCATAGTTTACATGTGAGTGTGAGGGATTCTCTCCGCAA ATTGCAAACTGATTGGATTGATATACTTTACGTTCACTGGTGGGATTATATGAGTTC AATCGAAGAAGTTATGGATAGTTTGCATATTTTAGTTCAGCAGGGCAAAGTCCTCT ATTTGGGTGTGTCTGATACACCTGCTTGGGTTGTTTCTGCGGCAAACTACTACGCC ACATCTCATGGGAAAACTCCTTTTAGTATCTATCAAGGTAAATGGAATGTGTTGAA CAGGGACTTTGAGCGCGATATCATTCCAATGGCCAGACATTTTGGTATGGCTCTAG CCCCATGGGATGTTATGGGAGGTGGAAGATTTCAGAGTAAAAAAGCAATGGAGGA ACGGAAGAAGAATGGAGAGGGTCTGCGTACTGTTTCGGGTACTTCTAAACAGACG GATAAAGAGGTTAAGATCAGTGAAGCATTGGCCAAGGTTGCTGAGGAACATGGCA CTGAGTCTGTTACTGCTATTGCTATTGCCTATGTTCGCTCTAAGGCGAAAAATGTTT TCCCATTGGTTGGTGGAAGGAAAATTGAACACCTCAAACAGAACATTGAGGCTTT AAGTATCAAACTGACACCAGAACAGATAGAATACTTAGAAAGTATTATTCCTTTTG ATGTTGGTTTTCCTACTAATTTTATCGGTGATGATCCGGCTGTTACCAAGAAGGCTT CACTTCTCACGGCAATGTCTGCGCAGATTTCCTTCGATTAA (SEQ ID NO: 56) |

**[Table 1-29]**

| AAD10/NP_012689.1/Saccharomyces cerevisiae S288C | |
|---|---|
| Amino acid sequence | MASRKLRDQIVIATKFTTDYKGYDVGKGKSANFCGNHKRSLHVSVRDSLRKLQTDW IDILYVHWWDYMSSIEEVMDSLHILVQQGKVLYLGVSDTPAWVVSAANYYATSHGKT PFSIYQGKWNVLNRDFERDIIPMARHFGMALAPWDVMGGGRFQSKKAVEERKKKGE GLRTFFGTSEQTDMEVKISEALLKVAEEHGTESVTAIAIAYVRSKAKHVFPLVGGRKIE HLKQNIEALSIKLTPEQIKYLESIVPFDVGFPTNFIGDDPAVTKKPSFLTEMSAKISFED (SEQ ID NO: 57) |
| Base sequence | ATGGCATCAAGAAAACTGCGTGACCAGATTGTAATTGCCACTAAATTTACCACGGA TTATAAGGGGTATGATGTAGGCAAGGGGAAGAGTGCCAATTTCTGTGGGAATCACA AGCGCAGTTTGCATGTAAGTGTGAGAGATTCCCTTCGTAAGTTGCAAACTGATTGG ATTGATATTCTTTACGTTCACTGGTGGGATTATATGAGCTCCATTGAGGAAGTTATG GATAGTTTGCACATTCTTGTGCAGCAGGGCAAAGTACTCTATCTAGGTGTGTCTGA TACTCCTGCCTGGGTTGTTTCTGCAGCAAATTACTACGCCACATCTCATGGTAAAA CTCCCTTTAGTATCTATCAAGGTAAATGGAATGTATTGAACAGGGACTTTGAACGT GATATCATTCCAATGGCTAGGCATTTTGGTATGGCTCTTGCTCCATGGGATGTTATG GGAGGCGGGAGATTTCAGAGTAAAAAGGCAGTGGAAGAGCGGAAGAAGAAAGG AGAAGGCTTGCGTACCTTTTTTGGTACTTCGGAACAGACGGATATGGAGGTTAAAA TCAGCGAAGCATTGTTAAAAGTTGCGGAAGAACATGGCACTGAGTCTGTCACTGC TATTGCCATAGCTTATGTTCGGTCTAAAGCGAAACATGTTTTCCCATTAGTGGGAGG AAGAAAGATCGAACATCTCAAACAGAACATTGAGGCTTTGAGCATTAAATTAACA CCAGAACAAATAAAGTACTTAGAAAGTATTGTTCCTTTTGATGTCGGATTTCCCAC TAATTTTATTGGAGATGACCCAGCTGTTACCAAGAAACCTTCATTTCTCACCGAAAT GTCTGCCAAGATTAGCTTCGAAGATTAG (SEQ ID NO: 58) |

**[Table 1-30]**

| AAD14/NP_014068.1/Saccharomyces cerevisiae S288C | |
|---|---|
| Amino acid sequence | MTDLFKPLPEPPTELGRLRVLSKTAGIRVSPLILGGASIGDAWSGFMGSMNKEQAFEL LDAFYEAGGNCIDTANSYQNEESEIWIGEWMASRKLRDQIVIATKFTGDYKKYEVGG GKSANYCGNHKRSLHVSVRDSLRKLQTDWIDILYIHWWDYMSSIEEVMDSLHILVQQ GKVLYLGVSDTPAWVVSAANYYATSHGKTPFSVYQGKWNVLNRDFERDIIPMARHF GMALAPWDVMGGGRFQSKKAMEERKKNGEGLRTFVGGPEQTELEVKISEALTKIAE EHGTESVTAIAIAYVRSKAKNVFPLIGGRKIEHLKQNIEALSIKLTPEQIEYLESIVPFDV GFPKSLIGDDPAVTKKLSPLTSMSARIAFDN (SEQ ID NO: 59) |
| Base sequence | ATGACTGACTTGTTTAAACCTCTACCTGAACCACCTACCGAATTGGACGTCTCAG GGTTCTTTCTAAAACTGCCGGCATAAGGGTTTCACCGCTAATTCTGGGAGGAGCTT CAATCGGCGACGCATGGTCAGGCTTTATGGGCTCTATGAATAAGGAACAGGCCTTT GAACTTCTTGATGCTTTTTATGAAGCTGGAGGTAATTGTATTGATACTGCAAACAGT TACCAAAATGAAGAGTCAGAGATTTGGATAGGTGAATGGATGGCATCAAGAAAAC TGCGTGACCAGATTGTAATTGCCACCAAGTTTACCGGAGATTATAAGAAGTATGAA GTAGGTGGTGGTAAAAGTGCCAACTACTGTGGTAATCACAAGCGTAGTTTACATGT GAGTGTGAGGGATTCTCTCCGCAAATTGCAAACTGATTGGATTGATATACTTTACAT TCACTGGTGGGATTATATGAGTTCAATCGAAGAAGTTATGGATAGTTTGCATATTTT AGTTCAGCAGGGCAAGGTCCTATATTTAGGAGTATCTGATACACCTGCTTGGGTTG TTTCTGCGGCAAATTACTACGCTACATCTCATGGTAAAACTCCTTTTAGCGTCTATC AAGGTAAATGGAATGTATTGAACAGGGACTTTGAGCGTGATATTATTCCAATGGCT AGGCATTTTGGTATGGCTCTAGCCCCATGGGATGTCATGGGAGGTGGAAGATTTCA GAGTAAAAAAGCAATGGAAGAACGGAAGAAGAATGGAGAGGGTCTGCGTACTTT TGTGGGTGGCCCCGAACAAACAGAATTGGAGGTTAAAATCAGCGAAGCATTGACT AAAATTGCTGAGGAACATGGAACAGAGTCTGTTACTGCTATCGCTATTGCCTATGT |
| | TCGCTCTAAAGCGAAAAATGTTTTCCCATTGATTGGAGGAAGGAAAATTGAACATC TCAAGCAGAACATTGAGGCTTTGAGTATTAAATTAACACCGGAACAAATAGAATAC CTGGAAAGTATTGTTCCTTTTGATGTTGGCTTTCCCAAAAGTTTAATAGGAGATGA CCCAGCGGTAACCAAGAAGCTTTCACCCCTCACATCGATGTCTGCCAGGATAGCTT TTGACAATTAG (SEQ ID NO: 60) |

**[Table 1-31]**

| AAD15/NP_014477.1/Saccharomyces cerevisiae S288C | |
|---|---|
| Amino acid sequence | MARHFGMALAPWDVMGGGRFQSKKAMEERRKNGECIRSFVGASEQTDAEIKISEAL AKVAEEHGTESVTAIAIAYVRSKAKNVFPSVEGGKIEDLKENIKALSIDLTPDNIKYLE NVVPFDIGFPNTFIVLNSLTQKYGTNNV (SEQ ID NO: 61) |
| Base sequence | ATGGCTAGGCATTTCGGTATGGCCCTCGCCCCATGGGATGTCATGGGAGGTGGAAG ATTTCAGAGTAAAAAAGCAATGGAGGAACGGAGGAAGAATGGAGAGTGTATTCGT TCTTTCGTTGGCGCCTCCGAACAAACAGATGCAGAAATCAAGATTAGTGAAGCAT TAGCCAAGGTTGCTGAGGAACATGGCACTGAGTCTGTTACTGCTATTGCTATTGCC TATGTTCGCTCTAAGGCGAAAAATGTTTTTCCGTCGGTTGAAGGAGGAAAAATTGA GGATCTCAAAGAGAACATTAAGGCTCTCAGTATCGATCTAACGCCGGACAATATAA AATACTTGGAAAATGTAGTTCCTTTTGACATCGGATTTCCTAACACTTTTATCGTGT TAAATTCCTTGACTCAAAAATATGGTACGAATAATGTTTAG (SEQ ID NO: 62) |

**[Table 1-32]**

| YPR1/NP_010656.1/Saccharomyces cerevisiae S288C | |
|---|---|
| Amino acid sequence | MPATLKNSSATLKLNTGASIPVLGFGTWRSVDNNGYHSVIAALKAGYRHIDAAAIYL NEEEVGRAIKDSGVPREEIFITTKLWGTEQRDPEAALNKSLKRLGLDYVDLYLMHWP VPLKTDRVTDGNVLCIPTLEDGTVDIDTKEWNFIKTWELMQELPKTGKTKAVGVSNF SINNIKELLESPNNKVVPATNQIEIHPLLPQDELIAFCKEKGIVVEAYSPFGSANAPLLK EQAIIDMAKKHGVEPAQLIISWSIQRGYVVLAKSVNPERIVSNFKIFTLPEDDFKTISNL SKVHGTKRVVDMKWGSFPIFQ (SEQ ID NO: 63) |
| Base sequence | ATGCCTGCTACGTTAAAGAATTCTTCTGCTACATTAAAACTAAATACTGGTGCCTCC ATTCCAGTGTTGGGTTTCGGCACTTGGCGTTCCGTTGACAATAACGGTTACCATTC TGTAATTGCAGCTTTGAAAGCTGGATACAGACACATTGATGCTGCGGCTATCTATTT GAATGAAGAAGAAGTTGGCAGGGCTATTAAAGATTCCGGAGTCCCTCGTGAGGAA ATTTTTATTACTACTAAGCTTTGGGGTACGGAACAACGTGATCCGGAAGCTGCTCT AAACAAGTCTTTGAAAAGACTAGGCTTGGATTATGTTGACCTATATCTGATGCATTG GCCAGTGCCTTTGAAAACCGACAGAGTTACTGATGGTAACGTTCTGTGCATTCCAA CATTAGAAGATGGCACTGTTGACATCGATACTAAGGAATGGAATTTTATCAAGACG TGGGAGTTGATGCAAGAGTTGCCAAAGACGGGCAAAACTAAAGCCGTTGGTGTC TCTAATTTTTCTATTAACAACATTAAAGAATTATTAGAATCTCCAAATAACAAGGTG GTACCAGCTACTAATCAAATTGAAATTCATCCATTGCTACCACAAGACGAATTGATT GCCTTTTGTAAGGAAAAGGGTATTGTTGTTGAAGCCTACTCACCATTTGGGAGTGC TAATGCTCCTTTACTAAAAGAGCAAGCAATTATTGATATGGCTAAAAAGCACGGCG TTGAGCCAGCACAGCTTATTATCAGTTGGAGTATTCAAAGAGGCTACGTTGTTCTG GCCAAATCGGTTAATCCTGAAAGAATTGTATCCAATTTTAAGATTTTCACTCTGCCT GAGGATGATTTCAAGACTATTAGTAACCTATCCAAAGTGCATGGTACAAAGAGAGT CGTTGATATGAAGTGGGGATCCTTCCCAATTTTCCAATGA (SEQ ID NO: 64) |

**[Table 1-33]**

| NCg10324/NP_599582.1/Corynebacterium glutamicum ATCC 13032 | |
|---|---|
| Amino acid sequence | MSISVKALQKSGPEAPFEVKIIERRDPRADDVVIDIKAAGICHSDIHTIRNEWGEAHFP LTVGHEIAGVVSAVGSDVTKWKVGDRVGVGCLVNSCGECEQCVAGFENNCLRGNV GTYNSNDVDGTITQGGYAEKVVVNERFLCSIPEELNFDVAAPLLCAGITTYSPIARWN VKEGDKVAVMGLGGLGHMGVQIAAAKGAEVTVLSRSLRKAELAKELGAARTLATS DEDFFTEHAGEFDFILNTISASIPVDKYLSLLKPHGVMAVVGLPPEKQPLSFGALIGGG KVLTGSNIGGIPETQEMLDFCAKHGLGAMIETVGVNDVDAAYDRVVAGDVQFRVVID TASFAEVEAV (SEQ ID NO: 65) |
| Base sequence | GTGAGTATCTCAGTAAAAGCACTACAAAAGTCCGGCCCAGAAGCACCTTTCGAGG TCAAGATCATTGAACGCCGTGACCCACGCAGATGATGTGGTTATTGATATCAAA GCTGCGGGCATCTGCCACAGCGATATCCACACCATCCGCAACGAATGGGGCGAGG CGCACTTCCCGCTCACCGTCGGCCACGAAATCGCAGGCGTTGTCTCTGCGGTTGG ATCCGATGTAACCAAATGGAAAGTCGGCGACCGCGTGGGCGTCGGCTGCCTCGTT AACTCCTGCGGCGAATGCGAACAGTGCGTCGCAGGATTTGAAAACAACTGCCTTC GCGGAAACGTCGGAACCTACAACTCTAACGACGTCGACGGCACCATCACCCAAG GCGGCTACGCTGAAAAGGTAGTGGTCAACGAACGTTTCCTGTGCAGCATCCCAGA GGAACTTAACTTCGATGTCGCAGCACCACTGCTGTGCGCAGGCATCACCACCTACT CCCCAATCGCTCGCTGGAACGTTAAAGAAGGCGACAAAGTAGCAGTCATGGGCCT CGGCGGACTCGGACACATGGGTGTCCAGATCGCTGCAGCCAAGGGTGCTGAGGTT ACCGTTCTGTCCCGTTCCCTGCGCAAGGCAGAACTTGCCAAGGAACTCGGCGCAG CTCGCACGCTTGCGACTTCTGATGAGGATTTCTTCACCGAACACGCCGGTGAATTC GACTTCATCCTCAACACCATTAGCGCATCCATCCCAGTCGACAAGTACCTGAGCCT TCTCAAGCCACACGGTGTCATGGCTGTTGTCGGTCTGCCACCAGAGAAGCAGCCA CTGAGCTTCGGTGCGCTCATCGGCGGCGGAAAAGTCCTCACCGGATCCAACATTG |
| | GCGGCATCCCTGAAACCCAGGAAATGCTCGACTTCTGTGCAAAACACGGCCTCGG TGCGATGATCGAAACTGTCGGCGTCAACGATGTTGATGCAGCCTACGACCGTGTTG TTGCCGGCGACGTTCAGTTCCGCGTTGTCATTGATACTGCTTCGTTTGCTGAGGTT GAGGCGGTTTAG (SEQ ID NO: 66) |

**[Table 1-34]**

| NCg10313/NP_599571.1/Corynebacterium glutamicum ATCC 13032 | |
|---|---|
| Amino acid sequence | MSTVVPGIVALSKGAPVEKVNVVVPDPGANDVIVKIQACGVCHTDLAYRDGDISDEF PYLLGHEAAGIVEEVGESVTHVEVGDFVILNWRAVCGECRACKKGEPKYCFNTHNA SKKMTLEDGTELSPALGIGAFLEKTLVHEGQCTKVNPEEDPAAAGLLGCGIMAGLGA AVNTGDIKRGESVAVFGLGGVGMAAIAGAKIAGASKIIAVDIDEKKLEWAKEFGATHT INSSGLGGEGDASEVVAKVRELTDGFGTDVSIDAVGIMPTWQQAFYSRDHAGRMVM VGVPNLTSRVDVPAIDFYGRGGSVRPAWYGDCLPERDFPTYVDLHLQGRFPLDKFVS ERIGLDDVEEAFNTMKAGDVLRSVVEI (SEQ ID NO: 67) |
| Base sequence | ATGAGCACTGTAGTGCCTGGAATTGTCGCACTGTCCAAGGGTGCACCGGTAGAAA AAGTAAACGTTGTTGTCCCTGATCCAGGTGCTAACGATGTCATCGTCAAGATTCAG GCCTGCGGTGTGTGCCACACCGACTTGGCCTACCGCGATGGCGATATTTCAGATGA GTTCCCTTACCTCCTCGGCCACGAGGCAGCAGGCATTGTTGAGGAGGTAGGCGAG TCCGTCACCCACGTTGAGGTCGGCGATTTCGTCATCTTGAACTGGCGTGCAGTGTG CGGCGAGTGCCGTGCATGTAAGAAGGGCGAGCCAAAGTACTGCTTTAACACCCAC AACGCCTCTAAGAAGATGACCCTGGAAGACGGCACCGAGCTGTCCCCAGCACTG GGTATTGGCGCGTTCTTGGAAAAGACCCTGGTCCACGAAGGCCAGTGCACCAAGG TTAACCCTGAGGAAGATCCAGCAGCAGCTGGCCTTCTGGGTTGTGGCATCATGGC AGGCCTTGGCGCTGCGGTGAACACCGGTGATATTAAGCGTGGCGAGTCCGTAGCA |
| | GTCTTCGGCCTTGGTGGCGTGGGCATGGCAGCTATTGCTGGCGCCAAGATTGCTGG CGCTTCCAAGATCATTGCTGTTGATATCGATGAGAAGAAGCTGGAGTGGGCGAAG GAATTCGGCGCAACCCACACCATTAATTCCTCTGGTCTTGGTGGCGAAGGTGATGC CTCTGAGGTCGTGGCAAAGGTTCGTGAGCTCACCGATGGTTTCGGCACCGATGTC TCCATCGATGCGGTAGGCATCATGCCGACCTGGCAGCAGGCGTTTTACTCCCGTGA CCATGCAGGCCGCATGGTGATGGTGGGCGTTCCAAACCTGACGTCTCGCGTAGAT GTTCCTGCGATTGATTTTTACGGTCGCGGTGGATCCGTGCGCCCTGCATGGTACGG CGACTGCCTGCCTGAGCGTGATTTCCCAACTTATGTGGATCTGCACCTGCAGGGTC GTTTCCCACTGGATAAGTTTGTTTCTGAGCGTATTGGTCTTGATGATGTTGAAGAG GCTTTCAACACCATGAAGGCTGGCGACGTGCTGCGTTCTGTGGTGGAGATCTAA (SEQ ID NO: 68) |

**[Table 1-35]**

| NCg10219/NP_599475.1/Corynebacterium glutamicum ATCC 13032 | |
|---|---|
| Amino acid sequence | MPKYIAMQVSESGAPLAANLVQPAPLKSREVRVEIAASGVCHADIGTAAASGKHTVF PVTPGHEIAGTIAEIGENVSRWTVGDRVAIGWFGGNCGDCAFCRAGDPVHCRERKIP GVSYAGGWAQNIVVPAEALAAIPDGMDFYEAAPMGCAGVTTFNALRNLKLDPGAAV AVFGIGGLVRLAIQFAAKMGYRTITIARGLEREELARQLGANHYIDSNDLHPGQALFE LGGADLILSTASTTEPLSELSTGLSIGGQLTIIGVDGGDITVSAAQLMMNRQIITGHLTG SANDTEQTMKFAHLHGVKPLIERMPLDQANEAIARISAGKPRFRIVLEPNS (SEQ ID NO: 69) |
| Base sequence | ATGCCCAAATACATTGCCATGCAGGTATCCGAATCCGGTGCACCGTTAGCCGCGAA TCTCGTGCAACCTGCTCCGTTGAAATCGAGGGAAGTCCGCGTGGAAATCGCTGCT AGTGGTGTGTGCCATGCAGATATTGGCACGGCAGCAGCATCGGGGAAGCACACTG |
| | TTTTTCCTGTTACCCCTGGTCATGAGATTGCAGGAACCATCGCGGAAATTGGTGAA AACGTATCTCGGTGGACGGTTGGTGATCGCGTTGCAATCGGTTGGTTTGGTGGCAA TTGCGGTGACTGCGCTTTTTGTCGTGCAGGTGATCCTGTGCATTGCAGAGAGCGG AAGATTCCTGGCGTTTCTTATGCGGGTGGTTGGGCACAGAATATTGTTGTTCCAGC GGAGGCTCTTGCTGCGATTCCAGATGGCATGGACTTTTACGAGGCCGCCCCGATGG GCTGCGCAGGTGTGACAACATTCAATGCGTTGCGAAACCTGAAGCTGGATCCCGG TGCGGCTGTCGCGGTCTTTGGAATCGGCGGTTTAGTGCGCCTAGCTATTCAGTTTG CTGCGAAAATGGGTTATCGAACCATCACCATCGCCCGCGGTTTAGAGCGTGAGGA GCTAGCTAGGCAACTTGGCGCCAACCACTACATCGATAGCAATGATCTGCACCCTG GCCAGGCGTTATTTGAACTTGGCGGGGCTGACTTGATCTTGTCTACTGCGTCCACC ACGGAGCCTCTTTCGGAGTTGTCTACCGGTCTTTCTATTGGCGGGCAGCTAACCAT TATCGGAGTTGATGGGGGAGATATCACCGTTTCGGCAGCCCAATTGATGATGAACC GTCAGATCATCACAGGTCACCTCACTGGAAGTGCGAATGACACGGAACAGACTAT GAAATTTGCTCATCTCCATGGCGTGAAACCGCTTATTGAACGGATGCCTCTCGATC AAGCCAACGAGGCTATTGCACGTATTTCAGCTGGTAAACCACGTTTCCGTATTGTC TTGGAGCCGAATTCATAA (SEQ ID NO: 70) |

**[Table 1-36]**

| NCg12709/NP_601999.1/Corynebacterium glutamicum ATCC 13032 | |
|---|---|
| Amino acid sequence | MTTAAPQEFTAAVVEKFGHDVTVKDIDLPKPGPHQALVKVLTSGICHTDLHALEGDW PVKPEPPFVPGHEGVGEVVELGPGEHDVKVGDIVGNAWLWSACGTCEYCITGRETQ CNEAEYGGYTQNGSFGQYMLVDTRYAARIPDGVDYLEAAPILCAGVTVYKALKVSE TRPGQFMVISGVGGLGHIAVQYAAAMGMRVIAVDIADDKLELARKHGAEFTVNARN EDSGEAVQKYTNGGAHGVLVTAVHEAAFGQALDMARRAGTIVFNGLPPGEFPASVF |
| | NIVFKGLTIRGSLVGTRQDLAEALDFFARGLIKPTVSECSLDEVNGVLDRMRNGKIDG RVAIRF (SEQ ID NO: 71) |
| Base sequence | ATGACCACTGCTGCACCCCAAGAATTTACCGCTGCTGTTGTTGAAAAATTCGGTCA TGACGTGACCGTGAAGGATATTGACCTTCCAAAGCCAGGGCCACACCAGGCATTG GTGAAGGTACTCACCTCCGGCATCTGCCACACCGACCTCCACGCCTTGGAGGGCG ATTGGCCAGTAAAGCCGGAACCACCATTCGTACCAGGACACGAAGGTGTAGGTGA AGTTGTTGAGCTCGGACCAGGTGAACACGATGTGAAGGTCGGCGATATTGTCGGC AATGCGTGGCTCTGGTCAGCGTGTGGCACCTGCGAATACTGCATCACCGGCAGGG AAACTCAGTGCAACGAAGCTGAGTATGGTGGCTACACCCAAAATGGATCCTTCGG CCAGTACATGCTGGTGGATACCCGTTACGCCGCTCGCATCCCAGACGGCGTGGACT ACCTCGAAGCAGCACCAATTCTGTGTGCAGGCGTGACTGTCTACAAGGCACTCAA AGTCTCTGAAACCCGCCCGGGCCAATTCATGGTGATCTCCGGTGTCGGCGGACTT GGCCACATCGCAGTCCAATACGCAGCGGCGATGGGCATGCGTGTCATTGCGGTAG ATATTGCCGATGACAAGCTGGAACTTGCCCGTAAGCACGGTGCGGAATTTACCGTG AATGCGCGTAATGAAGATTCAGGCGAAGCTGTACAGAAGTACACCAACGGTGGCG CACACGGCGTGCTTGTGACTGCAGTTCACGAGGCAGCATTCGGCCAGGCACTGGA TATGGCTCGACGTGCAGGAACAATTGTGTTCAACGGTCTGCCACCGGGAGAGTTC CCAGCATCCGTGTTCAACATCGTATTCAAGGGCCTGACCATCCGTGGATCCCTCGT GGGAACCCGCCAAGACTTGGCCGAAGCGCTCGATTTCTTTGCACGCGGACTAATC AAGCCAACCGTGAGTGAGTGCTCCCTCGATGAGGTCAATGGTGTGCTTGACCGCA TGCGAAACGGCAAGATCGATGGTCGTGTGGCGATTCGTTTCTAA (SEQ ID NO: 72) |

**[Table 1-37]**

| NCgl1112/NP_600385.1/Corynebacterium glutamicum ATCC 13032 | |
|---|---|
| Amino acid sequence | MSLQFDHETLGQRVLFGSGEAAQNLAAEISRLDAKNVMVVAGDFELPMARQVAADI DVKVWHSNVVMHVPIETAEEARSVAKENDIDVVVCVGGGSTTGLAKAIAMTTALPII AVPTTYAGSEATNVWGLTEAARKTTGVDNKVLPVTVIYDSALTMSLPVEMSVASGLN GLAHCIDSLWGPKADPINAAMAAEGIRALSAGLPKIVADAQDVDGRDEALYGAYLA AVSFASAGSGLHHKICHVLGGTFNLPHAQTHATVLPYVLAFNAPYAPQAEQRAAAAF GSATALEGLQQLRAQVGAPQRLSDYGFTAAGIPEAVEIILEKVPANNPRTVTEENLTAL LTTALNGDDPATLN (SEQ ID NO: 73) |
| Base sequence | ATGTCTTTACAGTTCGATCATGAAACCCTCGGTCAACGAGTTCTGTTCGGTTCAGG TGAGGCGGCGCAAAATCTCGCCGCTGAAATTAGCCGACTCGATGCCAAAAACGTC ATGGTGGTTGCCGGTGATTTCGAGCTTCCCATGGCACGGCAAGTAGCAGCAGATAT TGATGTCAAGGTGTGGCATTCAAATGTCGTGATGCATGTGCCCATCGAAACAGCAG AAGAAGCACGCAGTGTTGCGAAAGAAAACGACATTGATGTTGTGGTGTGTGTGG GCGGTGGATCCACAACAGGTCTAGCTAAAGCGATTGCCATGACCACCGCATTGCC GATCATTGCGGTACCCACTACTTATGCAGGTTCTGAAGCAACAAATGTGTGGGGAT TGACCGAAGCCGCGCGCAAAACAACTGGTGTTGATAACAAAGTGCTGCCAGTGA CAGTTATCTACGATTCAGCGTTAACCATGTCTTTGCCGGTAGAAATGTCGGTTGCTT CTGGTCTCAATGGTTTGGCTCACTGCATTGATTCTTTGTGGGGACCGAAGGCGGAT CCCATCAATGCGGCTATGGCTGCTGAGGGAATTCGAGCACTTTCTGCTGGCCTTCC CAAGATTGTGGCAGATGCTCAGGACGTAGATGGTCGCGATGAAGCGCTCTACGGT GCCTACCTGGCTGCGGTGTCTTTTGCCTCTGCTGGCTCTGGTCTCCACCACAAGAT CTGCCACGTGTTGGGTGGAACTTTTAACCTTCCACACGCGCAAACCCATGCAACA GTACTGCCTTATGTTCTTGCCTTCAACGCGCCATATGCGCCACAGGCAGAACAACG |
| | CGCAGCGGCAGCTTTCGGTTCTGCGACAGCACTTGAAGGATTGCAACAGCTGCGT GCCCAAGTGGGAGCACCACAGCGACTATCCGATTACGGATTCACCGCAGCAGGAA TCCCAGAGGCAGTGGAAATCATCTTGGAGAAAGTACCGGCGAATAATCCACGGAC GGTCACAGAAGAAAACCTCACTGCGCTGCTTACCACAGCGCTCAACGGCGACGAT CCAGCAACTTTGAATTAA (SEQ ID NO: 74) |

**[Table 1-38]**

| NCgl2382/NP_601669.1/Corynebacterium glutamicum ATCC 13032 | |
|---|---|
| Amino acid sequence | MQTLAAIVRATKQPFEITTIDLDAPRPDEVQIRVIAAGVRHTDAIVRDQIYPTFLPAVFG HEGAGVVVAVGSAVTSVKPDDKVVLGFNSCGQCLKCLGGKPAYCEKFYDRNFACTR DAGHTTLFTRATKEQAEAIIDTLDDVFYDADAGFLAYPATPPEASGVSVLVVAAGTSD LPQAKEALHTASYLGRSTSLIVDFGVAGIHRLLSYEEELRAAGVLIVAAGMDGALPGV VAGLVSAPVVALPTSVGYGAGAGGIAPLLTMLNACAPGVGVVNIDNGYGAGHLAAQ IAAR (SEQ ID NO: 75) |
| Base sequence | ATGCAAACCCTTGCTGCTATTGTTCGTGCCACGAAGCAACCTTTTGAGATCACCAC CATTGATCTGGATGCACCACGACCAGATGAAGTTCAAATCCGTGTTATTGCTGCCG GAGTGCGCCACACTGACGCAATTGTTCGTGATCAGATTTACCCAACTTTTCTTCCC GCAGTTTTCGGCCACGAAGGCGCCGGAGTAGTTGTCGCCGTGGGTTCTGCAGTCA CCTCGGTGAAACCAGATGACAAGGTAGTGCTGGGATTCAACTCTTGTGGCCAGTG CTTGAAGTGTTTGGGCGGTAAGCCTGCGTACTGTGAGAAATTCTATGACCGCAACT TCGCATGCACCCGCGATGCCGGGCACACTACTTTGTTTACCCGTGCAACAAAAGA GCAGGCAGAGGCCATCATCGACACCCTTGATGATGTTTTCTACGATGCGGATGCGG GTTTCCTGGCATACCCAGCAACTCCCCCAGAGGCTTCGGGAGTAAGCGTGTTGGT TGTCGCGGCTGGTACCTCTGATCTCCCCCAAGCAAAGGAAGCACTACACACTGCC |
| | TCCTACTTGGGGCGCTCCACCTCACTGATTGTTGATTTTGGAGTGGCTGGCATCCA CCGCCTGCTTTCATACGAAGAAGAACTCCGCGCTGCGGGCGTGCTCATCGTTGCC GCTGGAATGGATGGTGCGCTACCCGGAGTTGTCGCAGGCTTAGTGTCCGCACCTG TCGTCGCACTGCCAACCTCCGTGGGATACGGCGCAGGTGCTGGAGGAATCGCACC ACTTCTGACCATGCTTAACGCCTGCGCGCCGGGAGTTGGAGTGGTCAACATTGATA ACGGCTATGGAGCAGGACACCTGGCTGCGCAGATTGCGGCGAGGTAA (SEQ ID NO: 76) |

**[Table 1-39]**

| NCgl0186/NP_599442.1/Corynebacterium glutamicum ATCC 13032 | |
|---|---|
| Amino acid sequence | MLNAVGKAQNILLLGGTSEIGISIVSRFLKQGPSHVTLAARKDSPRVDAAVAEIKAAG AASVAVVDFDALDTESHPAAIDAAFENGDVDVAIVAFGILGDNEAQWRDQALAVEAT TVNYTAGVSVGVLLGQKFEQQGHGTIVALSSVAGQRVRRSNFVYGSAKAGFDGFYT QLGEALRGSGANVLVVRPGQVRTKMSADGGEAPLTVNREDVADAVYDAVVNKKDII FVHPLFQYVSFAFQFIPRAIFRKLPF (SEQ ID NO: 77) |
| Base sequence | ATGCTTAACGCAGTGGGCAAAGCCCAAAACATTCTCCTTCTTGGTGGAACCTCTG AGATCGGTATTTCCATTGTCTCCCGCTTCCTCAAGCAGGGTCCATCCCATGTGACCT TGGCAGCGCGTAAAGATTCCCCACGCGTGGACGCAGCAGTCGCAGAGATCAAAG CAGCTGGCGCTGCTTCCGTTGCTGTTGTTGATTTCGATGCGCTCGACACCGAATCC CACCCTGCAGCCATCGACGCAGCCTTTGAAAACGGCGACGTTGACGTAGCAATCG TGGCTTTCGGCATCCTCGGCGACAACGAAGCACAGTGGCGCGACCAAGCACTAGC AGTGGAAGCAACCACCGTGAACTACACCGCCGGCGTTTCCGTAGGTGTACTGCTG GGCCAGAAATTTGAGCAGCAGGGCCACGGCACCATCGTGGCATTGTCCTCTGTGG CAGGCCAGCGAGTCCGCCGCTCCAACTTTGTCTACGGCTCCGCCAAGGCAGGTTT |
| | CGACGGTTTCTACACCCAGCTCGGCGAAGCCCTGCGTGGATCCGGTGCCAACGTA TTGGTGGTTCGCCCAGGCCAGGTACGCACCAAGATGTCCGCAGATGGTGGCGAAG CCCCACTGACCGTCAACCGCGAAGACGTGGCAGATGCTGTTTATGATGCAGTGGT GAACAAGAAGGACATCATCTTTGTCCACCCACTGTTCCAGTACGTCTCTTTTGCGT TCCAATTCATTCCGCGAGCAATCTTCCGCAAGCTGCCGTTCTAA (SEQ ID NO: 78) |

**[Table 1-40]**

| NCgl0099/NP_599352.1/Corynebacterium glutamicum ATCC 13032 | |
|---|---|
| Amino acid sequence | MEHGVTVIKGTEFDVFPLNLGGNTFGWTSNREQTFAVLDAFVAAGGNFVDTADSYS AWVEGNEGGESERELGAWIKERGADKLIIATKSGALEPVAGRSREATFKAVEGSLERL GVESIDIFYYHYDDEAVSIDEQVAIANDLIAQGKIKHLALSNYSAERLAEFFEKSVGTP AQPVALQPHYNLVSRKDYEENVQPLAEKHGVAVFPYFALAAGLLTGKYTSKEDISGK ARAGQLDRYASDEAFAVVTELRAVADELGVAPTTVALAWLVAHGVTAPIASVSKVEQ LKDLMAVKDVELSAEQLARLDKVSEPFA (SEQ ID NO: 79) |
| Base sequence | ATGGAGCACGGCGTGACCGTTATTAAAGGCACTGAATTTGATGTTTTCCCACTAAA CCTCGGTGGAAATACCTTTGGCTGGACCTCGAATAGGGAACAGACCTTCGCGGTT TTGGATGCATTCGTGGCAGCGGGAGGAAACTTTGTTGACACCGCCGATTCTTATTC TGCATGGGTTGAAGGCAATGAGGGTGGCGAGTCGGAGCGGGAGCTCGGCGCGTG GATTAAGGAACGTGGCGCAGACAAGCTGATCATTGCTACCAAGTCTGGTGCGTTG GAGCCTGTTGCTGGTCGTTCCCGTGAGGCAACTTTCAAGGCTGTCGAGGGTTCCC TGGAGCGTTTGGGCGTGGAATCGATCGATATTTTTTACTACCACTACGACGATGAG GCAGTCAGCATTGATGAGCAGGTTGCTATCGCTAATGATCTGATTGCACAGGGCAA GATTAAGCACCTCGCATTGTCTAACTACAGCGCGGAGCGTTTAGCTGAGTTCTTTG |
| | AGAAGTCTGTAGGCACTCCAGCGCAGCCGGTTGCTCTGCAACCGCACTACAACCT GGTGTCGAGGAAGGATTATGAGGAGAACGTGCAGCCACTCGCCGAGAAGCATGG CGTTGCAGTCTTCCCTTATTTCGCGCTTGCCGCGGGTCTTTTGACCGGAAAGTACA CCTCCAAGGAGGATATTTCGGGTAAAGCGCGTGCGGGGCAGTTGGATCGTTACGC CAGCGATGAGGCGTTTGCCGTGGTGACAGAGTTGCGTGCTGTTGCCGATGAGTTG GGTGTTGCGCCAACGACTGTGGCGCTTGCGTGGTTGGTTGCGCATGGTGTGACCG CACCGATTGCGTCCGTGTCCAAGGTAGAGCAGTTGAAGGATTTGATGGCTGTGAA GGATGTGGAGCTGAGCGCTGAGCAGCTTGCACGTTTGGATAAGGTTTCGGAGCCT TTCGCTTAA (SEQ ID NO: 80) |

**[Table 1-41]**

| NCgl2952/NP_602249.1/Corynebacterium glutamicum ATCC 13032 | |
|---|---|
| Amino acid sequence | MNNSLAFNHDTLPQKVMFGYGKSSAFLKQEVERRGSAKVMVIAGEREMSIAHKVAS EIEVAIWHDEVVMHVPIEVAERARAVATDNEIDLLVCVGGGSTIGLAKAIAMTTALPIV AIPTTYAGSEATNVWGLTEAARKTTGVDLKVLPETVIYDSELTMSLPVEMSVASGLNG LAHCIDSLWGPNADPINAVLAAEGIRALNQGLPKIVANPHSIEGRDEALYGAYLAAVSF ASAGSGLHHKICHTLGGTFNLPHAQTHATVLPYVLAFNAGDAPEAERRAAAAFGTD TALEGLQRLRLSVNAPKRLSDYGFEASGIAEAVDVTLEKVPANNPRPVTRENLSRLLE AALNGEDPAVLSAVLSN (SEQ ID NO: 81) |
| Base sequence | GTGAACAACTCACTCGCATTCAACCACGACACCCTCCCACAGAAAGTCATGTTTG GATATGGCAAGTCCAGTGCATTCTTAAAGCAGGAAGTTGAACGCCGCGGCTCAGC CAAGGTCATGGTCATTGCGGGTGAACGAGAAATGTCGATCGCCCATAAGGTGGCC TCAGAAATTGAGGTGGCGATCTGGCACGACGAAGTTGTCATGCACGTGCCCATCG |
| | AAGTAGCCGAACGTGCGCGTGCAGTGGCAACCGACAATGAGATTGATCTGCTGGT GTGTGTTGGCGGCGGATCCACCATAGGTTTGGCCAAAGCAATTGCCATGACCACT GCCCTGCCCATCGTCGCGATCCCCACCACCTACGCAGGATCGGAAGCAACCAACG TGTGGGGTCTGACGGAAGCAGCGCAAAACAACCGGTGTTGATCTGAAGGTGC TCCCCGAAACAGTCATTTACGATTCCGAACTCACCATGTCGCTTCCAGTGGAGATG TCCGTGGCATCCGGACTCAACGGCCTGGCGCACTGCATTGATTCTTTGTGGGACC CAACGCCGATCCCATCAACGCAGTGCTTGCAGCCGAAGGAATCCGCGCACTCAAC CAGGGACTGCCGAAAATTGTTGCGAACCCGCACAGCATCGAAGGACGCGACGAA GCCCTCTACGGCGCCTACCTCGCAGCAGTATCCTTCGCCTCCGCAGGCTCCGGACT ACACCACAAAATCTGCCACACCTTGGGAGGCACCTTCAACCTCCCCCACGCCCAA ACCCACGCAACCGTGCTGCCGTATGTTTTGGCATTCAACGCAGGCGACGCACCAG AAGCTGAACGCCGCGCAGCCGCAGCCTTTGGAACTGACACCGCACTAGAAGGCC TGCAACGCCTCCGCTTGTCAGTCAACGCACCGAAACGACTTTCCGACTACGGCTT CGAGGCTTCAGGAATTGCTGAGGCAGTGGACGTCACGTTGGAGAAAGTTCCCGCC AACAATCCTCGCCCAGTGACCCGGGAAAACCTCAGCAGATTGCTCGAAGCAGCAC TCAACGGTGAGGATCCGGCAGTTCTTAGCGCAGTACTCAGTAACTAA (SEQ ID NO: 82) |

**[Table 1-42]**

| NCgl1459/NP_600732.2/Corynebacterium glutamicum ATCC 13032 | |
|---|---|
| Amino acid sequence | MKQRMVGSSGLRVSRLGLGTSTWGSGTELAEAGDIFKAFINSGGTLIDVSPNYTTGV AEEMLGTMLDAEVSRSAVVISSSAGVNPALPLGRRVDCSRRNLIAQLDVTLRALNTD YLDLWSVGYWDEGTPPHEVADTLDYAVRTGRVRYAGVRGYSGWQLAVTHAASNHA AASARPVVVAQNEYSLLERRAEQELLPATQHLGVGFFAGAPLGQGVLTAKYRSEIPHD SRAASTGRDAEVQSYLDNRGRIIVDALDTAAKGLGISPAVTATTWVRDRPGVTAVIVG ARTHEQLSHLLKAESVTLPTPITQALDDVSL (SEQ ID NO: 83) |
| Base sequence | GTGAAACAGCGAATGGTCGGTTCAAGTGGTTTGCGGGTATCCAGGCTCGGTTTGG GCACCTCAACATGGGGCTCGGGCACCGAGCTGGCTGAGGCAGGCGATATCTTTAA GGCGTTCATCAATTCTGGTGGCACGCTTATCGACGTCTCCCCCAACTACACCACCG GCGTCGCGGAAGAAATGCTCGGCACGATGTTGGATGCGGAAGTCTCTCGTTCGGC TGTCGTCATTTCCTCCAGCGCAGGTGTCAACCCCGCTCTGCCGCTCGGCCGACGTG TGGATTGCTCCCGCCGCAATTTGATTGCCCAATTAGATGTCACCCTGCGGGCATTAA ACACTGACTATTTGGATTTGTGGTCTGTGGGCTATTGGGATGAGGGCACCCCACCG CATGAGGTGGCCGATACTTTGGATTACGCCGTGCGCACCGGCCGAGTCCGATATGC CGGTGTCCGAGGATATTCCGGTTGGCAGTTAGCGGTCACCCACGCTGCATCCAATC ATGCAGCGGCCTCCGCCCGCCCCGTGGTCGTTGCACAAAATGAATACAGCCTGCT GGAACGCCGCGCAGAACAAGAACTCCTCCCTGCCACCCAACACCTAGGTGTCGG ATTCTTTGCTGGCGCTCCGCTGGGGCAAGGCGTGCTGACTGCTAAATACCGCTCCG AAATTCCCCATGATTCCAGAGCTGCATCCACAGGACGCGACGCAGAAGTCCAAAG CTACCTAGATAATCGAGGCCGCATCATTGTCGATGCTCTTGATACTGCAGCCAAAG GATTAGGCATTAGCCCCGCTGTCACAGCCACCACCTGGGTGCGTGATCGTCCCGGA GTGACAGCTGTCATCGTGGGCGCTCGCACACATGAACAGCTGTCACATCTTCTCA AGGCGGAATCGGTGACTTTGCCAACACCAATCACACAAGCCCTTGATGATGTCTC CCTGTGA (SEQ ID NO: 84) |

**[Table 1-43]**

| yogA/NP_389725.1/Bacillus subtilis subsp. subtilis str. 168 | |
|---|---|
| Amino acid sequence | MKAVIHNGKAGLLGLSVQDVPSTKPGYGEVKVKLKSAGLNHRDLFLMKNKSEQDP HMILGSDGAGIIEEIGEGVKNVTVQTEVVIFPTLNWDLTENVPPVPEILGGPSDGTLAE YVIIPSQNAIKKPAYLSWEEAGVLPLSALTAYRALFTKGQLKKGEHLLIPGIGSGVATY ALFMAKAIGATVSVTSRSEEKRKKALKLGADYAFDSYSNWDEQLQGKKIDVVLDSIG PALFSEYFRHVKPNGRIVSFGASSGDNLSFPVRSLFFPQVNVLGTSMGSGEEFQAMLA FIDKHKLRPVIDRIYPLEKACEAYKRMQEGRQFGNIGIVME (SEQ ID NO: 85) |
| Base sequence | ATGAAAGCTGTAATTCACAACGGAAAAGCCGGTCTTCTGGGGTTATCAGTTCAGG ACGTTCCATCAACAAAGCCTGGATACGGAGAGGTAAAGGTTAAATTAAAATCTGC AGGCCTGAATCATCGTGACTTGTTTCTTATGAAAAACAAATCTGAACAAGATCCTC ACATGATACTGGGTTCTGACGGCGCGGGTATCATCGAAGAGATTGGTGAAGGCGT GAAAAATGTTACTGTTCAGACAGAAGTAGTCATTTTCCCGACATTGAACTGGGATT TGACAGAAAATGTTCCACCTGTACCTGAGATTCTGGGAGGTCCTTCGGACGGAAC ACTTGCTGAATATGTGATCATTCCTTCACAAAATGCAATCAAAAAACCTGCTTATTT ATCTTGGGAAGAAGCGGGCGTTTTACCTTTATCCGCTTTAACTGCATATCGGGCTCT GTTTACAAAGGGGCAATTAAAAAAAGGCGAGCATCTATTGATACCCGGCATCGGC AGCGGTGTAGCAACCTACGCTTTATTTATGGCTAAGGCGATTGGGGCAACAGTAAG CGTGACCTCCCGCAGTGAGGAGAAAAGAAAAAAGGCGCTGAAATTAGGTGCTGA TTACGCATTTGACAGCTACAGCAATTGGGATGAGCAGTTGCAGGGAAAAAAGATA GATGTTGTTCTTGACAGCATAGGACCTGCCCTCTTTTCGGAATACTTCCGCCATGTA AAACCAAATGGCCGTATTGTCAGCTTTGGGGCAAGCTCAGGGGATAATCTCAGTTT TCCGGTGCGTTCTTTATTCTTTCCTCAGGTCAATGTTTTGGGAACCTCGATGGGAA |
| | GCGGTGAGGAATTTCAAGCTATGCTCGCTTTCATTGACAAACATAAGCTGCGGCCT GTAATTGACCGGATATATCCTTTAGAAAAAGCATGTGAAGCATATAAAAGAATGCA GGAAGGCAGACAGTTTGGAAACATCGGGATCGTAATGGAATAA (SEQ ID NO: 86) |

**[Table 1-44]**

| bdhK/NP_391014.1/Bacillus subtilis subsp. subtilis str. 168 | |
|---|---|
| Amino acid sequence | MENFTYYNPTKLIFGKGQLEQLRKEFKRYGKNVLLVYGGGSIKRNGLYDQVTGILKE EGAVVHELSGVEPNPRLATVEKGIGLCREHDIDFLLAVGGGSVIDCTKAIAAGVKYDG DAWDIFSKKVTAEDALPFGTVLTLAATGSEMNPDSVITNWETNEKFVWGSNVTHPRF SILDPENTFTVPENQTVYGMVDMMSHVFEQYFHNVENTPLQDRMCFAVLQTVIETAP KLLEDLENYELRETILYAGTIALNGTLQMGYFGDWASHTMEHAVSAVYDIPHAGGLA ILFPNWMRYTLDTNVGRFKNLMLNMFDIDTEGKTDKEIALEGIDKLSAFWTSLGAPS RLADYNIGEEKLELIADIAAKEMEHGGFGNFQKLNKDDVLAILRASL (SEQ ID NO: 87) |
| Base sequence | ATGGAAAATTTCACTTATTATAATCCGACAAAGCTGATTTTTGGAAAAGGTCAGCT TGAACAATTAAGAAAAGAATTCAAACGATACGGCAAGAATGTACTGCTTGTTTAC GGGGGCGGCAGCATTAAACGCAACGGCCTTTATGATCAAGTCACAGGCATTTTAA AAGAAGAGGGCGCTGTTGTTCATGAGCTGTCAGGTGTAGAGCCAAACCCGCGTCT TGCGACAGTGGAAAAAGGCATAGGACTTTGCAGAGAGCATGACATTGATTTTCTG CTTGCTGTCGGCGGAGGCAGTGTGATTGACTGTACAAAGGCAATCGCAGCTGGCG TCAAATATGACGGTGACGCTTGGGATATTTTCAGCAAAAAAGTAACAGCGGAGGA TGCGCTGCCGTTTGGCACTGTTTTAACTCTTGCTGCAACAGGGTCTGAAATGAACC CTGATTCCGTGATTACAAACTGGGAAACAAACGAGAAATTTGTATGGGGCAGCAA |
| | TGTCACTCATCCGCGTTTCTCTATTTTAGACCCTGAAAATACGTTCACCGTTCCAGA AAATCAAACAGTATACGGCATGGTTGACATGATGAGCCACGTATTCGAACAATACT TCCATAATGTTGAAAACACGCCGCTTCAGGATAGAATGTGCTTTGCTGTTTTGCAG ACGGTCATCGAAACAGCTCCTAAGCTTCTTGAAGATCTGGAAAACTACGAACTTC GTGAAACGATTTTGTATGCTGGTACTATTGCTTTAAACGGCACGCTCCAAATGGGA TACTTCGGTGACTGGGCTTCTCATACAATGGAACACGCTGTTTCAGCTGTATATGAT ATTCCGCACGCGGGCGGTTTGGCAATACTGTTCCCAAATTGGATGAGATACACGCT TGATACAAATGTAGGCCGTTTTAAAAACCTTATGCTCAACATGTTTGACATTGATAC TGAAGGCAAAACAGATAAAGAAATTGCGCTTGAAGGAATCGATAAACTGTCTGCG TTCTGGACAAGCCTCGGTGCACCTTCTCGTCTTGCTGATTACAATATTGGAGAAGA AAAGCTTGAGCTGATTGCTGATATCGCAGCCAAGGAAATGGAACACGGCGGCTTC GGCAACTTCCAAAAACTGAACAAAGATGACGTGCTTGCCATCCTTCGCGCGTCTC TATAA (SEQ ID NO: 88) |

**[Table 1-45]**

| bdhJ/NP_391015.1/Bacillus subtilis subsp. subtilis str. 168 | |
|---|---|
| Amino acid sequence | MQNFTYWNPTKLIFGRGEVERLPEELKPYGKNVLLVYGGGSIKRSGLYDQVIEQLNK AGATVHELAGVEPNPRVSTVNKGVAICKEQNIDFLLAVGGGSVIDCTKAIAAGAKYD GDAWDIVTKKHQPKDALPFGTVLTLAATGSEMNSGSVITNWETKEKYGWGSPLVFP KFSILDPVNTFTVPKNHTIYGMVDMMSHVFEQYFHHVSNTPYQDRMCESLLRTVIET APKLINDLENYELRETILYTGTIALNGMLSMGARGDWATHNIEHAVSAVYDIPHAGGL AILFPNWMRHTLSENPARMKQLAVRVFDVEEAGKTDEEIALEGIDKLSAFWTSLGAP NRLADYDINDEQLDTIADKAMANGTFGQFKSLNKEDVLSILKASL (SEQ ID NO: 89) |
| Base sequence | ATGCAAAACTTTACATACTGGAATCCGACCAAATTAATTTTCGGGCGCGGCGAAGT GGAAAGACTTCCGGAGGAACTCAAACCTTACGGAAAAAACGTATTGCTTGTGTAC GGAGGCGGCAGCATTAAACGCAGCGGCCTGTATGATCAAGTGATTGAACAGCTGA ATAAAGCCGGAGCGACCGTGCATGAATTAGCAGGTGTGGAACCGAATCCTCGTGT GTCGACTGTTAATAAAGGTGTTGCCATCTGTAAAGAACAAAACATTGATTTCTTGC TGGCTGTCGGAGGCGGAAGCGTAATCGACTGTACAAAAGCGATTGCCGCAGGAGC GAAGTATGATGGTGATGCGTGGGATATCGTTACGAAAAAGCATCAGCCAAAAGAT GCTTTGCCATTCGGAACGGTATTGACTCTCGCTGCAACTGGTTCAGAAATGAACTC AGGATCTGTTATTACAAACTGGGAAACAAAAGAAAAATACGGCTGGGGCAGCCCG CTCGTATTCCCTAAATTCTCGATTCTTGATCCGGTGAATACATTCACCGTACCTAAA AACCACACGATCTACGGGATGGTTGACATGATGAGCCACGTATTCGAACAATACTT CCATCATGTATCAAACACGCCGTATCAGGACCGCATGTGTGAATCACTTTTGCGTA CAGTCATTGAAACAGCGCCTAAGCTGATCAATGATCTCGAAAATTACGAATTGCGT GAGACGATCCTGTACACAGGAACAATCGCATTAAACGGCATGCTTTCTATGGGGGC AAGAGGGGATTGGGCTACTCATAATATTGAACATGCAGTATCAGCCGTTTATGATAT TCCGCATGCCGGCGGACTGGCGATTCTGTTCCCGAATTGGATGAGACACACATTGT CTGAAAACCCTGCCCGCATGAAACAGCTTGCAGTTCGCGTGTTTGATGTTGAAGA AGCAGGTAAAACGGATGAAGAAATTGCCCTTGAAGGTATCGATAAGCTGTCCGCA TTCTGGACAAGTCTTGGCGCTCCGAACCGTCTTGCTGATTATGATATTAATGATGAG CAGCTTGACACAATCGCTGACAAGGCAATGGCTAACGGTACATTCGGCCAATTTAA ATCACTCAACAAAGAAGATGTGCTGTCAATTTTGAAAGCATCACTATAA (SEQ ID NO: 90) |

**[Table 1-46]**

| akrN/NP_388834.1/Bacillus subtilis subsp. subtilis str. 168 | |
|---|---|
| Amino acid sequence | MEYTSIADTGIEASRIGLGTWAIGGTMWGGTDEKTSIETIRAALDQGITLIDTAPAYGF GQSEEIVGKAIKEYGKRDQVILATKTALDWKNNQLFRHANRARIVEEVENSLKRLQT DYIDLYQVHWPDPLVPIEETAEVMKELYDAGKIRAIGVSNFSIEQMDTFRAVAPLHTIQ PPYNLFEREMEESVLPYAKDNKITTLLYGSLCRGLLTGKMTEEYTFEGDDLRNHDPKF QKPRFKEYLSAVNQLDKLAKTRYGKSVIHLAVRWILDQPGADIALWGARKPGQLEAL SEITGWTLNSEDQKDINTILENTISDPVGPEFMAPPTREEI (SEQ ID NO: 91) |
| Base sequence | ATGGAATATACCAGTATAGCAGATACAGGAATAGAAGCCTCCAGAATCGGCCTCGG CACATGGGCCATTGGCGGAACGATGTGGGGAGGCACTGACGAAAAACATCGATT GAAACAATCCGCGCCGCTCTTGATCAGGGGATTACACTGATTGACACCGCACCGG CTTACGGCTTCGGGCAGTCCGAGGAAATTGTCGGAAAGGCAATCAAAGAGTACGG CAAAAGAGACCAGGTGATTCTCGCAACGAAAACGGCTCTGGACTGGAAGAACAA CCAGCTGTTCCGCCATGCGAACAGAGCGAGAATTGTAGAGGAAGTTGAGAATTCT TTGAAGCGGCTTCAAACAGACTATATTGATCTTTATCAGGTGCATTGGCCCGATCCG CTTGTGCCAATTGAAGAAACGGCTGAAGTCATGAAGGAATTATATGATGCGGGAA AAATCCGGGCGATTGGCGTCAGCAATTTTTCAATTGAGCAAATGGATACATTTCGC GCCGTCGCACCTCTCCATACGATTCAGCCTCCATATAATCTGTTTGAAAGAGAGAT GGAAGAGAGTGTCCTTCCTTATGCGAAAGATAACAAGATAACAACATTATTATACG GCAGTTTATGCAGAGGGCTGTTAACAGGCAAAATGACTGAAGAATATACATTTGAG GGCGATGATCTGCGTAATCACGATCCAAAATTCCAGAAGCCCCGCTTTAAAGAGTA TCTTTCTGCTGTGAATCAATTGGATAAGCTGGCGAAGACACGTTATGGAAAATCAG TGATTCACTTGGCTGTCAGATGGATCTTAGATCAGCCGGGAGCGGATATCGCTCTTT GGGGAGCAAGAAAGCCTGGGCAGCTTGAGGCCCTATCTGAGATTACAGGCTGGAC GCTGAACAGTGAAGATCAGAAAGATATCAATACTATATTGGAAAATACGATATCAG |
| | ACCCTGTCGGACCGGAGTTTATGGCCCCGCCGACCAGAGAGGAAATATAA (SEQ ID NO: 92) |

**[Table 1-47]**

| yqkF/NP_390243.1/Bacillus subtilis subsp. subtilis str. 168 | |
|---|---|
| Amino acid sequence | MRKRKLGTSDLDISEVGLGCMSLGTEKNKALSILDEAIELGINYLDTADLYDRGRNEE IVGDAIQNRRHDIILATKAGNRWDDGSEGWYWDPSKAYIKEAVKKSLTRLKTDYIDL YQLHGGTIEDNIDETIEAFEELKQEGVIRYYGISSIRPNVIKEYVKKSNIVSIMMQFSLF DRRPEEWLPLLEEHQISVVARGPVAKGLLTEKPLDQASESMKQNGYLSYSFEELTNAR KAMEEVAPDLSMTEKSLQYLLAQPAVASVITGASKIEQLRENIQAANARRLTEEEIKAL QSHTKQDIYKAHRS (SEQ ID NO: 93) |
| Base sequence | ATGAGAAAGCGCAAATTGGGTACATCTGATTTAGACATTAGCGAAGTCGGACTCG GCTGTATGTCTCTTGGAACTGAAAAAACAAAGCATTGTCCATTCTGGATGAAGC GATCGAGCTTGGCATCAACTATTTGGACACAGCGGATTTGTATGACCGGGACGCA ATGAAGAAATTGTCGGTGATGCGATCCAAAACAGACGCCATGATATTATTCTGGCA ACAAAAGCGGGAAACCGTTGGGATGACGGAAGCGAGGGCTGGTATTGGACCCT TCAAAAGCTTACATAAAAGAGGCGGTAAAAAAGAGCCTTACACGTCTGAAAACA GATTATATCGACCTTTATCAGCTCCACGGCGGCACGATAGAGGACAACATTGATGA AACGATTGAAGCGTTTGAGGAATTAAAACAAGAAGGTGTCATCCGCTACTACGGC ATTTCTTCCATCCGCCCGAATGTGATTAAAGAATATGTAAAAAAATCAAACATCGTC AGCATTATGATGCAATTCAGCCTGTTTGACAGACGCCCTGAGGAATGGCTCCCGCT TTTAGAGGAACATCAAATCAGCGTAGTCGCCAGAGGTCCTGTTGCCAAAGGGCTC TTAACTGAAAAACCGCTTGATCAAGCTTCAGAAAGTATGAAACAAAACGGGTACT |
| | TGTCCTATTCATTCGAGGAACTGACAAATGCCCGTAAGGCAATGGAGGAAGTGGC TCCCGATCTTTCCATGACAGAAAAGTCGCTGCAGTATCTGCTAGCACAGCCGGCTG TCGCTTCAGTGATTACAGGCGCCAGTAAGATTGAGCAGTTACGGAAAATATTCAG GCAGCAAATGCACGGCGTTTAACCGAAGAGGAAATCAAAGCGCTCCAATCTCATA CGAAACAAGACATTTACAAAGCTCATCGCTCATAG (SEQ ID NO: 94) |

**[Table 1-48]**

| yccK/NP_388159.1/Bacillus subtilis subsp. subtilis str. 168 | |
|---|---|
| Amino acid sequence | MDQTRTLGKTKLKVKRIGFGANAVGGHNLFPNLNDETGKDLVRTALDGGVNFIDTAF IYGLGRSEELIGEVVQERGVRNELIIATKGAHKEVDGSIELDNSREFLRSEVEKSLKRL KTDYIDLYYVHFPDGKTPLAEVAGTLKELKDEGKIKAIGASNLDYQQLQDFNADGYL EVFQAEYSLIQRDAEKELLPYCEKQGISFIPYFPLASGLLTGKFTQDTVFDDFRKDKPQ FQGETFIHNLKKVDKLKAVAEEKQADTAHVALAWLLTRPAIDAIIPGAKRPEQLQDNL KTLNIELTEDEVNFISDIFK (SEQ ID NO: 95) |
| Base sequence | ATGGATCAAACACGTACACTCGGCAAAACGAAGCTGAAGGTGAAGCGGATCGGA TTCGGCGCGAATGCGGTCGGCGGGCATAATCTATTTCCAAATCTAAATGATGAAAC AGGGAAGGATTTAGTGCGCACGGCATTGGATGGCGGCGTCAATTTTATCGATACCG CCTTTATATATGGATTGGGGCGATCTGAAGAATTAATCGGCGAAGTCGTACAGGAA CGCGGCGTGCGGAATGAGCTCATCATCGCCACCAAAGGAGCTCATAAAGAAGTGG ACGGCAGCATTGAATTAGACAATAGCCGGGAGTTTCTTCGCAGCGAGGTGGAGAA GAGCCTGAAGCGGCTGAAAACAGATTACATTGATTTGTATTATGTTCACTTTCCGG ATGGAAAAACACCTCTCGCTGAAGTGGCGGGCACTTTGAAAGAGCTGAAGGATG AGGGGAAAATCAAAGCGATCGGCGCTTCGAACCTCGATTATCAGCAATTGCAGGA |
| | TTTTAATGCTGACGGCTATTTGGAGGTCTTCCAGGCCGAATATTCTCTCATACAGCG TGATGCCGAGAAAGAGCTTCTTCCATACTGTGAAAAACAAGGCATCTCCTTTATTC CTTACTTTCCGCTTGCGTCCGGACTGCTGACAGGAAAATTCACGCAAGACACAGT CTTTGATGATTTCAGAAAGGATAAACCTCAATTTCAGGGTGAAACGTTTATCCACA ATCTCAAAAAAGTAGATAAGCTGAAAGCAGTAGCGGAGGAAAAACAAGCGGATA CGGCACATGTCGCCTTGGCGTGGCTGTTAACGAGACCGGCGATTGATGCCATTATT CCAGGAGCTAAACGACCGGAGCAATTACAGGATAACCTGAAAACCTTGAACATTG AACTGACCGAAGATGAAGTGAATTTCATCAGCGACATTTTCAAATAA (SEQ ID NO: 96) |

**[Table 1-49]**

| iolS/NP_391857.1/Bacillus subtilis subsp. subtilis str. 168 | |
|---|---|
| Amino acid sequence | MKKAKLGKSDLQVFPIGLGTNAVGGHNLYPNLNEETGKELVREAIRNGVTMLDTAYI YGIGRSEELIGEVLREFNREDVVIATKAAHRKQGNDFVFDNSPDFLKKSVDESLKRLN TDYIDLFYIHFPDEHTPKDEAVNALNEMKKAGKIRSIGVSNFSLEQLKEANKDGLVDV LQGEYNLLNREAEKTFFPYTKEHNISFIPYFPLVSGLLAGKYTEDTTFPEGDLRNEQEH FKGERFKENIRKVNKLAPIAEKHNVDIPHIVLAWYLARPEIDILIPGAKRADQLIDNIKT ADVTLSQEDISFIDKLFA (SEQ ID NO: 97) |
| Base sequence | ATGAAAAAAGCGAAGCTCGGAAAATCAGACTTGCAGGTATTCCCTATCGGATTAG GAACAAATGCTGTCGGAGGACATAACCTCTACCCGAACCTAAATGAAGAAACCGG AAAAGAATTGGTGCGCGAGGCGATCCGTAATGGCGTGACAATGTTAGACACCGCT TACATTTACGGGATCGGCCGTTCCGAAGAATTAATTGGTGAAGTGCTGCGTGAATT CAACCGTGAAGATGTTGTCATCGCGACAAAAGCCGCTCACAGAAAACAAGGCAA |
| | TGACTTTGTCTTTGATAATTCACCAGATTTTCTTAAAAAATCAGTTGATGAAAGCCT GAAGCGCTTGAATACCGATTATATTGATTTGTTCTACATTCACTTCCCTGACGAACA TACGCCTAAGGATGAAGCCGTTAACGCGCTGAATGAGATGAAGAAAGCCGGAAA AATCCGTTCCATCGGTGTATCCAACTTCTCTTTAGAGCAATTGAAAGAAGCAAACA AAGACGGTTTGGTAGATGTATTGCAAGGCGAATACAACCTGTTAAACCGTGAAGC GGAAAAAACATTCTTCCCGTATACGAAGGAGCATAATATTTCATTTATCCCTTACTT CCCTCTCGTATCAGGTTTATTGGCAGGAAAGTATACAGAAGATACAACGTTCCCAG AAGGCGACCTGCGAAACGAACAGGAACACTTCAAGGGTGAGCGTTTCAAAGAAA ATATCAGAAAGGTCAACAAGCTTGCGCCGATTGCCGAAAAACACAACGTGGATAT CCCTCACATCGTATTGGCCTGGTATTTAGCAAGACCGGAAATTGATATTTTAATCCC AGGAGCAAAACGTGCCGATCAGCTGATTGATAACATCAAAACAGCTGACGTGACG CTTTCTCAAGAGGATATTTCATTTATTGATAAGCTGTTCGCATAA (SEQ ID NO: 98) |

**[TABLE 1-50]**

| yrpG/NP_390562.2/Bacillus subtilis subsp. subtilis str. 168 | |
|---|---|
| Amino acid sequence | MEYTYLGRTGLRVSRLCLGTMNFGVDTDEKTAFRIMDEALDNGIQFFDTANIYGWG KNAGLTESIIGKWFAQGGQRREKVVLATKVYEPISDPNDGPNDMRGLSLYKIRRHLE GSLKRLQTDHIELYQMHHIDRRTPWDEIWEAFETQVRSGKVDYIGSSNFAGWHLVKA QAEAEKRRFMGLVTEQHKYSLLERTAEMEVLPAARDLGLGVVAWSPLAGGLLGGKA LKSNAGTRTAKRADLIEKHRLQLEKFSDLCKELGEKEANVALAWVLANPVLTAPIIGP RTVEQLRDTIKAVEISLDKEILRMLNDIFPGPGGETPEAYAW (SEQ ID NO: 99) |
| Base sequence | GTGGAGTATACCTATTTAGGGAGAACAGGATTGCGGGTGAGCCGTTTATGTTTAGG CACGATGAATTTTGGAGTTGATACAGACGAAAAGACTGCGTTCCGTATCATGGATG |
| | AAGCACTTGATAACGGCATTCAATTTTTGATACTGCCAATATTTACGGCTGGGGCA AAAACGCAGGATTGACAGAGAGCATCATTGGAAAATGGTTTGCACAAGGAGGAC AGCGCCGCGAGAAAGTTGTTCTGGCGACAAAAGTATATGAACCGATTTCTGATCC GAATGACGGACCAAATGATATGAGGGGCTTGTCTCTATACAAAATCAGACGTCATC TGGAAGGATCACTGAAGCGGCTTCAGACAGATCATATCGAATTGTACCAAATGCAT CATATCGATAGGCGGACACCGTGGGATGAGATATGGGAAGCTTTTGAGACTCAGGT TCGCTCCGGCAAAGTAGACTATATTGGATCCAGTAATTTTGCAGGCTGGCATTTAGT TAAAGCGCAAGCTGAAGCTGAAAAACGGCGATTCATGGGACTCGTCACTGAACA GCATAAGTATAGTTTATTAGAACGAACAGCTGAAATGGAAGTGCTGCCGGCTGCAC GGGATCTTGGTTTAGGAGTAGTGGCGTGGAGTCCCCTTGCAGGAGGGCTTCTTGG CGGGAAGGCATTGAAAAGCAATGCCGGAACTCGTACAGCAAAAAGAGCAGATTT AATTGAAAAACATCGTTTGCAACTCGAGAAATTTTCAGATTTATGCAAAGAACTAG GAGAAAAAGAAGCAAATGTGGCTTTGGCATGGGTGCTGGCAAATCCAGTTTTAAC TGCGCCGATCATCGGACCACGAACGGTTGAGCAGCTGCGTGATACGATAAAAGCC GTTGAAATCAGTCTGGATAAGGAGATTCTCCGCATGTTAAATGATATCTTTCCCGGA CCTGGAGGAGAGACACCTGAGGCATACGCCTGGTGA (SEQ ID NO: 100) |

In the present invention, an activity of one kind of ADH may be reduced, and activities of two or more kinds of ADH may be reduced. It is preferable that activities of two or more kinds of ADH are reduced from the viewpoint of further reducing production of an alcohol form as a by-product.

In the present invention, "ADH non-reduced strain" (alternatively, also simply referred to as a "non-reduced strain") refers to a strain which is not modified such that the ADH activity is reduced. Examples of the ADH non-reduced strain include, but are not limited to, a wild-type strain or a reference strain of each microbial strain and a derivative strain including a strain obtained by breeding. Examples of E. coli strains include, but are not limited to, K-12 strain, B strain, C strain, W strain, and derivative strains thereof such as BL21 (DE3) strain, W3110 strain, MG1655 strain, JM109 strain, DH5α strain, and HB101 strain.

The genetically modified microorganism "modified to reduce an activity of an alcohol dehydrogenase" means that a modification is performed at least to suppress expression of a gene encoding an alcohol dehydrogenase. The "modification to reduce an activity of an alcohol dehydrogenase" includes a modification to suppress an activity of the enzyme, in addition to the modification to suppress expression of a gene encoding an alcohol dehydrogenase. That is, the modified microorganism of the present invention contains a modification to suppress expression of a gene encoding an alcohol dehydrogenase compared to a non-reduced strain (for example, a host microorganism) or a modification to suppress an activity of the enzyme. More specifically, the "modification to reduce an activity of an alcohol dehydrogenase" means that a modification is performed at least to suppress expression of a gene encoding an alcohol dehydrogenase, and a modification is preferably performed to suppress expression of a gene encoding an alcohol dehydrogenase and to suppress an activity of an alcohol dehydrogenase. Since a host microorganism may have plural genes each encoding an alcohol dehydrogenase and plural alcohol dehydrogenases exhibiting an activity against the same substrate may be present, even in the case where "the modification is performed to reduce an activity of an alcohol dehydrogenase", the activity of the alcohol dehydrogenase may be maintained in a decomposition activity of alcohol species compared to a non-reduced strain. Therefore, as described above, the case where "the modification is performed to reduce an activity of an alcohol dehydrogenase" includes a case where an activity of an alcohol dehydrogenase is maintained compared to a non-reduced strain even though the modification aimed to reduce the activity is performed. In the present invention, with regard to the gene encoding an enzyme, "suppression of expression" also includes "reduction in expression". In addition, with regard to the enzyme, "suppression of activity" is synonymous with "suppression of function", "reduction in function", and "reduction in activity", and these are used interchangeably.

The genetically modified microorganism of the present invention preferably contains a modification to suppress expression of two or more genes encoding an alcohol dehydrogenase. In the production of the diamine compound, the production amount of the diamine compound can be significantly increased, and production of an alcohol form that is a by-product can also be suppressed, by modifying the microorganism to suppress expression of a plurality of kinds of genes.

The modification performed to reduce the activity of an ADH can be achieved by, for example, reducing expression of a gene encoding an ADH. More specifically, the reduction in the expression of the gene may mean that a transcription amount of a gene (mRNA amount) is reduced and/or that a translation amount of a gene (protein amount) is reduced. The reduction in the expression of the gene also includes a case where a gene is not expressed at all.

The reduction in the expression of the gene may be, for example, a reduction in transcription amount, a reduction in translation amount, or a combination thereof. The reduction in the transcription amount can be achieved by, for example, a method of modifying an expression regulatory region such as a promoter region or a ribosome binding site (RBS) of an ADH gene. The reduction in the transcription amount of the gene can be evaluated by a method known to those skilled in the art, and examples of the method include a quantitative RT-PCR method and a Northern blotting method. The transcription amount of the gene may be reduced to, for example, 50% or less, 20% or less, 10% or less, 5% or less, or 0% compared to the ADH non-reduced strain.

An example of a method of reducing the translation amount includes a method of suppressing translation by inserting a riboswitch region into the upstream of a gene. The riboswitch refers to an RNA that selectively binds to a specific low-molecular compound, and the low-molecular compound refers to a ligand. A secondary structure is formed by RNA base pairs in the absence of a ligand to affect nucleic acids around the riboswitch. In particular, in a case where a ribosome binding site is included in the downstream of the riboswitch, the access of the ribosome to the ribosome binding site is blocked, which interferes with translation of mRNA of a gene located further downstream. On the other hand, the ribosome can access the ribosome binding site in the presence of a ligand through secondary-structure elimination according to ligand binding. Therefore, when a ligand is not added, mRNA of a gene is not translated, and expression of a target gene is suppressed. The reduction in the translation amount of the gene can be evaluated by a method known to those skilled in the art, and an example of the method includes a Western blotting method. The translation amount of the gene may be reduced to, for example, 50% or less, 20% or less, 10% or less, 5% or less, or 0% compared to the ADH non-reduced strain.

In addition, the modification to reduce the activity of the ADH is achieved by disrupting a gene encoding an ADH. The disruption of the ADH gene means that a gene is modified so that a protein having an ADH activity is not expressed, and includes a case where a protein is not produced at all and a case where a protein with reduced or eliminated ADH activity is produced. For example, the disruption can be achieved by deficiency of a part or all of a coding region of a gene on a chromosome. Furthermore, the entire gene containing sequences before and after a gene on a chromosome may be deleted. As long as the reduction of the ADH activity can be achieved, the deleted region may be any one of the N-terminus region, the internal region, and the C-terminus region.

In addition, the disruption of the ADH gene can be achieved by a method of introducing amino acid substitution (missense mutation) or introducing a stop codon (nonsense mutation) into a coding region of an ADH gene on a chromosome, or a method of introducing a frameshift mutation that adds or deletes one or two bases.

Furthermore, the disruption of the ADH gene can be achieved by inserting another sequence into a coding region of a gene on a chromosome. Examples of other sequences include an antibiotic-resistant gene or a transposon, but are not particularly limited as long as the ADH activity is reduced.

The disruption of the ADH gene can be performed by using a method of homologous recombination, and examples of the method include, but are not limited to, a method of using Red reconbinase of A-phage (Datsenko, Kirill A., and Barry L. Wanner. "One-step inactivation of chromosomal genes in Escherichia coli K-12 using PCR products." Proceedings of the National Academy of Sciences 97.12 (2000): 6640-6645.), a method of using a Suicide vector containing a temperature sensitive origin of replication (Blomfield et al., Molecular microbiology 5.6 (1991): 1447-1457.), and a method of using a CRISPR-Cas9 system (Jiang, Yu, et al. "Multigene editing in the Escherichia coli genome via the CRISPR-Cas9 system." Appl. Environ. Microbiol. 81.7 (2015): 2506-2514.).

In addition, the disruption of the ADH gene can be performed by a mutation treatment. Examples of the mutation treatment include physical treatments such as an X-ray treatment, an ultraviolet ray treatment, and a γ-ray treatment, and chemical treatments with mutagens such as N-methyl-N'-nitro-N-nitrosoguanidine, ethyl methanesulfonate, and methyl methanesulfonate, but are not particularly limited as long as the ADH activity is reduced.

The ADH activity can be evaluated by a method known to those skilled in the art. For example, an example of the evaluation method includes a method of monitoring oxidation of NAD(P)H by incubating a substrate (an aldehyde or a ketone) and NAD(P)H and measuring an absorbance at 340 nm (Pick, et al., Applied microbiology and biotechnology 97.13 (2013): 5815-5824). The ADH activity may be reduced to, for example, 50% or less, 20% or less, 10% or less, 5% or less, or 0% compared to an ADH of an ADH non-reduced strain.

In the genetically modified microorganism according to the present invention, the enzyme involved in synthesis of a diamine compound may have an endogenous property, an exogenous property, or a combination thereof.

The genetically modified microorganism according to the present invention preferably expresses a carboxylic acid reductase as an enzyme gene involved in synthesis of a diamine compound. The carboxylic acid reductase (CAR) generally refers to an arbitrary protein having an activity of reducing a carboxylic acid to convert into an aldehyde. In the present invention, the carboxylic acid reductase has an activity of converting, for example, a carboxyl group of a carboxylic acid semialdehyde, a dicarboxylic acid, or an aminocarboxylic acid into an aldehyde. Examples of the carboxylic acid reductase include, but are not limited to, enzymes classified as EC 1.2.1.30, EC 1.2.1.31, EC 1.2.1.95, EC 1.2.99.6 or the like.

A source of a gene encoding the enzyme is not particularly limited as long as it has a carboxylic acid reducing activity, and examples thereof include, but are not limited to, Nocardia iowensis, Nocardia asteroides, Nocardia brasiliensis, Nocardia farcinica, Segniliparus rugosus, Segniliparus rotundus, Tsukamurella paurometabola, Mycobacterium marinum, Mycobacterium neoaurum, Mycobacterium abscessus, Mycobacterium avium, Mycobacterium chelonae, Mycobacterium immunogenum, Mycobacterium smegmatis, Serpula lacrymans, Heterobasidion annosum, Coprinopsis cinerea, Aspergillus flavus, Aspergillus terreus, Neurospora crassa, and Saccharomyces cerevisiae. In the present invention, for example, a gene encoding a protein consisting of an amino acid sequence set forth in any one of SEQ ID NOs: 101 to 104 is used. A gene encoding a carboxylic acid reductase MaCar derived from Mycobacterium abscessus is preferably used. A base sequence of a coding region of a MaCar gene is set forth in SEQ ID NO: 105, and an amino acid sequence of MaCar is set forth in SEQ ID NO: 103.

**[Table 2-1]**

| Accession No. / origin (name of protein) | Amino acid sequence |
|---|---|
| AAR91681 / Nocardia iowensis | MAVDSPDERLQRRIAQLFAEDEQVKAARPLEAVSAAVSAPGMRLAQIAATVMAGY ADRPAAGQRAFELNTDDATGRTSLRLLPRFETITYRELWQRVGEVAAAWHHDPENP LRAGDFVALLGFTSIDYATLDLADIHLGAVTVPLQASAAVSQLIAILTETSPRLLASTP |
| | EHLDAAVECLLAGTTPERLVVFDYHPEDDDQRAAFESARRRLADAGSSVIVETLDA VRARGRDLPAAPLFVPDTDDDPLALLIYTSGSTGTPKGAMYTNRLAATMWQGNS MLQGNSQRVGINLNYMPMSHIAGRISLFGVLARGGTAYFAAKSDMSTLFEDIGLVR PTEIFFVPRVCDMVFQRYQSELDRRSVAGADLDTLDREVKADLRQNYLGGRFLVAV VGSAPLAAEMKTFMESVLDLPLHDGYGSTEAGASVLLDNQIQRPPVLDYKLVDVP ELGYFRTDRPHPRGELLLKAETTIPGYYKRPEVTAEIFDEDGFYKTGDIVAELEHDR LVYVDRRNNVLKLSQGEFVTVAHLEAVFASSPLIRQIFIYGSSERSYLLAVIVPTDDA LRGRDTATLKSALAESIQRIAKDANLQPYEIPRDFLIETEPFTIANGLLSGIAKLLRPN LKERYGAQLEQMYTDLATGQADELLALRREAADLPVLETVSRAAKAMLGVASAD MRPDAHFTDLGGDSLSALSFSNLLHEIFGVEVPVGVVVSPANELRDLANYIEAERN SGAKRPTFTSVHGGGSEIRAADLTLDKFIDARTLAAADSIPHAPVPAQTVLLTGANG YLGRFLCLEWLERLDKTGGTLICVVRGSDAAAARKRLDSAFDSGDPGLLEHYQQL AARTLEVLAGDIGDPNLGLDDATWQRLAETVDLIVHPAALVNHVLPYTQLFGPNV VGTAEIVRLAITARRKPVTYLSTVGVADQVDPAEYQEDSDVREMSAVRVVRESYAN GYGNSKWAGEVLLREAHDLCGLPVAVFRSDMILAHSRYAGQLNVQDVFTRLILSLV ATGIAPYSFYRTDADGNRQRAHYDGLPADFTAAAITALGIQATEGFRTYDVLNPYD DGISLDEFVDWLVESGHPIQRITDYSDWFHRFETAIRALPEKQRQASVLPLLDAYRN PCPAVRGAILPAKEFQAAVQTAKIGPEQDIPHLSAPLIDKYVSDLELLQLL (SEQ ID NO: 101) |
| ACC40567 / Mycobacterium marinum | MSPITREERLERRIQDLYANDPQFAAAKPATAITAAIERPGLPLPQIIETVMTGYADRP ALAQRSVEFVTDAGTGHTTLRLLPHFETISYGELWDRISALADVLSTEQTVKPGDR VCLLGFNSVDYATIDMTLARLGAVAVPLQTSAAITQLQPIVAETQPTMIAASVDALA DATELALSGQTATRVLVFDHHRQVDAHRAAVESARERLAGSAVVETLAEAIARGD VPRGASAGSAPGTDVSDDSLALLIYTSGSTGAPKGAMYPRRNVATFWRKRTWFEG |
| | GYEPSITLNFMPMSHVMGRQILYGTLCNGGTAYFVAKSDLSTLFEDLALVRPTELTF VPRVWDMVFDEFQSEVDRRLVDGADRVALEAQVKAEIRNDVLGGRYTSALTGSAP ISDEMKAWVEELLDMHLVEGYGSTEAGMILIDGAIRRPAVLDYKLVDVPDLGYFLT DRPHPRGELLVKTDSLFPGYYQRAEVTADVFDADGFYRTGDIMAEVGPEQFVYLD RRNNVLKLSQGEFVTVSKLEAVFGDSPLVRQIYIYGNSARAYLLAVIVPTQEALDAV PVEELKARLGDSLQEVAKAAGLQSYEIPRDFIIETTPWTLENGLLTGIRKLARPQLK KHYGELLEQIYTDLAHGQADELRSLRQSGADAPVLVTVCRAAAALLGGSASDVQP DAHFTDLGGDSLSALSFTNLLHEIFDIEVPVGVIVSPANDLQALADYVEAARKPGSS RPTFASVHGASNGQVTEVHAGDLSLDKFIDAATLAEAPRLPAANTQVRTVLLTGAT GFLGRYLALEWLERMDLVDGKLICLVRAKSDTEARARLDKTFDSGDPELLAHYRA LAGDHLEVLAGDKGEADLGLDRQTWQRLADTVDLIVDPAALVNHVLPYSQLFGP NALGTAELLRLALTSKIKPYSYTSTIGVADQIPPSAFTEDADIRVISATRAVDDSYANG YSNSKWAGEVLLREAHDLCGLPVAVFRCDMILADTTWAGQLNVPDMFTRMILSLA ATGIAPGSFYELAADGARQRAHYDGLPVEFIAEAISTLGAQSQDGFHTYHVMNPY DDGIGLDEFVDWLNESGCPIQRIADYGDWLQRFETALRALPDRQRHSSLLPLLHNY RQPERPVRGSIAPTDRFRAAVQEAKIGPDKDIPHVGAPIIVKYVSDLRLLGLL (SEQ ID NO: 102) |
| CAM64782 / Mycobacterium abcessus | MTETISTAAVPTTDLEEQVKRRIEQVVSNDPQLAALLPEDSVTEAVNEPDLPLVEVI RRLLEGYGDRPALGQRAFEFVTGDDGATVIALKPEYTTVSYRELWERAEAIAAAW HEQGIRDGDFVAQLGFTSTDFASLDVAGLRLGTVSVPLQTGASLQQRNAILEETRPA VFAASIEYLDAAVDSVLATPSVRLLSVFDYHAEVDSQREALEAVRARLESAGRTIVV |
| (MaCar) | EALAEALARGRDLPAAPLPSADPDALRLLIYTSGSTGTPKGAMYPQWLVANLWQK KWLTDDVIPSIGVNFMPMSHLAGRLTLMGTLSGGGTAYYIASSDLSTFFEDIALIRPS EVLFVPRVVEMVFQRFQAELDRSLAPGESNSEIAERIKVRIREQDFGGRVLSAGSGS APLSPEMTEFMESLLQVPLRDGYGSTEAGGVWRDGVLQRPPVTDYKLVDVPELGY FTTDSPHPRGELRLKSETMFPGYYKRPETTADVFDDEGYYKTGDVVAELGPDHLK YLDRVKNVLKLAQGEFVAVSKLEAAYTGSPLVRQIFVYGNSERSFLLAVVVPTPEV LERYADSPDALKPLIQDSLQQVAKDAELQSYEIPRDFIVETVPFTVESGLLSDARKLL RPKLKDHYGERLEALYAELAESQNERLRQLAREAATRPVLETVTDAAAALLGASSS DLAPDVRFIDLGGDSLSALSYSELLRDIFEVDVPVGVINSVANDLAAIARHIEAQRT GAATQPTFASVHGKDATVITAGELTLDKFLDESLLKAAKDVQPATADVKTVLVTGG NGWLGRWLVLDWLERLAPNGGKVYALIRGADAEAARARLDAVYESGDPKLSAH YRQLAQQSLEVIAGDFGDQDLGLSQEVWQKLAKDVDLIVHSGALVNHVLPYSQLF GPNVAGTAEIIKLAISERLKPVTYLSTVGIADQIPVTEFEEDSDVRVMSAERQINDGY ANGYGNSKWAGEVLLREAHDLAGLPVRVFRSDMILAHSDYHGQLNVTDVFTRSIQ SLLLTGVAPASFYELDADGNRQRAHYDGVPGDFTAASITAIGGVNVVDGYRSFDVF NPHHDGVSMDTFVDWLIDAGYKIARIDDYDQWLARFELALKGLPEQQRQQSVLP LLKMYEKPQPAIDGSALPTAEFSRAVHEAKVGDSGEIPHVTKELILKYASDIQLLGL V (SEQ ID NO: 103) |
| AFP42026 / Mycobacterium smegmatis | MHQLTVTGMNICEVQRLFPRMTSDVHDATDGVTETALDDEQSTRRIAELYATDPEF AAAAPLPAVVDAAHKPGLRLAEILQTLFTGYGDRPALGYRARELATDEGGRTVTRL LPRFDTLTYAQVWSRVQAVAAALRHNFAQPIYPGDAVATIGFASPDYLTLDLVCAYL GLVSVPLQHNAPVSRLAPILAEVEPRILTVSAEYLDLAVESVRDVNSVSQLVVFDHH PEVDDHRDALARAREQLAGKGIAVTTLDAIADEGAGLPAEPIYTADHDQRLAMILY TSGSTGAPKGAMYTEAMVARLWTMSFITGDPTPVINVNFMPLNHLGGRIPISTAVQ |
| | NGGTSYFVPESDMSTLFEDLALVRPTELGLVPRVADMLYQHHLATVDRLVTQGADE LTAEKQAGAELREQVLGGRVITGFVSTAPLAAEMRAFLDITLGAHIVDGYGLTETG AVTRDGVIVRPPVIDYKLIDVPELGYFSTDKPYPRGELLVRSQTLTPGYYKRPEVTA SVFDRDGYYHTGDVMAETAPDHLVYVDRRNNVLKLAQGEFVAVANLEAVFSGAA LVRQIFVYGNSERSFLLAVVVPTPEALEQYDPAALKAALADSLQRTARDAELQSYE VPADFIVETEPFSAANGLLSGVGKLLRPNLKDRYGQRLEQMYADIAATQANQLREL RRAAATQPVIDTLTQAAATILGTGSEVASDAHFTDLGGDSLSALTLSNLLSDFFGFE VPVGTIVNPATNLAQLAQHIEAQRTAGDRRPSFTTVHGADATEIRASELTLDKFIDA ETLRAAPGLPKVTTEPRTVLLSGANGWLGRFLTLQWLERLAPVGGTLITIVRGRDD AAARARLTQAYDTDPELSRRFAELADRHLRVVAGDIGDPNLGLTPEIWHRLAAEVD LVVHPAALVNHVLPYRQLFGPNVVGTAEVIKLALTERIKPVTYLSTVSVAMGIPDFE EDGDIRTVSPVRPLDGGYANGYGNSKWAGEVLLREAHDLCGLPVATFRSDMILAHP RYRGQVNVPDMFTRLLLSLLITGVAPRSFYIGDGERPRAHYPGLTVDFVAEAVTTLG AQQREGYVSYDVMNPHDDGISLDVFVDWLIRAGHPIDRVDDYDDWVRRFETALT ALPEKRRAQTVLPLLHAFRAPQAPLRGAPEPTEVFHAAVRTAKVGPGDIPHLDEALI DKYIRDLREFGLI (SEQ ID NO: 104) |

**[Table 2-2]**

| Base sequence of a region encoding MaCar |
|---|
| ATGACTGAAACGATCTCCACAGCGGCTGTCCCACTACGGATCTCGAAGAGCAGG TGAAGCGACGCATCGAGCAGGTCGTGTCCAACGATCCGCAGCTGGCGGCTTCT CCCGGAAGATTCGGTCACCGAGGCGGTCAACGAGCCCGATCTACCGCTGGTCGAG GTGATCAGGCGACTGCTGGAGGGCTACGGTGACCGCCCGGCACTCGGCCAGCGC GCCTTCGAGTTCGTCACCGGGGACGACGGTGCGACCGTGATCGCGCTGAAGCCCG AATACACCACCGTCTCCTACCGCGAGTTGTGGAACGTGCCGAGGCTATCGCTGC CGCGTGGCACGAGCAGGGCATCCGTGACGGCGACTTCGTCGCTCAGTTGGGTTTC ACCAGCACGGACTTCGCGTCGCTCGACGTCGCGGATTGCGTCTGGGCACCGTCT CGGTGCCCCTGCAGACGGGCGCGTCGCTGCAGCAGCGCAACGCGATTCTCGAAG AGACCCGGCCCGCAGTCTTTGCCGCGAGTATCGAATACCTTGATGCCGCCGTCGAT TCGGTGCTTGCGACCCCCTCGGTGCGACTCCTCTCGGTTTTCGACTATCACGCGGA GGTCGACAGCCAGCGCGAAGCGCTGGAGGCTGTGCGGGCCCGGCTTGAGAGTGC CGGCCGGACGATCGTCGTCGAGGCCCTGGCGGAGGCTCTCGCGCGGGGGCGGGA CCTGCCCGCCGCGCCGCTGCCCAGTGCAGATCCCGATGCCTTGCGTCTGCTCATCT ACACCTCCGGCAGCACCGGTACCCCCAAGGGCGCCATGTATCCGCAATGGCTGGT CGCCAACTTGTGGCAGAAGAAGTGGCTCACCGACGATGTGATTCCGTCCATAGGC GTGAACTTCATGCCCATGAGCCACCTGGCGGGTCGCCTCACTCTCATGGGCACCCT TTCCGGTGGCGGAACCGCCTACTACATCGCTTCGAGCGATCTTTCGACTTTCTTCG AGGACATCGCGCTCATCCGCCCCTCCGAAGTGCTCTTCGTGCCGCGTGTGGTGGA GATGGTGTTCCAGCGTTTTCAGGCAGAATTGGACCGGTCCCTTGCCCCGGGTGAG AGCAACTCCGAGATCGCGGAGCGAATCAAGGTCCGCATCCGGGAACAGGACTTC GGCGGGCGTGTGCTCAGTGCTGGCTCCGGGTCGGCCCCGTTGTCTCCTGAGATGA CGGAGTTCATGGAGTCGCTGCTGCAGGTGCCGTTGCGCGACGGGTATGGGTCCAC |
| CGAGGCCGGTGGTGTGTGGCGTGACGGAGTCCTGCAGCGTCCGCCCGTCACCGA CTACAAGCTGGTTGACGTTCCGGAACTCGGATACTTCACCACAGATTCGCCGCATC CCCGTGGCGAGCTGCGGTTGAAGTCGGAGACGATGTTCCCCGGCTACTACAAGCG CCCGGAGACCACTGCCGATGTCTTCGATGACGAGGGGTACTACAAGACCGGTGAC GTGGTCGCCGAGCTCGGGCCGGATCACCTCAAGTACCTCGACCGCGTCAAGAACG TCCTCAAGCTCGCGCAGGGAGAGTTTGTCGCGGTGTCAAAGCTGGAGGCCGCTTA CACCGGCAGCCCGCTGGTCCGGCAGATCTTTGTGTACGGGAACAGTGAACGCTCG TTCCTGCTGGCTGTCGTGGTCCCGACACCCGAAGTCCTTGAGCGGTACGCAGATT CGCCAGATGCGCTCAAGCCCTTGATCCAGGATTCGCTGCAGCAGGTCGCCAAGGA CGCGGAGCTGCAATCCTATGAGATACCGCGCGACTTCATCGTTGAGACGGTGCCGT TCACCGTCGAGTCCGGATTGCTATCGGACGCGCGAAAGCTGCTGCGCCCCAAGCT GAAGGATCACTACGGAGAGAGGCTGGAGGCGCTGTACGCCGAACTGGCGGAAAG CCAGAATGAGCGGCTGCGCCAGTTGGCCAGGGAGGCAGCCACGCGCCCGGTCCT GGAGACGGTGACCGATGCGGCCGCCGCGCTGCTGGGCGCATCGTCCTCGGATCTG GCTCCTGATGTGCGATTCATCGACCTCGGTGGCGACTCACTGTCGGCGCTGTCGTA CTCCGAGCTGCTGCGCGACATCTTTGAGGTGGACGTTCCGGTGGGCGTCATCAAC AGCGTCGCCAACGACCTTGCCGCGATCGCCCGGCACATCGAGGCGCAGCGGACC GGCGCCGCTACGCAGCCGACCTTTGCGTCGGTCCACGGCAAGGACGCGACGGTC ATCACCGCCGGTGAACTCACCCTCGACAAGTTCTTGGACGAGTCACTGTTGAAAG CGGCCAAGGACGTTCAGCCGGCAACGGCCGATGTCAAGACCGTTCTAGTGACCG GCGGCAACGGCTGGTTGGGTCGTTGGCTGGTGCTCGATTGGCTGGAGCGGTTGGC ACCCAATGGTGGCAAGGTCTACGCCCTCATTCGTGGCGCCGATGCCGAAGCAGCC CGGGCACGGTTGGACGCCGTGTACGAATCGGGTGATCCCAAGCTGTCCGCGCATT ATCGTCAGCTGGCGCAACAGAGTCTGGAAGTTATCGCCGGCGATTTCGGCGACCA |
| GGATCTCGGTCTATCCCAGGAAGTTTGGCAGAAGCTGGCCAAGGACGTGGACCTG ATCGTGCACTCCGGTGCCTTGGTGAACCACGTGCTGCCGTACAGCCAGTTGTTCG GTCCGAATGTGGCGGGTACCGCCGAGATCATCAAGCTGGCAATTTCGGAGCGGCT CAAGCCGGTCACCTACCTGTCGACGGTGGGCATCGCCGACCAGATTCCGGTGACG GAGTTCGAGGAAGACTCCGATGTTCGTGTGATGTCGGCCGAGCGCCAGATCAATG ACGGCTACGCGAACGGATACGGCAACTCAAAATGGGCCGGCGAGGTGCTGTTGC GGGAGGCTCATGACCTAGCGGGGCTGCCGGTGCGTGTGTTCCGCTCCGACATGAT CCTGGCGCACAGTGACTACCACGGACAGCTCAACGTCACCGACGTGTTCACCCGG AGCATCCAGAGTCTGCTGCTCACCGGTGTTGCACCGGCCAGCTTCTATGAATTGGA TGCCGACGGCAATCGGCAGCGCGCTCACTATGACGGTGTGCCCGGCGATTTCACC GCCGCATCGATCACCGCCATCGGCGGTGTGAACGTGGTAGACGGTTACCGCAGCT TCGACGTGTTCAACCCGCACCATGACGGTGTCTCGATGGATACCTTCGTCGACTGG CTGATCGACGCAGGCTACAAGATCGCGCGGATCGACGATTACGACCAGTGGCTCG CCCGGTTCGAGCTGGCCCTCAAGGGATTGCCCGAGCAGCAGCGGCAACAGTCGG TGTTGCCACTTCTCAAGATGTACGAGAAGCCGCAACCGGCGATCGACGGAAGTGC ACTTCCGACCGCAGAATTCAGTCGCGCCGTGCACGAGGCGAAGGTCGGAGACAG CGGTGAGATACCGCACGTCACCAAGGAGCTGATCCTCAAGTACGCCAGCGATATT CAGCTGTTGGGCCTGGTGTAG (SEQ ID NO: 105) |

The activity of the carboxylic acid reductase can be evaluated by a method known to those skilled in the art, and examples of the evaluation method include a method of monitoring oxidation of NADPH by incubating a substrate (a carboxylic acid) and an enzyme in the presence of ATP and NADPH and measuring an absorbance at 340 nm, and a method of quantifying a consumption amount of a substrate and/or a production amount of a product (an aldehyde) (Venkitasubramanian et al., Journal of Biological Chemistry, Vol. 282, No. 1, 478-485 (2007)).

In addition, the carboxylic acid reductase can be converted into an active holoenzyme by being phosphopantetinylated (Venkitasubramanian et al., Journal of Biological Chemistry, Vol. 282, No. 1, 478-485 (2007)). The phosphopantetinylation is catalyzed by a phosphopantetheinyl transferase (PT) (for example, an example thereof includes an enzyme classified as EC 2.7.8.7). Therefore, the microorganism of the present invention may be further modified such that an activity of the phosphopantetheinyl transferase is increased. Examples of a method of increasing the activity of the phosphopantetheinyl transferase include, but are not limited to, a method of introducing an exogenous phosphopantetheinyl transferase and a method of enhancing expression of an endogenous phosphopantetheinyl transferase. An example of a donor of the phosphopantetheinyl group includes a coenzyme A (CoA).

A source of a PT gene is not particularly limited as long as it has a phosphopantetheinyl group transfer activity, and examples of a gene encoding a typical phosphopantetheinyl transferase include Sfp of Bacillus subtilis, Npt of Nocardia iowensis (Venkitasubramanian et al., Journal of Biological Chemistry,Vol.282,No.1,478-485 (2007)), and Lys5 of Saccharomyces cerevisiae (Ehmann et al., Biochemistry 38.19 (1999): 6171-6177). In the present invention, for example, a gene encoding a protein consisting of an amino acid sequence set forth in any one of SEQ ID NOs: 106 to 108 is used. An Npt gene of Nocardia iowensis derived from Nocardia iowensis is preferably used. A base sequence of a coding region of the Npt gene is set forth in SEQ ID NO: 109, and an amino acid sequence of Npt is set forth in SEQ ID NO: 107.

**[Table 3-1]**

| Accession No. / origin (name of protein) | Amino acid sequence |
|---|---|
| CAA44858 / derived from Bacillus subtilis/ (Sfp) | MKIYGIYMDRPLSQEENERFMTFISPEKREKCRRFYHKEDAHRTLLGDV LVRSVISRQYQLDKSDIRFSTQEYGKPCIPDLPDAHFNISHSGRWVIGAF DSQPIGIDIEKTKPISLEIAKRFFSKTEYSDLLAKDKDEQTDYFYHLWSM KESFIKQEGKGLSLPLDSFSVRLHQDGQVSIELPDSHSPCYIKTYEVDPG |
| | YKMAVCAAHPDFPEDITMVSYEELL (SEQ ID NO: 106) |
| ABI83656 / derived from Nocardia iowensis (Npt) | MIETILPAGVESAELLEYPEDLKAHPAEEHLIAKSVEKRRRDFIGARHCA RLALAELGEPPVAIGKGERGAPIWPRGVVGSLTHCDGYRAAAVAHKMR FRSIGIDAEPHATLPEGVLDSVSLPPEREWLKTTDSALHLDRLLFCAKEA TYKAWWPLTARWLGFEEAHITFEIEDGSADSGNGTFHSELLVPGQTNDG GTPLLSFDGRWLIADGFILTAIAYA (SEQ ID NO: 107) |
| CAA96866 / derived from Sacharomyces cerevisiae (Lys5) | MVKTTEVVSEVSKVAGVRPWAGIFVVEIQEDILADEFTFEALMRTLPLA SQARILNKKSFHDRCSNLCSQLLQLFGCSIVTGLNFQELKFDKGSFGKPF LDNNRFLPFSMTIGEQYVAMFLVKCVSTDEYQDVGIDIASPCNYGGREE LELFKEVFSEREFNGLLKASDPCTIFTYLWSLKESYTKFTGTGLNTDLSL IDFGAISFFPAEGASMCITLDEVPLIFHSQWFNNEIVTICMPKSISDKINTN RPKLYNISLSTLIDYFIENDGL (SEQ ID NO: 108) |

**[Table 3-2]**

| Base sequence of a region encoding Npt |
|---|
| ATGATCGAGACAATTTTGCCTGCTGGTGTCGAGTCGGCTGAGCTGCTGGAGTATCC GGAGGACCTGAAGGCGCATCCGGCGGAGGAGCATCTCATCGCGAAGTCGGTGGA GAAGCGGCGCCGGGACTTCATCGGGGCCAGGCATTGTGCCCGGCTGGCGCTGGCT GAGCTCGGCGAGCCGCCGGTGGCGATCGGCAAAGGGGAGCGGGGTGCGCCGATC TGGCCGCGCGGCGTCGTCGGCAGCCTCACCCATTGCGACGGATATCGGGCCGCGG CGGTGGCGCACAAGATGCGCTTCCGTTCGATCGGCATCGATGCCGAGCCGCACGC GACGCTGCCCGAAGGCGTGCTGGATTCGGTCAGCCTGCCGCCGGAGCGGGAGTG |
| GTTGAAGACCACCGATTCCGCACTGCACCTGGACCGTTTACTGTTCTGCGCCAAG GAAGCCACCTACAAGGCGTGGTGGCCGCTGACCGCGCTGGCTCGGCTTCGAG GAAGCGCACATCACCTTCGAGATCGAAGACGGCTCCGCCGATTCCGGCAACGGCA CCTTTCACAGCGAGCTGCTGGTGCCGGACAGACGAATGACGGTGGACGCCGC TGCTTTCGTTCGACGGCCGGTGGCTGATCGCCGACGGGTTCATCCTCACCGCGATC GCGTACGCCTGA (SEQ ID NO: 109) |

As another aspect of producing an aldehyde, the genetically modified microorganism of the present invention may express acyl-(acyl carrier protein (ACP)) reductase (AAR). AAR is an enzyme that converts acyl-ACP into an aldehyde. A gene encoding AAR is not particularly limited, and an example of a typical AAR gene includes AAR of Synechococcus elongatus (Schirmer, Andreas, et al., Science 329.5991 (2010): 559-562).

In addition, as another aspect, an enzyme that produces an aldehyde from acyl-CoA may be expressed. Examples of a gene encoding an enzyme that catalyzes this reaction include, but are not limited to, acr1 of Acinetobacter baylyi encoding fatty acid acyl-CoA dehydrogenase (ZHENG, Yan-Ning, et al., Microbial cell factories, 2012, 11.1:65) and an sucD gene of Clostridium kluyveri encoding succinic acid semialdehyde dehydrogenase (Sohling, B., and Gerhard Gottschalk., Journal of bacteriology 178.3 (1996): 871-880).

Furthermore, an enzyme that produces an aldehyde from acyl phosphate may be expressed, and for example, aspartic acid semialdehyde dehydrogenase (ASD; EC 1.2.1.11) that catalyzes a reaction of aspartic acid semialdehyde from NADPH-dependant 4-aspartyl phosphate can catalyze the same reaction, and an asd gene of E. coli or the like can be used.

The genetically modified microorganism according to the present invention expresses an aminotransferase as an enzyme gene involved in synthesis of a diamine compound.

The aminotransferase refers to an arbitrary enzyme that catalyzes an amino group transfer reaction in the presence of an amino group donor and a receptor. An example of the aminotransferase includes an enzyme classified as EC 2.6.1.p (wherein, p is an integer of 1 or more). Examples of the amino group donor include, but are not limited to, L-glutamic acid, L-alanine, and glycine.

A gene encoding the aminotransferase is not particularly limited as long as it has an amino group transfer activity, and putrescine aminotransferase or other diamine transferases can be preferably used. Examples thereof include a ygjG gene encoding putrescine aminotransferase of E. coli, which is reported to perform amino group transfer on cadaverine and spermidine (Samsonova., et al., BMC microbiology 3.1 (2003): 2), an SpuC gene encoding putrescine aminotransferase of the genus Pseudomonas (Lu et al., Journal of bacteriology 184.14 (2002): 3765-3773, Galman et al., Green Chemistry 19.2 (2017): 361-366.), and a gabT gene and a puuE gene encoding GABA aminotransferase of E. coli. Furthermore, it is reported that an ω-transaminase derived from Ruegeria pomeroyi, Chromobacterium violaceum, Arthrobacter citreus, Sphaerobacter thermophilus, Aspergillus fischeri, Vibrio fluvialis, Agrobacterium tumefaciens, Mesorhizobium loti, or the like also has an amino group transfer activity to a diamine compound such as 1,8-diaminooctane and 1,10-diaminodecane, and can be preferably used (Sung et al., Green Chemistry 20.20 (2018): 4591-4595, Sattler et al., Angewandte Chemie 124.36 (2012): 9290-9293).

In the present invention, as the gene encoding an aminotransferase, for example, a gene encoding a protein consisting of an amino acid sequence set forth in any one of SEQ ID NOs: 110 to 114 is used. A putrescine aminotransferase ygjG gene derived from E. coli is preferably used. A base sequence of a coding region of the ygjG gene is set forth in SEQ ID NO: 115, and an amino acid sequence of ygjG is set forth in SEQ ID NO: 110.

**[Table 4-1]**

| Accession No. / origin (name of protein) | Amino acid sequence |
|---|---|
| BAE77123 / derived from Escherichia coli K-12 (ygjG) | MIREPPEHILNRLPSSASALACSAHALNLIEKRTLDHEEMKALNREVIEYFKEHVNP GFLEYRKSVTAGGDYGAVEWQAGSLNTLVDTQGQEFIDCLGGFGIFNVGHRNPVV VSAVQNQLAKQPLHSQELLDPLRAMLAKTLAALTPGKLKYSFFCNSGTESVEAAL KLAKAYQSPRGKFTFIATSGAFHGKSLGALSATAKSTFRKPFMPLLPGFRHVPFGNI EAMRTALNECKKTGDDVAAVILEPIQGEGGVILPPPGYLTAVRKLCDEFGALMILDE VQTGMGRTGKMFACEHENVQPDILCLAKALGGGVMPIGATIATEEVFSVLFDNPFL HTTTFGGNPLACAAALATINVLLEQNLPAQAEQKGDMLLDGFRQLAREYPDLVQE ARGKGMLMAIEFVDNEIGYNFASEMFRQRVLVAGTLNNAKTIRIEPPLTLTIEQCEL VIKAARKALAAMRVSVEEA (SEQ ID NO: 110) |
| AAG03688 / derived from Pseudomonas aeruginosa PAO1 | MNSQITNAKTREWQALSRDHHLPPFTDYKQLNEKGARIITKAEGVYIWDSEGNKIL DAMAGLWCVNVGYGREELVQAATRQMRELPFYNLFFQTAHPPVVELAKAIADVA PEGMNHVFFTGSGSEANDTVLRMVRHYWATKGQPQKKVVIGRWNGYHGSTVAG VSLGGMKALHEQGDFPIPGIVHIAQPYWYGEGGDMSPDEFGVWAAEQLEKKILEV GEENVAAFIAEPIQGAGGVIVPPDTYWPKIREILAKYDILFIADEVICGFGRTGEWFG SQYYGNAPDLMPIAKGLTSGYIPMGGVVVRDEIVEVLNQGGEFYHGFTYSGHPVA |
| | AAVALENIRILREEKIIEKVKAETAPYLQKRWQELADHPLVGEARGVGMVAALELV KNKKTRERFTDKGVGMLCREHCFRNGLIMRAVGDTMIISPPLVIDPSQIDELITLAR KCLDQTAAAVLA (SEQ ID NO: 111) |
| BAA16525 / derived from Escherichia coli K-12 (gabT) | MNSNKELMQRRSQAIPRGVGQIHPIFADRAENCRVWDVEGREYLDFAGGIAVLNT GHLHPKVVAAVEAQLKKLSHTCFQVLAYEPYLELCEIMNQKVPGDFAKKTLLVTT GSEAVENAVKIARAATKRSGTIAFSGAYHGRTHYTLALTGKVNPYSAGMGLMPGH VYRALYPCPLHGISEDDAIASIHRIFKNDAAPEDIAAIVIEPVQGEGGFYASSPAFMQ RLRALCDEHGIMLIADEVQSGAGRTGTLFAMEQMGVAPDLTTFAKSIAGGFPLAGV TGRAEVMDAVAPGGLGGTYAGNPIACVAALEVLKVFEQENLLQKANDLGQKLKD GLLAIAEKHPEIGDVRGLGAMIAIELFEDGDHNKPDAKLTAEIVARARDKGLILLSC GPYYNVLRILVPLTIEDAQIRQGLEIISQCFDEAKQ (SEQ ID NO: 112) |
| BAA14871 / derived from Escherichia coli K-12 (puuE) | MSNNEFHQRRLSATPRGVGVMCNFFAQSAENATLKDVEGNEYIDFAAGIAVLNTG HRHPDLVAAVEQQLQQFTHTAYQIVPYESYVTLAEKINALAPVSGQAKTAFFTTGA EAVENAVKIARAHTGRPGVIAFSGGFHGRTYMTMALTGKVAPYKIGFGPFPGSVYH VPYPSDLHGISTQDSLDAIERLFKSDIEAKQVAAIIFEPVQGEGGFNVAPKELVAAIRR LCDEHGIVMIADEVQSGFARTGKLFAMDHYADKPDLMTMAKSLAGGMPLSGVVG NANIMDAPAPGGLGGTYAGNPLAVAAAHAVLNIIDKESLCERANQLGQRLKNTLID AKESVPAIAAVRGLGSMIAVEFNDPQTGEPSAAIAQKIQQRALAQGLLLLTCGAYGN VIRFLYPLTIPDAQFDAAMKILQDALSD (SEQ ID NO: 113) |
| WP_011049154 / derived from Ruegeria pomeroyi | MATITNHMPTAELQALDAAHHLHPFSANNALGEEGTRVITRARGVWLNDSEGEEIL DAMAGLWCVNIGYGRDELAEVAARQMRELPYYNTFFKTTHVPAIALAQKLAELAP GDLNHVFFAGGGSEANDTNIRMVRTYWQNKGQPEKTVIISRKNAYHGSTVASSAL GGMAGMHAQSGLIPDVHHINQPNWWAEGGDMDPEEFGLARARELEEAILELGEN RVAAFIAEPVQGAGGVIVAPDSYWPEIQRICDKYDILLIADEVICGFGRTGNWFGTQ |
| | TMGIRPHIMTIAKGLSSGYAPIGGSIVCDEVAHVIGKDEFNHGYTYSGHPVAAAVAL ENLRILEEENILDHVRNVAAPYLKEKWEALTDHPLVGEAKIVGMMASIALTPNKAS RAKFASEPGTIGYICRERCFANNLIMRHVGDRMIISPPLVITPAEIDEMFVRIRKSLDE AQAEIEKQGLMKSAA (SEQ ID NO: 114) |

**[Table 4-2]**

| Base sequence of a region encoding ygjG |
|---|
| ATGATACGCGAGCCTCCGGAGCATATTTTGAACAGGTTACCTTCGAGCGCATCGGC TTTAGCGTGCAGCGCCCACGCCCTGAATCTCATTGAGAAGCGAACGCTGGATCAT GAGGAGATGAAAGCACTTAACCGAGAGGTGATTGAATACTTCAAAGAGCATGTCA ATCCGGGGTTTTTAGAGTATCGCAAATCTGTTACCGCCGGCGGGGATTACGGAGCC GTAGAGTGGCAAGCGGGAAGTTTAAATACGCTTGTCGACACCCAGGGCCAGGAG TTTATCGACTGCCTGGGAGGTTTTGGAATTTTCAACGTGGGGCACCGTAATCCAGT TGTGGTTTCCGCCGTACAGAATCAACTTGCGAAACAACCGCTGCACAGCCAGGAG CTGCTCGATCCGTTACGGGCGATGTTGGCGAAAACCCTTGCTGCGCTAACGCCCG GTAAACTGAAATACAGCTTCTTCTGTAATAGCGGCACCGAGTCCGTTGAAGCAGC GCTGAAGCTGGCGAAAGCTTACCAGTCACCGCGCGGCAAGTTTACTTTTATTGCC ACCAGCGGCGCGTTCCACGGTAAATCACTTGGCGCGCTGTCGGCCACGGCGAAAT CGACCTTCCGCAAACCGTTTATGCCGTTACTGCCGGGCTTCCGTCATGTGCCGTTT GGCAATATCGAAGCCATGCGCACGGCTCTTAACGAGTGCAAAAAAACCGGTGATG ATGTGGCTGCGGTGATCCTCGAACCGATTCAGGGTGAAGGTGGCGTAATTCTGCC GCCGCCGGGCTATCTCACCGCCGTACGTAAGCTATGCGATGAGTTCGGCGCACTGA TGATCCTCGATGAAGTACAAACGGGCATGGGGCGCACGGGCAAGATGTTCGCCTG CGAGCATGAGAACGTACAGCCGGATATCCTCTGCCTTGCCAAAGCGCTCGGCGGC |
| GGCGTGATGCCGATTGGCGCGACCATCGCCACTGAAGAGGTGTTTCAGTTCTGTT CGACAACCCATTCCTGCATACCACCACCTTTGGCGGCAACCCGCTGGCCTGTGCG GCGGCGCTGGCGACCATCAATGTGTTGCTGGAGCAGAACTTACCGGCTCAGGCTG AGCAAAAAGGCGATATGTTGCTGGACGGTTTCCGTCAACTGGCGCGGGAATATCC CGATCTGGTACAGGAAGCGCGTGGTAAAGGGATGTTGATGGCGATTGAGTTTGTT GATAACGAAATCGGCTATAACTTTGCCAGCGAGATGTTCCGCCAGCGCGTACTGGT GGCCGGAACGCTCAATAACGCCAAAACGATCCGCATTGAACCGCCACTGACACTG ACCATTGAACAGTGTGAACTGGTGATCAAAGCGGCGCGTAAGGCGCTGGCGGCC ATGCGAGTAAGTGTCGAAGAAGCGTAA (SEQ ID NO: 115) |

The gene encoding the enzyme that can be used in the present invention may be derived from an organism other than the exemplified organism or artificially synthesized, and may be any gene that can express a substantial enzyme activity in a host microorganism cell.

In addition, the enzyme gene that can be used for the present invention may have all naturally occurring mutations or artificially introduced mutations and modifications as long as it can express a substantial enzyme activity in the host microorganism cell. For example, it is known that extra codons are present in various codons encoding a specific amino acid. Therefore, in the present invention, alternative codons that are to be finally translated into the same amino acid may also be used. That is, since a genetic code degenerates, a plurality of codons can be used to encode a particular amino acid, so that the amino acid sequence can be encoded by an arbitrary set of similar DNA oligonucleotides. Only the member of the set is identical to the gene sequence of the natural enzyme; however, even mismatched DNA oligonucleotides can hybridize to natural sequences under an appropriate stringent condition (for example, hybridize at 3xSSC and 68°C and wash at 2xSSC and 0.1%SDS and 68°C), DNA encoding a natural sequence can by identified and isolated, and such a gene can also be used in the present invention. In particular, since most organisms are known to preferentially use a subset of a specific codon (optimal codon) (Gene, Vol. 105, pp. 61-72, 1991, and the like), performing of "codon optimization" depending on a host microorganism can also be useful in the present invention.

Therefore, the genetically modified microorganism according to the present invention can contain a base sequence having, for example, 80%, 85%, 90%, 95%, 97%, 98%, or 99% or more of sequence identity with the base sequence of the enzyme gene under a condition in which a substantial enzyme activity can be expressed. Alternatively, the genetically modified microorganism according to the present invention can contain a base sequence having, for example, 80%, 85%, 90%, 95%, 97%, 98%, or 99% or more of sequence identity with the base sequence encoding the amino acid sequence of the enzyme.

In the present invention, when the diamine compound synthetase gene group is introduced in a host microorganism cell as an "expression cassette", a more stable and high level of enzyme activity can be obtained. In the present specification, the "expression cassette" refers to a nucleotide containing a nucleic acid sequence that is functionally linked to a nucleic acid to be expressed or a gene to be expressed and regulates transcription and translation. Typically, the expression cassette of the present invention contains a promoter sequence on the 5' upstream from the coding sequence, a terminator sequence on the 3' upstream, and an optionally additional normal regulatory element in a functionally linked state, and in such a case, a nucleic acid to be expressed or a gene to be expressed is introduced into a host microorganism.

The promoter is defined as a DNA sequence that allows RNA polymerase to bind to DNA to initiate RNA synthesis, regardless of whether the promoter is a constitutive expression promoter or an inductive expression promoter. A strong promoter is a promoter that initiates mRNA synthesis at a high frequency, and is also preferably used in the present invention. In E. coli, a major operator and promoter region of a lac system, a trp system, a tac or trc system, or A-phage, a control region for fd coat protein, a promoter for a glycolytic enzyme (for example, 3-phosphoglycerate kinase or glyceraldehyde-3-phosphate dehydrogenase), glutamate decarboxylase A, or serine hydroxymethyltransferase, a promoter region of RNA polymerase derived from T7 phage, and the like can be used. In Corynebacterium glutamicum, a high-level constitutive expression (HCE) promoter, a cspB promoter, a sodA promoter, an elongation factor Tu (EF-Tu) promoter, and the like can be used. As the terminator, a T7 terminator, an rrnBT1T2 terminator, a lac terminator, and the like can be used. In addition to the promoter and terminator sequences, other examples of regulatory elements may include a selection marker, an amplification signal, and a replication point. A preferred regulatory sequence is described in, for example, "Gene Expression Technology: Methods in Enzymology 185" Academic Press (1990)".

The expression cassette described above is incorporated into a vector consisting of, for example, a plasmid, a phage, a transposon, an IS element, a fosmid, a cosmid, or a linear or cyclic DNA and is inserted into a host microorganism. A plasmid and a phage are preferred. The vector may be self-replicated in a host microorganism or may be replicated by a chromosome. Examples of a preferred plasmid include pLG338, pACYC184, pBR322, pUC18, pUC19, pKC30, pRep4, pHS1, pKK223-3, pDHE19.2, pHS2, pPLc236, pMBL24, pLG200, pUR290, pIN-III113-B1, λgt11, or pBdCI of E. coli; pUB110, pC194, or pBD214 of a bacillus; and pSA77 or pAJ667 of the genus Corynebacterium. Other plasmids that can also be used are described in "Cloning Vectors", Elsevier, 1985. The expression cassette can be introduced into the vector by a conventional method of including cutting with an appropriate restriction enzyme, cloning, and ligation. Each expression cassette may be located on one vector or on two or more vectors.

After the vector having the expression cassette of the present invention is constructed as described above, a conventional method can be used as a method that can be applied when the vector is introduced into a host microorganism. Examples of the method include, but are not limited to, a calcium chloride method, an electroporation method, a conjugation transfer method, and a protoplast fusion method, and a method suitable for a host microorganism can be selected.

The modified microorganism obtained as described above is cultured and maintained under conditions suitable for growth and/or maintenance of the modified microorganism for production of the diamine compound of the present invention. A medium composition, culture conditions, and culture time suitable for the modified microorganisms derived from various host microorganisms can be easily set by those skilled in the art.

Another embodiment of the present invention relates to a method of producing a diamine compound using the modified microorganism described above. The method of producing a diamine includes, for example, the following steps.

### (a) Culture step

The method of producing a diamine compound includes a culture step of culturing the modified microorganism according to the embodiment described above. For example, the modified microorganism is cultured in a medium containing a carbon source and a nitrogen source to obtain a culture medium containing bacterial cells.

The present production method may include bringing the genetically modified microorganism into contact with a precursor of a diamine compound. Examples of a method of supplying the precursor of the diamine compound to the modified microorganism include a method of producing a precursor of a diamine compound in a modified microorganism and a method of supplying a precursor of a diamine compound from the outside of a cell without relying on a modified microorganism.

In the culture step, in a case where the modified microorganism is brought into contact with a precursor of a diamine compound, the medium may further contain a precursor, or a precursor may be added to the medium during the culture step.

The medium may be a natural, semi-synthetic, or synthetic medium containing one or more carbon sources, nitrogen sources, inorganic salts, vitamins, and optionally a trace component such as a trace element or vitamin. However, the medium to be used is required to appropriately satisfy nutritional requirements of microorganisms to be cultured.

Examples of the carbon source include D-glucose, sucrose, lactose, fructose, maltose, oligosaccharides, polysaccharides, starch, cellulose, rice bran, waste molasses, fats and oils (for example, soybean oil, sunflower oil, peanut oil, palm oil, and the like), fatty acids (for example, palmitic acid, linoleic acid, oleic acid, linolenic acid, and the like), alcohols (for example, glycerol, ethanol, and the like), and organic acids (for example, acetic acid, lactic acid, succinic acid, and the like). Furthermore, the carbon source may be biomass containing D-glucose. Examples of preferred biomass include a corn decomposition liquid and a cellulose decomposition liquid. These carbon sources can be used alone or as a mixture.

The diamine compound produced using a raw material derived from biomass can be clearly distinguished from a synthetic raw material derived from, for example, petroleum, natural gas, or coal, by measurement of a biomass carbon content based on Carbon-14 (radiocarbon) analysis defined in ISO 16620-2 or ASTM D6866.

Examples of the nitrogen source include nitrogen-containing organic compounds (for example, peptone, casamino acid, tryptone, a yeast extract, a meat extract, a malt extract, a corn steep liquor, soy flour, an amino acid, urea, and the like) and inorganic compounds (for example, an aqueous ammonia solution, ammonium sulfate, ammonium chloride, ammonium phosphate, ammonium carbonate, sodium nitrate, ammonium nitrate, and the like). These nitrogen sources can be used alone or as a mixture.

In addition, the medium may contain a corresponding antibiotic in a case where a modified microorganism expresses a useful additional trait, for example, in a case where a modified microorganism contains a marker resistant to an antibiotic. Therefore, a risk of contamination by various bacteria during culture is reduced. Examples of the antibiotic include, but are not limited to, a β-lactam antibiotic such as ampicillin, an aminoglycoside antibiotic such as kanamycin, a macrolide antibiotic such as erythromycin, a tetracycline antibiotic, and chloramphenicol.

The "precursor" of the diamine compound refers to a compound that can induce a diamine compound by the enzyme involved in synthesis of a diamine compound of the present invention. Examples of the precursor include, but are not limited to, a dicarboxylic acid, a carboxylic acid semialdehyde, a dialdehyde, an aminocarboxylic acid, an aminoaldehyde, acyl-ACP, acyl-CoA, and acyl phosphate.

As a specific precursor that can induce hexamethylenediamine, for example, adipic acid, adipic acid semialdehyde, adipaldehyde, 6-aminohexanoic acid, 6-aminohexanal, adipyl-CoA, adipyl phosphate, or the like can be used.

For example, in a case where a diamine compound is hexamethylenediamine, the modified microorganism is brought into contact with adipic acid that is a precursor, such that the adipic acid is converted into hexamethylenediamine by a carboxylic acid reductase and an aminotransferase produced by the modified microorganism.

In addition, for example, in a case where a diamine compound is 1,10-decanediamine, the modified microorganism is brought into contact with sebacic acid that is a precursor, such that the sebacic acid is converted into 1,10-decanediamine by a carboxylic acid reductase and an aminotransferase produced by the modified microorganism.

As the precursor, one precursor may be used, or two or more precursors may be combined. In addition, in a case where the compound is a compound that can adopt a salt form, the precursor may be used as a salt, a free form, or a mixture thereof.

The method of producing a precursor is not particularly limited, and a precursor can be produced by, for example, a chemical synthesis method, an enzyme method, a bioconversion method, a fermentation method, or a combination thereof.

In the culture step, the genetically modified microorganism of the present invention can be brought into contact with a precursor of a diamine compound to generate and accumulate a diamine compound in a medium, thereby producing a diamine compound. In addition, as described below, in a reaction step, the genetically modified microorganism of the present invention may be allowed to act in an aqueous solution containing a precursor of a diamine compound to produce and accumulate a diamine compound in the reaction solution.

### (b) Reaction step

The present step is a step of bringing a precursor of a diamine compound into contact with the modified microorganism to produce a target diamine compound from the precursor of the diamine compound. The contact of the precursor of the diamine compound may be performed, for example, in the culture step described above, or may be performed after the culture step. In a case where the present reaction step is performed after the culture step, the culture medium and/or bacterial cells obtained in the culture step can be brought into contact with an aqueous solution containing a precursor of a diamine compound to obtain a reaction solution containing a diamine compound. The diamine compound is produced and accumulated in the reaction solution by being brought into contact with such a precursor.

In one aspect, in the present step, the culture medium containing the bacterial cells obtained in the culture step and/or the bacterial cells from which a supernatant is removed by centrifugation or the like from the culture medium obtained in the culture step are brought into contact with an aqueous solution containing a precursor to obtain a reaction solution.

In addition, in another aspect, bacteria that produce a precursor by fermentation and the modified microorganism according to the present invention may be co-cultured. By co-culturing the bacteria and the modified microorganism, the precursor produced by the bacteria can be efficiently converted into a target diamine compound by the enzyme produced by the modified composition according to the present invention.

In still another aspect, the genetically modified microorganism according to the present invention is allowed to have an ability of producing a precursor of a diamine compound so that a diamine compound may be produced and accumulated from components in the medium.

As an example of a precursor production pathway by the genetically modified microorganism, a method of producing 6-aminohexanoic acid using glucose as a starting material (Turk et al., ACS synthetic biology 5.1 (2015): 65-73) and a method of producing adipic acid using glucose or glycerol as a starting material (Zhao et al., Metabolic engineering 47 (2018): 254-262) are disclosed, and the method is not limited as long as a precursor can be produced.

For example, the modified microorganism has an ability of producing a dicarboxylic acid, a carboxylic acid semialdehyde, or an aminocarboxylic acid, and further expresses an aminotransferase and a carboxylic acid reductase so that a diamine compound can be produced.

In addition, for example, the modified microorganism has an ability of producing adipic acid, adipic acid semialdehyde, or 6-aminohexanoic acid, and further expresses an aminotransferase and a carboxylic acid reductase so that hexamethylenediamine can be produced.

According to the genetically modified microorganism and the method of producing a diamine compound using the microorganism of the present invention, production of a by-product can be suppressed, and a diamine compound can be more efficiently produced. Specifically, for example, the microorganism that expresses an enzyme required to produce a diamine compound is modified to reduce an activity of an alcohol dehydrogenase compared to a non-reduced strain, thereby suppressing production of an alcohol form that is a by-product and efficiently producing a diamine compound.

Hereinafter, examples are given for the purpose of further description, and the present invention is not limited to the examples. In the present specification, unless otherwise specified, nucleotide sequences are described from 5' directions to 3' direction.

### Examples

Hereinafter, the present invention will be explained based on examples, but the present invention is not limited to these examples.

### 1: Construction of ADH gene-disrupted strain

### <1-a Construction of plasmid for disrupting gene>

Disruption of an ADH gene was performed by a homologous recombination method using pHAK1 (deposited with biotechnology division of National Institute of Technology and Evaluation (NITE), Patent Microorganisms Depositary (NPMD) (address: #122, 2-5-8, Kazusakamatari, Kisarazu-shi, Chiba, Japan) on March 18, 2019, under the accession number NITE P-02919). pHAK1 contains a temperature-sensitive variant repA gene, a kanamycin resistant gene, and a levansucrase gene SacB derived from Bacillus subtilis. The levansucrase gene lethally acts on a host microorganism under the presence of sucrose. The PCR fragment was amplified using PrimeSTAR Max DNA Polymerase (trade name, manufactured by TAKARA BIO INC.), and the plasmid was prepared using an E. coli HST08 strain. Using the genomic DNA of the E. coli BL21 (DE3) strain as a template, a PCR product containing an upstream region, a coding region, and a downstream region of a disruption target gene was obtained. The combinations of the target gene and the primer sequence are shown in the following table.

**[Table 5]**

| Target gene | Forward primer (SEQ ID NO) | Reverse primer (SEQ ID NO) |
|---|---|---|
| yqhD | TTGTTGAATAAATCGTCTGTTTGCCGAGAATAC GCG (SEQ ID NO: 116) | TATCGCATGCAGATCTGCGCTAATGGGCTCCAGGA (SEQ ID NO: 117) |
| fucO | TTGTTGAATAAATCGCGCGCCAGCGCTGGCTGT TT (SEQ ID NO: 118) | TATCGCATGCAGATCCCGCCAATGCCGGAAGAGTT (SEQ ID NO: 119) |
| adhP | TTGTTGAATAAATCGCGATCGTGATGCCGCTGT CT (SEQ ID NO: 120) | TATCGCATGCAGATCGACAACGTAGGCTTTGTTCA (SEQ ID NO: 121) |
| eutG | TTGTTGAATAAATCGCGCCATCTCGACACTCTT GA (SEQ ID NO: 122) | TATCGCATGCAGATCGCGAACATCGATGGGTTAGC (SEQ ID NO: 123) |
| ybbO | TTGTTGAATAAATCGTGGCATTTGCCCTTCCTG TT (SEQ ID NO: 124) | TATCGCATGCAGATCGTCCTGATCCTGCAACGGAA (SEQ ID NO: 125) |
| ahr | TTGTTGAATAAATCGCTCATAACGGTACTGCAA AC (SEQ ID NO: 126) | TATCGCATGCAGATCTCGCAGCAGGTAAGATGATT (SEQ ID NO: 127) |
| yahK | TTGTTGAATAAATCGATATTCGTCCTAACGAAC AG (SEQ ID NO: 128) | TATCGCATGCAGATCTTTTTGATTTTCAAGTATGT (SEQ ID NO: 129) |

Next, the present PCR product was inserted into the pHAK1 plasmid fragment amplified using primers of SEQ ID NOs: 130 and 131 using an In-Fusion HD cloning kit (trade name, manufactured by Clontech Laboratories, Inc.) to circularize it.

**[Table 6]**

| |
|---|
| GATCTGCATGCGATATCTCTAGAACGCGTAAGCTT (SEQ ID NO: 130) |
| TCTCGAGCCGATTTATTCAACAAAGCCGC (SEQ ID NO: 131) |

PCR was performed using the pHAK1 plasmid into which the DNA fragment containing the upstream region, the coding region, and the downstream region of the obtained disruption target gene as a template and using primers shown in the following table, thereby obtaining a plasmid fragment in which the coding region of the disruption target gene was partially or entirely removed.

**[Table 7]**

| Target gene | Forward primer (SEQ ID NO) | Reverse primer (SEQ ID NO) |
|---|---|---|
| yqhD | ACTTTCGTTTTCGGGCATTTCGTCC (SEQ ID NO: 132) | CCAATATGAGGGCAGAGAACGATC SEQ ID NO: 133) |
| fucO | ATGCGCTGATGTGATAATGC (SEQ ID NO: 134) | CCTTCTCCTTGTTGCTTTA (SEQ ID NO: 135) |
| adhP | GAGGCCTTTGCTGCGACTGC (SEQ ID NO: 136) | AGTTCCTCCTTTTCGGATGAT (SEQ ID NO: 137) |
| eutG | ATGCCGGATGCGACGCTT (SEQ ID NO: 138) | TCATTTTGCATATAGCCCCT (SEQ ID NO: 139) |
| ybbO | TGACCTGGGCAGTAATGGTG (SEQ ID NO: 140) | CAGGATCTCCGTTGCTTTATGAGTC (SEQ ID NO: 141) |
| ahr | CGTGGTGTTGAAAGCCGATTATTG (SEQ ID NO: 142) | CATAAACTTCCAGTTCTCCGCCC (SEQ ID NO: 143) |
| yahK | TCGCACACTAACAGACTGAA (SEQ ID NO: 144) | TGTGTTTACTCCTGATTAGC (SEQ ID NO: 145) |

The obtained plasmid fragment was subjected to terminal phosphorylation and circularization by self-ligation to obtain a plasmid for disrupting a gene.

### <1-b Construction of ADH gene-disrupted E. coli strain>

A plasmid for disrupting a desired gene was transformed into the E. coli BL21 (DE3) strain by a calcium chloride method (refer to Genetic Engineering Laboratory Notebook, by Takaaki Tamura, Yodosha), and then applied to an LB agar medium (10 g/L of tryptone, 5 g/L of yeast extract, 5 g/L of sodium chloride, and 15 g/L of agar powder) containing 100 mg/L of kanamycin sulfate, and culture was performed at 30°C overnight to obtain a single colony, thereby obtaining a transformant. The present transformant was inoculated into 1 mL of an LB liquid medium (10 g/L of tryptone, 5 g/L of yeast extract, and 5 g/L of sodium chloride) containing 100 mg/L of kanamycin sulfate with a platinum loop, and shaking culture was performed at 30°C. The obtained culture medium was applied to an LB agar medium containing 100 mg/L of kanamycin sulfate, and culture was performed at 42°C overnight. In the obtained colony, a plasmid was inserted into the genome by single crossover. The colony was inoculated into 1 mL of an LB liquid medium with a platinum loop, and shaking culture was performed at 30°C. The obtained culture medium was applied to an LB agar medium containing 10% sucrose, and culture was performed overnight. Disruption of a desired gene in the obtained colony was observed by colony direct PCR using the primer set shown in Table 8. The constructed ADH gene-disrupted E. coli strain is shown in Table 9. In the table, Δ indicates that the enzyme gene is deficient.

**[Table 8]**

| Target gene | Forward primer (SEQ ID NO) | Reverse primer (SEQ ID NO) |
|---|---|---|
| yqhD | TATTCTCAATCCGTTTCAGCACGCG (SEQ ID NO: 146) | TTCGGGATCACCACCAGGCCG (SEQ ID NO: 147) |
| fucO | CGGAAATGGACGAACAGTGG (SEQ ID NO:148) | CGTCATCAGCGTTTACCAGATT (SEQ ID NO: 149) |
| adhP | GCATAAACACTGTCCGCGTC (SEQ ID NO:150) | GAAATCGAGAAGGCAGAAGCGAAA (SEQ ID NO: 151) |
| eutG | GGTGCGGTCACCATTGTTCG (SEQ ID NO:152) | CATATCGCACGCCAGCAGTG (SEQ ID NO: 153) |
| ybbO | GGTGAGGATGGAGAGTTCATG (SEQ ID NO:154) | CAGTTCGATTTGCGCCACCAG (SEQ ID NO: 155) |
| ahr | TCCGCTAGTGTGATTTCAGG (SEQ ID NO: 156) | GAAATTATTATGCCGCCAGGCGT (SEQ ID NO: 157) |
| yahK | TTATGGTCTGGGCGACATGC (SEQ ID NO: 158) | GCATCATCCTGGTCATATACCC (SEQ ID NO: 159) |

**[Table 9]**

| | |
|---|---|
| BL21(DE3) | *ΔyqhD* |
| BL21(DE3) | *ΔfucO* |
| BL21(DE3) | *ΔadhP* |
| BL21(DE3) | *ΔeutG* |
| BL21(DE3) | *ΔybbO* |
| BL21(DE3) | *Δahr* |
| BL21(DE3) | *ΔyahK* |
| BL21(DE3) | *ΔyqhD ΔfucO* |
| BL21(DE3) | *ΔyqhD ΔadhP* |
| BL21(DE3) | *ΔyqhD ΔeutG* |
| BL21(DE3) | *ΔyqhD ΔybbO* |
| BL21(DE3) | *ΔyqhD Δahr* |
| BL21(DE3) | *ΔyqhD ΔyahK* |
| BL21(DE3) | *ΔyqhD ΔfucO ΔadhP* |
| BL21(DE3) | *ΔyqhD ΔfucO ΔadhP ΔeutG* |
| BL21 (DE3) | *ΔyqhD ΔfucO ΔadhP ΔeutG ΔybbO* |
| BL21(DE3) | *ΔyqhD ΔfucO ΔadhP ΔeutG ΔybbO Δahr* |
| BL21(DE3) | *ΔyqhD ΔfucO ΔadhP ΔeutG ΔybbO Δahr ΔyahK* |

### <1-c Test for reducing decomposition activity of 1,6-hexanediol>

The reduction in decomposition activity of 1,6-hexanediol of the constructed ADH gene-disrupted E. coli strain was confirmed by the progress of the oxidation reaction of 1,6-hexanediol. 1,6-Hexanediol is one of alcohol forms that are by-products of the production reaction of hexamethylenediamine. In this test, based on the fact that the reaction between the aldehyde and the alcohol catalyzed by ADH illustrated in Fig. 1 is a reversible reaction, a conversion reaction from the alcohol (here, 1,6-hexanediol) to the aldehyde was focused on, and the consumption of 1,6-hexanediol was used as an index of the decomposition activity of 1,6-hexanediol by ADH. In this test, the bacterial cells of each of the ADH gene-disrupted E. coli strains were inoculated into 2 mL of an LB liquid medium with a platinum loop, and shaking culture was performed at 37°C overnight as pre-culture. The obtained pre-culture medium was inoculated into 2 mL of an LB liquid medium containing 10 mM 1,6-hexanediol in an amount corresponding to 1%, and shaking culture was performed at 37°C for 48 hours as a main culture. The culture medium was separated into the bacterial cells and the supernatant by centrifugation, and a concentration of 1,6-hexanediol in the supernatant was analyzed.

The analysis of the concentration of 1,6-hexanediol was performed using gas chromatograph.

The conditions are as follows.
GC system: GC-2010 (manufactured by Shimadzu Corporation)
Detector: Hydrogen flame ionization detector
Column: DB-WAX (manufactured by Agilent Technologies, column length: 30 m, inner diameter: 0.25 mm, film thickness: 0.25 mm)
Carrier gas: He
Gas pressure: 100 kPa
Column temperature: 50°C-(25°C/min)-230°C-(holding for 20 min)
Detector temperature: 250°C
Inlet temperature: 250°C
Injection amount: 1 µL
Injection method: Split injection method (split ratio: 36.3)

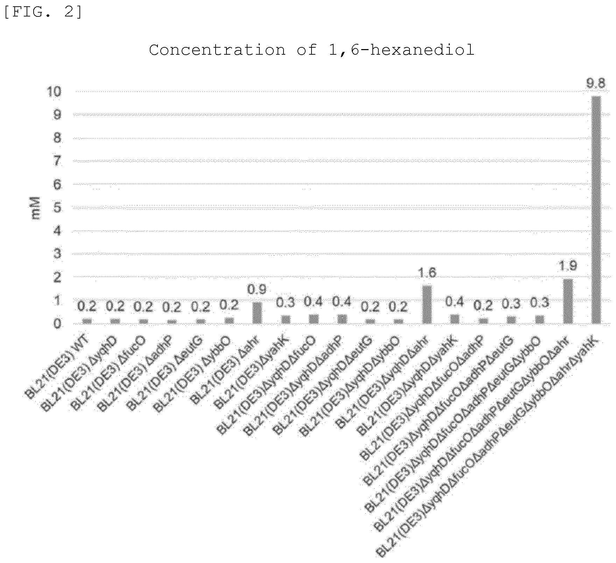

The concentration of 1,6-hexanediol in the culture supernatant 48 hours after the main culture is shown in Fig. 2. In the wild-type strain (BL21(DE3) strain, WT is an abbreviation of Wild type and indicates wild type) in which the ADH gene was not disrupted, 1,6-hexanediol was consumed by the action of the ADH, whereas in the ADH gene-disrupted strain, particularly regarding two genes: the ahr gene and the yahK gene, consumption of 1,6-hexanediol was suppressed by single gene disruption. It was confirmed from the present results that the decomposition activity of 1,6-hexanediol was reduced in the ADH gene-disrupted strain.

### 2: Production of diamine compound in ADH gene-disrupted strain

### <2-a Construction of MaCar gene, Npt gene, and ygjG gene expression plasmids>

The PCR fragment was amplified using PrimeSTAR Max DNA Polymerase (trade name, manufactured by TAKARA BIO INC.), and the plasmid was prepared using an E. coli JM109 strain. PCR was performed using the genome DNA of the Eshcherichia coli W3110 strain (NBRC12713) as a template, and using oligonucleotides of SEQ ID NOs: 160 and 161 as primers, thereby obtaining a PCR product containing a coding region of a ygjG gene. Next, the present PCR product was inserted between restriction enzymes NcoI and HindIII cleavage sites of plasmid pACYCDuet (trademark)-1 (trade name, manufactured by Merck & Co., Inc.) using an In-Fusion HD cloning kit (trade name, manufactured by Clontech Laboratories, Inc.), and the PCR product was named "pDA50".

**[Table 10]**

| |
|---|
| ATAAGGAGATATACCATGATACGCGAGCCTCCGGA (SEQ ID NO: 160) |
| ATGCGGCCGCAAGCTTTACGCTTCTTCGACACTTA (SEQ ID NO: 161) |

PCR was performed using the genome DNA of the Mycobacterium abscessus JCM13569 strain (provided by RIKEN BRC through Ministry of Education, Culture, Sports, Science and Technology/the National BioResource Project of Japan Agency for Medical Research and Development) as a template and using oligonucleotides of SEQ ID NOs: 162 and 163 as primers, thereby obtaining a PCR product containing a coding region of an MaCar gene. Next, the PCR product was inserted between restriction enzymes NdeI and AvrII cleavage sites of pDA50 using an In-Fusion HD cloning kit (trade name, manufactured by Clontech Laboratories, Inc.), and the PCR product was named "pDA52".

**[Table 11]**

| |
|---|
| TAAGAAGGAGATATACATATGACTGAAACGATCTC (SEQ ID NO: 162) |
| GTGGCAGCAGCCTAGCTACACCAGGCCCAACAGCT (SEQ ID NO: 163) |

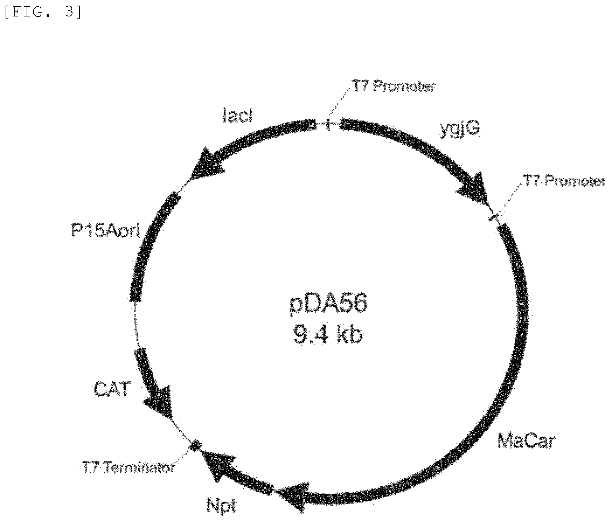

PCR was performed using the genome DNA of the Nocardia iowensis JCM18299 strain (provided by RIKEN BRC through Ministry of Education, Culture, Sports, Science and Technology/the National BioResource Project of Japan Agency for Medical Research and Development) as a template and using oligonucleotides of SEQ ID NOs: 164 and 165 as primers, thereby obtaining a PCR product containing a coding region of an Npt gene. Next, PCR was performed using pDA52 as a template and using oligonucleotides of SEQ ID NOs: 166 and 167 as primers, thereby obtaining a pDA52 fragment. The PCR products were connected to each other using an In-Fusion HD cloning kit (trade name, manufactured by Clontech Laboratories, Inc.). The plasmid was extracted from the obtained transformant, and the product into which the Npt gene was inserted was named "pDA56". The plasmid map of pDA56 is illustrated in Fig. 3.

**[Table 12]**

| |
|---|
| TTTAAGGAGTTCGATATGATCGAGACAATTTTGCC (SEQ ID NO: 164) |
| GGTGGCAGCAGCCTAGTCAGGCGTACGCGATCGCG (SEQ ID NO: 165) |
| CTAGGCTGCTGCCACCGCTG (SEQ ID NO: 166) |
| ATCGAACTCCTTAAATTTATCTACACCAGGCCCAACAGCT (SEQ ID NO: 167) |

### <2-b Preparation of transformant>

pDA56 was introduced into an ADH gene non-disrupted E. coli strain or an ADH gene-disrupted strain by a calcium chloride method (refer to Genetic Engineering Laboratory Notebook, by Takaaki Tamura, Yodosha), and culture was performed in an LB agar medium containing 34 mg/L of chloramphenicol overnight, thereby obtaining a transformant. The obtained transformants were named transformants A to S as shown in the following table. As shown in the table, the transformant A is an ADH gene non-disrupted strain, the transformants B to H are strains in which any one of genes encoding an ADH are disrupted, and the transformants I to S are strains in which at least two of genes encoding ADH are disrupted (multiply disrupted).

**[Table 13]**

| Name of transformant | Microorganism strain | Plasmid |
|---|---|---|
| A | BL21(DE3) | |
| B | BL21(DE3) *ΔyqhD* | |
| C | BL21(DE3) *ΔfucO* | |
| D | BL21(DE3) *ΔadhP* | |
| E | BL21(DE3) *ΔeutG* | |
| F | BL21(DE3) *ΔybbO* | |
| G | BL21(DE3) *Δahr* | |
| H | BL21(DE3) *ΔyahK* | |
| I | BL21(DE3) *ΔyqhDΔfucO* | |
| J | BL21(DE3) *ΔyqhDΔadhP* | pDA56 |
| K | BL21(DE3) *ΔyqhDΔeutG* | |
| L | BL21(DE3) *ΔyqhDΔybbO* | |
| M | BL21(DE3) *ΔyqhDΔahr* | |
| N | BL21(DE3) *ΔyqhDΔyahK* | |
| O | BL21(DE3) *ΔyqhDΔfucOΔadhP* | |
| P | BL21(DE3) *ΔyqhDΔfucOΔadhPΔeutG* | |
| Q | BL21(DE3) *ΔyqhDΔfucOΔadhPΔeutGΔybbO* | |
| R | BL21(DE3) *ΔyqhDΔfucOΔadhPΔeutGΔybbOΔahr* | |
| S | BL21(DE3) *ΔyqhDΔfucOΔadhPΔeutGΔybbOΔahrΔyahK* | |

### <2-c Production of hexamethylenediamine from adipic acid (Comparative example 1 and Examples 1 to 18)>

The bacterial cells of the transformants A to S were inoculated in 2 mL of an LB liquid medium containing 34 mg/L of chloramphenicol with a platinum loop, and shaking culture was performed at 37°C overnight as pre-culture. The obtained pre-culture medium was inoculated in 1 mL of an SOB liquid medium (20 g/L of tryptone, 5 g/L of yeast extract, 0.5 g/L of sodium chloride, 2.5 mM calcium chloride, 10 mM magnesium sulfate, and 10 mM magnesium chloride) containing 50 mM diammonium adipate, 34 mg/L of chloramphenicol, and 2% glucose in an amount corresponding to 1%, and shaking culture was performed at 37°C. After 2 hours of culture, isopropyl β-thiogalactopyranoside (IPTG) was added so that the final concentration was 0.2 mM, and shaking culture was performed at 30°C for 48 hours. The culture medium was separated into the bacterial cells and the supernatant by centrifugation, and a concentration of hexamethylenediamine and a concentration of 1,6-hexanediol in the supernatant were analyzed.

The analysis of the concentration of hexamethylenediamine was performed using ion chromatograph. The conditions are as follows.
Apparatus: ICS-3000 (manufactured by Dionex Corporation)
Detector: Electrical conductivity detector
Column: IonPac CG19 (2 × 50 mm)/CS19(2 × 250 mm) (manufactured by Thermo Fisher Scientific)
Oven temperature: 30°C
Mobile phase: 8 mM aqueous methanesulfonic acid solution (A), 70 mM aqueous methanesulfonic acid solution (B)
Gradient condition: (A: 100%, B: 0%) - (10 min)-(A: 0%, B: 100%)-(holding for 1 min)
Flow rate: 0.35 mL/min
Injection amount: 20 µL

The concentration of 1,6-hexanediol was performed using gas chromatograph under the same conditions as in (1-c).

The concentration of hexamethylenediamine and the concentration of 1,6-hexanediol in each of the culture media are shown in Table 14. First, as for the concentration of hexamethylenediamine, an increase in production amount of hexamethylenediamine was observed in the ADH gene-disrupted strains of Examples 1, 3, and 8 to 18 compared to the ADH gene non-disrupted strain (Comparative example 1). In addition, in the ADH gene-disrupted strains of Examples 1, 2, and 4 to 18, the production amount of 1,6-hexanediol was reduced. In addition, the production amount of hexamethylenediamine was further increased by multiple disruption of the ADH gene (Examples 8 to 18). Regarding the concentration of 1,6-hexanediol, it was observed that the production was suppressed compared to the ADH gene non-disrupted strain (Comparative example 1).

**[Table 14]**

| | Transformant | Concentration of hexamethylenediamine (µM) | Concentration of 1,6-hexanediol (mM) |
|---|---|---|---|
| Comparative example 1 | A | 2 | 1.70 |
| Example 1 | B | 10 | 0.22 |
| Example 2 | C | 2 | 1.41 |
| Example 3 | D | 4 | 2.01 |
| Example 4 | E | 2 | 1.57 |
| Example 5 | F | 1 | 1.18 |
| Example 6 | G | 2 | 1.30 |
| Example 7 | H | 2 | 1.28 |
| Example 8 | I | 38 | 0.27 |
| Example 9 | J | 81 | 0.38 |
| Example 10 | K | 48 | 0.29 |
| Example 11 | L | 18 | 0.18 |
| Example 12 | M | 69 | 0.08 |
| Example 13 | N | 63 | 0.26 |
| Example 14 | O | 68 | 0.31 |
| Example 15 | P | 54 | 0.28 |
| Example 16 | Q | 42 | 0.24 |
| Example 17 | R | 38 | 0.09 |
| Example 18 | S | 47 | 0.05 |

### <2-d Production of 1,10-decanediamine from sebacic acid (Comparative example 2 and Examples 19 and 20)>

The bacterial cells of the transformants A to S were inoculated in 2 mL of an LB liquid medium containing 34 mg/L of chloramphenicol with a platinum loop, and shaking culture was performed at 37°C overnight as pre-culture. The obtained pre-culture medium was inoculated in 1 mL of an SOB liquid medium containing 50 mM sodium sebacate, 34 mg/L of chloramphenicol, and 2% glucose in an amount corresponding to 1%, and shaking culture was performed at 37°C. After 2 hours of culture, IPTG was added so that the final concentration was 0.2 mM, and shaking culture was performed at 30°C for 48 hours. The culture medium was separated into the bacterial cells and the supernatant by centrifugation, and a concentration of 1,10-decanediamine and a concentration of 1,10-decanediol in the supernatant were analyzed.

The analysis of the concentration of 1,10-decanediamine was performed by ion chromatography under the same conditions as in (2-c). The concentration of 1,10-decanediamine in each of the culture media is shown in Table 15. It was observed that the production amount of 1,10-diaminodecane in the ADH gene-disrupted strain of each of Examples 19 and 20 was increased compared to the ADH gene non-disrupted strain (Comparative Example 2).

The concentration of 1,10-decanediol was performed using gas chromatograph under the same conditions as in (1-c). The concentration of 1,10-decanediamine in each of the culture media is shown in Table 15. It was observed that the production amount of 1,10-decanediol was suppressed in the ADH gene-disrupted strains of Examples 19 and 20 compared to the ADH gene non-disrupted strain (Comparative example 2).

**[Table 15]**

| | Transformant | Concentration of 1,10-decanediamine (µM) | Concentration of 1,10-decanediol (mM) |
|---|---|---|---|
| Comparative example 2 | A | 107 | 1.03 |
| Example 19 | B | 199 | 0.55 |
| Example 20 | S | 228 | 0.57 |

### Deposit of biological material

The plasmid pHAK1 was deposited with biotechnology division of National Institute of Technology and Evaluation (NITE), Patent Microorganisms Depositary (NPMD) (address: #122, 2-5-8, Kazusakamatari, Kisarazu-shi, Chiba, Japan) on July 21, 2020, under "NITE ABP-02919 (accession number)" (the demand for conversion from NITE P-02919 to the deposit under Budapest Treaty was filed).

### Industrial Applicability

The genetically modified microorganism of the present invention can be suitably used in production of a diamine compound.
20200722111720202007161145140240_P1AP101_19_73.app

## Claims

1. A genetically modified microorganism that expresses an enzyme involved in synthesis of a diamine compound, wherein
the diamine compound is represented by Formula: H₂N-R-NH₂
(wherein, R is a chain or cyclic organic group comprised of one or more atoms selected from the group consisting of C, H, O, N, and S), and
the genetically modified microorganism is modified to reduce an activity of an alcohol dehydrogenase compared to a non-reduced strain.

2. The genetically modified microorganism according to claim 1, wherein the modification performed to reduce the activity of the alcohol dehydrogenase compared to the non-reduced strain is
a modification to suppress expression of a gene encoding an alcohol dehydrogenase or
a modification to suppress expression of a gene encoding an alcohol dehydrogenase and to suppress an activity of an alcohol dehydrogenase.

3. The modified microorganism according to claim 1 or 2, wherein the alcohol dehydrogenase is a protein encoded by
DNA consisting of a base sequence selected from SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, and 100,
DNA consisting of a base sequence having 85% or more of sequence identity with a base sequence selected from SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, and 100 and encoding a protein having an alcohol dehydrogenase activity,
DNA consisting of a base sequence encoding a protein consisting of an amino acid sequence obtained by deleting, substituting, inserting, and/or adding 1 to 10 amino acids with respect to an amino acid sequence of a protein encoded by a base sequence selected from SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, and 100 and encoding a protein having an alcohol dehydrogenase activity, or
DNA consisting of a degenerate isomer of a base sequence selected from SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, and 100.

4. The modified microorganism according to any one of claims 1 to 3, wherein the alcohol dehydrogenase is a protein containing an amino acid sequence having 80% or more of sequence identity with an amino acid sequence selected from SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, and 99 and having an alcohol dehydrogenase activity.

5. The genetically modified microorganism according to any one of claims 1 to 4, wherein the alcohol dehydrogenase is a protein encoded by at least one gene selected from the group consisting of yqhD, fucO, adhP, ybbO, eutG, ahr, yahK, adhE, ybdR, dkgA, yiaY, frmA, dkgB, yghA, ydjG, gldA, yohF, yeaE, ADH1, ADH2, ADH3, ADH4, ADH5, ADH6, ADH7, SFA1, AAD3, AAD4, AAD10, AAD14, AAD15, YPR1, NCgl0324, NCgl0313, NCgl0219, NCgl2709, NCgl1112, NCgl2382, NCgl0186, NCgl0099, NCgl2952, NCgl1459, yogA, bdhK, bdhJ, akrN, yqkF, yccK, iolS, and yrpG.

6. The genetically modified microorganism according to any one of claims 1 to 5, wherein the alcohol dehydrogenase is a protein encoded by at least one gene selected from the group consisting of yqhD, fucO, adhP, eutG, ybbO, ahr, and yahK.

7. The genetically modified microorganism according to any one of claims 1 to 6, wherein the alcohol dehydrogenase is a protein encoded by at least one gene selected from the group consisting of yqhD and adhP.

8. The genetically modified microorganism according to claim 7, wherein the alcohol dehydrogenase is a protein encoded by an adhP gene.

9. The genetically modified microorganism according to any one of claims 1 to 6, wherein the alcohol dehydrogenase is a protein encoded by at least one gene selected from the group consisting of yqhD, fucO, eutG, ybbO, ahr, and yahK.

10. The genetically modified microorganism according to claim 9, wherein the alcohol dehydrogenase is a protein encoded by at least one gene selected from the group consisting of eutG, ybbO, ahr, and yahK.

11. The genetically modified microorganism according to any one of claims 1 to 6, wherein the alcohol dehydrogenase is a protein encoded by two or more genes selected from the group consisting of yqhD, fucO, adhP, eutG, ybbO, ahr, and yahK.

12. The genetically modified microorganism according to any one of claims 1 to 6, wherein the alcohol dehydrogenase is a protein encoded by a gene of one combination selected from the group consisting of:
- yqhD and fucO,
- yqhD and adhP,
- yqhD and eutG,
- yqhD and ybbO,
- yqhD and ahr,
- yqhD and yahK,
- yqhD, fucO, and adhP,
- yqhD, fucO, adhP, and eutG,
- yqhD, fucO, adhP, eutG, and ybbO,
- yqhD, fucO, adhP, eutG, ybbO, and ahr,
and
- yqhD, fucO, adhP, eutG, ybbO, ahr, and yahK.

13. The modified microorganism according to any one of claims 1 to 12, wherein the modification performed to reduce the activity of the alcohol dehydrogenase compared to the non-reduced strain is performed by one or more selected from the group consisting of
a reduction in transcription amount and/or translation amount of a gene encoding the alcohol dehydrogenase in the microorganism and
a disruption of a gene encoding the alcohol dehydrogenase in the microorganism.

14. The genetically modified microorganism according to any one of claims 1 to 13, wherein the genetically modified microorganism belongs to a genus selected from the group consisting of the genus Escherichia, the genus Corynebacterium, the genus Bacillus, the genus Acinetobacter, the genus Burkholderia, the genus Pseudomonas, the genus Clostridium, the genus Saccharomyces, the genus Schizosaccharomyces, the genus Yarrowia, the genus Candida, the genus Pichia, and the genus Aspergillus.

15. The genetically modified microorganism according to any one of claims 1 to 14, wherein the genetically modified microorganism is Escherichia coli.

16. The genetically modified microorganism according to any one of claims 1 to 15, wherein the genetically modified microorganism expresses an aminotransferase as the enzyme involved in the synthesis of the diamine compound.

17. The genetically modified microorganism according to any one of claims 1 to 16, wherein the genetically modified microorganism expresses a carboxylic acid reductase as the enzyme involved in the synthesis of the diamine compound.

18. The genetically modified microorganism according to claim 17, wherein the carboxylic acid reductase has an activity of converting a carboxyl group of a carboxylic acid semialdehyde, a dicarboxylic acid, or an aminocarboxylic acid into an aldehyde.

19. The genetically modified microorganism according to any one of claims 1 to 18, wherein the genetically modified microorganism
has an ability of producing a dicarboxylic acid, a carboxylic acid semialdehyde, or an aminocarboxylic acid, and
further expresses an aminotransferase and a carboxylic acid reductase.

20. The genetically modified microorganism according to any one of claims 1 to 19, wherein the genetically modified microorganism
has an ability of producing adipic acid, adipic acid semialdehyde, or 6-aminohexanoic acid, and
further expresses an aminotransferase and a carboxylic acid reductase.

21. The genetically modified microorganism according to any one of claims 17 to 20, wherein the genetically modified microorganism is further modified to increase an activity of a phosphopantetheinyl transferase.

22. The genetically modified microorganism according to any one of claims 16 to 21, wherein a gene encoding the aminotransferase is ygjG.

23. The genetically modified microorganism according to any one of claims 17 to 22, wherein a gene encoding the carboxylic acid reductase is MaCar.

24. The genetically modified microorganism according to any one of claims 21 to 23, wherein a gene encoding the phosphopantetheinyl transferase is Npt.

25. The modified microorganism according to any one of claims 1 to 24, wherein the modified microorganism contains
a base sequence having 85% or more of sequence identity with a base sequence set forth in SEQ ID NO: 115 and encoding a protein having an aminotransferase activity or
a base sequence having 85% or more of sequence identity with a base sequence encoding an amino acid sequence set forth in any one of SEQ ID NOs: 110 to 114 and encoding a protein having an aminotransferase activity.

26. The modified microorganism according to any one of claims 1 to 25, wherein the modified microorganism contains
a base sequence having 85% or more of sequence identity with a base sequence set forth in SEQ ID NO: 105 and encoding a protein having a carboxylic acid reductase activity or
a base sequence having 85% or more of sequence identity with a base sequence encoding an amino acid sequence set forth in any one of SEQ ID NOs: 101 to 104 and encoding a protein having a carboxylic acid reductase activity.

27. The modified microorganism according to any one of claims 21 to 26, wherein the modified microorganism contains
a base sequence having 85% or more of sequence identity with a base sequence set forth in SEQ ID NO: 109 and encoding a protein having a phosphopantetheinyl transferase activity or
a base sequence having 80% or more of sequence identity with a base sequence encoding an amino acid sequence set forth in any one of SEQ ID NOs: 106 to 108 and encoding a protein having a phosphopantetheinyl transferase activity.

28. The genetically modified microorganism according to any one of claims 1 to 27, wherein the genetically modified microorganism expresses one or more enzymes selected from the group consisting of
acyl-(acyl carrier protein (ACP)) reductase (AAR),
an enzyme that produces an aldehyde from acyl-CoA, and
an enzyme that produces an aldehyde from acyl phosphate.

29. A method of producing a diamine compound using the genetically modified microorganism according to any one of claims 1 to 28.

30. A method of producing a diamine compound, the method comprising a culture step of culturing the genetically modified microorganism according to any one of claims 1 to 28 in a medium containing a carbon source and a nitrogen source to obtain a culture medium containing bacterial cells.

31. The method of producing a diamine compound according to claim 30, wherein
the medium further contains a precursor of a diamine compound, or
in the culture step, the precursor is added to the medium.

32. The method of producing a diamine compound according to claim 30 or 31, further comprising a reaction step of bringing the culture medium and/or the bacterial cells into contact with an aqueous solution containing a precursor of a diamine compound to obtain a reaction solution containing a diamine compound.

33. The method of producing a diamine compound according to claim 31 or 32, wherein the precursor is selected from the group consisting of a dicarboxylic acid, a carboxylic acid semialdehyde, an aminocarboxylic acid, an aminoaldehyde, a dialdehyde, acyl-ACP, acyl-CoA, and acyl phosphate.


SEQUENCE LISTING

<110>  ASAHI KASEI KABUSHIKI KAISHA

<120>  Gene recombinant microorganism and method for producing diamine
       compounds

<130>  190379WO01

<150>  JP2019-134306
<151>  2019-07-22

<160>  167

<170>  PatentIn version 3.5

<210>  1
<211>  387
<212>  PRT
<213>  Escherichia coli

<400>  1

Met Asn Asn Phe Asn Leu His Thr Pro Thr Arg Ile Leu Phe Gly Lys
1               5                   10                  15

Gly Ala Ile Ala Gly Leu Arg Glu Gln Ile Pro His Asp Ala Arg Val
            20                  25                  30

Leu Ile Thr Tyr Gly Gly Gly Ser Val Lys Lys Thr Gly Val Leu Asp
        35                  40                  45

Gln Val Leu Asp Ala Leu Lys Gly Met Asp Val Leu Glu Phe Gly Gly
    50                  55                  60

Ile Glu Pro Asn Pro Ala Tyr Glu Thr Leu Met Asn Ala Val Lys Leu
65                  70                  75                  80

Val Arg Glu Gln Lys Val Thr Phe Leu Leu Ala Val Gly Gly Gly Ser
                85                  90                  95

Val Leu Asp Gly Thr Lys Phe Ile Ala Ala Ala Ala Asn Tyr Pro Glu
            100                 105                 110

Asn Ile Asp Pro Trp His Ile Leu Gln Thr Gly Gly Lys Glu Ile Lys
        115                 120                 125

Ser Ala Ile Pro Met Gly Cys Val Leu Thr Leu Pro Ala Thr Gly Ser
    130                 135                 140

Glu Ser Asn Ala Gly Ala Val Ile Ser Arg Lys Thr Thr Gly Asp Lys
145                 150                 155                 160

Gln Ala Phe His Ser Ala His Val Gln Pro Val Phe Ala Val Leu Asp
                165                 170                 175

Pro Val Tyr Thr Tyr Thr Leu Pro Pro Arg Gln Val Ala Asn Gly Val
            180                 185                 190

Val Asp Ala Phe Val His Thr Val Glu Gln Tyr Val Thr Lys Pro Val
        195                 200                 205

Asp Ala Lys Ile Gln Asp Arg Phe Ala Glu Gly Ile Leu Leu Thr Leu
    210                 215                 220

Ile Glu Asp Gly Pro Lys Ala Leu Lys Glu Pro Glu Asn Tyr Asp Val
225                 230                 235                 240

Arg Ala Asn Val Met Trp Ala Ala Thr Gln Ala Leu Asn Gly Leu Ile
                245                 250                 255

Gly Ala Gly Val Pro Gln Asp Trp Ala Thr His Met Leu Gly His Glu
            260                 265                 270

Leu Thr Ala Met His Gly Leu Asp His Ala Gln Thr Leu Ala Ile Val
        275                 280                 285

Leu Pro Ala Leu Trp Asn Glu Lys Arg Asp Thr Lys Arg Ala Lys Leu
    290                 295                 300

Leu Gln Tyr Ala Glu Arg Val Trp Asn Ile Thr Glu Gly Ser Asp Asp
305                 310                 315                 320

Glu Arg Ile Asp Ala Ala Ile Ala Ala Thr Arg Asn Phe Phe Glu Gln
                325                 330                 335

Leu Gly Val Pro Thr His Leu Ser Asp Tyr Gly Leu Asp Gly Ser Ser
            340                 345                 350

Ile Pro Ala Leu Leu Lys Lys Leu Glu Glu His Gly Met Thr Gln Leu
        355                 360                 365

Gly Glu Asn His Asp Ile Thr Leu Asp Val Ser Arg Arg Ile Tyr Glu
    370                 375                 380

Ala Ala Arg
385

<210>  2
<211>  1164
<212>  DNA

<213>  Escherichia coli

<400>  2
atgaacaact ttaatctgca caccccaacc cgcattctgt ttggtaaagg cgcaatcgct      60

ggtttacgcg aacaaattcc tcacgatgct cgcgtattga ttacctacgg cggcggcagc     120

gtgaaaaaaa ccggcgttct cgatcaagtt ctggatgccc tgaaaggcat ggacgtgctg     180

gaatttggcg gtattgagcc aaacccggct tatgaaacgc tgatgaacgc cgtgaaactg     240

gttcgcgaac agaaagtgac tttcctgctg gcggttggcg gcggttctgt actggacggc     300

accaaattta tcgccgcagc ggctaactat ccggaaaata tcgatccgtg gcacattctg     360

caaacgggcg gtaaagagat taaaagcgcc atcccgatgg gctgtgtgct gacgctgcca     420

gcaaccggtt cagaatccaa cgcaggcgcg gtgatctccc gtaaaaccac aggcgacaag     480

caggcgttcc attctgccca tgttcagccg gtatttgccg tgctcgatcc ggtttatacc     540

tacaccctgc cgccgcgtca ggtggctaac ggcgtagtgg acgcctttgt acacaccgtg     600

gaacagtatg ttaccaaacc ggttgatgcc aaaattcagg accgtttcgc agaaggcatt     660

ttgctgacgc taatcgaaga tggtccgaaa gccctgaaag agccagaaaa ctacgatgtg     720

cgcgccaacg tcatgtgggc ggcgactcag gcgctgaacg gtttgattgg cgctggcgta     780

ccgcaggact gggcaacgca tatgctgggc cacgaactga ctgcgatgca cggtctggat     840

cacgcgcaaa cactggctat cgtcctgcct gcactgtgga atgaaaaacg cgataccaag     900

cgcgctaagc tgctgcaata tgctgaacgc gtctggaaca tcactgaagg ttccgatgat     960

gagcgtattg acgccgcgat tgccgcaacc cgcaatttct ttgagcaatt aggcgtgccg    1020

acccacctct ccgactacgg tctggacggc agctccatcc cggctttgct gaaaaaactg    1080

gaagagcacg gcatgaccca actgggcgaa aatcatgaca ttacgttgga tgtcagccgc    1140

cgtatatacg aagccgcccg ctaa                                           1164

<210>  3
<211>  383
<212>  PRT
<213>  Escherichia coli

<400>  3

Met Met Ala Asn Arg Met Ile Leu Asn Glu Thr Ala Trp Phe Gly Arg
1               5                   10                  15

Gly Ala Val Gly Ala Leu Thr Asp Glu Val Lys Arg Arg Gly Tyr Gln
            20                  25                  30

Lys Ala Leu Ile Val Thr Asp Lys Thr Leu Val Gln Cys Gly Val Val
        35                  40                  45

Ala Lys Val Thr Asp Lys Met Asp Ala Ala Gly Leu Ala Trp Ala Ile
    50                  55                  60

Tyr Asp Gly Val Val Pro Asn Pro Thr Ile Thr Val Val Lys Glu Gly
65                  70                  75                  80

Leu Gly Val Phe Gln Asn Ser Gly Ala Asp Tyr Leu Ile Ala Ile Gly
                85                  90                  95

Gly Gly Ser Pro Gln Asp Thr Cys Lys Ala Ile Gly Ile Ile Ser Asn
            100                 105                 110

Asn Pro Glu Phe Ala Asp Val Arg Ser Leu Glu Gly Leu Ser Pro Thr
        115                 120                 125

Asn Lys Pro Ser Val Pro Ile Leu Ala Ile Pro Thr Thr Ala Gly Thr
    130                 135                 140

Ala Ala Glu Val Thr Ile Asn Tyr Val Ile Thr Asp Glu Glu Lys Arg
145                 150                 155                 160

Arg Lys Phe Val Cys Val Asp Pro His Asp Ile Pro Gln Val Ala Phe
                165                 170                 175

Ile Asp Ala Asp Met Met Asp Gly Met Pro Pro Ala Leu Lys Ala Ala
            180                 185                 190

Thr Gly Val Asp Ala Leu Thr His Ala Ile Glu Gly Tyr Ile Thr Arg
        195                 200                 205

Gly Ala Trp Ala Leu Thr Asp Ala Leu His Ile Lys Ala Ile Glu Ile
    210                 215                 220

Ile Ala Gly Ala Leu Arg Gly Ser Val Ala Gly Asp Lys Asp Ala Gly
225                 230                 235                 240

Glu Glu Ile Ala Leu Gly Gln Tyr Val Ala Gly Met Gly Phe Ser Asn
                245                 250                 255

Val Gly Leu Gly Leu Val His Gly Met Ala His Pro Leu Gly Ala Phe
            260                 265                 270

Tyr Asn Thr Pro His Gly Val Ala Asn Ala Ile Leu Leu Pro His Val
        275                 280                 285

Met Arg Tyr Asn Ala Asp Phe Thr Gly Glu Lys Tyr Arg Asp Ile Ala
    290                 295                 300

Arg Val Met Gly Val Lys Val Glu Gly Met Ser Leu Glu Glu Ala Arg
305                 310                 315                 320

Asn Ala Ala Val Glu Ala Val Phe Ala Leu Asn Arg Asp Val Gly Ile
                325                 330                 335

Pro Pro His Leu Arg Asp Val Gly Val Arg Lys Glu Asp Ile Pro Ala
            340                 345                 350

Leu Ala Gln Ala Ala Leu Asn Asp Val Cys Thr Gly Gly Asn Pro Arg
        355                 360                 365

Glu Ala Thr Leu Glu Asp Ile Val Glu Leu Tyr His Thr Ala Trp
    370                 375                 380

<210>  4
<211>  1152
<212>  DNA
<213>  Escherichia coli

<400>  4
atgatggcta acagaatgat tctgaacgaa acggcatggt ttggtcgggg tgctgttggg      60

gctttaaccg atgaggtgaa acgccgtggt tatcagaagg cgctgatcgt caccgataaa     120

acgctggtgc aatgcggcgt ggtggcgaaa gtgaccgata agatggatgc tgcagggctg     180

gcatgggcga tttacgacgg cgtagtgccc aacccaacaa ttactgtcgt caaagaaggg     240

ctcggtgtat tccagaatag cggcgcggat tacctgatcg ctattggtgg tggttctcca     300

caggatactt gtaaagcgat tggcattatc agcaacaacc cggagtttgc cgatgtgcgt     360

agcctggaag ggctttcccc gaccaataaa cccagtgtac cgattctggc aatccccacc     420

acagcaggca ctgcggcaga agtgaccatt aactacgtga tcactgacga agaaaaacgg     480

cgcaagtttg tttgcgttga tccgcatgat atcccgcagg tggcgtttat tgacgctgac     540

atgatggatg gtatgcctcc agcgctgaaa gctgcgacgg gtgtcgatgc gctcactcat     600

gctattgagg ggtatattac ccgtggcgcg tgggcgctaa ccgatgcact gcacattaaa     660

gcgattgaaa tcattgctgg ggcgctgcga ggatcggttg ctggtgataa ggatgccgga     720

gaagaaatag cgctcgggca gtatgttgcg ggtatgggct tctcgaatgt tgggttaggg     780

ttggtgcatg gtatggcgca tccactgggc gcgttttata acactccaca cggtgttgca     840

aacgccatcc tgctaccgca tgtcatgcgc tataacgctg actttaccgg tgagaagtac     900

cgcgatatcg cgcgcgttat gggcgtgaaa gtggaaggta tgagcctgga agaggcgcgt     960

aatgccgctg ttgaagcggt gtttgctctc aaccgtgatg tcggtattcc gccacatttg    1020

cgtgatgttg gggtacgcaa ggaagacatt ccggcactgg cgcaggcggc actgaatgat    1080

gtttgtaccg gtggcaaccc gcgtgaagca acgcttgagg atattgtaga gctttaccat 1140

accgcctggt aa 1152

<210> 5
<211> 336
<212> PRT
<213> Escherichia coli

<400> 5

Met Lys Ala Ala Val Val Thr Lys Asp His His Val Asp Val Thr Tyr
1               5                   10                  15

Lys Thr Leu Arg Ser Leu Lys His Gly Glu Ala Leu Leu Lys Met Glu
            20                  25                  30

Cys Cys Gly Val Cys His Thr Asp Leu His Val Lys Asn Gly Asp Phe
        35                  40                  45

Gly Asp Lys Thr Gly Val Ile Leu Gly His Glu Gly Ile Gly Val Val
    50                  55                  60

Ala Glu Val Gly Pro Gly Val Thr Ser Leu Lys Pro Gly Asp Arg Ala
65                  70                  75                  80

Ser Val Ala Trp Phe Tyr Glu Gly Cys Gly His Cys Glu Tyr Cys Asn
                85                  90                  95

Ser Gly Asn Glu Thr Leu Cys Arg Ser Val Lys Asn Ala Gly Tyr Ser
            100                 105                 110

Val Asp Gly Gly Met Ala Glu Glu Cys Ile Val Val Ala Asp Tyr Ala
        115                 120                 125

Val Lys Val Pro Asp Gly Leu Asp Ser Ala Ala Ala Ser Ser Ile Thr
    130                 135                 140

Cys Ala Gly Val Thr Thr Tyr Lys Ala Val Lys Leu Ser Lys Ile Arg
145                 150                 155                 160

Pro Gly Gln Trp Ile Ala Ile Tyr Gly Leu Gly Gly Leu Gly Asn Leu
                165                 170                 175

Ala Leu Gln Tyr Ala Lys Asn Val Phe Asn Ala Lys Val Ile Ala Ile
            180                 185                 190

Asp Val Asn Asp Glu Gln Leu Lys Leu Ala Thr Glu Met Gly Ala Asp
        195                 200                 205

Leu Ala Ile Asn Ser His Thr Glu Asp Ala Ala Lys Ile Val Gln Glu
    210                 215                 220

Lys Thr Gly Gly Ala His Ala Ala Val Val Thr Ala Val Ala Lys Ala
225                 230                 235                 240

Ala Phe Asn Ser Ala Val Asp Ala Val Arg Ala Gly Gly Arg Val Val
                245                 250                 255

Ala Val Gly Leu Pro Pro Glu Ser Met Ser Leu Asp Ile Pro Arg Leu
            260                 265                 270

Val Leu Asp Gly Ile Glu Val Val Gly Ser Leu Val Gly Thr Arg Gln
        275                 280                 285

Asp Leu Thr Glu Ala Phe Gln Phe Ala Ala Glu Gly Lys Val Val Pro
    290                 295                 300

Lys Val Ala Leu Arg Pro Leu Ala Asp Ile Asn Thr Ile Phe Thr Glu
305                 310                 315                 320

Met Glu Glu Gly Lys Ile Arg Gly Arg Met Val Ile Asp Phe Arg His
                325                 330                 335

<210>  6
<211>  1011
<212>  DNA
<213>  Escherichia coli

<400>  6
atgaaggctg cagttgttac gaaggatcat catgttgacg ttacgtataa aacactgcgc      60

tcactgaaac atggcgaagc cctgctgaaa atggagtgtt gtggtgtatg tcataccgat     120

cttcatgtta agaatggcga ttttggtgac aaaaccggcg taattctggg ccatgaaggc     180

atcggtgtgg tggcagaagt gggtccaggt gtcacctcat taaaaccagg cgatcgtgcc     240

agcgtggcgt ggttctacga aggatgcggt cattgcgaat actgtaacag tggtaacgaa     300

acgctctgcc gttcagttaa aaatgccgga tacagcgttg atggcgggat ggcggaagag     360

tgcatcgtgg tcgccgatta cgcggtaaaa gtgccagatg gtctggactc ggcggcggcc     420

agcagcatta cctgtgcggg agtcaccacc tacaaagccg ttaagctgtc aaaaattcgt     480

ccagggcagt ggattgctat ctacggtctt ggcggtctgg gtaacctcgc cctgcaatac     540

gcgaagaatg tctttaacgc caaagtgatc gccattgatg tcaatgatga gcagttaaaa     600

ctggcaaccg aaatgggcgc agatttagcg attaactcac acaccgaaga cgccgccaaa     660

attgtgcagg agaaaactgg tggcgctcac gctgcggtgg taacagcggt agctaaagct     720

gcgtttaact cggcagttga tgctgtccgt gcaggcggtc gtgttgtggc tgtcggtcta 780

ccgccggagt ctatgagcct ggatatccca cgtcttgtgc tggatggtat tgaagtggtc 840

ggttcgctgg tcggcacgcg ccaggattta actgaagcct tccagtttgc cgccgaaggt 900

aaagtggtgc cgaaagtcgc cctgcgtccg ttagcggaca tcaacaccat ctttactgag 960

atggaagaag gcaaaatccg tggccgcatg gtgattgatt tccgtcacta a 1011

<210> 7
<211> 269
<212> PRT
<213> Escherichia coli

<400> 7

Met Thr His Lys Ala Thr Glu Ile Leu Thr Gly Lys Val Met Gln Lys
1               5                   10                  15

Ser Val Leu Ile Thr Gly Cys Ser Ser Gly Ile Gly Leu Glu Ser Ala
            20                  25                  30

Leu Glu Leu Lys Arg Gln Gly Phe His Val Leu Ala Gly Cys Arg Lys
        35                  40                  45

Pro Asp Asp Val Glu Arg Met Asn Asn Met Gly Phe Thr Gly Val Leu
    50                  55                  60

Ile Asp Leu Asp Ser Pro Glu Ser Val Asp Arg Ala Ala Asp Glu Val
65                  70                  75                  80

Ile Ala Leu Thr Asp Asn Cys Leu Tyr Gly Ile Phe Asn Asn Ala Gly
                85                  90                  95

Phe Gly Met Tyr Gly Pro Leu Ser Thr Ile Ser Arg Ala Gln Met Glu
            100                 105                 110

Gln Gln Phe Ser Ala Asn Phe Phe Gly Ala His Gln Leu Thr Met Arg
        115                 120                 125

Leu Leu Pro Ala Met Leu Pro His Gly Glu Gly Arg Ile Val Met Thr
    130                 135                 140

Ser Ser Val Met Gly Leu Ile Ser Thr Pro Gly Arg Gly Ala Tyr Ala
145                 150                 155                 160

Ala Ser Lys Tyr Ala Leu Glu Ala Trp Ser Asp Ala Leu Arg Met Glu
                165                 170                 175

Leu Arg His Ser Gly Ile Lys Val Ser Leu Ile Glu Pro Gly Pro Ile

180                 185                 190

Arg Thr Arg Phe Thr Asp Asn Val Asn Gln Thr Gln Ser Asp Lys Pro
        195                 200                 205

Val Glu Asn Pro Gly Ile Ala Ala Arg Phe Thr Leu Gly Pro Glu Ala
    210                 215                 220

Val Val Asp Lys Val Arg His Ala Phe Ile Ser Glu Lys Pro Lys Met
225                 230                 235                 240

Arg Tyr Pro Val Thr Leu Val Thr Trp Ala Val Met Val Leu Lys Arg
                245                 250                 255

Leu Leu Pro Gly Arg Val Met Asp Lys Ile Leu Gln Gly
            260                 265

<210>  8
<211>  810
<212>  DNA
<213>  Escherichia coli

<400>  8
atgactcata aagcaacgga gatcctgaca ggtaaagtta tgcaaaaatc ggtcttaatt      60

accggatgtt ccagtggaat tggcctggaa agcgcgctcg aattaaaacg ccagggtttt     120

catgtgctgg caggttgccg aaaaccggat gatgttgagc gtatgaacaa catgggattt     180

accggcgtgt tgatcgatct ggattcacca gaaagtgttg atcgcgcagc agacgaggtg     240

atcgccctga ccgataattg tctgtatggg atctttaaca atgccggatt cggcatgtat     300

ggcccccttt ccaccatcag ccgtgcgcag atggaacagc agttttccgc caactttttc     360

ggcgcacacc agctcaccat gcgcctgtta cccgcgatgt taccgcacgg tgaagggcgt     420

attgtgatga catcatcggt gatgggatta atctccacgc cgggtcgtgg cgcttacgcg     480

gccagtaaat atgcgctgga ggcgtggtca gacgcgctgc gcatggagct acgccacagc     540

ggaattaaag tcagcctgat cgaacccggt cccattcgta ctcgcttcac cgacaacgtc     600

aaccagacgc aaagtgataa accagtcgaa aatcccggca tcgccgcccg ctttacgttg     660

ggaccggaag cggtggtgga caaagtacgc catgctttta ttagcgagaa gccgaagatg     720

cgctatccgg taacgctggt gacctgggca gtaatggtgc ttaagcgcct gctgccgggg     780

cgcgtgatgg acaaaatatt gcaggggtga                                      810

<210>  9
<211>  395
<212>  PRT
<213>  Escherichia coli

<400> 9

Met Gln Asn Glu Leu Gln Thr Ala Leu Phe Gln Ala Phe Asp Thr Leu
1               5                   10                  15

Asn Leu Gln Arg Val Lys Thr Phe Ser Val Pro Pro Val Thr Leu Cys
            20                  25                  30

Gly Pro Gly Ala Val Ser Ser Cys Gly Gln Gln Ala Gln Thr Arg Gly
        35                  40                  45

Leu Lys His Leu Phe Val Met Ala Asp Ser Phe Leu His Gln Ala Gly
    50                  55                  60

Met Thr Ala Gly Leu Thr Arg Ser Leu Ala Val Lys Gly Ile Ala Met
65                  70                  75                  80

Thr Leu Trp Pro Cys Pro Val Gly Glu Pro Cys Ile Thr Asp Val Cys
                85                  90                  95

Ala Ala Val Ala Gln Leu Arg Glu Ser Gly Cys Asp Gly Val Ile Ala
            100                 105                 110

Phe Gly Gly Gly Ser Val Leu Asp Ala Ala Lys Ala Val Ala Leu Leu
        115                 120                 125

Val Thr Asn Pro Asp Ser Thr Leu Ala Glu Met Ser Glu Thr Ser Val
    130                 135                 140

Leu Gln Pro Arg Leu Pro Leu Ile Ala Ile Pro Thr Thr Ala Gly Thr
145                 150                 155                 160

Gly Ser Glu Thr Thr Asn Val Thr Val Ile Ile Asp Ala Val Ser Gly
                165                 170                 175

Arg Lys Gln Val Leu Ala His Ala Ser Leu Met Pro Asp Val Ala Ile
            180                 185                 190

Leu Asp Ala Ala Leu Thr Glu Gly Val Pro Ser His Val Thr Ala Met
        195                 200                 205

Thr Gly Ile Asp Ala Leu Thr His Ala Ile Glu Ala Tyr Ser Ala Leu
    210                 215                 220

Asn Ala Thr Pro Phe Thr Asp Ser Leu Ala Ile Gly Ala Ile Ala Met
225                 230                 235                 240

Ile Gly Lys Ser Leu Pro Lys Ala Val Gly Tyr Gly His Asp Leu Ala

245                 250                 255

Ala Arg Glu Ser Met Leu Leu Ala Ser Cys Met Ala Gly Met Ala Phe
            260                 265                 270

Ser Ser Ala Gly Leu Gly Leu Cys His Ala Met Ala His Gln Pro Gly
        275                 280                 285

Ala Ala Leu His Ile Pro His Gly Leu Ala Asn Ala Met Leu Leu Pro
    290                 295                 300

Thr Val Met Glu Phe Asn Arg Met Val Cys Arg Glu Arg Phe Ser Gln
305                 310                 315                 320

Ile Gly Arg Ala Leu Arg Thr Lys Lys Ser Asp Asp Arg Asp Ala Ile
                325                 330                 335

Asn Ala Val Ser Glu Leu Ile Ala Glu Val Gly Ile Gly Lys Arg Leu
            340                 345                 350

Gly Asp Val Gly Ala Thr Ser Ala His Tyr Gly Ala Trp Ala Gln Ala
        355                 360                 365

Ala Leu Glu Asp Ile Cys Leu Arg Ser Asn Pro Arg Thr Ala Ser Leu
    370                 375                 380

Glu Gln Ile Val Gly Leu Tyr Ala Ala Ala Gln
385                 390                 395

<210>  10
<211>  1188
<212>  DNA
<213>  Escherichia coli

<400>  10
atgcaaaatg aattgcagac cgcgctcttt caggcgttcg ataccctgaa tctgcaacgg      60

gtaaaaacat ttagcgttcc accggtgacg ctttgcggtc cgggcgcggt gagcagttgc     120

gggcagcaag cgcaaacgcg tgggctgaaa catctgttcg tgatggcaga cagctttttg     180

catcaggcgg ggatgaccgc cgggctgacg cgcagcctgg ctgttaaagg catcgccatg     240

acgctctggc catgtccggt gggcgaaccg tgcattaccg acgtgtgtgc agccgtggcg     300

cagttgcgtg agtcaggctg tgatggggtg atcgcatttg gcggcggctc ggtgctggat     360

gcggcgaaag ccgtggcgtt gctggtgacg aaccccgata gcacgctggc agagatgtca     420

gaaaccagcg ttctgcaacc gcgcttgccg ctgattgcca ttccaacgac cgccggaacc     480

ggctctgaaa ccaccaatgt aacggtgatt atcgacgcgg tgagcgggcg caagcaggtg     540

ttagcccatg cctcgctgat gccggatgtg gcgatcctcg acgccgcatt gaccgaaggt 600

gtgccgtcgc atgtcacggc gatgaccggc attgatgcgt taacccatgc cattgaagca 660

tacagcgccc tgaacgctac accgtttacc gacagcctgg cgattggtgc cattgcgatg 720

attggcaaat cgctgccgaa agcggtgggc tacggtcacg accttgccgc gcgcgagagc 780

atgttactgg cttcatgtat ggcgggaatg gcgttttcca gtgcgggtct tgggttgtgc 840

cacgcgatgg cgcatcagcc gggcgcggcg ctgcatattc cgcacggtct cgcgaacgcc 900

atgttgctgc caacggtgat ggaatttaac cggatggttt gtcgtgaacg ctttagtcag 960

attggtcggg cactgcgaac taaaaaatcc gacgatcgtg acgctattaa cgcggtaagt 1020

gagctgattg cggaagttgg gattggtaaa cgactgggcg atgttggtgc gacatctgcg 1080

cattacggcg catgggcgca ggccgcgctg gaagatattt gtctgcgcag taacccgcgt 1140

accgccagcc tggagcagat tgtcggcctg tacgcagcgg cgcaataa 1188

<210> 11
<211> 339
<212> PRT
<213> Escherichia coli

<400> 11

Met Ser Met Ile Lys Ser Tyr Ala Ala Lys Glu Ala Gly Gly Glu Leu
1               5                   10                  15

Glu Val Tyr Glu Tyr Asp Pro Gly Glu Leu Arg Pro Gln Asp Val Glu
            20                  25                  30

Val Gln Val Asp Tyr Cys Gly Ile Cys His Ser Asp Leu Ser Met Ile
        35                  40                  45

Asp Asn Glu Trp Gly Phe Ser Gln Tyr Pro Leu Val Ala Gly His Glu
    50                  55                  60

Val Ile Gly Arg Val Val Ala Leu Gly Ser Ala Ala Gln Asp Lys Gly
65                  70                  75                  80

Leu Gln Val Gly Gln Arg Val Gly Ile Gly Trp Thr Ala Arg Ser Cys
                85                  90                  95

Gly His Cys Asp Ala Cys Ile Ser Gly Asn Gln Ile Asn Cys Glu Gln
            100                 105                 110

Gly Ala Val Pro Thr Ile Met Asn Arg Gly Gly Phe Ala Glu Lys Leu
        115                 120                 125

Arg Ala Asp Trp Gln Trp Val Ile Pro Leu Pro Glu Asn Ile Asp Ile

130                 135                 140

Glu Ser Ala Gly Pro Leu Leu Cys Gly Gly Ile Thr Val Phe Lys Pro
145                 150                 155                 160

Leu Leu Met His His Ile Thr Ala Thr Ser Arg Val Gly Val Ile Gly
                165                 170                 175

Ile Gly Gly Leu Gly His Ile Ala Ile Lys Leu Leu His Ala Met Gly
            180                 185                 190

Cys Glu Val Thr Ala Phe Ser Ser Asn Pro Ala Lys Glu Gln Glu Val
        195                 200                 205

Leu Ala Met Gly Ala Asp Lys Val Val Asn Ser Arg Asp Pro Gln Ala
    210                 215                 220

Leu Lys Ala Leu Ala Gly Gln Phe Asp Leu Ile Ile Asn Thr Val Asn
225                 230                 235                 240

Val Ser Leu Asp Trp Gln Pro Tyr Phe Glu Ala Leu Thr Tyr Gly Gly
                245                 250                 255

Asn Phe His Thr Val Gly Ala Val Leu Thr Pro Leu Ser Val Pro Ala
            260                 265                 270

Phe Thr Leu Ile Ala Gly Asp Arg Ser Val Ser Gly Ser Ala Thr Gly
        275                 280                 285

Thr Pro Tyr Glu Leu Arg Lys Leu Met Arg Phe Ala Ala Arg Ser Lys
    290                 295                 300

Val Ala Pro Thr Thr Glu Leu Phe Pro Met Ser Lys Ile Asn Asp Ala
305                 310                 315                 320

Ile Gln His Val Arg Asp Gly Lys Ala Arg Tyr Arg Val Val Leu Lys
                325                 330                 335

Ala Asp Tyr

<210>  12
<211>  1020
<212>  DNA
<213>  Escherichia coli

<400>  12
atgtcgatga taaaaagcta tgccgcaaaa gaagcgggcg gagaactgga agtttatgag        60

tacgatcccg gtgagctgag gccacaagat gttgaagtgc aggtggatta ctgcgggatc 120

tgccattccg atctgtcgat gatcgataac gaatggggat tttcacaata tccgctggtt 180

gccgggcatg aggtgattgg gcgcgtggtg gcactcggga gcgccgcgca ggataaaggt 240

ttgcaggtcg gtcagcgtgt cgggattggc tggacggcgc gtagctgtgg tcactgcgac 300

gcctgtatta gcggtaatca gatcaactgc gagcaaggtg cggtgccgac gattatgaat 360

cgcggtggct ttgccgagaa gttgcgtgcg gactggcaat gggtgattcc actgccagaa 420

aatattgata tcgagtccgc cgggccgctg ttgtgcggcg gtatcacggt ctttaaacca 480

ctgttgatgc accatatcac tgctaccagc cgcgttgggg taattggtat tggcgggctg 540

gggcatatcg ctataaaact tctgcacgca atgggatgcg aggtgacagc ctttagttct 600

aatccggcga aagagcagga agtgctggcg atgggtgccg ataaagtggt gaatagccgc 660

gatccgcagg cactgaaagc actggcgggg cagtttgatc tcattatcaa caccgtcaac 720

gtcagcctcg actggcagcc ctattttgag gcgctgacct atggcggtaa tttccatacg 780

gtcggtgcgg ttctcacgcc gctgtctgtt ccggccttta cgttaattgc gggcgatcgc 840

agcgtctctg gttctgctac cggcacgcct tatgagctgc gtaagctgat gcgttttgcc 900

gcccgcagca aggttgcgcc gaccaccgaa ctgttcccga tgtcgaaaat taacgacgcc 960

atccagcatg tgcgcgacgg taaggcgcgt taccgcgtgg tgttgaaagc cgattattga 1020

<210> 13
<211> 349
<212> PRT
<213> Escherichia coli

<400> 13

Met Lys Ile Lys Ala Val Gly Ala Tyr Ser Ala Lys Gln Pro Leu Glu
1               5                   10                  15

Pro Met Asp Ile Thr Arg Arg Glu Pro Gly Pro Asn Asp Val Lys Ile
            20                  25                  30

Glu Ile Ala Tyr Cys Gly Val Cys His Ser Asp Leu His Gln Val Arg
        35                  40                  45

Ser Glu Trp Ala Gly Thr Val Tyr Pro Cys Val Pro Gly His Glu Ile
    50                  55                  60

Val Gly Arg Val Val Ala Val Gly Asp Gln Val Glu Lys Tyr Ala Pro
65                  70                  75                  80

Gly Asp Leu Val Gly Val Gly Cys Ile Val Asp Ser Cys Lys His Cys
                85                  90                  95

Glu Glu Cys Glu Asp Gly Leu Glu Asn Tyr Cys Asp His Met Thr Gly
            100                 105                 110

Thr Tyr Asn Ser Pro Thr Pro Asp Glu Pro Gly His Thr Leu Gly Gly
        115                 120                 125

Tyr Ser Gln Gln Ile Val Val His Glu Arg Tyr Val Leu Arg Ile Arg
    130                 135                 140

His Pro Gln Glu Gln Leu Ala Ala Val Ala Pro Leu Leu Cys Ala Gly
145                 150                 155                 160

Ile Thr Thr Tyr Ser Pro Leu Arg His Trp Gln Ala Gly Pro Gly Lys
                165                 170                 175

Lys Val Gly Val Val Gly Ile Gly Gly Leu Gly His Met Gly Ile Lys
            180                 185                 190

Leu Ala His Ala Met Gly Ala His Val Val Ala Phe Thr Thr Ser Glu
        195                 200                 205

Ala Lys Arg Glu Ala Ala Lys Ala Leu Gly Ala Asp Glu Val Val Asn
    210                 215                 220

Ser Arg Asn Ala Asp Glu Met Ala Ala His Leu Lys Ser Phe Asp Phe
225                 230                 235                 240

Ile Leu Asn Thr Val Ala Ala Pro His Asn Leu Asp Asp Phe Thr Thr
                245                 250                 255

Leu Leu Lys Arg Asp Gly Thr Met Thr Leu Val Gly Ala Pro Ala Thr
            260                 265                 270

Pro His Lys Ser Pro Glu Val Phe Asn Leu Ile Met Lys Arg Arg Ala
        275                 280                 285

Ile Ala Gly Ser Met Ile Gly Gly Ile Pro Glu Thr Gln Glu Met Leu
    290                 295                 300

Asp Phe Cys Ala Glu His Gly Ile Val Ala Asp Ile Glu Met Ile Arg
305                 310                 315                 320

Ala Asp Gln Ile Asn Glu Ala Tyr Glu Arg Met Leu Arg Gly Asp Val
                325                 330                 335

Lys Tyr Arg Phe Val Ile Asp Asn Arg Thr Leu Thr Asp
            340                 345

<210>  14
<211>  1050
<212>  DNA
<213>  Escherichia coli

<400>  14
atgaagatca aagctgttgg tgcatattcc gctaaacaac cacttgaacc gatggatatc      60

acccggcgtg aaccgggacc gaatgatgtc aaaatcgaaa tcgcttactg tggcgtttgc     120

cattccgatc tccaccaggt ccgttccgag tgggcgggga cggtttaccc ctgcgtgccg     180

ggtcatgaaa ttgtggggcg tgtggtagcc gttggtgatc aggtagaaaa atatgcgccg     240

ggcgatctgg tcggtgtcgg ctgcattgtc gacagttgta aacattgcga agagtgtgaa     300

gacgggttgg aaaactactg tgatcacatg accggcacct ataactcgcc gacgccggac     360

gaaccgggcc atactctggg cggctactca caacagatcg tcgttcatga gcgatatgtt     420

ctgcgtattc gtcacccgca agagcagctg gcggcggtgg ctcctttgtt gtgtgcaggg     480

atcaccacgt attcgccgct acgtcactgg caggccgggc cgggtaaaaa agtgggcgtg     540

gtcggcatcg gcggtctggg acatatgggg attaagctgg cccacgcgat gggggcacat     600

gtggtggcat ttaccacttc tgaggcaaaa cgcgaagcgg caaaagccct gggggccgat     660

gaagttgtta actcacgcaa tgccgatgag atggcggctc atctgaagag tttcgatttc     720

attttgaata cagtagctgc gccacataat ctcgacgatt ttaccacctt gctgaagcgt     780

gatggcacca tgacgctggt tggtgcgcct gcgacaccgc ataaatcgcc ggaagttttc     840

aacctgatca tgaaacgccg tgcgatagcc ggttctatga ttggcggcat tccagaaact     900

caggagatgc tcgatttttg cgccgaacat ggcatcgtgg ctgatataga gatgattcgg     960

gccgatcaaa ttaatgaagc ctatgagcga atgctgcgcg gtgatgtgaa atatcgtttt    1020

gttatcgata atcgcacact aacagactga                                     1050


<210>  15
<211>  891
<212>  PRT
<213>  Escherichia coli

<400>  15

Met Ala Val Thr Asn Val Ala Glu Leu Asn Ala Leu Val Glu Arg Val
1               5                   10                  15


Lys Lys Ala Gln Arg Glu Tyr Ala Ser Phe Thr Gln Glu Gln Val Asp
            20                  25                  30


Lys Ile Phe Arg Ala Ala Ala Leu Ala Ala Ala Asp Ala Arg Ile Pro
        35                  40                  45

Leu Ala Lys Met Ala Val Ala Glu Ser Gly Met Gly Ile Val Glu Asp
    50                  55                  60

Lys Val Ile Lys Asn His Phe Ala Ser Glu Tyr Ile Tyr Asn Ala Tyr
65                  70                  75                  80

Lys Asp Glu Lys Thr Cys Gly Val Leu Ser Glu Asp Asp Thr Phe Gly
                85                  90                  95

Thr Ile Thr Ile Ala Glu Pro Ile Gly Ile Ile Cys Gly Ile Val Pro
            100                 105                 110

Thr Thr Asn Pro Thr Ser Thr Ala Ile Phe Lys Ser Leu Ile Ser Leu
        115                 120                 125

Lys Thr Arg Asn Ala Ile Ile Phe Ser Pro His Pro Arg Ala Lys Asp
    130                 135                 140

Ala Thr Asn Lys Ala Ala Asp Ile Val Leu Gln Ala Ala Ile Ala Ala
145                 150                 155                 160

Gly Ala Pro Lys Asp Leu Ile Gly Trp Ile Asp Gln Pro Ser Val Glu
                165                 170                 175

Leu Ser Asn Ala Leu Met His His Pro Asp Ile Asn Leu Ile Leu Ala
            180                 185                 190

Thr Gly Gly Pro Gly Met Val Lys Ala Ala Tyr Ser Ser Gly Lys Pro
        195                 200                 205

Ala Ile Gly Val Gly Ala Gly Asn Thr Pro Val Val Ile Asp Glu Thr
    210                 215                 220

Ala Asp Ile Lys Arg Ala Val Ala Ser Val Leu Met Ser Lys Thr Phe
225                 230                 235                 240

Asp Asn Gly Val Ile Cys Ala Ser Glu Gln Ser Val Val Val Asp
                245                 250                 255

Ser Val Tyr Asp Ala Val Arg Glu Arg Phe Ala Thr His Gly Gly Tyr
            260                 265                 270

Leu Leu Gln Gly Lys Glu Leu Lys Ala Val Gln Asp Val Ile Leu Lys
        275                 280                 285

Asn Gly Ala Leu Asn Ala Ala Ile Val Gly Gln Pro Ala Tyr Lys Ile
    290                 295                 300

Ala Glu Leu Ala Gly Phe Ser Val Pro Glu Asn Thr Lys Ile Leu Ile
305                 310                 315                 320

Gly Glu Val Thr Val Val Asp Glu Ser Glu Pro Phe Ala His Glu Lys
                325                 330                 335

Leu Ser Pro Thr Leu Ala Met Tyr Arg Ala Lys Asp Phe Glu Asp Ala
            340                 345                 350

Val Glu Lys Ala Glu Lys Leu Val Ala Met Gly Gly Ile Gly His Thr
        355                 360                 365

Ser Cys Leu Tyr Thr Asp Gln Asp Asn Gln Pro Ala Arg Val Ser Tyr
    370                 375                 380

Phe Gly Gln Lys Met Lys Thr Ala Arg Ile Leu Ile Asn Thr Pro Ala
385                 390                 395                 400

Ser Gln Gly Gly Ile Gly Asp Leu Tyr Asn Phe Lys Leu Ala Pro Ser
                405                 410                 415

Leu Thr Leu Gly Cys Gly Ser Trp Gly Gly Asn Ser Ile Ser Glu Asn
            420                 425                 430

Val Gly Pro Lys His Leu Ile Asn Lys Lys Thr Val Ala Lys Arg Ala
        435                 440                 445

Glu Asn Met Leu Trp His Lys Leu Pro Lys Ser Ile Tyr Phe Arg Arg
    450                 455                 460

Gly Ser Leu Pro Ile Ala Leu Asp Glu Val Ile Thr Asp Gly His Lys
465                 470                 475                 480

Arg Ala Leu Ile Val Thr Asp Arg Phe Leu Phe Asn Asn Gly Tyr Ala
                485                 490                 495

Asp Gln Ile Thr Ser Val Leu Lys Ala Ala Gly Val Glu Thr Glu Val
            500                 505                 510

Phe Phe Glu Val Glu Ala Asp Pro Thr Leu Ser Ile Val Arg Lys Gly
        515                 520                 525

Ala Glu Leu Ala Asn Ser Phe Lys Pro Asp Val Ile Ile Ala Leu Gly
    530                 535                 540

Gly Gly Ser Pro Met Asp Ala Ala Lys Ile Met Trp Val Met Tyr Glu

545                 550                 555                 560

His Pro Glu Thr His Phe Glu Glu Leu Ala Leu Arg Phe Met Asp Ile
                565                 570                 575

Arg Lys Arg Ile Tyr Lys Phe Pro Lys Met Gly Val Lys Ala Lys Met
            580                 585                 590

Ile Ala Val Thr Thr Thr Ser Gly Thr Gly Ser Glu Val Thr Pro Phe
        595                 600                 605

Ala Val Val Thr Asp Asp Ala Thr Gly Gln Lys Tyr Pro Leu Ala Asp
    610                 615                 620

Tyr Ala Leu Thr Pro Asp Met Ala Ile Val Asp Ala Asn Leu Val Met
625                 630                 635                 640

Asp Met Pro Lys Ser Leu Cys Ala Phe Gly Gly Leu Asp Ala Val Thr
                645                 650                 655

His Ala Met Glu Ala Tyr Val Ser Val Leu Ala Ser Glu Phe Ser Asp
            660                 665                 670

Gly Gln Ala Leu Gln Ala Leu Lys Leu Leu Lys Glu Tyr Leu Pro Ala
        675                 680                 685

Ser Tyr His Glu Gly Ser Lys Asn Pro Val Ala Arg Glu Arg Val His
    690                 695                 700

Ser Ala Ala Thr Ile Ala Gly Ile Ala Phe Ala Asn Ala Phe Leu Gly
705                 710                 715                 720

Val Cys His Ser Met Ala His Lys Leu Gly Ser Gln Phe His Ile Pro
                725                 730                 735

His Gly Leu Ala Asn Ala Leu Leu Ile Cys Asn Val Ile Arg Tyr Asn
            740                 745                 750

Ala Asn Asp Asn Pro Thr Lys Gln Thr Ala Phe Ser Gln Tyr Asp Arg
        755                 760                 765

Pro Gln Ala Arg Arg Arg Tyr Ala Glu Ile Ala Asp His Leu Gly Leu
    770                 775                 780

Ser Ala Pro Gly Asp Arg Thr Ala Ala Lys Ile Glu Lys Leu Leu Ala
785                 790                 795                 800

Trp Leu Glu Thr Leu Lys Ala Glu Leu Gly Ile Pro Lys Ser Ile Arg
                805                 810                 815

Glu Ala Gly Val Gln Glu Ala Asp Phe Leu Ala Asn Val Asp Lys Leu
            820                 825                 830

Ser Glu Asp Ala Phe Asp Asp Gln Cys Thr Gly Ala Asn Pro Arg Tyr
        835                 840                 845

Pro Leu Ile Ser Glu Leu Lys Gln Ile Leu Leu Asp Thr Tyr Tyr Gly
    850                 855                 860

Arg Asp Tyr Val Glu Gly Glu Thr Ala Ala Lys Lys Glu Ala Ala Pro
865                 870                 875                 880

Ala Lys Ala Glu Lys Lys Ala Lys Lys Ser Ala
                885                 890

<210>  16
<211>  2676
<212>  DNA
<213>  Escherichia coli

<400>  16
atggctgtta ctaatgtcgc tgaacttaac gcactcgtag agcgtgtaaa aaaagcccag      60

cgtgaatatg ccagtttcac tcaagagcaa gtagacaaaa tcttccgcgc cgccgctctg     120

gctgctgcag atgctcgaat cccactcgcg aaaatggccg ttgccgaatc cggcatgggt     180

atcgtcgaag ataaagtgat caaaaaccac tttgcttctg aatatatcta caacgcctat     240

aaagatgaaa aaacctgtgg tgttctgtct gaagacgaca cttttggtac catcactatc     300

gctgaaccaa tcggtattat ttgcggtatc gttccgacca ctaacccgac ttcaactgct     360

atcttcaaat cgctgatcag tctgaagacc cgtaacgcca ttatcttctc cccgcacccg     420

cgtgcaaaag atgccaccaa caaagcggct gatatcgttc tgcaggctgc tatcgctgcc     480

ggtgctccga aagatctgat cggctggatc gatcaacctt ctgttgaact gtctaacgca     540

ctgatgcacc acccagacat caacctgatc ctcgcgactg gtggtccggg catggttaaa     600

gccgcataca gctccggtaa accagctatc ggtgtaggcg cgggcaacac tccagttgtt     660

atcgatgaaa ctgctgatat caaacgtgca gttgcatctg tactgatgtc caaaaccttc     720

gacaacggcg taatctgtgc ttctgaacag tctgttgttg ttgttgactc tgtttatgac     780

gctgtacgtg aacgttttgc aacccacggc ggctatctgt tgcagggtaa agagctgaaa     840

gctgttcagg atgttatcct gaaaaacggt gcgctgaacg cggctatcgt tggtcagcca     900

gcctataaaa ttgctgaact ggcaggcttc tctgtaccag aaaacaccaa gattctgatc     960

ggtgaagtga ccgttgttga tgaaagcgaa ccgttcgcac atgaaaaact gtccccgact    1020

ctggcaatgt accgcgctaa agatttcgaa gacgcggtag aaaaagcaga gaaactggtt 1080

gctatgggcg gtatcggtca tacctcttgc ctgtacactg accaggataa ccaaccggct 1140

cgcgtttctt acttcggtca gaaaatgaaa acggcgcgta tcctgattaa caccccagcg 1200

tctcagggtg gtatcggtga cctgtataac ttcaaactcg caccttccct gactctgggt 1260

tgtggttctt ggggtggtaa ctccatctct gaaaacgttg gtccgaaaca cctgatcaac 1320

aagaaaaccg ttgctaagcg agctgaaaac atgttgtggc acaaacttcc gaaatctatc 1380

tacttccgcc gtggctccct gccaatcgcg ctggatgaag tgattactga tggccacaaa 1440

cgtgcgctca tcgtgactga ccgcttcctg ttcaacaatg gttatgctga tcagatcact 1500

tccgtactga aagcagcagg cgttgaaact gaagtcttct tcgaagtaga agcggacccg 1560

accctgagca tcgttcgtaa aggtgcagaa ctggcaaact ccttcaaacc agacgtgatt 1620

atcgcgctgg gtggtggttc cccgatggac gccgcgaaga tcatgtgggt tatgtacgaa 1680

catccggaaa ctcacttcga agagctggcg ctgcgcttta tggatatccg taaacgtatc 1740

tacaagttcc cgaaaatggg cgtgaaagcg aaaatgatcg ctgtcaccac cacttctggt 1800

acaggttctg aagtcactcc gtttgcggtt gtaactgacg acgctactgg tcagaaatat 1860

ccgctggcag actatgcgct gactccggat atggcgattg tcgacgccaa cctggttatg 1920

gacatgccga agtccctgtg tgctttcggt ggtctggacg cagtaactca cgccatggaa 1980

gcttatgttt ctgtactggc atctgagttc tctgatggtc aggctctgca ggcactgaaa 2040

ctgctgaaag aatatctgcc agcgtcctac cacgaagggt ctaaaaatcc ggtagcgcgt 2100

gaacgtgttc acagtgcagc gactatcgcg ggtatcgcgt ttgcgaacgc cttcctgggt 2160

gtatgtcact caatggcgca caaactgggt tcccagttcc atattccgca cggtctggca 2220

aacgccctgc tgatttgtaa cgttattcgc tacaatgcga acgacaaccc gaccaagcag 2280

actgcattca gccagtatga ccgtccgcag gctcgccgtc gttatgctga aattgccgac 2340

cacttgggtc tgagcgcacc gggcgaccgt actgctgcta agatcgagaa actgctggca 2400

tggctggaaa cgctgaaagc tgaactgggt attccgaaat ctatccgtga agctggcgtt 2460

caggaagcag acttcctggc gaacgtggat aaactgtctg aagatgcatt cgatgaccag 2520

tgcaccggcg ctaacccgcg ttacccgctg atctccgagc tgaaacagat cctgctggat 2580

acctactacg gtcgtgatta tgtagaaggt gaaactgcag cgaaaaaaga agccgctccg 2640

gctaaagctg agaaaaaagc gaaaaaatcc gcttaa 2676

<210> 17
<211> 412
<212> PRT
<213> Escherichia coli

<400> 17

Met Lys Ala Leu Thr Tyr His Gly Pro His His Val Gln Val Glu Asn
1               5                   10                  15

Val Pro Asp Pro Gly Ile Glu Gln Ala Asp Asp Ile Ile Leu Arg Ile
            20                  25                  30

Thr Ala Thr Ala Ile Cys Gly Ser Asp Leu His Leu Tyr Arg Gly Lys
        35                  40                  45

Ile Pro Gln Val Lys His Gly Asp Ile Phe Gly His Glu Phe Met Gly
    50                  55                  60

Glu Val Val Glu Thr Gly Lys Asp Val Lys Asn Leu Gln Lys Gly Asp
65                  70                  75                  80

Arg Val Val Ile Pro Phe Val Ile Ala Cys Gly Asp Cys Phe Phe Cys
                85                  90                  95

Arg Leu Gln Gln Tyr Ala Ala Cys Glu Asn Thr Asn Ala Gly Lys Gly
            100                 105                 110

Ala Ala Leu Asn Lys Lys Gln Ile Pro Ala Pro Ala Ala Leu Phe Gly
        115                 120                 125

Tyr Ser His Leu Tyr Gly Gly Val Pro Gly Gly Gln Ala Glu Tyr Val
    130                 135                 140

Arg Val Pro Lys Gly Asn Val Gly Pro Phe Lys Val Pro Pro Leu Leu
145                 150                 155                 160

Ser Asp Asp Lys Ala Leu Phe Leu Ser Asp Ile Leu Pro Thr Ala Trp
                165                 170                 175

Gln Ala Ala Lys Asn Ala Gln Ile Gln Gln Gly Ser Ser Val Ala Val
            180                 185                 190

Tyr Gly Ala Gly Pro Val Gly Leu Leu Thr Ile Ala Cys Ala Arg Leu
        195                 200                 205

Leu Gly Ala Glu Gln Ile Phe Val Val Asp His His Pro Tyr Arg Leu
    210                 215                 220

His Phe Ala Ala Asp Arg Tyr Gly Ala Ile Pro Ile Asn Phe Asp Glu
225                 230                 235                 240

Asp Ser Asp Pro Ala Gln Ser Ile Ile Glu Gln Thr Ala Gly His Arg

245                 250                 255

Gly Val Asp Ala Val Ile Asp Ala Val Gly Phe Glu Ala Lys Gly Ser
            260                 265                 270

Thr Thr Glu Thr Val Leu Thr Asn Leu Lys Leu Glu Gly Ser Ser Gly
        275                 280                 285

Lys Ala Leu Arg Gln Cys Ile Ala Ala Val Arg Arg Gly Gly Ile Val
    290                 295                 300

Ser Val Pro Gly Val Tyr Ala Gly Phe Ile His Gly Phe Leu Phe Gly
305                 310                 315                 320

Asp Ala Phe Asp Lys Gly Leu Ser Phe Lys Met Gly Gln Thr His Val
                325                 330                 335

His Ala Trp Leu Gly Glu Leu Leu Pro Leu Ile Glu Lys Gly Leu Leu
            340                 345                 350

Lys Pro Glu Glu Ile Val Thr His Tyr Met Pro Phe Glu Glu Ala Ala
        355                 360                 365

Arg Gly Tyr Glu Ile Phe Glu Lys Arg Glu Glu Glu Cys Arg Lys Val
    370                 375                 380

Ile Leu Val Pro Gly Ala Gln Ser Ala Glu Ala Ala Gln Lys Ala Val
385                 390                 395                 400

Ser Gly Leu Val Asn Ala Met Pro Gly Gly Thr Ile
                405                 410

<210>  18
<211>  1239
<212>  DNA
<213>  Escherichia coli

<400>  18
atgaaagcat tgacttatca cggcccacat cacgttcagg tagaaaatgt tcccgatccg      60

ggcattgaac aggcagatga tattattctg cgtattacgg caacggcgat ctgtggctct     120

gacctccatc tttatcgagg caaaataccc caggttaaac atggcgatat ttttggtcat     180

gaatttatgg gggaagtcgt tgaaaccgga aaggacgtaa aaaatttgca aaaaggcgac     240

cgggtggtaa ttccgtttgt cattgcttgt ggcgactgtt ttttctgtcg attacagcaa     300

tatgccgcct gcgaaaatac caatgcgggt aaaggcgctg cgctcaataa aaaacagata     360

ccagctcccg cggcattgtt tggttatagt cacctgtatg gcggcgttcc tggtgggcag     420

gcggaatatg tccgcgtccc taaagggaat gtggggccgt ttaaagtacc gcctttgctt 480

tcagatgata aagcgctttt cctttctgat attctgccaa cggcatggca ggcagcaaaa 540

aatgcgcaga tccaacaagg ttcaagcgtt gcagtctatg gtgctggtcc tgtgggattg 600

ttgacaatcg cctgtgcacg gttgctcggt gcggaacaga tttttgttgt tgatcatcat 660

ccctaccgct tgcatttcgc cgccgaccgc tacggcgcga tcccgattaa ttttgatgaa 720

gacagcgatc cggcacagtc aattattgaa caaacggcag gtcaccgggg cgtggatgca 780

gtaatagacg ccgtcggttt tgaagcgaaa ggcagcacca cggaaacggt gctgactaac 840

ctgaaactgg agggcagcag cggtaaagcg ttgcgtcagt gtattgcggc ggtcaggcgt 900

ggcggcattg ttagcgtacc gggcgtctac gctggattta ttcacggttt cctgtttggc 960

gacgcctttg ataaagggtt gtcgtttaaa atgggacaga cccacgttca cgcatggctg 1020

ggagaattat taccgttaat tgagaaagga ttactgaaac cagaagaaat tgttacccac 1080

tatatgccgt ttgaagaggc cgcccgggga tatgagattt tcgaaaaacg tgaagaggag 1140

tgccgtaagg tgattctggt acccggtgca caaagcgcag aggcggcgca gaaggcggtt 1200

tcaggtctag tgaatgcgat gccgggggga acaatatga 1239

<210> 19
<211> 275
<212> PRT
<213> Escherichia coli

<400> 19

Met Ala Asn Pro Thr Val Ile Lys Leu Gln Asp Gly Asn Val Met Pro
1               5                   10                  15

Gln Leu Gly Leu Gly Val Trp Gln Ala Ser Asn Glu Glu Val Ile Thr
            20                  25                  30

Ala Ile Gln Lys Ala Leu Glu Val Gly Tyr Arg Ser Ile Asp Thr Ala
        35                  40                  45

Ala Ala Tyr Lys Asn Glu Glu Gly Val Gly Lys Ala Leu Lys Asn Ala
    50                  55                  60

Ser Val Asn Arg Glu Glu Leu Phe Ile Thr Thr Lys Leu Trp Asn Asp
65                  70                  75                  80

Asp His Lys Arg Pro Arg Glu Ala Leu Leu Asp Ser Leu Lys Lys Leu
                85                  90                  95

Gln Leu Asp Tyr Ile Asp Leu Tyr Leu Met His Trp Pro Val Pro Ala
            100                 105                 110

Ile Asp His Tyr Val Glu Ala Trp Lys Gly Met Ile Glu Leu Gln Lys
        115                 120                 125

Glu Gly Leu Ile Lys Ser Ile Gly Val Cys Asn Phe Gln Ile His His
    130                 135                 140

Leu Gln Arg Leu Ile Asp Glu Thr Gly Val Thr Pro Val Ile Asn Gln
145                 150                 155                 160

Ile Glu Leu His Pro Leu Met Gln Gln Arg Gln Leu His Ala Trp Asn
                165                 170                 175

Ala Thr His Lys Ile Gln Thr Glu Ser Trp Ser Pro Leu Ala Gln Gly
            180                 185                 190

Gly Lys Gly Val Phe Asp Gln Lys Val Ile Arg Asp Leu Ala Asp Lys
        195                 200                 205

Tyr Gly Lys Thr Pro Ala Gln Ile Val Ile Arg Trp His Leu Asp Ser
    210                 215                 220

Gly Leu Val Val Ile Pro Lys Ser Val Thr Pro Ser Arg Ile Ala Glu
225                 230                 235                 240

Asn Phe Asp Val Trp Asp Phe Arg Leu Asp Lys Asp Glu Leu Gly Glu
                245                 250                 255

Ile Ala Lys Leu Asp Gln Gly Lys Arg Leu Gly Pro Asp Pro Asp Gln
            260                 265                 270

Phe Gly Gly
        275

<210>  20
<211>  828
<212>  DNA
<213>  Escherichia coli

<400>  20
atggctaatc caaccgttat taagctacag gatggcaatg tcatgcccca gctgggactg      60

ggcgtctggc aagcaagtaa tgaggaagta atcaccgcca ttcaaaaagc gttagaagtg     120

ggttatcgct cgattgatac cgccgcggcc tacaagaacg aagaaggtgt cggcaaagcc     180

ctgaaaaatg cctcagtcaa cagagaagaa ctgttcatca ccactaagct gtggaacgac     240

gaccacaagc gcccccgcga agccctgctc gacagcctga aaaaactcca gcttgattat     300

atcgacctct acttaatgca ctggcccgtt cccgctatcg accattatgt cgaagcatgg     360

aaaggcatga tcgaattgca aaaagaggga ttaatcaaaa gcatcggcgt gtgcaacttc 420

cagatccatc acctgcaacg cctgattgat gaaactggcg tgacgcctgt gataaaccag 480

atcgaacttc atccgctgat gcaacaacgc cagctacacg cctggaacgc gacacacaaa 540

atccagaccg aatcctggag cccattagcg caaggaggga aaggcgtttt cgatcagaaa 600

gtcattcgcg atctggcaga taaatacggc aaaaccccgg cgcagattgt tatccgctgg 660

catctggata gcggcctggt ggtgatcccg aaatcggtca caccttcacg tattgccgaa 720

aactttgatg tctgggattt ccgtctcgac aaagacgaac tcggcgaaat tgcaaaactc 780

gatcagggca agcgtctcgg tcccgatcct gaccagttcg gcggctaa 828

<210> 21
<211> 383
<212> PRT
<213> Escherichia coli

<400> 21

Met Ala Ser Ser Thr Phe Phe Ile Pro Ser Val Asn Val Ile Gly Ala
1               5                   10                  15

Asp Ser Leu Thr Asp Ala Met Asn Met Met Ala Asp Tyr Gly Phe Thr
            20                  25                  30

Arg Thr Leu Ile Val Thr Asp Asn Met Leu Thr Lys Leu Gly Met Ala
        35                  40                  45

Gly Asp Val Gln Lys Ala Leu Glu Glu Arg Asn Ile Phe Ser Val Ile
    50                  55                  60

Tyr Asp Gly Thr Gln Pro Asn Pro Thr Thr Glu Asn Val Ala Ala Gly
65                  70                  75                  80

Leu Lys Leu Leu Lys Glu Asn Asn Cys Asp Ser Val Ile Ser Leu Gly
                85                  90                  95

Gly Gly Ser Pro His Asp Cys Ala Lys Gly Ile Ala Leu Val Ala Ala
            100                 105                 110

Asn Gly Gly Asp Ile Arg Asp Tyr Glu Gly Val Asp Arg Ser Ala Lys
        115                 120                 125

Pro Gln Leu Pro Met Ile Ala Ile Asn Thr Thr Ala Gly Thr Ala Ser
    130                 135                 140

Glu Met Thr Arg Phe Cys Ile Ile Thr Asp Glu Ala Arg His Ile Lys
145                 150                 155                 160

Met Ala Ile Val Asp Lys His Val Thr Pro Leu Leu Ser Val Asn Asp
                    165                 170                 175

Ser Ser Leu Met Ile Gly Met Pro Lys Ser Leu Thr Ala Ala Thr Gly
            180                 185                 190

Met Asp Ala Leu Thr His Ala Ile Glu Ala Tyr Val Ser Ile Ala Ala
        195                 200                 205

Thr Pro Ile Thr Asp Ala Cys Ala Leu Lys Ala Val Thr Met Ile Ala
    210                 215                 220

Glu Asn Leu Pro Leu Ala Val Glu Asp Gly Ser Asn Ala Lys Ala Arg
225                 230                 235                 240

Glu Ala Met Ala Tyr Ala Gln Phe Leu Ala Gly Met Ala Phe Asn Asn
                    245                 250                 255

Ala Ser Leu Gly Tyr Val His Ala Met Ala His Gln Leu Gly Gly Phe
            260                 265                 270

Tyr Asn Leu Pro His Gly Val Cys Asn Ala Val Leu Leu Pro His Val
        275                 280                 285

Gln Val Phe Asn Ser Lys Val Ala Ala Ala Arg Leu Arg Asp Cys Ala
    290                 295                 300

Ala Ala Met Gly Val Asn Val Thr Gly Lys Asn Asp Ala Glu Gly Ala
305                 310                 315                 320

Glu Ala Cys Ile Asn Ala Ile Arg Glu Leu Ala Lys Lys Val Asp Ile
                    325                 330                 335

Pro Ala Gly Leu Arg Asp Leu Asn Val Lys Glu Glu Asp Phe Ala Val
            340                 345                 350

Leu Ala Thr Asn Ala Leu Lys Asp Ala Cys Gly Phe Thr Asn Pro Ile
        355                 360                 365

Gln Ala Thr His Glu Glu Ile Val Ala Ile Tyr Arg Ala Ala Met
    370                 375                 380

<210>  22
<211>  1152
<212>  DNA
<213>  Escherichia coli

<400>  22

atggcatctt caactttctt tattccttct gtgaatgtca tcggcgctga ttcattgact 60

gatgcaatga atatgatggc agattatgga tttacccgta ccttaattgt cactgacaat 120

atgttaacga aattaggtat ggcgggtgat gtgcaaaaag cactggaaga acgcaatatt 180

tttagcgtta tttatgatgg cacccaacct aacccaacca cggaaaacgt cgccgcaggt 240

ttgaaattac ttaaagaaaa taattgcgat agcgtgattt ccttaggcgg tggttctccg 300

catgactgtg caaaaggtat tgcgctggtg gcagccaatg gtggtgatat ccgtgattat 360

gaaggcgttg accgctctgc aaaaccgcag ctgccgatga tcgccatcaa taccactgcg 420

ggtacagcat cagaaatgac tcgtttctgc atcatcaccg acgaagcgcg tcacatcaaa 480

atggcgattg ttgataagca cgtgactccg ctgctttctg tcaatgactc ctcgctgatg 540

atcggtatgc cgaagtcact gaccgccgcc actggtatgg acgccttaac gcacgctatc 600

gaagcgtatg tttctattgc cgccacgccg atcactgacg cttgtgcact gaaagccgtg 660

accatgattg ccgaaaacct gccgttagcc gttgaagatg gcagtaatgc gaaagcgcgt 720

gaagcaatgg cttatgccca gttcctcgcc ggtatggcgt tcaataatgc ttctctgggt 780

tatgttcatg cgatggcgca ccagctgggc ggtttctaca acctgccaca cggtgtatgt 840

aacgccgttt tgctgccgca tgttcaggta ttcaacagca aagtcgccgc cgcacgtctg 900

cgtgactgtg ccgctgcaat gggcgtgaac gtgacaggta aaaacgatgc ggaaggtgct 960

gaagcctgca ttaacgccat ccgtgaactg gcgaagaaag tggatatccc ggcaggccta 1020

cgcgacctga acgtgaaaga agaagatttc gcggttctgg cgactaatgc cctgaaagat 1080

gcctgtggtt ttactaaccc gatccaggca actcacgaag aaattgtggc gatttatcgc 1140

gcagcgatgt aa 1152

<210> 23
<211> 369
<212> PRT
<213> Escherichia coli

<400> 23

Met Lys Ser Arg Ala Ala Val Ala Phe Ala Pro Gly Lys Pro Leu Glu
1               5                   10                  15

Ile Val Glu Ile Asp Val Ala Pro Pro Lys Lys Gly Glu Val Leu Ile
            20                  25                  30

Lys Val Thr His Thr Gly Val Cys His Thr Asp Ala Phe Thr Leu Ser
        35                  40                  45

Gly Asp Asp Pro Glu Gly Val Phe Pro Val Val Leu Gly His Glu Gly
    50                  55                  60

Ala Gly Val Val Val Glu Val Gly Glu Gly Val Thr Ser Val Lys Pro
65                  70                  75                  80

Gly Asp His Val Ile Pro Leu Tyr Thr Ala Glu Cys Gly Glu Cys Glu
                85                  90                  95

Phe Cys Arg Ser Gly Lys Thr Asn Leu Cys Val Ala Val Arg Glu Thr
            100                 105                 110

Gln Gly Lys Gly Leu Met Pro Asp Gly Thr Thr Arg Phe Ser Tyr Asn
        115                 120                 125

Gly Gln Pro Leu Tyr His Tyr Met Gly Cys Ser Thr Phe Ser Glu Tyr
    130                 135                 140

Thr Val Val Ala Glu Val Ser Leu Ala Lys Ile Asn Pro Glu Ala Asn
145                 150                 155                 160

His Glu His Val Cys Leu Leu Gly Cys Gly Val Thr Thr Gly Ile Gly
                165                 170                 175

Ala Val His Asn Thr Ala Lys Val Gln Pro Gly Asp Ser Val Ala Val
            180                 185                 190

Phe Gly Leu Gly Ala Ile Gly Leu Ala Val Val Gln Gly Ala Arg Gln
        195                 200                 205

Ala Lys Ala Gly Arg Ile Ile Ala Ile Asp Thr Asn Pro Lys Lys Phe
    210                 215                 220

Asp Leu Ala Arg Arg Phe Gly Ala Thr Asp Cys Ile Asn Pro Asn Asp
225                 230                 235                 240

Tyr Asp Lys Pro Ile Lys Asp Val Leu Leu Asp Ile Asn Lys Trp Gly
                245                 250                 255

Ile Asp His Thr Phe Glu Cys Ile Gly Asn Val Asn Val Met Arg Ala
            260                 265                 270

Ala Leu Glu Ser Ala His Arg Gly Trp Gly Gln Ser Val Ile Ile Gly
        275                 280                 285

Val Ala Gly Ala Gly Gln Glu Ile Ser Thr Arg Pro Phe Gln Leu Val
    290                 295                 300

Thr Gly Arg Val Trp Lys Gly Ser Ala Phe Gly Gly Val Lys Gly Arg
305                 310                 315                 320

Ser Gln Leu Pro Gly Met Val Glu Asp Ala Met Lys Gly Asp Ile Asp
                325                 330                 335

Leu Glu Pro Phe Val Thr His Thr Met Ser Leu Asp Glu Ile Asn Asp
            340                 345                 350

Ala Phe Asp Leu Met His Glu Gly Lys Ser Ile Arg Thr Val Ile Arg
        355                 360                 365

Tyr

<210>  24
<211>  1110
<212>  DNA
<213>  Escherichia coli

<400>  24
atgaaatcac gtgctgccgt tgcatttgct cccggtaaac cgctggaaat cgttgaaatt      60

gacgttgcac caccgaaaaa aggtgaagtg ctgattaaag tcacccatac cggcgtttgc     120

cataccgacg catttaccct ctccggggat gacccggaag gtgtattccc ggtggttctc     180

ggtcacgaag gggccggcgt tgtggttgaa gtcggtgaag gcgtaaccag cgtcaaacct     240

ggcgaccatg tgatcccgct ttacaccgcg gagtgcggcg agtgtgagtt ctgtcgttct     300

ggcaaaacta acctctgtgt tgcggttcgc gaaacccagg gtaaaggctt gatgccagac     360

ggcaccaccc gtttttctta caacgggcag ccgctttatc actacatggg atgctcaaca     420

ttcagtgaat acaccgtggt cgcggaagtg tctctggcca aaattaatcc agaagcaaac     480

catgaacacg tctgcctgct gggctgtggc gtgaccaccg gtattggcgc ggtgcacaac     540

acagctaaag tccagccagg tgattctgtt gccgtgtttg gtcttggcgc gattggtctg     600

gcagtggttc agggcgcgcg tcaggcgaaa gcgggacgga ttatcgctat cgataccaac     660

ccgaagaaat tcgatctggc tcgtcgcttc ggtgctaccg actgcattaa cccgaatgac     720

tacgacaaac cgattaaaga tgtcctgctg gatatcaaca aatggggtat cgaccatacc     780

tttgaatgca tcggtaacgt caacgtgatg cgtgcggcgc tggaaagtgc gcaccgcggc     840

tggggtcagt cggtgatcat cggggtagca ggtgccggtc aggaaatctc cacccgacca     900

ttccagttgg tcaccggtcg cgtatggaaa ggttccgcgt ttggcggcgt gaaaggtcgt     960

tcccagttac cgggtatggt tgaagatgcg atgaaaggtg atatcgatct ggaaccgttt    1020

gtcacgcata ccatgagcct tgatgaaatt aatgacgcct tcgacctgat gcatgaaggc    1080

aaatccattc gaaccgtaat tcgttactga                                     1110

<210>  25
<211>  267
<212>  PRT
<213>  Escherichia coli

<400>  25

Met Ala Ile Pro Ala Phe Gly Leu Gly Thr Phe Arg Leu Lys Asp Asp
1               5                   10                  15

Val Val Ile Ser Ser Val Lys Thr Ala Leu Glu Leu Gly Tyr Arg Ala
            20                  25                  30

Ile Asp Thr Ala Gln Ile Tyr Asp Asn Glu Ala Ala Val Gly Gln Ala
        35                  40                  45

Ile Ala Glu Ser Gly Val Pro Arg His Glu Leu Tyr Ile Thr Thr Lys
    50                  55                  60

Ile Trp Ile Glu Asn Leu Ser Lys Asp Lys Leu Ile Pro Ser Leu Lys
65                  70                  75                  80

Glu Ser Leu Gln Lys Leu Arg Thr Asp Tyr Val Asp Leu Thr Leu Ile
                85                  90                  95

His Trp Pro Ser Pro Asn Asp Glu Val Ser Val Glu Glu Phe Met Gln
            100                 105                 110

Glu Leu Leu Glu Ala Lys Lys Glu Gly Leu Thr Arg Glu Ile Gly Ile
        115                 120                 125

Ser Asn Phe Thr Ile Pro Leu Met Glu Lys Ala Ile Ala Ala Val Gly
    130                 135                 140

Ala Glu Asn Ile Ala Thr Asn Gln Ile Glu Leu Ser Pro Tyr Leu Gln
145                 150                 155                 160

Asn Arg Lys Val Val Ala Trp Ala Lys Gln His Gly Ile His Ile Thr
                165                 170                 175

Ser Tyr Met Thr Leu Ala Tyr Gly Lys Ala Leu Lys Asp Glu Val Ile
            180                 185                 190

Ala Arg Ile Ala Ala Lys His Asn Ala Thr Pro Ala Gln Val Ile Leu
        195                 200                 205

Ala Trp Ala Met Gly Glu Gly Tyr Ser Val Ile Pro Ser Ser Thr Lys
    210                 215                 220

Arg Lys Asn Leu Glu Ser Asn Leu Lys Ala Gln Asn Leu Gln Leu Asp
225                 230                 235                 240

Ala Glu Asp Lys Lys Ala Ile Ala Ala Leu Asp Cys Asn Asp Arg Leu
                245                 250                 255

Val Ser Pro Glu Gly Leu Ala Pro Glu Trp Asp
            260                 265

<210>  26
<211>  804
<212>  DNA
<213>  Escherichia coli

<400>  26
atggctatcc ctgcatttgg tttaggtact ttccgtctga aagacgacgt tgttatttca      60

tctgtgaaaa cggcgcttga acttggttat cgcgcaattg atactgcaca aatctatgat     120

aacgaagccg cagtaggtca ggcgattgca gaaagtggcg tgccacgtca tgaactctac     180

atcaccacta aaatctggat tgaaaatctc agcaaagaca aattgatccc gagtctgaaa     240

gagagcctgc aaaaattgcg tactgattat gttgatctga ctctaatcca ctggccgtca     300

ccaaacgatg aagtctctgt tgaagagttt atgcaggagc tgctggaagc caaaaaagaa     360

gggttgacgc gtgagatcgg tatttccaac ttcacgatcc cattgatgga aaaggcgatt     420

gctgctgttg gcgctgaaaa catcgctact aaccagattg aactctctcc ttatctgcaa     480

aaccgtaaag tggttgcctg ggctaaacag cacggcatcc atattacttc ctatatgacg     540

ctggcgtatg gtaaggccct gaaagatgag gttattgctc gtatcgcagc taaacacaat     600

gcgactccgg cacaagtgat tctggcgtgg gctatggggg aaggttactc agtaattcct     660

tcttctacta aacgtaaaaa cctggaaagt aatcttaagg cacaaaattt acagcttgat     720

gccgaagata aaaaagcgat cgccgcactg gattgcaacg accgcctggt tagcccggaa     780

ggtctggctc ctgaatggga ttaa                                            804

<210>  27
<211>  294
<212>  PRT
<213>  Escherichia coli

<400>  27

Met Ser His Leu Lys Asp Pro Thr Thr Gln Tyr Tyr Thr Gly Glu Tyr
1               5                   10                  15

Pro Lys Gln Lys Gln Pro Thr Pro Gly Ile Gln Ala Lys Met Thr Pro
            20                  25                  30

Val Pro Asp Cys Gly Glu Lys Thr Tyr Val Gly Ser Gly Arg Leu Lys

35                  40                  45

Asp Arg Lys Ala Leu Val Thr Gly Gly Asp Ser Gly Ile Gly Arg Ala
    50                  55                  60

Ala Ala Ile Ala Tyr Ala Arg Glu Gly Ala Asp Val Ala Ile Ser Tyr
65                  70                  75                  80

Leu Pro Val Glu Glu Glu Asp Ala Gln Asp Val Lys Lys Ile Ile Glu
                85                  90                  95

Glu Cys Gly Arg Lys Ala Val Leu Leu Pro Gly Asp Leu Ser Asp Glu
            100                 105                 110

Lys Phe Ala Arg Ser Leu Val His Glu Ala His Lys Ala Leu Gly Gly
        115                 120                 125

Leu Asp Ile Met Ala Leu Val Ala Gly Lys Gln Val Ala Ile Pro Asp
    130                 135                 140

Ile Ala Asp Leu Thr Ser Glu Gln Phe Gln Lys Thr Phe Ala Ile Asn
145                 150                 155                 160

Val Phe Ala Leu Phe Trp Leu Thr Gln Glu Ala Ile Pro Leu Leu Pro
                165                 170                 175

Lys Gly Ala Ser Ile Ile Thr Thr Ser Ser Ile Gln Ala Tyr Gln Pro
            180                 185                 190

Ser Pro His Leu Leu Asp Tyr Ala Ala Thr Lys Ala Ala Ile Leu Asn
        195                 200                 205

Tyr Ser Arg Gly Leu Ala Lys Gln Val Ala Glu Lys Gly Ile Arg Val
    210                 215                 220

Asn Ile Val Ala Pro Gly Pro Ile Trp Thr Ala Leu Gln Ile Ser Gly
225                 230                 235                 240

Gly Gln Thr Gln Asp Lys Ile Pro Gln Phe Gly Gln Gln Thr Pro Met
                245                 250                 255

Lys Arg Ala Gly Gln Pro Ala Glu Leu Ala Pro Val Tyr Val Tyr Leu
            260                 265                 270

Ala Ser Gln Glu Ser Ser Tyr Val Thr Ala Glu Val His Gly Val Cys
        275                 280                 285

Gly Gly Glu His Leu Gly
    290

<210>  28
<211>  885
<212>  DNA
<213>  Escherichia coli

<400>  28
atgtctcatt taaaagaccc gaccacgcag tattacactg gtgaatatcc caaacagaaa        60

caaccgacgc caggcatcca ggcgaagatg acaccggtac cggattgcgg cgagaaaacc       120

tatgttggta gcggtcgcct gaaagatcgt aaagcactgg tgacaggggg cgattccgga       180

ataggtcgcg ctgccgccat cgcttacgcg cgtgaagggg ctgacgtggc gatcagttat       240

cttcccgtgg aagaagaaga cgctcaggat gtgaaaaaga tcattgaaga atgcggacgc       300

aaagccgttc tgctgccagg cgatttaagc gatgagaaat ttgcccgttc gctggttcac       360

gaagcgcaca aggcgttagg cgggctggat attatggcgc tggtcgccgg gaaacaggtt       420

gccattccgg atattgcaga cctcaccagc gaacagtttc aaaagacctt tgccattaac       480

gttttcgcgc tgttctggct aacccaggaa gcgatccccc tgctaccgaa aggtgcaagt       540

atcatcacca cttcgtcaat ccaggcatac cagccaagtc cgcatttact ggactatgcg       600

gctacgaagg cggcgattct gaactacagc cgtggcttgg caaaacaggt cgcggagaaa       660

ggtattcggg tgaatattgt cgcgccaggc ccgatctgga cagcactgca aatttccggc       720

ggacaaacgc aggataagat cccgcagttt ggtcagcaaa cgccgatgaa acgtgcgggg       780

caaccggcgg aactggcccc tgtatatgtt tatctggcaa gtcaggagtc gagctacgtc       840

accgcagaag tgcacggcgt gtgcggcggc gagcatttag gttaa                       885

<210>  29
<211>  326
<212>  PRT
<213>  Escherichia coli

<400>  29

Met Lys Lys Ile Pro Leu Gly Thr Thr Asp Ile Thr Leu Ser Arg Met
1               5                   10                  15

Gly Leu Gly Thr Trp Ala Ile Gly Gly Gly Pro Ala Trp Asn Gly Asp
            20                  25                  30

Leu Asp Arg Gln Ile Cys Ile Asp Thr Ile Leu Glu Ala His Arg Cys
        35                  40                  45

Gly Ile Asn Leu Ile Asp Thr Ala Pro Gly Tyr Asn Phe Gly Asn Ser
    50                  55                  60

Glu Val Ile Val Gly Gln Ala Leu Lys Lys Leu Pro Arg Glu Gln Val
65                  70                  75                  80

Val Val Glu Thr Lys Cys Gly Ile Val Trp Glu Arg Lys Gly Ser Leu
                85                  90                  95

Phe Asn Lys Val Gly Asp Arg Gln Leu Tyr Lys Asn Leu Ser Pro Glu
            100                 105                 110

Ser Ile Arg Glu Glu Val Glu Ala Ser Leu Gln Arg Leu Gly Ile Asp
        115                 120                 125

Tyr Ile Asp Ile Tyr Met Thr His Trp Gln Ser Val Pro Pro Phe Phe
    130                 135                 140

Thr Pro Ile Ala Glu Thr Val Ala Val Leu Asn Glu Leu Lys Ala Glu
145                 150                 155                 160

Gly Lys Ile Arg Ala Ile Gly Ala Ala Asn Val Asp Ala Asp His Ile
                165                 170                 175

Arg Glu Tyr Leu Gln Tyr Gly Glu Leu Asp Ile Ile Gln Ala Lys Tyr
            180                 185                 190

Ser Ile Leu Asp Arg Ala Met Glu Asn Glu Leu Leu Pro Leu Cys Arg
        195                 200                 205

Asp Asn Gly Ile Val Val Gln Val Tyr Ser Pro Leu Glu Gln Gly Leu
    210                 215                 220

Leu Thr Gly Thr Ile Thr Arg Asp Tyr Val Pro Gly Gly Ala Arg Ala
225                 230                 235                 240

Asn Lys Val Trp Phe Gln Arg Glu Asn Met Leu Lys Val Ile Asp Met
                245                 250                 255

Leu Glu Gln Trp Gln Pro Leu Cys Ala Arg Tyr Gln Cys Thr Ile Pro
            260                 265                 270

Thr Leu Ala Leu Ala Trp Ile Leu Lys Gln Ser Asp Leu Ile Ser Ile
        275                 280                 285

Leu Ser Gly Ala Thr Ala Pro Glu Gln Val Arg Glu Asn Val Ala Ala
    290                 295                 300

Leu Asn Ile Asn Leu Ser Asp Ala Asp Ala Thr Leu Met Arg Glu Met
305                 310                 315                 320

Ala Glu Ala Leu Glu Arg
                325

<210>  30
<211>  981
<212>  DNA
<213>  Escherichia coli

<400>  30
atgaaaaaaa tacctttagg cacaacggat attacgcttt cgcgaatggg gttggggaca        60

tgggccattg gcggcggtcc tgcatggaat ggcgatctcg atcggcaaat atgtattgat       120

acgattcttg aagcccatcg ttgcggcatt aatctgattg atactgcacc aggatataac       180

tttggcaata gtgaagttat cgtcggtcag gcgttaaaaa aactgccccg tgaacaggtt       240

gtagtagaaa ccaaatgcgg cattgtctgg gaacgaaaag gaagtttatt caacaaagtt       300

ggcgatcggc agttgtataa aaacctttcc ccggaatcta tccgcgaaga ggtagaagcc       360

agcttgcaac gtctgggtat tgattacatc gatatctaca tgacgcactg gcagtcggtg       420

ccgccatttt ttacgccgat agctgaaact gtcgcagtgc ttaatgagtt aaaagccgaa       480

gggaaaattc gcgcgatagg cgctgctaac gtcgatgctg accatatccg cgagtatctg       540

caatatggtg aactggatat tattcaggcg aaatacagta tcctcgaccg ggcaatggaa       600

aacgaactgc tgccgctatg tcgtgataat ggcattgtgg ttcaggttta ttccccgcta       660

gagcagggat tgttgaccgg caccatcact cgtgattacg ttccgggcgg cgctcgggca       720

aataaagtct ggttccagcg tgaaaacatg ctgaaagtga ttgatatgct tgaacagtgg       780

cagccacttt gtgctcgtta tcagtgcaca attcccactc tggcactggc gtggatatta       840

aaacagagtg atttaatctc cattcttagt ggggctactg caccggaaca ggtacgcgaa       900

aatgtcgcgg cactgaatat caacttatcg gatgcagacg caacattgat gagggaaatg       960

gcagaggccc tggagcgtta a                                                 981

<210>  31
<211>  367
<212>  PRT
<213>  Escherichia coli

<400>  31

Met Asp Arg Ile Ile Gln Ser Pro Gly Lys Tyr Ile Gln Gly Ala Asp
1               5                   10                  15

Val Ile Asn Arg Leu Gly Glu Tyr Leu Lys Pro Leu Ala Glu Arg Trp
            20                  25                  30

Leu Val Val Gly Asp Lys Phe Val Leu Gly Phe Ala Gln Ser Thr Val

        35                  40                  45

Glu Lys Ser Phe Lys Asp Ala Gly Leu Val Val Glu Ile Ala Pro Phe
    50                  55                  60

Gly Gly Glu Cys Ser Gln Asn Glu Ile Asp Arg Leu Arg Gly Ile Ala
65                  70                  75                  80

Glu Thr Ala Gln Cys Gly Ala Ile Leu Gly Ile Gly Gly Gly Lys Thr
                85                  90                  95

Leu Asp Thr Ala Lys Ala Leu Ala His Phe Met Gly Val Pro Val Ala
            100                 105                 110

Ile Ala Pro Thr Ile Ala Ser Thr Asp Ala Pro Cys Ser Ala Leu Ser
        115                 120                 125

Val Ile Tyr Thr Asp Glu Gly Glu Phe Asp Arg Tyr Leu Leu Leu Pro
    130                 135                 140

Asn Asn Pro Asn Met Val Ile Val Asp Thr Lys Ile Val Ala Gly Ala
145                 150                 155                 160

Pro Ala Arg Leu Leu Ala Ala Gly Ile Gly Asp Ala Leu Ala Thr Trp
                165                 170                 175

Phe Glu Ala Arg Ala Cys Ser Arg Ser Gly Ala Thr Thr Met Ala Gly
            180                 185                 190

Gly Lys Cys Thr Gln Ala Ala Leu Ala Leu Ala Glu Leu Cys Tyr Asn
        195                 200                 205

Thr Leu Leu Glu Glu Gly Glu Lys Ala Met Leu Ala Ala Glu Gln His
    210                 215                 220

Val Val Thr Pro Ala Leu Glu Arg Val Ile Glu Ala Asn Thr Tyr Leu
225                 230                 235                 240

Ser Gly Val Gly Phe Glu Ser Gly Gly Leu Ala Ala Ala His Ala Val
                245                 250                 255

His Asn Gly Leu Thr Ala Ile Pro Asp Ala His His Tyr Tyr His Gly
            260                 265                 270

Glu Lys Val Ala Phe Gly Thr Leu Thr Gln Leu Val Leu Glu Asn Ala
        275                 280                 285

Pro Val Glu Glu Ile Glu Thr Val Ala Ala Leu Ser His Ala Val Gly
    290                 295                 300

Leu Pro Ile Thr Leu Ala Gln Leu Asp Ile Lys Glu Asp Val Pro Ala
305                 310                 315                 320

Lys Met Arg Ile Val Ala Glu Ala Ala Cys Ala Glu Gly Glu Thr Ile
                325                 330                 335

His Asn Met Pro Gly Gly Ala Thr Pro Asp Gln Val Tyr Ala Ala Leu
            340                 345                 350

Leu Val Ala Asp Gln Tyr Gly Gln Arg Phe Leu Gln Glu Trp Glu
        355                 360                 365

<210>  32
<211>  1104
<212>  DNA
<213>  Escherichia coli

<400>  32
atggaccgca ttattcaatc accgggtaaa tacatccagg gcgctgatgt gattaatcgt      60

ctgggcgaat acctgaagcc gctggcagaa cgctggttag tggtgggtga caaatttgtt     120

ttaggttttg ctcaatccac tgtcgagaaa agctttaaag atgctggact ggtagtagaa     180

attgcgccgt ttggcggtga atgttcgcaa aatgagatcg accgtctgcg tggcatcgcg     240

gagactgcgc agtgtggcgc aattctcggt atcggtggcg gaaaaaccct cgatactgcc     300

aaagcactgg cacatttcat gggtgttccg gtagcgatcg caccgactat cgcctctacc     360

gatgcaccgt gcagcgcatt gtctgttatc tacaccgatg agggtgagtt tgaccgctat     420

ctgctgttgc caaataaccc gaatatggtc attgtcgaca ccaaaatcgt cgctggcgca     480

cctgcacgtc tgttagcggc gggtatcggc gatgcgctgg caacctggtt tgaagcgcgt     540

gcctgctctc gtagcggcgc gaccaccatg gcgggcggca agtgcaccca ggctgcgctg     600

gcactggctg aactgtgcta caacaccctg ctggaagaag gcgaaaaagc gatgcttgct     660

gccgaacagc atgtagtgac tccggcgctg gagcgcgtga ttgaagcgaa cacctatttg     720

agcggtgttg gttttgaaag tggtggtctg gctgcggcgc acgcagtgca taacggcctg     780

accgctatcc cggacgcgca tcactattat cacggtgaaa aagtggcatt cggtacgctg     840

acgcagctgg ttctggaaaa cgcgccggtg gaggaaatcg aaaccgtagc tgcgcttagc     900

catgcggtag gtttgccaat aactctcgct caactggata ttaaagaaga tgtcccggcg     960

aaaatgcgaa ttgtggcaga agcggcatgt gcagaaggtg aaaccatcca caacatgcct    1020

ggcggcgcga cgccagatca ggtttacgcc gctctgctgg tagccgacca gtacggtcag    1080

cgtttcctgc aagagtggga ataa                                           1104

<210>  33
<211>  253
<212>  PRT
<213>  Escherichia coli

<400>  33

Met Ala Gln Val Ala Ile Ile Thr Ala Ser Asp Ser Gly Ile Gly Lys
1               5                   10                  15

Glu Cys Ala Leu Leu Leu Ala Gln Gln Gly Phe Asp Ile Gly Ile Thr
            20                  25                  30

Trp His Ser Asp Glu Glu Gly Ala Lys Asp Thr Ala Arg Glu Val Val
        35                  40                  45

Ser His Gly Val Arg Ala Glu Ile Val Gln Leu Asp Leu Gly Asn Leu
    50                  55                  60

Pro Glu Gly Ala Leu Ala Leu Glu Lys Leu Ile Gln Arg Leu Gly Arg
65                  70                  75                  80

Ile Asp Val Leu Val Asn Asn Ala Gly Ala Met Thr Lys Ala Pro Phe
                85                  90                  95

Leu Asp Met Ala Phe Asp Glu Trp Arg Lys Ile Phe Thr Val Asp Val
            100                 105                 110

Asp Gly Ala Phe Leu Cys Ser Gln Ile Ala Ala Arg Gln Met Val Lys
        115                 120                 125

Gln Gly Gln Gly Gly Arg Ile Ile Asn Ile Thr Ser Val His Glu His
    130                 135                 140

Thr Pro Leu Pro Asp Ala Ser Ala Tyr Thr Ala Ala Lys His Ala Leu
145                 150                 155                 160

Gly Gly Leu Thr Lys Ala Met Ala Leu Glu Leu Val Arg His Lys Ile
                165                 170                 175

Leu Val Asn Ala Val Ala Pro Gly Ala Ile Ala Thr Pro Met Asn Gly
            180                 185                 190

Met Asp Asp Ser Asp Val Lys Pro Asp Ala Glu Pro Ser Ile Pro Leu
        195                 200                 205

Arg Arg Phe Gly Ala Thr His Glu Ile Ala Ser Leu Val Val Trp Leu
    210                 215                 220

Cys Ser Glu Gly Ala Asn Tyr Thr Thr Gly Gln Ser Leu Ile Val Asp
225                 230                 235                 240

Gly Gly Phe Met Leu Ala Asn Pro Gln Phe Asn Pro Glu
                245                 250

<210>  34
<211>  762
<212>  DNA
<213>  Escherichia coli

<400>  34
atggcacagg ttgcgattat taccgcctcc gattcgggga tcggcaaaga gtgcgcgtta      60

ttactggcgc agcaggggtt tgatattggt attacctggc actcagatga agaaggggca     120

aaagataccg cgcgtgaggt agttagccac ggcgtacgtg cggagatcgt gcagctggat     180

ctcggcaatc taccagaagg ggcactggcg ctggagaaac tcattcaacg gctggggcgc     240

attgatgtgc tggtgaataa tgcgggtgca atgaccaaag cgccgtttct tgatatggct     300

tttgatgagt ggcgcaagat ttttaccgtt gatgtcgatg gtgcattctt atgctcgcaa     360

attgcggctc gtcagatggt gaaacaaggg cagggcggtc gcatcatcaa cattacgtcg     420

gtacatgaac atacgccgct gccggatgcc agcgcctaca cagccgctaa acatgcgctc     480

ggtgggttaa ccaaagcgat ggcgctggag ctggtcaggc ataagatttt ggtgaacgca     540

gtcgcgcctg gggcgatcgc cacgccaatg aatggcatgg atgacagcga cgtgaagccc     600

gacgcggagc cttcgattcc cttgcggcgt tttggcgcaa cgcatgagat tgccagcctg     660

gtggtgtggc tttgttcgga gggcgcaaat tacaccaccg ggcagtcgtt gatagtggat     720

ggcggcttta tgttggcgaa tccacagttc aacccagaat ag                        762

<210>  35
<211>  284
<212>  PRT
<213>  Escherichia coli

<400>  35
   Met Gln Gln Lys Met Ile Gln Phe Ser Gly Asp Val Ser Leu Pro Ala
   1               5                   10                  15
   Val Gly Gln Gly Thr Trp Tyr Met Gly Glu Asp Ala Ser Gln Arg Lys
               20                  25                  30
   Thr Glu Val Ala Ala Leu Arg Ala Gly Ile Glu Leu Gly Leu Thr Leu
           35                  40                  45
   Ile Asp Thr Ala Glu Met Tyr Ala Asp Gly Gly Ala Glu Lys Val Val
   50                  55                  60
   Gly Glu Ala Leu Thr Gly Leu Arg Glu Lys Val Phe Leu Val Ser Lys
   65                  70                  75                  80
   Val Tyr Pro Trp Asn Ala Gly Gly Gln Lys Ala Ile Asn Ala Cys Glu
               85                  90                  95
   Ala Ser Leu Arg Arg Leu Asn Thr Asp Tyr Leu Asp Leu Tyr Leu Leu
           100                 105                 110
   His Trp Ser Gly Ser Phe Ala Phe Glu Glu Thr Val Ala Ala Met Glu
           115                 120                 125
   Lys Leu Ile Ala Gln Gly Lys Ile Arg Arg Trp Gly Val Ser Asn Leu
       130                 135                 140
   Asp Tyr Ala Asp Met Gln Glu Leu Trp Gln Leu Pro Gly Gly Asn Gln
   145                 150                 155                 160
   Cys Ala Thr Asn Gln Val Leu Tyr His Leu Gly Ser Arg Gly Ile Glu
                   165                 170                 175
   Tyr Asp Leu Leu Pro Trp Cys Gln Gln Gln Gln Met Pro Val Met Ala
               180                 185                 190
   Tyr Ser Pro Leu Ala Gln Ala Gly Arg Leu Arg Asn Gly Leu Leu Lys
           195                 200                 205
   Asn Ala Val Val Asn Glu Ile Ala His Ala His Asn Ile Ser Ala Ala
       210                 215                 220
   Gln Val Leu Leu Ala Trp Val Ile Ser His Gln Gly Val Met Ala Ile
   225                 230                 235                 240
   Pro Lys Ala Ala Thr Ile Ala His Val Gln Gln Asn Ala Ala Val Leu
                   245                 250                 255
   Glu Val Glu Leu Ser Ser Ala Glu Leu Ala Met Leu Asp Lys Ala Tyr
               260                 265                 270
   Pro Ala Pro Lys Gly Lys Thr Ala Leu Asp Met Val
           275                 280

<210>  36
<211>  855
<212>  DNA
<213>  Escherichia coli

<400>  36
atgcaacaaa aaatgattca atttagtggc gatgtctcac tgccagccgt agggcaggga      60

acatggtata tgggcgaaga tgccagtcag cgcaaaacag aagttgctgc actacgcgcg     120

ggcattgaac tcggtttaac cctcattgat accgccgaaa tgtatgccga tggcggtgcc     180

gaaaaggtgg ttggggaagc attaaccggt ctgcgagaga aggtctttct cgtctctaaa     240

gtctatccgt ggaatgctgg cgggcaaaaa gcgataaatg catgcgaagc cagtttacgc     300

cgtctcaata ctgattatct cgatctttac ttattacact ggtctggcag tttcgctttt     360

gaagagactg tcgcagcgat ggaaaaattg atcgcccagg gaaaaatccg ccgctggggc     420

gtttctaacc ttgattatgc tgatatgcag gaactctggc agctgccggg gggaaatcag     480

tgtgccacta atcaggtgct ttaccatctc ggttcacgag gaattgagta cgatctactc     540

ccctggtgcc agcaacagca gatgccggtg atggcttaca gtccgttagc ccaggccggg     600

cggttgcgca atggactgtt aaaaaacgcg gtagtcaacg aaattgcaca tgctcacaat     660

atcagcgcgg cacaagtatt gttggcgtgg gtgatcagtc atcagggtgt gatggcgatt     720

ccaaaagcgg ccacgattgc ccatgtccaa caaaatgcgg ctgtgcttga ggtcgaactt     780

tcttcagcgg aattagctat gctggataag gcatatccgg caccaaaagg aaaaactgcg     840

ctggatatgg tgtga                                                      855

<210>  37
<211>  348
<212>  PRT
<213>  Saccharomyces cerevisiae

<400>  37

Met Ser Ile Pro Glu Thr Gln Lys Gly Val Ile Phe Tyr Glu Ser His
1               5                   10                  15

Gly Lys Leu Glu Tyr Lys Asp Ile Pro Val Pro Lys Pro Lys Ala Asn
            20                  25                  30

Glu Leu Leu Ile Asn Val Lys Tyr Ser Gly Val Cys His Thr Asp Leu
        35                  40                  45

His Ala Trp His Gly Asp Trp Pro Leu Pro Val Lys Leu Pro Leu Val
    50                  55                  60

Gly Gly His Glu Gly Ala Gly Val Val Val Gly Met Gly Glu Asn Val
65                  70                  75                  80

Lys Gly Trp Lys Ile Gly Asp Tyr Ala Gly Ile Lys Trp Leu Asn Gly
                85                  90                  95

Ser Cys Met Ala Cys Glu Tyr Cys Glu Leu Gly Asn Glu Ser Asn Cys
            100                 105                 110

Pro His Ala Asp Leu Ser Gly Tyr Thr His Asp Gly Ser Phe Gln Gln
        115                 120                 125

Tyr Ala Thr Ala Asp Ala Val Gln Ala Ala His Ile Pro Gln Gly Thr
    130                 135                 140

Asp Leu Ala Gln Val Ala Pro Ile Leu Cys Ala Gly Ile Thr Val Tyr
145                 150                 155                 160

Lys Ala Leu Lys Ser Ala Asn Leu Met Ala Gly His Trp Val Ala Ile
                165                 170                 175

Ser Gly Ala Ala Gly Gly Leu Gly Ser Leu Ala Val Gln Tyr Ala Lys
            180                 185                 190

Ala Met Gly Tyr Arg Val Leu Gly Ile Asp Gly Gly Glu Gly Lys Glu
        195                 200                 205

Glu Leu Phe Arg Ser Ile Gly Gly Glu Val Phe Ile Asp Phe Thr Lys
    210                 215                 220

Glu Lys Asp Ile Val Gly Ala Val Leu Lys Ala Thr Asp Gly Gly Ala
225                 230                 235                 240

His Gly Val Ile Asn Val Ser Val Ser Glu Ala Ala Ile Glu Ala Ser
                245                 250                 255

Thr Arg Tyr Val Arg Ala Asn Gly Thr Thr Val Leu Val Gly Met Pro
            260                 265                 270

Ala Gly Ala Lys Cys Cys Ser Asp Val Phe Asn Gln Val Val Lys Ser
        275                 280                 285

Ile Ser Ile Val Gly Ser Tyr Val Gly Asn Arg Ala Asp Thr Arg Glu
    290                 295                 300

Ala Leu Asp Phe Phe Ala Arg Gly Leu Val Lys Ser Pro Ile Lys Val
305                 310                 315                 320

Val Gly Leu Ser Thr Leu Pro Glu Ile Tyr Glu Lys Met Glu Lys Gly
                325                 330                 335

Gln Ile Val Gly Arg Tyr Val Val Asp Thr Ser Lys
            340                 345

<210>  38
<211>  1047
<212>  DNA
<213>  Saccharomyces cerevisiae

<400>  38
atgtctatcc cagaaactca aaaaggtgtt atcttctacg aatcccacgg taagttggaa      60

tacaaagata ttccagttcc aaagccaaag gccaacgaat tgttgatcaa cgttaaatac     120

tctggtgtct gtcacactga cttgcacgct tggcacggtg actggccatt gccagttaag     180

ctaccattag tcggtggtca cgaaggtgcc ggtgtcgttg tcggcatggg tgaaaacgtt     240

aagggctgga agatcggtga ctacgccggt atcaaatggt tgaacggttc ttgtatggcc     300

tgtgaatact gtgaattggg taacgaatcc aactgtcctc acgctgactt gtctggttac     360

acccacgacg gttctttcca acaatacgct accgctgacg ctgttcaagc cgctcacatt     420

cctcaaggta ccgacttggc ccaagtcgcc cccatcttgt gtgctggtat caccgtctac     480

aaggctttga agtctgctaa cttgatggcc ggtcactggg ttgctatctc cggtgctgct     540

ggtggtctag gttctttggc tgttcaatac gccaaggcta tgggttacag agtcttgggt     600

attgacggtg gtgaaggtaa ggaagaatta ttcagatcca tcggtggtga agtcttcatt     660

gacttcacta aggaaaagga cattgtcggt gctgttctaa aggccactga cggtggtgct     720

cacggtgtca tcaacgtttc cgtttccgaa gccgctattg aagcttctac cagatacgtt     780

agagctaacg gtaccaccgt tttggtcggt atgccagctg gtgccaagtg ttgttctgat     840

gtcttcaacc aagtcgtcaa gtccatctct attgttggtt cttacgtcgg taacagagct     900

gacaccagag aagctttgga cttcttcgcc agaggtttgg tcaagtctcc aatcaaggtt     960

gtcggcttgt ctaccttgcc agaaatttac gaaaagatgg aaaagggtca aatcgttggt    1020

agatacgttg ttgacacttc taaataa                                        1047

<210>  39
<211>  348
<212>  PRT
<213>  Saccharomyces cerevisiae

<400>  39

Met Ser Ile Pro Glu Thr Gln Lys Ala Ile Ile Phe Tyr Glu Ser Asn
1               5                   10                  15

Gly Lys Leu Glu His Lys Asp Ile Pro Val Pro Lys Pro Lys Pro Asn
            20                  25                  30

Glu Leu Leu Ile Asn Val Lys Tyr Ser Gly Val Cys His Thr Asp Leu
        35                  40                  45

His Ala Trp His Gly Asp Trp Pro Leu Pro Thr Lys Leu Pro Leu Val
    50                  55                  60

Gly Gly His Glu Gly Ala Gly Val Val Val Gly Met Gly Glu Asn Val
65                  70                  75                  80

Lys Gly Trp Lys Ile Gly Asp Tyr Ala Gly Ile Lys Trp Leu Asn Gly
                85                  90                  95

Ser Cys Met Ala Cys Glu Tyr Cys Glu Leu Gly Asn Glu Ser Asn Cys
            100                 105                 110

Pro His Ala Asp Leu Ser Gly Tyr Thr His Asp Gly Ser Phe Gln Glu
        115                 120                 125

Tyr Ala Thr Ala Asp Ala Val Gln Ala Ala His Ile Pro Gln Gly Thr
    130                 135                 140

Asp Leu Ala Glu Val Ala Pro Ile Leu Cys Ala Gly Ile Thr Val Tyr
145                 150                 155                 160

Lys Ala Leu Lys Ser Ala Asn Leu Arg Ala Gly His Trp Ala Ala Ile
                165                 170                 175

Ser Gly Ala Ala Gly Gly Leu Gly Ser Leu Ala Val Gln Tyr Ala Lys
            180                 185                 190

Ala Met Gly Tyr Arg Val Leu Gly Ile Asp Gly Gly Pro Gly Lys Glu
        195                 200                 205

Glu Leu Phe Thr Ser Leu Gly Gly Glu Val Phe Ile Asp Phe Thr Lys
    210                 215                 220

Glu Lys Asp Ile Val Ser Ala Val Val Lys Ala Thr Asn Gly Gly Ala
225                 230                 235                 240

His Gly Ile Ile Asn Val Ser Val Ser Glu Ala Ala Ile Glu Ala Ser
                245                 250                 255

Thr Arg Tyr Cys Arg Ala Asn Gly Thr Val Val Leu Val Gly Leu Pro
            260                 265                 270

Ala Gly Ala Lys Cys Ser Ser Asp Val Phe Asn His Val Val Lys Ser
        275                 280                 285

Ile Ser Ile Val Gly Ser Tyr Val Gly Asn Arg Ala Asp Thr Arg Glu
    290                 295                 300

Ala Leu Asp Phe Phe Ala Arg Gly Leu Val Lys Ser Pro Ile Lys Val
305                 310                 315                 320

Val Gly Leu Ser Ser Leu Pro Glu Ile Tyr Glu Lys Met Glu Lys Gly
                325                 330                 335

Gln Ile Ala Gly Arg Tyr Val Val Asp Thr Ser Lys
            340                 345

<210>  40
<211>  1047
<212>  DNA
<213>  Saccharomyces cerevisiae

<400>  40
atgtctattc cagaaactca aaaagccatt atcttctacg aatccaacgg caagttggag      60

cataaggata tcccagttcc aaagccaaag cccaacgaat tgttaatcaa cgtcaagtac     120

tctggtgtct gccacaccga tttgcacgct tggcatggtg actggccatt gccaactaag     180

ttaccattag ttggtggtca cgaaggtgcc ggtgtcgttg tcggcatggg tgaaaacgtt     240

aagggctgga agatcggtga ctacgccggt atcaaatggt tgaacggttc ttgtatggcc     300

tgtgaatact gtgaattggg taacgaatcc aactgtcctc acgctgactt gtctggttac     360

acccacgacg gttctttcca agaatacgct accgctgacg ctgttcaagc cgctcacatt     420

cctcaaggta ctgacttggc tgaagtcgcg ccaatcttgt gtgctggtat caccgtatac     480

aaggctttga agtctgccaa cttgagagca ggccactggg cggccatttc tggtgctgct     540

ggtggtctag gttctttggc tgttcaatat gctaaggcga tgggttacag agtcttaggt     600

attgatggtg gtccaggaaa ggaagaattg tttacctcgc tcggtggtga agtattcatc     660

gacttcacca aagagaagga cattgttagc gcagtcgtta aggctaccaa cggcggtgcc     720

cacggtatca tcaatgtttc cgtttccgaa gccgctatcg aagcttctac cagatactgt     780

agggcgaacg gtactgttgt cttggttggt ttgccagccg gtgcaaagtg ctcctctgat     840

gtcttcaacc acgttgtcaa gtctatctcc attgtcggct cttacgtggg gaacagagct     900

gataccagag aagccttaga tttctttgcc agaggtctag tcaagtctcc aataaaggta     960

gttggcttat ccagtttacc agaaatttac gaaaagatgg agaagggcca aattgctggt    1020

agatacgttg ttgacacttc taaataa                                        1047

<210>  41
<211>  375
<212>  PRT
<213>  Saccharomyces cerevisiae

<400>  41

<210>  42
<211>  1128
<212>  DNA
<213>  Saccharomyces cerevisiae

<400>  42
atgttgagaa cgtcaacatt gttcaccagg cgtgtccaac caagcctatt ttctagaaac      60

attcttagat tgcaatccac agctgcaatc cctaagactc aaaaaggtgt catcttttat     120

gagaataagg ggaagctgca ttacaaagat atccctgtcc ccgagcctaa gccaaatgaa     180

attttaatca acgttaaata ttctggtgta tgtcacaccg atttacatgc ttggcacggc     240

gattggccat tacctgttaa actaccatta gtaggtggtc atgaaggtgc tggtgtagtt     300

gtcaaactag gttccaatgt caagggctgg aaagtcggtg atttagcagg tatcaaatgg     360

ctgaacggtt cttgtatgac atgcgaattc tgtgaatcag gtcatgaatc aaattgtcca     420

gatgctgatt tatctggtta cactcatgat ggttctttcc aacaatttgc gaccgctgat     480

gctattcaag ccgccaaaat tcaacagggt accgacttgg ccgaagtagc cccaatatta     540

tgtgctggtg ttactgtata taaagcacta aaagaggcag acttgaaagc tggtgactgg     600

gttgccatct ctggtgctgc aggtggcttg ggttccttgg ccgttcaata tgcaactgcg     660

atgggttaca gagttctagg tattgatgca ggtgaggaaa aggaaaaact tttcaagaaa     720

ttggggggtg aagtattcat cgactttact aaaacaaaga atatggtttc tgacattcaa 780

gaagctacca aaggtggccc tcatggtgtc attaacgttt ccgtttctga agccgctatt 840

tctctatcta cggaatatgt tagaccatgt ggtaccgtcg ttttggttgg tttgcccgct 900

aacgcctacg ttaaatcaga ggtattctct catgtggtga agtccatcaa tatcaagggt 960

tcttatgttg gtaacagagc tgatacgaga gaagccttag acttctttag cagaggtttg 1020

atcaaatcac caatcaaaat tgttggatta tctgaattac caaaggttta tgacttgatg 1080

gaaaagggca agattttggg tagatacgtc gtcgatacta gtaaataa 1128

<210> 43
<211> 382
<212> PRT
<213> Saccharomyces cerevisiae

<400> 43

Met Ser Ser Val Thr Gly Phe Tyr Ile Pro Pro Ile Ser Phe Phe Gly
1               5                   10                  15

Glu Gly Ala Leu Glu Glu Thr Ala Asp Tyr Ile Lys Asn Lys Asp Tyr
            20                  25                  30

Lys Lys Ala Leu Ile Val Thr Asp Pro Gly Ile Ala Ala Ile Gly Leu
        35                  40                  45

Ser Gly Arg Val Gln Lys Met Leu Glu Glu Arg Asp Leu Asn Val Ala
    50                  55                  60

Ile Tyr Asp Lys Thr Gln Pro Asn Pro Asn Ile Ala Asn Val Thr Ala
65                  70                  75                  80

Gly Leu Lys Val Leu Lys Glu Gln Asn Ser Glu Ile Val Val Ser Ile
                85                  90                  95

Gly Gly Gly Ser Ala His Asp Asn Ala Lys Ala Ile Ala Leu Leu Ala
            100                 105                 110

Thr Asn Gly Gly Glu Ile Gly Asp Tyr Glu Gly Val Asn Gln Ser Lys
        115                 120                 125

Lys Ala Ala Leu Pro Leu Phe Ala Ile Asn Thr Thr Ala Gly Thr Ala
    130                 135                 140

Ser Glu Met Thr Arg Phe Thr Ile Ile Ser Asn Glu Glu Lys Lys Ile
145                 150                 155                 160

Lys Met Ala Ile Ile Asp Asn Asn Val Thr Pro Ala Val Ala Val Asn
                165                 170                 175

Asp Pro Ser Thr Met Phe Gly Leu Pro Pro Ala Leu Thr Ala Ala Thr
            180                 185                 190

Gly Leu Asp Ala Leu Thr His Cys Ile Glu Ala Tyr Val Ser Thr Ala
        195                 200                 205

Ser Asn Pro Ile Thr Asp Ala Cys Ala Leu Lys Gly Ile Asp Leu Ile
    210                 215                 220

Asn Glu Ser Leu Val Ala Ala Tyr Lys Asp Gly Lys Asp Lys Lys Ala
225                 230                 235                 240

Arg Thr Asp Met Cys Tyr Ala Glu Tyr Leu Ala Gly Met Ala Phe Asn
                245                 250                 255

Asn Ala Ser Leu Gly Tyr Val His Ala Leu Ala His Gln Leu Gly Gly
            260                 265                 270

Phe Tyr His Leu Pro His Gly Val Cys Asn Ala Val Leu Leu Pro His
        275                 280                 285

Val Gln Glu Ala Asn Met Gln Cys Pro Lys Ala Lys Lys Arg Leu Gly
    290                 295                 300

Glu Ile Ala Leu His Phe Gly Ala Ser Gln Glu Asp Pro Glu Glu Thr
305                 310                 315                 320

Ile Lys Ala Leu His Val Leu Asn Arg Thr Met Asn Ile Pro Arg Asn
                325                 330                 335

Leu Lys Glu Leu Gly Val Lys Thr Glu Asp Phe Glu Ile Leu Ala Glu
            340                 345                 350

His Ala Met His Asp Ala Cys His Leu Thr Asn Pro Val Gln Phe Thr
        355                 360                 365

Lys Glu Gln Val Val Ala Ile Ile Lys Lys Ala Tyr Glu Tyr
    370                 375                 380

<210>  44
<211>  1149
<212>  DNA
<213>  Saccharomyces cerevisiae

<400>  44
atgtcttccg ttactgggtt ttacattcca ccaatctctt tctttggtga aggtgcttta      60

gaagaaaccg ctgattacat caaaaacaag gattacaaaa aggctttgat cgttactgat 120

cctggtattg cagctattgg tctctccggt agagtccaaa agatgttgga agaacgtgac 180

ttaaacgttg ctatctatga caaaactcaa ccaaacccaa atattgccaa tgtcacagct 240

ggtttgaagg ttttgaagga acaaaactct gaaattgttg tttccattgg tggtggttct 300

gctcacgaca atgctaaggc cattgcttta ttggctacta acggtgggga aatcggagac 360

tatgaaggtg tcaatcaatc taagaaggct gctttaccac tatttgccat caacactact 420

gctggtactg cttccgaaat gaccagattc actattatct ctaatgaaga aaagaaaatc 480

aagatggcta tcattgacaa caacgtcact ccagctgttg ctgtcaacga tccatctacc 540

atgtttggtt tgccacctgc tttgactgct gctactggtc tagatgcttt gactcactgt 600

atcgaagctt atgtttccac cgcctctaac ccaatcaccg atgcctgtgc tttgaagggt 660

attgatttga tcaatgaaag cttagtcgct gcatacaaag acggtaaaga caagaaggcc 720

agaactgaca tgtgttacgc tgaatacttg gcaggtatgg ctttcaacaa tgcttctcta 780

ggttatgttc atgcccttgc tcatcaactt ggtggtttct accacttgcc tcatggtgtt 840

tgtaacgctg tcttgttgcc tcatgttcaa gaggccaaca tgcaatgtcc aaaggccaag 900

aagagattag gtgaaattgc tttgcatttc ggtgcttctc aagaagatcc agaagaaacc 960

atcaaggctt tgcacgtttt aaacagaacc atgaacattc caagaaactt gaaagaatta 1020

ggtgttaaaa ccgaagattt tgaaattttg gctgaacacg ccatgcatga tgcctgccat 1080

ttgactaacc cagttcaatt caccaaagaa caagtggttg ccattatcaa gaaagcctat 1140

gaatattaa 1149

<210> 45
<211> 351
<212> PRT
<213> Saccharomyces cerevisiae

<400> 45

Met Pro Ser Gln Val Ile Pro Glu Lys Gln Lys Ala Ile Val Phe Tyr
1               5                   10                  15

Glu Thr Asp Gly Lys Leu Glu Tyr Lys Asp Val Thr Val Pro Glu Pro
            20                  25                  30

Lys Pro Asn Glu Ile Leu Val His Val Lys Tyr Ser Gly Val Cys His
        35                  40                  45

Ser Asp Leu His Ala Trp His Gly Asp Trp Pro Phe Gln Leu Lys Phe
    50                  55                  60

Pro Leu Ile Gly Gly His Glu Gly Ala Gly Val Val Val Lys Leu Gly
65                  70                  75                  80

Ser Asn Val Lys Gly Trp Lys Val Gly Asp Phe Ala Gly Ile Lys Trp
                85                  90                  95

Leu Asn Gly Thr Cys Met Ser Cys Glu Tyr Cys Glu Val Gly Asn Glu
            100                 105                 110

Ser Gln Cys Pro Tyr Leu Asp Gly Thr Gly Phe Thr His Asp Gly Thr
        115                 120                 125

Phe Gln Glu Tyr Ala Thr Ala Asp Ala Val Gln Ala Ala His Ile Pro
    130                 135                 140

Pro Asn Val Asn Leu Ala Glu Val Ala Pro Ile Leu Cys Ala Gly Ile
145                 150                 155                 160

Thr Val Tyr Lys Ala Leu Lys Arg Ala Asn Val Ile Pro Gly Gln Trp
                165                 170                 175

Val Thr Ile Ser Gly Ala Cys Gly Gly Leu Gly Ser Leu Ala Ile Gln
            180                 185                 190

Tyr Ala Leu Ala Met Gly Tyr Arg Val Ile Gly Ile Asp Gly Gly Asn
        195                 200                 205

Ala Lys Arg Lys Leu Phe Glu Gln Leu Gly Gly Glu Ile Phe Ile Asp
    210                 215                 220

Phe Thr Glu Glu Lys Asp Ile Val Gly Ala Ile Ile Lys Ala Thr Asn
225                 230                 235                 240

Gly Gly Ser His Gly Val Ile Asn Val Ser Val Ser Glu Ala Ala Ile
                245                 250                 255

Glu Ala Ser Thr Arg Tyr Cys Arg Pro Asn Gly Thr Val Val Leu Val
            260                 265                 270

Gly Met Pro Ala His Ala Tyr Cys Asn Ser Asp Val Phe Asn Gln Val
        275                 280                 285

Val Lys Ser Ile Ser Ile Val Gly Ser Cys Val Gly Asn Arg Ala Asp
    290                 295                 300

Thr Arg Glu Ala Leu Asp Phe Phe Ala Arg Gly Leu Ile Lys Ser Pro
305                 310                 315                 320

Ile His Leu Ala Gly Leu Ser Asp Val Pro Glu Ile Phe Ala Lys Met
                325                 330                 335

Glu Lys Gly Glu Ile Val Gly Arg Tyr Val Val Glu Thr Ser Lys
            340                 345                 350

<210>  46
<211>  1056
<212>  DNA
<213>  Saccharomyces cerevisiae

<400>  46
atgccttcgc aagtcattcc tgaaaaacaa aaggctattg tcttttatga gacagatgga      60

aaattggaat ataaagacgt cacagttccg gaacctaagc ctaacgaaat tttagtccac     120

gttaaatatt ctggtgtttg tcatagtgac ttgcacgcgt ggcacggtga ttggccattt     180

caattgaaat ttccattaat cggtggtcac gaaggtgctg gtgttgttgt taagttggga     240

tctaacgtta agggctggaa agtcggtgat tttgcaggta taaaatggtt gaatgggact     300

tgcatgtcct gtgaatattg tgaagtaggt aatgaatctc aatgtcctta tttggatggt     360

actggcttca cacatgatgg tacttttcaa gaatacgcaa ctgccgatgc cgttcaagct     420

gcccatattc caccaaacgt caatcttgct gaagttgccc caatcttgtg tgcaggtatc     480

actgtttata aggcgttgaa aagagccaat gtgataccag gccaatgggt cactatatcc     540

ggtgcatgcg gtggcttggg ttctctggca atccaatacg cccttgctat gggttacagg     600

gtcattggta tcgatggtgg taatgccaag cgaaagttat ttgaacaatt aggcggagaa     660

atattcatcg atttcacgga agaaaaagac attgttggtg ctataataaa ggccactaat     720

ggcggttctc atggagttat taatgtgtct gtttctgaag cagctatcga ggcttctacg     780

aggtattgta ggcccaatgg tactgtcgtc ctggttggta tgccagctca tgcttactgc     840

aattccgatg ttttcaatca agttgtaaaa tcaatctcca tcgttggatc ttgtgttgga     900

aatagagctg atacaaggga ggctttagat ttcttcgcca gaggtttgat caaatctccg     960

atccacttag ctggcctatc ggatgttcct gaaatttttg caaagatgga gaagggtgaa    1020

attgttggta gatatgttgt tgagacttct aaatga                              1056

<210>  47
<211>  360
<212>  PRT
<213>  Saccharomyces cerevisiae

<400>  47

Met Ser Tyr Pro Glu Lys Phe Glu Gly Ile Ala Ile Gln Ser His Glu
1               5                   10                  15

Asp Trp Lys Asn Pro Lys Lys Thr Lys Tyr Asp Pro Lys Pro Phe Tyr
            20                  25                  30

Asp His Asp Ile Asp Ile Lys Ile Glu Ala Cys Gly Val Cys Gly Ser
        35                  40                  45

Asp Ile His Cys Ala Ala Gly His Trp Gly Asn Met Lys Met Pro Leu
    50                  55                  60

Val Val Gly His Glu Ile Val Gly Lys Val Val Lys Leu Gly Pro Lys
65                  70                  75                  80

Ser Asn Ser Gly Leu Lys Val Gly Gln Arg Val Gly Val Gly Ala Gln
                85                  90                  95

Val Phe Ser Cys Leu Glu Cys Asp Arg Cys Lys Asn Asp Asn Glu Pro
            100                 105                 110

Tyr Cys Thr Lys Phe Val Thr Thr Tyr Ser Gln Pro Tyr Glu Asp Gly
        115                 120                 125

Tyr Val Ser Gln Gly Gly Tyr Ala Asn Tyr Val Arg Val His Glu His
    130                 135                 140

Phe Val Val Pro Ile Pro Glu Asn Ile Pro Ser His Leu Ala Ala Pro
145                 150                 155                 160

Leu Leu Cys Gly Gly Leu Thr Val Tyr Ser Pro Leu Val Arg Asn Gly
                165                 170                 175

Cys Gly Pro Gly Lys Lys Val Gly Ile Val Gly Leu Gly Gly Ile Gly
            180                 185                 190

Ser Met Gly Thr Leu Ile Ser Lys Ala Met Gly Ala Glu Thr Tyr Val
        195                 200                 205

Ile Ser Arg Ser Ser Arg Lys Arg Glu Asp Ala Met Lys Met Gly Ala
    210                 215                 220

Asp His Tyr Ile Ala Thr Leu Glu Glu Gly Asp Trp Gly Glu Lys Tyr
225                 230                 235                 240

Phe Asp Thr Phe Asp Leu Ile Val Val Cys Ala Ser Ser Leu Thr Asp
                245                 250                 255

Ile Asp Phe Asn Ile Met Pro Lys Ala Met Lys Val Gly Gly Arg Ile
            260                 265                 270

Val Ser Ile Ser Ile Pro Glu Gln His Glu Met Leu Ser Leu Lys Pro
        275                 280                 285

Tyr Gly Leu Lys Ala Val Ser Ile Ser Tyr Ser Ala Leu Gly Ser Ile
    290                 295                 300

Lys Glu Leu Asn Gln Leu Leu Lys Leu Val Ser Glu Lys Asp Ile Lys
305                 310                 315                 320

Ile Trp Val Glu Thr Leu Pro Val Gly Glu Ala Gly Val His Glu Ala
                325                 330                 335

Phe Glu Arg Met Glu Lys Gly Asp Val Arg Tyr Arg Phe Thr Leu Val
            340                 345                 350

Gly Tyr Asp Lys Glu Phe Ser Asp
        355                 360

<210>  48
<211>  1083
<212>  DNA
<213>  Saccharomyces cerevisiae

<400>  48
atgtcttatc ctgagaaatt tgaaggtatc gctattcaat cacacgaaga ttggaaaaac      60

ccaaagaaga caaagtatga cccaaaacca ttttacgatc atgacattga cattaagatc     120

gaagcatgtg gtgtctgcgg tagtgatatt cattgtgcag ctggtcattg gggcaatatg     180

aagatgccgc tagtcgttgg tcatgaaatc gttggtaaag ttgtcaagct agggcccaag     240

tcaaacagtg ggttgaaagt cggtcaacgt gttggtgtag gtgctcaagt cttttcatgc     300

ttggaatgtg accgttgtaa gaatgataat gaaccatact gcaccaagtt tgttaccaca     360

tacagtcagc cttatgaaga cggctatgtg tcgcagggtg gctatgcaaa ctacgtcaga     420

gttcatgaac attttgtggt gcctatccca gagaatattc catcacattt ggctgctcca     480

ctattatgtg gtggtttgac tgtgtactct ccattggttc gtaacggttg cggtccaggt     540

aaaaaagttg gtatagttgg tcttggtggt atcggcagta tgggtacatt gatttccaaa     600

gccatggggg cagagacgta tgttatttct cgttcttcga gaaaaagaga agatgcaatg     660

aagatgggcg ccgatcacta cattgctaca ttagaagaag gtgattgggg tgaaaagtac     720

tttgacacct tcgacctgat tgtagtctgt gcttcctccc ttaccgacat tgacttcaac     780

attatgccaa aggctatgaa ggttggtggt agaattgtct caatctctat accagaacaa     840

cacgaaatgt tatcgctaaa gccatatggc ttaaaggctg tctccatttc ttacagtgct     900

ttaggttcca tcaaagaatt gaaccaactc ttgaaattag tctctgaaaa agatatcaaa     960

atttgggtgg aaacattacc tgttggtgaa gccggcgtcc atgaagcctt cgaaaggatg 1020

gaaaagggtg acgttagata tagatttacc ttagtcggct acgacaaaga attttcagac 1080

tag 1083

<210> 49
<211> 361
<212> PRT
<213> Saccharomyces cerevisiae

<400> 49

Met Leu Tyr Pro Glu Lys Phe Gln Gly Ile Gly Ile Ser Asn Ala Lys
1               5                   10                  15

Asp Trp Lys His Pro Lys Leu Val Ser Phe Asp Pro Lys Pro Phe Gly
            20                  25                  30

Asp His Asp Val Asp Val Glu Ile Glu Ala Cys Gly Ile Cys Gly Ser
        35                  40                  45

Asp Phe His Ile Ala Val Gly Asn Trp Gly Pro Val Pro Glu Asn Gln
    50                  55                  60

Ile Leu Gly His Glu Ile Ile Gly Arg Val Val Lys Val Gly Ser Lys
65                  70                  75                  80

Cys His Thr Gly Val Lys Ile Gly Asp Arg Val Gly Val Gly Ala Gln
                85                  90                  95

Ala Leu Ala Cys Phe Glu Cys Glu Arg Cys Lys Ser Asp Asn Glu Gln
            100                 105                 110

Tyr Cys Thr Asn Asp His Val Leu Thr Met Trp Thr Pro Tyr Lys Asp
        115                 120                 125

Gly Tyr Ile Ser Gln Gly Gly Phe Ala Ser His Val Arg Leu His Glu
    130                 135                 140

His Phe Ala Ile Gln Ile Pro Glu Asn Ile Pro Ser Pro Leu Ala Ala
145                 150                 155                 160

Pro Leu Leu Cys Gly Gly Ile Thr Val Phe Ser Pro Leu Leu Arg Asn
                165                 170                 175

Gly Cys Gly Pro Gly Lys Arg Val Gly Ile Val Gly Ile Gly Gly Ile
            180                 185                 190

Gly His Met Gly Ile Leu Leu Ala Lys Ala Met Gly Ala Glu Val Tyr

195                 200                 205

Ala Phe Ser Arg Gly His Ser Lys Arg Glu Asp Ser Met Lys Leu Gly
    210                 215                 220

Ala Asp His Tyr Ile Ala Met Leu Glu Asp Lys Gly Trp Thr Glu Gln
225                 230                 235                 240

Tyr Ser Asn Ala Leu Asp Leu Leu Val Val Cys Ser Ser Ser Leu Ser
                245                 250                 255

Lys Val Asn Phe Asp Ser Ile Val Lys Ile Met Lys Ile Gly Gly Ser
            260                 265                 270

Ile Val Ser Ile Ala Ala Pro Glu Val Asn Glu Lys Leu Val Leu Lys
        275                 280                 285

Pro Leu Gly Leu Met Gly Val Ser Ile Ser Ser Ser Ala Ile Gly Ser
    290                 295                 300

Arg Lys Glu Ile Glu Gln Leu Leu Lys Leu Val Ser Glu Lys Asn Val
305                 310                 315                 320

Lys Ile Trp Val Glu Lys Leu Pro Ile Ser Glu Glu Gly Val Ser His
                325                 330                 335

Ala Phe Thr Arg Met Glu Ser Gly Asp Val Lys Tyr Arg Phe Thr Leu
            340                 345                 350

Val Asp Tyr Asp Lys Lys Phe His Lys
        355                 360

<210>  50
<211>  1086
<212>  DNA
<213>  Saccharomyces cerevisiae

<400>  50
atgctttacc cagaaaaatt tcagggcatc ggtatttcca acgcaaagga ttggaagcat      60

cctaaattag tgagttttga cccaaaaccc tttggcgatc atgacgttga tgttgaaatt     120

gaagcctgtg gtatctgcgg atctgatttt catatagccg ttggtaattg gggtccagtc     180

ccagaaaatc aaatccttgg acatgaaata attggccgcg tggtgaaggt tggatccaag     240

tgccacactg gggtaaaaat cggtgaccgt gttggtgttg gtgcccaagc cttggcgtgt     300

tttgagtgtg aacgttgcaa aagtgacaac gagcaatact gtaccaatga ccacgttttg     360

actatgtgga ctccttacaa ggacggctac atttcacaag gaggctttgc ctcccacgtg     420

aggcttcatg aacactttgc tattcaaata ccagaaaata ttccaagtcc gctagccgct 480

ccattattgt gtggtggtat tacagttttc tctccactac taagaaatgg ctgtggtcca 540

ggtaagaggg taggtattgt tggcatcggt ggtattgggc atatggggat tctgttggct 600

aaagctatgg gagccgaggt ttatgcgttt tcgcgaggcc actccaagcg ggaggattct 660

atgaaactcg gtgctgatca ctatattgct atgttggagg ataaaggctg gacagaacaa 720

tactctaacg ctttggacct tcttgtcgtt tgctcatcat ctttgtcgaa agttaatttt 780

gacagtatcg ttaagattat gaagattgga ggctccatcg tttcaattgc tgctcctgaa 840

gttaatgaaa agcttgtttt aaaaccgttg ggcctaatgg gagtatcaat ctcaagcagt 900

gctatcggat ctaggaagga aatcgaacaa ctattgaaat tagtttccga aaagaatgtc 960

aaaatatggg tggaaaaact tccgatcagc gaagaaggcg tcagccatgc ctttacaagg 1020

atggaaagcg gagacgtcaa atacagattt actttggtcg attatgataa gaaattccat 1080

aaatag 1086

<210> 51
<211> 386
<212> PRT
<213> Saccharomyces cerevisiae

<400> 51

Met Ser Ala Ala Thr Val Gly Lys Pro Ile Lys Cys Ile Ala Ala Val
1               5                   10                  15

Ala Tyr Asp Ala Lys Lys Pro Leu Ser Val Glu Glu Ile Thr Val Asp
            20                  25                  30

Ala Pro Lys Ala His Glu Val Arg Ile Lys Ile Glu Tyr Thr Ala Val
        35                  40                  45

Cys His Thr Asp Ala Tyr Thr Leu Ser Gly Ser Asp Pro Glu Gly Leu
    50                  55                  60

Phe Pro Cys Val Leu Gly His Glu Gly Ala Gly Ile Val Glu Ser Val
65                  70                  75                  80

Gly Asp Asp Val Ile Thr Val Lys Pro Gly Asp His Val Ile Ala Leu
                85                  90                  95

Tyr Thr Ala Glu Cys Gly Lys Cys Lys Phe Cys Thr Ser Gly Lys Thr
            100                 105                 110

Asn Leu Cys Gly Ala Val Arg Ala Thr Gln Gly Lys Gly Val Met Pro
        115                 120                 125

Asp Gly Thr Thr Arg Phe His Asn Ala Lys Gly Glu Asp Ile Tyr His
    130                 135                 140

Phe Met Gly Cys Ser Thr Phe Ser Glu Tyr Thr Val Val Ala Asp Val
145                 150                 155                 160

Ser Val Val Ala Ile Asp Pro Lys Ala Pro Leu Asp Ala Ala Cys Leu
                165                 170                 175

Leu Gly Cys Gly Val Thr Thr Gly Phe Gly Ala Ala Leu Lys Thr Ala
            180                 185                 190

Asn Val Gln Lys Gly Asp Thr Val Ala Val Phe Gly Cys Gly Thr Val
        195                 200                 205

Gly Leu Ser Val Ile Gln Gly Ala Lys Leu Arg Gly Ala Ser Lys Ile
    210                 215                 220

Ile Ala Ile Asp Ile Asn Asn Lys Lys Lys Gln Tyr Cys Ser Gln Phe
225                 230                 235                 240

Gly Ala Thr Asp Phe Val Asn Pro Lys Glu Asp Leu Ala Lys Asp Gln
                245                 250                 255

Thr Ile Val Glu Lys Leu Ile Glu Met Thr Asp Gly Gly Leu Asp Phe
            260                 265                 270

Thr Phe Asp Cys Thr Gly Asn Thr Lys Ile Met Arg Asp Ala Leu Glu
        275                 280                 285

Ala Cys His Lys Gly Trp Gly Gln Ser Ile Ile Ile Gly Val Ala Ala
    290                 295                 300

Ala Gly Glu Glu Ile Ser Thr Arg Pro Phe Gln Leu Val Thr Gly Arg
305                 310                 315                 320

Val Trp Lys Gly Ser Ala Phe Gly Gly Ile Lys Gly Arg Ser Glu Met
                325                 330                 335

Gly Gly Leu Ile Lys Asp Tyr Gln Lys Gly Ala Leu Lys Val Glu Glu
            340                 345                 350

Phe Ile Thr His Arg Arg Pro Phe Lys Glu Ile Asn Gln Ala Phe Glu
        355                 360                 365

Asp Leu His Asn Gly Asp Cys Leu Arg Thr Val Leu Lys Ser Asp Glu
    370                 375                 380

Ile Lys
385

<210> 52
<211> 1161
<212> DNA
<213> Saccharomyces cerevisiae

<400> 52
atgtccgccg ctactgttgg taaacctatt aagtgcattg ctgctgttgc gtatgatgcg 60

aagaaaccat taagtgttga agaaatcacg gtagacgccc caaaagcgca cgaagtacgt 120

atcaaaattg aatatactgc tgtatgccac actgatgcgt acactttatc aggctctgat 180

ccagaaggac ttttcccttg cgttctgggc cacgaaggag ccggtatcgt agaatctgta 240

ggcgatgatg tcataacagt taagcctggt gatcatgtta ttgctttgta cactgctgag 300

tgtggcaaat gtaagttctg tacttccggt aaaaccaact tatgtggtgc tgttagagct 360

actcaaggga aaggtgtaat gcctgatggg accacaagat ttcataatgc gaaaggtgaa 420

gatatatacc atttcatggg ttgctctact ttttccgaat atactgtggt ggcagatgtc 480

tctgtggttg ccatcgatcc aaaagctccc ttggatgctg cctgtttact gggttgtggt 540

gttactactg gttttggggc ggctcttaag acagctaatg tgcaaaaagg cgataccgtt 600

gcagtatttg gctgcgggac tgtaggactc tccgttatcc aaggtgcaaa gttaaggggc 660

gcttccaaga tcattgccat tgacattaac aataagaaaa aacaatattg ttctcaattt 720

ggtgccacgg attttgttaa tcccaaggaa gatttggcca aagatcaaac tatcgttgaa 780

aagttaattg aaatgactga tgggggtctg gattttactt ttgactgtac tggtaatacc 840

aaaattatga gagatgcttt ggaagcctgt cataaaggtt ggggtcaatc tattatcatt 900

ggtgtggctg ccgctggtga agaaatttct acaaggccgt tccagctggt cactggtaga 960

gtgtggaaag gctctgcttt tggtggcatc aaaggtagat ctgaaatggg cggtttaatt 1020

aaagactatc aaaaaggtgc cttaaaagtc gaagaattta tcactcacag gagaccattc 1080

aaagaaatca atcaagcctt tgaagatttg cataacggtg attgcttaag aaccgtcttg 1140

aagtctgatg aaataaaata g 1161

<210> 53
<211> 363
<212> PRT
<213> Saccharomyces cerevisiae

<400> 53

Met Ile Gly Ser Ala Ser Asp Ser Ser Ser Lys Leu Gly Arg Leu Arg
1               5                   10                  15

Phe Leu Ser Glu Thr Ala Ala Ile Lys Val Ser Pro Leu Ile Leu Gly
            20                  25                  30

Glu Val Ser Tyr Asp Gly Ala Arg Ser Asp Phe Leu Lys Ser Met Asn
        35                  40                  45

Lys Asn Arg Ala Phe Glu Leu Leu Asp Thr Phe Tyr Glu Ala Gly Gly
    50                  55                  60

Asn Phe Ile Asp Ala Ala Asn Asn Cys Gln Asn Glu Gln Ser Glu Glu
65                  70                  75                  80

Trp Ile Gly Glu Trp Ile Gln Ser Arg Arg Leu Arg Asp Gln Ile Val
                85                  90                  95

Ile Ala Thr Lys Phe Ile Lys Ser Asp Lys Lys Tyr Lys Ala Gly Glu
            100                 105                 110

Ser Asn Thr Ala Asn Tyr Cys Gly Asn His Lys Arg Ser Leu His Val
        115                 120                 125

Ser Val Arg Asp Ser Leu Arg Lys Leu Gln Thr Asp Trp Ile Asp Ile
    130                 135                 140

Leu Tyr Val His Trp Trp Asp Tyr Met Ser Ser Ile Glu Glu Phe Met
145                 150                 155                 160

Asp Ser Leu His Ile Leu Val Gln Gln Gly Lys Val Leu Tyr Leu Gly
                165                 170                 175

Val Ser Asp Thr Pro Ala Trp Val Val Ser Ala Ala Asn Tyr Tyr Ala
            180                 185                 190

Thr Ser Tyr Gly Lys Thr Pro Phe Ser Ile Tyr Gln Gly Lys Trp Asn
        195                 200                 205

Val Leu Asn Arg Asp Phe Glu Arg Asp Ile Ile Pro Met Ala Arg His
    210                 215                 220

Phe Gly Met Ala Leu Ala Pro Trp Asp Val Met Gly Gly Gly Arg Phe
225                 230                 235                 240

Gln Ser Lys Lys Ala Met Glu Glu Arg Arg Lys Asn Gly Glu Gly Ile
                245                 250                 255

Arg Ser Phe Val Gly Ala Ser Glu Gln Thr Asp Ala Glu Ile Lys Ile
            260                 265                 270

Ser Glu Ala Leu Ala Lys Ile Ala Glu Glu His Gly Thr Glu Ser Val
        275                 280                 285

Thr Ala Ile Ala Ile Ala Tyr Val Arg Ser Lys Ala Lys Asn Phe Phe
    290                 295                 300

Pro Ser Val Glu Gly Gly Lys Ile Glu Asp Leu Lys Glu Asn Ile Lys
305                 310                 315                 320

Ala Leu Ser Ile Asp Leu Thr Pro Asp Asn Ile Lys Tyr Leu Glu Ser
                325                 330                 335

Ile Val Pro Phe Asp Ile Gly Phe Pro Asn Asn Phe Ile Val Leu Asn
            340                 345                 350

Ser Leu Thr Gln Lys Tyr Gly Thr Asn Asn Val
        355                 360

<210>  54
<211>  1092
<212>  DNA
<213>  Saccharomyces cerevisiae

<400>  54
atgattgggt ccgcgtccga ctcatctagc aagttaggac gcctccgatt tctttctgaa      60

actgccgcta ttaaagtatc cccgttaatc ctaggagaag tctcatacga tggagcacgt     120

tcggattttc tcaaatcaat gaacaagaat cgagcttttg aattgcttga tactttttac     180

gaggcaggtg gaaatttcat tgatgccgca aacaactgcc aaaacgagca atcagaagaa     240

tggattggtg aatggataca gtccagaagg ttacgtgatc aaattgtcat tgcaaccaag     300

tttataaaaa gcgataaaaa gtataaagca ggtgaaagta acactgccaa ctactgtggt     360

aatcacaagc gtagtttaca tgtgagtgtg agggattctc tccgcaaatt gcaaactgat     420

tggattgata tactttacgt tcactggtgg gattatatga gttcaatcga agaatttatg     480

gatagtttgc atattctggt ccagcagggc aaggtcctct atttgggtgt atctgataca     540

cctgcttggg ttgtttctgc ggcaaactac tacgctacat cttatggtaa aactcccttt     600

agtatctacc aaggtaaatg gaacgtgttg aacagagatt ttgagcgtga tattattcca     660

atggctaggc atttcggtat ggccctcgcc ccatgggatg tcatgggagg tggaagattt     720

cagagtaaaa aagcaatgga ggaacggagg aagaatggag agggtattcg ttctttcgtt     780

ggcgcctccg aacaaacaga tgcagaaatc aagattagtg aagcattggc caagattgct     840

gaggaacatg gcactgagtc tgttactgct attgctattg cctatgttcg ctctaaggcg     900

aaaaattttt ttccgtcggt tgaaggagga aaaattgagg atctcaaaga gaacattaag     960

gctctcagta tcgatctaac gccagacaat ataaaatact tagaaagtat agttcctttt 1020

gacatcggat ttcctaataa ttttatcgtg ttaaattcct tgactcaaaa atatggtacg 1080

aataatgttt ag 1092

<210> 55
<211> 329
<212> PRT
<213> Saccharomyces cerevisiae

<400> 55

Met Gly Ser Met Asn Lys Glu Gln Ala Phe Glu Leu Leu Asp Ala Phe
1               5                   10                  15

Tyr Glu Ala Gly Gly Asn Cys Ile Asp Thr Ala Asn Ser Tyr Gln Asn
            20                  25                  30

Glu Glu Ser Glu Ile Trp Ile Gly Glu Trp Met Lys Ser Arg Lys Leu
        35                  40                  45

Arg Asp Gln Ile Val Ile Ala Thr Lys Phe Thr Gly Asp Tyr Lys Lys
    50                  55                  60

Tyr Glu Val Gly Gly Gly Lys Ser Ala Asn Tyr Cys Gly Asn His Lys
65                  70                  75                  80

His Ser Leu His Val Ser Val Arg Asp Ser Leu Arg Lys Leu Gln Thr
                85                  90                  95

Asp Trp Ile Asp Ile Leu Tyr Val His Trp Trp Asp Tyr Met Ser Ser
            100                 105                 110

Ile Glu Glu Val Met Asp Ser Leu His Ile Leu Val Gln Gln Gly Lys
        115                 120                 125

Val Leu Tyr Leu Gly Val Ser Asp Thr Pro Ala Trp Val Val Ser Ala
    130                 135                 140

Ala Asn Tyr Tyr Ala Thr Ser His Gly Lys Thr Pro Phe Ser Ile Tyr
145                 150                 155                 160

Gln Gly Lys Trp Asn Val Leu Asn Arg Asp Phe Glu Arg Asp Ile Ile
                165                 170                 175

Pro Met Ala Arg His Phe Gly Met Ala Leu Ala Pro Trp Asp Val Met
            180                 185                 190

Gly Gly Gly Arg Phe Gln Ser Lys Lys Ala Met Glu Glu Arg Lys Lys
        195                 200                 205

Asn Gly Glu Gly Leu Arg Thr Val Ser Gly Thr Ser Lys Gln Thr Asp
    210                 215                 220

Lys Glu Val Lys Ile Ser Glu Ala Leu Ala Lys Val Ala Glu Glu His
225                 230                 235                 240

Gly Thr Glu Ser Val Thr Ala Ile Ala Ile Ala Tyr Val Arg Ser Lys
                245                 250                 255

Ala Lys Asn Val Phe Pro Leu Val Gly Gly Arg Lys Ile Glu His Leu
            260                 265                 270

Lys Gln Asn Ile Glu Ala Leu Ser Ile Lys Leu Thr Pro Glu Gln Ile
        275                 280                 285

Glu Tyr Leu Glu Ser Ile Ile Pro Phe Asp Val Gly Phe Pro Thr Asn
    290                 295                 300

Phe Ile Gly Asp Asp Pro Ala Val Thr Lys Lys Ala Ser Leu Leu Thr
305                 310                 315                 320

Ala Met Ser Ala Gln Ile Ser Phe Asp
                325

<210>  56
<211>  990
<212>  DNA
<213>  Saccharomyces cerevisiae

<400>  56
atgggctcta tgaataagga acaggctttt gaacttcttg atgcttttta tgaagcagga      60

ggtaattgca ttgatactgc aaacagttac caaaatgaag agtcagagat ttggataggt     120

gaatggatga aatcaagaaa gttgcgtgac caaattgtaa ttgccaccaa gtttaccgga     180

gattataaga agtatgaagt aggtggcggt aaaagtgcca actattgtgg taatcacaag     240

catagtttac atgtgagtgt gagggattct ctccgcaaat tgcaaactga ttggattgat     300

atactttacg ttcactggtg ggattatatg agttcaatcg aagaagttat ggatagtttg     360

catattttag ttcagcaggg caaagtcctc tatttgggtg tgtctgatac acctgcttgg     420

gttgtttctg cggcaaacta ctacgccaca tctcatggga aaactccttt tagtatctat     480

caaggtaaat ggaatgtgtt gaacagggac tttgagcgcg atatcattcc aatggccaga     540

cattttggta tggctctagc cccatgggat gttatgggag gtggaagatt tcagagtaaa     600

aaagcaatgg aggaacggaa gaagaatgga gagggtctgc gtactgtttc gggtacttct     660

aaacagacgg ataaagaggt taagatcagt gaagcattgg ccaaggttgc tgaggaacat 720

ggcactgagt ctgttactgc tattgctatt gcctatgttc gctctaaggc gaaaaatgtt 780

ttcccattgg ttggtggaag gaaaattgaa cacctcaaac agaacattga ggctttaagt 840

atcaaactga caccagaaca gatagaatac ttagaaagta ttattccttt tgatgttggt 900

tttcctacta attttatcgg tgatgatccg gctgttacca agaaggcttc acttctcacg 960

gcaatgtctg cgcagatttc cttcgattaa 990

<210> 57
<211> 288
<212> PRT
<213> Saccharomyces cerevisiae

<400> 57

Met Ala Ser Arg Lys Leu Arg Asp Gln Ile Val Ile Ala Thr Lys Phe
1               5                   10                  15

Thr Thr Asp Tyr Lys Gly Tyr Asp Val Gly Lys Gly Lys Ser Ala Asn
            20                  25                  30

Phe Cys Gly Asn His Lys Arg Ser Leu His Val Ser Val Arg Asp Ser
        35                  40                  45

Leu Arg Lys Leu Gln Thr Asp Trp Ile Asp Ile Leu Tyr Val His Trp
    50                  55                  60

Trp Asp Tyr Met Ser Ser Ile Glu Glu Val Met Asp Ser Leu His Ile
65                  70                  75                  80

Leu Val Gln Gln Gly Lys Val Leu Tyr Leu Gly Val Ser Asp Thr Pro
                85                  90                  95

Ala Trp Val Val Ser Ala Ala Asn Tyr Tyr Ala Thr Ser His Gly Lys
            100                 105                 110

Thr Pro Phe Ser Ile Tyr Gln Gly Lys Trp Asn Val Leu Asn Arg Asp
        115                 120                 125

Phe Glu Arg Asp Ile Ile Pro Met Ala Arg His Phe Gly Met Ala Leu
    130                 135                 140

Ala Pro Trp Asp Val Met Gly Gly Gly Arg Phe Gln Ser Lys Lys Ala
145                 150                 155                 160

Val Glu Glu Arg Lys Lys Lys Gly Glu Gly Leu Arg Thr Phe Phe Gly
                165                 170                 175

Thr Ser Glu Gln Thr Asp Met Glu Val Lys Ile Ser Glu Ala Leu Leu
            180                 185                 190

Lys Val Ala Glu Glu His Gly Thr Glu Ser Val Thr Ala Ile Ala Ile
        195                 200                 205

Ala Tyr Val Arg Ser Lys Ala Lys His Val Phe Pro Leu Val Gly Gly
    210                 215                 220

Arg Lys Ile Glu His Leu Lys Gln Asn Ile Glu Ala Leu Ser Ile Lys
225                 230                 235                 240

Leu Thr Pro Glu Gln Ile Lys Tyr Leu Glu Ser Ile Val Pro Phe Asp
                245                 250                 255

Val Gly Phe Pro Thr Asn Phe Ile Gly Asp Asp Pro Ala Val Thr Lys
            260                 265                 270

Lys Pro Ser Phe Leu Thr Glu Met Ser Ala Lys Ile Ser Phe Glu Asp
        275                 280                 285

<210>  58
<211>  867
<212>  DNA
<213>  Saccharomyces cerevisiae

<400>  58
atggcatcaa gaaaactgcg tgaccagatt gtaattgcca ctaaatttac cacggattat      60

aaggggtatg atgtaggcaa ggggaagagt gccaatttct gtgggaatca caagcgcagt     120

ttgcatgtaa gtgtgagaga ttcccttcgt aagttgcaaa ctgattggat tgatattctt     180

tacgttcact ggtgggatta tatgagctcc attgaggaag ttatggatag tttgcacatt     240

cttgtgcagc agggcaaagt actctatcta ggtgtgtctg atactcctgc ctgggttgtt     300

tctgcagcaa attactacgc cacatctcat ggtaaaactc cctttagtat ctatcaaggt     360

aaatggaatg tattgaacag ggactttgaa cgtgatatca ttccaatggc taggcatttt     420

ggtatggctc ttgctccatg ggatgttatg ggaggcggga gatttcagag taaaaaggca     480

gtggaagagc ggaagaagaa aggagaaggc ttgcgtacct tttttggtac ttcggaacag     540

acggatatgg aggttaaaat cagcgaagca ttgttaaaag ttgcggaaga acatggcact     600

gagtctgtca ctgctattgc catagcttat gttcggtcta aagcgaaaca tgttttccca     660

ttagtgggag gaagaaagat cgaacatctc aaacagaaca ttgaggcttt gagcattaaa     720

ttaacaccag aacaaataaa gtacttagaa agtattgttc cttttgatgt cggatttccc     780

actaatttta ttggagatga cccagctgtt accaagaaac cttcatttct caccgaaatg     840

tctgccaaga ttagcttcga agattag                                          867

<210>  59
<211>  376
<212>  PRT
<213>  Saccharomyces cerevisiae

<400>  59

Met Thr Asp Leu Phe Lys Pro Leu Pro Glu Pro Pro Thr Glu Leu Gly
1               5                   10                  15

Arg Leu Arg Val Leu Ser Lys Thr Ala Gly Ile Arg Val Ser Pro Leu
            20                  25                  30

Ile Leu Gly Gly Ala Ser Ile Gly Asp Ala Trp Ser Gly Phe Met Gly
        35                  40                  45

Ser Met Asn Lys Glu Gln Ala Phe Glu Leu Leu Asp Ala Phe Tyr Glu
    50                  55                  60

Ala Gly Gly Asn Cys Ile Asp Thr Ala Asn Ser Tyr Gln Asn Glu Glu
65                  70                  75                  80

Ser Glu Ile Trp Ile Gly Glu Trp Met Ala Ser Arg Lys Leu Arg Asp
                85                  90                  95

Gln Ile Val Ile Ala Thr Lys Phe Thr Gly Asp Tyr Lys Lys Tyr Glu
            100                 105                 110

Val Gly Gly Gly Lys Ser Ala Asn Tyr Cys Gly Asn His Lys Arg Ser
        115                 120                 125

Leu His Val Ser Val Arg Asp Ser Leu Arg Lys Leu Gln Thr Asp Trp
    130                 135                 140

Ile Asp Ile Leu Tyr Ile His Trp Trp Asp Tyr Met Ser Ser Ile Glu
145                 150                 155                 160

Glu Val Met Asp Ser Leu His Ile Leu Val Gln Gln Gly Lys Val Leu
                165                 170                 175

Tyr Leu Gly Val Ser Asp Thr Pro Ala Trp Val Val Ser Ala Ala Asn
            180                 185                 190

Tyr Tyr Ala Thr Ser His Gly Lys Thr Pro Phe Ser Val Tyr Gln Gly
        195                 200                 205

Lys Trp Asn Val Leu Asn Arg Asp Phe Glu Arg Asp Ile Ile Pro Met
    210                 215                 220

Ala Arg His Phe Gly Met Ala Leu Ala Pro Trp Asp Val Met Gly Gly
225                 230                 235                 240

Gly Arg Phe Gln Ser Lys Lys Ala Met Glu Glu Arg Lys Lys Asn Gly
                245                 250                 255

Glu Gly Leu Arg Thr Phe Val Gly Gly Pro Glu Gln Thr Glu Leu Glu
            260                 265                 270

Val Lys Ile Ser Glu Ala Leu Thr Lys Ile Ala Glu Glu His Gly Thr
        275                 280                 285

Glu Ser Val Thr Ala Ile Ala Ile Ala Tyr Val Arg Ser Lys Ala Lys
    290                 295                 300

Asn Val Phe Pro Leu Ile Gly Gly Arg Lys Ile Glu His Leu Lys Gln
305                 310                 315                 320

Asn Ile Glu Ala Leu Ser Ile Lys Leu Thr Pro Glu Gln Ile Glu Tyr
                325                 330                 335

Leu Glu Ser Ile Val Pro Phe Asp Val Gly Phe Pro Lys Ser Leu Ile
            340                 345                 350

Gly Asp Asp Pro Ala Val Thr Lys Lys Leu Ser Pro Leu Thr Ser Met
        355                 360                 365

Ser Ala Arg Ile Ala Phe Asp Asn
    370                 375

<210>  60
<211>  1131
<212>  DNA
<213>  Saccharomyces cerevisiae

<400>  60
atgactgact tgtttaaacc tctacctgaa ccacctaccg aattgggacg tctcagggtt      60

ctttctaaaa ctgccggcat aagggtttca ccgctaattc tgggaggagc ttcaatcggc     120

gacgcatggt caggctttat gggctctatg aataaggaac aggcctttga acttcttgat     180

gctttttatg aagctggagg taattgtatt gatactgcaa acagttacca aaatgaagag     240

tcagagattt ggataggtga atggatggca tcaagaaaac tgcgtgacca gattgtaatt     300

gccaccaagt ttaccggaga ttataagaag tatgaagtag gtggtggtaa aagtgccaac     360

tactgtggta atcacaagcg tagtttacat gtgagtgtga gggattctct ccgcaaattg     420

caaactgatt ggattgatat actttacatt cactggtggg attatatgag ttcaatcgaa 480

gaagttatgg atagtttgca tattttagtt cagcagggca aggtcctata tttaggagta 540

tctgatacac ctgcttgggt tgtttctgcg gcaaattact acgctacatc tcatggtaaa 600

actcctttta gcgtctatca aggtaaatgg aatgtattga acagggactt tgagcgtgat 660

attattccaa tggctaggca ttttggtatg gctctagccc catgggatgt catgggaggt 720

ggaagatttc agagtaaaaa agcaatggaa gaacggaaga agaatggaga gggtctgcgt 780

acttttgtgg gtggccccga acaaacagaa ttggaggtta aaatcagcga agcattgact 840

aaaattgctg aggaacatgg aacagagtct gttactgcta tcgctattgc ctatgttcgc 900

tctaaagcga aaaatgtttt cccattgatt ggaggaagga aaattgaaca tctcaagcag 960

aacattgagg ctttgagtat taaattaaca ccggaacaaa tagaatacct ggaaagtatt 1020

gttccttttg atgttggctt tcccaaaagt ttaataggag atgacccagc ggtaaccaag 1080

aagctttcac ccctcacatc gatgtctgcc aggatagctt ttgacaatta g 1131

<210> 61
<211> 143
<212> PRT
<213> Saccharomyces cerevisiae

<400> 61

Met Ala Arg His Phe Gly Met Ala Leu Ala Pro Trp Asp Val Met Gly
1               5                   10                  15

Gly Gly Arg Phe Gln Ser Lys Lys Ala Met Glu Glu Arg Arg Lys Asn
            20                  25                  30

Gly Glu Cys Ile Arg Ser Phe Val Gly Ala Ser Glu Gln Thr Asp Ala
        35                  40                  45

Glu Ile Lys Ile Ser Glu Ala Leu Ala Lys Val Ala Glu Glu His Gly
    50                  55                  60

Thr Glu Ser Val Thr Ala Ile Ala Ile Ala Tyr Val Arg Ser Lys Ala
65                  70                  75                  80

Lys Asn Val Phe Pro Ser Val Glu Gly Gly Lys Ile Glu Asp Leu Lys
                85                  90                  95

Glu Asn Ile Lys Ala Leu Ser Ile Asp Leu Thr Pro Asp Asn Ile Lys
            100                 105                 110

Tyr Leu Glu Asn Val Val Pro Phe Asp Ile Gly Phe Pro Asn Thr Phe
        115                 120                 125

Ile Val Leu Asn Ser Leu Thr Gln Lys Tyr Gly Thr Asn Asn Val
    130                 135                 140

<210>  62
<211>  432
<212>  DNA
<213>  Saccharomyces cerevisiae

<400>  62
atggctaggc atttcggtat ggccctcgcc ccatgggatg tcatgggagg tggaagattt      60

cagagtaaaa aagcaatgga ggaacggagg aagaatggag agtgtattcg ttctttcgtt     120

ggcgcctccg aacaaacaga tgcagaaatc aagattagtg aagcattagc caaggttgct     180

gaggaacatg gcactgagtc tgttactgct attgctattg cctatgttcg ctctaaggcg     240

aaaaatgttt ttccgtcggt tgaaggagga aaaattgagg atctcaaaga gaacattaag     300

gctctcagta tcgatctaac gccggacaat ataaaatact tggaaaatgt agttcctttt     360

gacatcggat ttcctaacac ttttatcgtg ttaaattcct tgactcaaaa atatggtacg     420

aataatgttt ag                                                         432

<210>  63
<211>  312
<212>  PRT
<213>  Saccharomyces cerevisiae

<400>  63

Met Pro Ala Thr Leu Lys Asn Ser Ser Ala Thr Leu Lys Leu Asn Thr
1               5                   10                  15

Gly Ala Ser Ile Pro Val Leu Gly Phe Gly Thr Trp Arg Ser Val Asp
            20                  25                  30

Asn Asn Gly Tyr His Ser Val Ile Ala Ala Leu Lys Ala Gly Tyr Arg
        35                  40                  45

His Ile Asp Ala Ala Ala Ile Tyr Leu Asn Glu Glu Glu Val Gly Arg
    50                  55                  60

Ala Ile Lys Asp Ser Gly Val Pro Arg Glu Glu Ile Phe Ile Thr Thr
65                  70                  75                  80

Lys Leu Trp Gly Thr Glu Gln Arg Asp Pro Glu Ala Ala Leu Asn Lys
                85                  90                  95

Ser Leu Lys Arg Leu Gly Leu Asp Tyr Val Asp Leu Tyr Leu Met His
            100                 105                 110

Trp Pro Val Pro Leu Lys Thr Asp Arg Val Thr Asp Gly Asn Val Leu
        115                 120                 125

Cys Ile Pro Thr Leu Glu Asp Gly Thr Val Asp Ile Asp Thr Lys Glu
    130                 135                 140

Trp Asn Phe Ile Lys Thr Trp Glu Leu Met Gln Glu Leu Pro Lys Thr
145                 150                 155                 160

Gly Lys Thr Lys Ala Val Gly Val Ser Asn Phe Ser Ile Asn Asn Ile
                165                 170                 175

Lys Glu Leu Leu Glu Ser Pro Asn Asn Lys Val Val Pro Ala Thr Asn
            180                 185                 190

Gln Ile Glu Ile His Pro Leu Leu Pro Gln Asp Glu Leu Ile Ala Phe
        195                 200                 205

Cys Lys Glu Lys Gly Ile Val Val Glu Ala Tyr Ser Pro Phe Gly Ser
    210                 215                 220

Ala Asn Ala Pro Leu Leu Lys Glu Gln Ala Ile Ile Asp Met Ala Lys
225                 230                 235                 240

Lys His Gly Val Glu Pro Ala Gln Leu Ile Ile Ser Trp Ser Ile Gln
                245                 250                 255

Arg Gly Tyr Val Val Leu Ala Lys Ser Val Asn Pro Glu Arg Ile Val
            260                 265                 270

Ser Asn Phe Lys Ile Phe Thr Leu Pro Glu Asp Asp Phe Lys Thr Ile
        275                 280                 285

Ser Asn Leu Ser Lys Val His Gly Thr Lys Arg Val Val Asp Met Lys
    290                 295                 300

Trp Gly Ser Phe Pro Ile Phe Gln
305                 310

<210>  64
<211>  939
<212>  DNA
<213>  Saccharomyces cerevisiae

<400>  64
atgcctgcta cgttaaagaa ttcttctgct acattaaaac taaatactgg tgcctccatt      60

ccagtgttgg gtttcggcac ttggcgttcc gttgacaata acggttacca ttctgtaatt     120

gcagctttga aagctggata cagacacatt gatgctgcgg ctatctattt gaatgaagaa 180

gaagttggca gggctattaa agattccgga gtccctcgtg aggaaatttt tattactact 240

aagctttggg gtacggaaca acgtgatccg gaagctgctc taaacaagtc tttgaaaaga 300

ctaggcttgg attatgttga cctatatctg atgcattggc cagtgccttt gaaaaccgac 360

agagttactg atggtaacgt tctgtgcatt ccaacattag aagatggcac tgttgacatc 420

gatactaagg aatggaattt tatcaagacg tgggagttga tgcaagagtt gccaaagacg 480

ggcaaaacta aagccgttgg tgtctctaat ttttctatta acaacattaa agaattatta 540

gaatctccaa ataacaaggt ggtaccagct actaatcaaa ttgaaattca tccattgcta 600

ccacaagacg aattgattgc cttttgtaag gaaaagggta ttgttgttga agcctactca 660

ccatttggga gtgctaatgc tcctttacta aaagagcaag caattattga tatggctaaa 720

aagcacggcg ttgagccagc acagcttatt atcagttgga gtattcaaag aggctacgtt 780

gttctggcca aatcggttaa tcctgaaaga attgtatcca attttaagat tttcactctg 840

cctgaggatg atttcaagac tattagtaac ctatccaaag tgcatggtac aaagagagtc 900

gttgatatga agtggggatc cttcccaatt ttccaatga 939

<210> 65
<211> 353
<212> PRT
<213> Corynebacterium glutamicum

<400> 65

Met Ser Ile Ser Val Lys Ala Leu Gln Lys Ser Gly Pro Glu Ala Pro
1               5                   10                  15

Phe Glu Val Lys Ile Ile Glu Arg Arg Asp Pro Arg Ala Asp Asp Val
            20                  25                  30

Val Ile Asp Ile Lys Ala Ala Gly Ile Cys His Ser Asp Ile His Thr
        35                  40                  45

Ile Arg Asn Glu Trp Gly Glu Ala His Phe Pro Leu Thr Val Gly His
    50                  55                  60

Glu Ile Ala Gly Val Val Ser Ala Val Gly Ser Asp Val Thr Lys Trp
65                  70                  75                  80

Lys Val Gly Asp Arg Val Gly Val Gly Cys Leu Val Asn Ser Cys Gly
                85                  90                  95

Glu Cys Glu Gln Cys Val Ala Gly Phe Glu Asn Asn Cys Leu Arg Gly
            100                 105                 110

Asn Val Gly Thr Tyr Asn Ser Asn Asp Val Asp Gly Thr Ile Thr Gln
        115                 120                 125

Gly Gly Tyr Ala Glu Lys Val Val Val Asn Glu Arg Phe Leu Cys Ser
    130                 135                 140

Ile Pro Glu Glu Leu Asn Phe Asp Val Ala Ala Pro Leu Leu Cys Ala
145                 150                 155                 160

Gly Ile Thr Thr Tyr Ser Pro Ile Ala Arg Trp Asn Val Lys Glu Gly
                165                 170                 175

Asp Lys Val Ala Val Met Gly Leu Gly Gly Leu Gly His Met Gly Val
            180                 185                 190

Gln Ile Ala Ala Ala Lys Gly Ala Glu Val Thr Val Leu Ser Arg Ser
        195                 200                 205

Leu Arg Lys Ala Glu Leu Ala Lys Glu Leu Gly Ala Ala Arg Thr Leu
    210                 215                 220

Ala Thr Ser Asp Glu Asp Phe Phe Thr Glu His Ala Gly Glu Phe Asp
225                 230                 235                 240

Phe Ile Leu Asn Thr Ile Ser Ala Ser Ile Pro Val Asp Lys Tyr Leu
                245                 250                 255

Ser Leu Leu Lys Pro His Gly Val Met Ala Val Val Gly Leu Pro Pro
            260                 265                 270

Glu Lys Gln Pro Leu Ser Phe Gly Ala Leu Ile Gly Gly Gly Lys Val
        275                 280                 285

Leu Thr Gly Ser Asn Ile Gly Gly Ile Pro Glu Thr Gln Glu Met Leu
    290                 295                 300

Asp Phe Cys Ala Lys His Gly Leu Gly Ala Met Ile Glu Thr Val Gly
305                 310                 315                 320

Val Asn Asp Val Asp Ala Ala Tyr Asp Arg Val Val Ala Gly Asp Val
                325                 330                 335

Gln Phe Arg Val Val Ile Asp Thr Ala Ser Phe Ala Glu Val Glu Ala
            340                 345                 350

Val

<210>  66
<211>  1062
<212>  DNA
<213>  Corynebacterium glutamicum

<400>  66
gtgagtatct cagtaaaagc actacaaaag tccggcccag aagcaccttt cgaggtcaag      60

atcattgaac gccgtgaccc acgcgcagat gatgtggtta ttgatatcaa agctgcgggc     120

atctgccaca gcgatatcca caccatccgc aacgaatggg gcgaggcgca cttcccgctc     180

accgtcggcc acgaaatcgc aggcgttgtc tctgcggttg gatccgatgt aaccaaatgg     240

aaagtcggcg accgcgtggg cgtcggctgc ctcgttaact cctgcggcga atgcgaacag     300

tgcgtcgcag gatttgaaaa caactgcctt cgcggaaacg tcggaaccta caactctaac     360

gacgtcgacg gcaccatcac ccaaggcggc tacgctgaaa aggtagtggt caacgaacgt     420

ttcctgtgca gcatcccaga ggaacttaac ttcgatgtcg cagcaccact gctgtgcgca     480

ggcatcacca cctactcccc aatcgctcgc tggaacgtta aagaaggcga caaagtagca     540

gtcatgggcc tcggcggact cggacacatg ggtgtccaga tcgctgcagc caagggtgct      600

gaggttaccg ttctgtcccg ttccctgcgc aaggcagaac ttgccaagga actcggcgca     660

gctcgcacgc ttgcgacttc tgatgaggat ttcttcaccg aacacgccgg tgaattcgac     720

ttcatcctca acaccattag cgcatccatc ccagtcgaca agtacctgag ccttctcaag     780

ccacacggtg tcatggctgt tgtcggtctg ccaccagaga agcagccact gagcttcggt     840

gcgctcatcg gcggcggaaa agtcctcacc ggatccaaca ttggcggcat ccctgaaacc     900

caggaaatgc tcgacttctg tgcaaaacac ggcctcggtg cgatgatcga aactgtcggc     960

gtcaacgatg ttgatgcagc ctacgaccgt gttgttgccg gcgacgttca gttccgcgtt    1020

gtcattgata ctgcttcgtt tgctgaggtt gaggcggttt ag                       1062

<210>  67
<211>  368
<212>  PRT
<213>  Corynebacterium glutamicum

<400>  67

Met Ser Thr Val Val Pro Gly Ile Val Ala Leu Ser Lys Gly Ala Pro
1               5                   10                  15

Val Glu Lys Val Asn Val Val Val Pro Asp Pro Gly Ala Asn Asp Val
            20                  25                  30

Ile Val Lys Ile Gln Ala Cys Gly Val Cys His Thr Asp Leu Ala Tyr
        35                  40                  45

Arg Asp Gly Asp Ile Ser Asp Glu Phe Pro Tyr Leu Leu Gly His Glu
    50                  55                  60

Ala Ala Gly Ile Val Glu Glu Val Gly Glu Ser Val Thr His Val Glu
65                  70                  75                  80

Val Gly Asp Phe Val Ile Leu Asn Trp Arg Ala Val Cys Gly Glu Cys
                85                  90                  95

Arg Ala Cys Lys Lys Gly Glu Pro Lys Tyr Cys Phe Asn Thr His Asn
            100                 105                 110

Ala Ser Lys Lys Met Thr Leu Glu Asp Gly Thr Glu Leu Ser Pro Ala
        115                 120                 125

Leu Gly Ile Gly Ala Phe Leu Glu Lys Thr Leu Val His Glu Gly Gln
    130                 135                 140

Cys Thr Lys Val Asn Pro Glu Glu Asp Pro Ala Ala Ala Gly Leu Leu
145                 150                 155                 160

Gly Cys Gly Ile Met Ala Gly Leu Gly Ala Ala Val Asn Thr Gly Asp
                165                 170                 175

Ile Lys Arg Gly Glu Ser Val Ala Val Phe Gly Leu Gly Gly Val Gly
            180                 185                 190

Met Ala Ala Ile Ala Gly Ala Lys Ile Ala Gly Ala Ser Lys Ile Ile
        195                 200                 205

Ala Val Asp Ile Asp Glu Lys Lys Leu Glu Trp Ala Lys Glu Phe Gly
    210                 215                 220

Ala Thr His Thr Ile Asn Ser Ser Gly Leu Gly Gly Glu Gly Asp Ala
225                 230                 235                 240

Ser Glu Val Val Ala Lys Val Arg Glu Leu Thr Asp Gly Phe Gly Thr
                245                 250                 255

Asp Val Ser Ile Asp Ala Val Gly Ile Met Pro Thr Trp Gln Gln Ala
            260                 265                 270

Phe Tyr Ser Arg Asp His Ala Gly Arg Met Val Met Val Gly Val Pro
        275                 280                 285

Asn Leu Thr Ser Arg Val Asp Val Pro Ala Ile Asp Phe Tyr Gly Arg
    290                 295                 300

Gly Gly Ser Val Arg Pro Ala Trp Tyr Gly Asp Cys Leu Pro Glu Arg
305                 310                 315                 320

Asp Phe Pro Thr Tyr Val Asp Leu His Leu Gln Gly Arg Phe Pro Leu
                325                 330                 335

Asp Lys Phe Val Ser Glu Arg Ile Gly Leu Asp Asp Val Glu Glu Ala
            340                 345                 350

Phe Asn Thr Met Lys Ala Gly Asp Val Leu Arg Ser Val Val Glu Ile
        355                 360                 365

<210>  68
<211>  1107
<212>  DNA
<213>  Corynebacterium glutamicum

<400>  68
atgagcactg tagtgcctgg aattgtcgca ctgtccaagg gtgcaccggt agaaaaagta      60

aacgttgttg tccctgatcc aggtgctaac gatgtcatcg tcaagattca ggcctgcggt     120

gtgtgccaca ccgacttggc ctaccgcgat ggcgatattt cagatgagtt cccttacctc     180

ctcggccacg aggcagcagg cattgttgag gaggtaggcg agtccgtcac ccacgttgag     240

gtcggcgatt tcgtcatctt gaactggcgt gcagtgtgcg gcgagtgccg tgcatgtaag     300

aagggcgagc caaagtactg ctttaacacc cacaacgcct ctaagaagat gaccctggaa     360

gacggcaccg agctgtcccc agcactgggt attggcgcgt tcttggaaaa gaccctggtc     420

cacgaaggcc agtgcaccaa ggttaaccct gaggaagatc cagcagcagc tggccttctg     480

ggttgtggca tcatggcagg ccttggcgct gcggtgaaca ccggtgatat taagcgtggc     540

gagtccgtag cagtcttcgg ccttggtggc gtgggcatgg cagctattgc tggcgccaag     600

attgctggcg cttccaagat cattgctgtt gatatcgatg agaagaagct ggagtgggcg     660

aaggaattcg gcgcaaccca caccattaat tcctctggtc ttggtggcga aggtgatgcc     720

tctgaggtcg tggcaaaggt tcgtgagctc accgatggtt tcggcaccga tgtctccatc     780

gatgcggtag gcatcatgcc gacctggcag caggcgtttt actcccgtga ccatgcaggc     840

cgcatggtga tggtgggcgt tccaaacctg acgtctcgcg tagatgttcc tgcgattgat     900

ttttacggtc gcggtggatc cgtgcgccct gcatggtacg gcgactgcct gcctgagcgt     960

gatttcccaa cttatgtgga tctgcacctg cagggtcgtt tcccactgga taagtttgtt    1020

tctgagcgta ttggtcttga tgatgttgaa gaggctttca acaccatgaa ggctggcgac    1080

gtgctgcgtt ctgtggtgga gatctaa                                        1107

<210>  69
<211>  339
<212>  PRT
<213>  Corynebacterium glutamicum

<400>  69

Met Pro Lys Tyr Ile Ala Met Gln Val Ser Glu Ser Gly Ala Pro Leu
1               5                   10                  15

Ala Ala Asn Leu Val Gln Pro Ala Pro Leu Lys Ser Arg Glu Val Arg
            20                  25                  30

Val Glu Ile Ala Ala Ser Gly Val Cys His Ala Asp Ile Gly Thr Ala
        35                  40                  45

Ala Ala Ser Gly Lys His Thr Val Phe Pro Val Thr Pro Gly His Glu
    50                  55                  60

Ile Ala Gly Thr Ile Ala Glu Ile Gly Glu Asn Val Ser Arg Trp Thr
65                  70                  75                  80

Val Gly Asp Arg Val Ala Ile Gly Trp Phe Gly Gly Asn Cys Gly Asp
                85                  90                  95

Cys Ala Phe Cys Arg Ala Gly Asp Pro Val His Cys Arg Glu Arg Lys
            100                 105                 110

Ile Pro Gly Val Ser Tyr Ala Gly Gly Trp Ala Gln Asn Ile Val Val
        115                 120                 125

Pro Ala Glu Ala Leu Ala Ala Ile Pro Asp Gly Met Asp Phe Tyr Glu
    130                 135                 140

Ala Ala Pro Met Gly Cys Ala Gly Val Thr Thr Phe Asn Ala Leu Arg
145                 150                 155                 160

Asn Leu Lys Leu Asp Pro Gly Ala Ala Val Ala Val Phe Gly Ile Gly
                165                 170                 175

Gly Leu Val Arg Leu Ala Ile Gln Phe Ala Ala Lys Met Gly Tyr Arg
            180                 185                 190

Thr Ile Thr Ile Ala Arg Gly Leu Glu Arg Glu Glu Leu Ala Arg Gln
        195                 200                 205

Leu Gly Ala Asn His Tyr Ile Asp Ser Asn Asp Leu His Pro Gly Gln
    210                 215                 220

Ala Leu Phe Glu Leu Gly Gly Ala Asp Leu Ile Leu Ser Thr Ala Ser
225                 230                 235                 240

Thr Thr Glu Pro Leu Ser Glu Leu Ser Thr Gly Leu Ser Ile Gly Gly
                245                 250                 255

Gln Leu Thr Ile Ile Gly Val Asp Gly Gly Asp Ile Thr Val Ser Ala
            260                 265                 270

Ala Gln Leu Met Met Asn Arg Gln Ile Ile Thr Gly His Leu Thr Gly
        275                 280                 285

Ser Ala Asn Asp Thr Glu Gln Thr Met Lys Phe Ala His Leu His Gly
    290                 295                 300

Val Lys Pro Leu Ile Glu Arg Met Pro Leu Asp Gln Ala Asn Glu Ala
305                 310                 315                 320

Ile Ala Arg Ile Ser Ala Gly Lys Pro Arg Phe Arg Ile Val Leu Glu
                325                 330                 335

Pro Asn Ser

<210>  70
<211>  1020
<212>  DNA
<213>  Corynebacterium glutamicum

<400>  70
atgcccaaat acattgccat gcaggtatcc gaatccggtg caccgttagc cgcgaatctc      60

gtgcaacctg ctccgttgaa atcgagggaa gtccgcgtgg aaatcgctgc tagtggtgtg     120

tgccatgcag atattggcac ggcagcagca tcggggaagc acactgtttt tcctgttacc     180

cctggtcatg agattgcagg aaccatcgcg gaaattggtg aaaacgtatc tcggtggacg     240

gttggtgatc gcgttgcaat cggttggttt ggtggcaatt gcggtgactg cgctttttgt     300

cgtgcaggtg atcctgtgca ttgcagagag cggaagattc ctggcgtttc ttatgcgggt     360

ggttgggcac agaatattgt tgttccagcg gaggctcttg ctgcgattcc agatggcatg     420

gacttttacg aggccgcccc gatgggctgc gcaggtgtga caacattcaa tgcgttgcga     480

aacctgaagc tggatcccgg tgcggctgtc gcggtctttg gaatcggcgg tttagtgcgc     540

ctagctattc agtttgctgc gaaaatgggt tatcgaacca tcaccatcgc ccgcggttta     600

gagcgtgagg agctagctag gcaacttggc gccaaccact acatcgatag caatgatctg     660

caccctggcc aggcgttatt tgaacttggc ggggctgact tgatcttgtc tactgcgtcc     720

accacggagc ctctttcgga gttgtctacc ggtctttcta ttggcgggca gctaaccatt     780

atcggagttg atgggggaga tatcaccgtt tcggcagccc aattgatgat gaaccgtcag     840

atcatcacag gtcacctcac tggaagtgcg aatgacacgg aacagactat gaaatttgct     900

catctccatg gcgtgaaacc gcttattgaa cggatgcctc tcgatcaagc caacgaggct     960

attgcacgta tttcagctgg taaaccacgt ttccgtattg tcttggagcc gaattcataa     1020

<210>  71
<211>  345
<212>  PRT
<213>  Corynebacterium glutamicum

<400>  71

Met Thr Thr Ala Ala Pro Gln Glu Phe Thr Ala Ala Val Val Glu Lys
1               5                   10                  15

Phe Gly His Asp Val Thr Val Lys Asp Ile Asp Leu Pro Lys Pro Gly
            20                  25                  30

Pro His Gln Ala Leu Val Lys Val Leu Thr Ser Gly Ile Cys His Thr
        35                  40                  45

Asp Leu His Ala Leu Glu Gly Asp Trp Pro Val Lys Pro Glu Pro Pro
    50                  55                  60

Phe Val Pro Gly His Glu Gly Val Gly Glu Val Val Glu Leu Gly Pro
65                  70                  75                  80

Gly Glu His Asp Val Lys Val Gly Asp Ile Val Gly Asn Ala Trp Leu
                85                  90                  95

Trp Ser Ala Cys Gly Thr Cys Glu Tyr Cys Ile Thr Gly Arg Glu Thr
            100                 105                 110

Gln Cys Asn Glu Ala Glu Tyr Gly Gly Tyr Thr Gln Asn Gly Ser Phe
        115                 120                 125

Gly Gln Tyr Met Leu Val Asp Thr Arg Tyr Ala Ala Arg Ile Pro Asp
    130                 135                 140

Gly Val Asp Tyr Leu Glu Ala Ala Pro Ile Leu Cys Ala Gly Val Thr
145                 150                 155                 160

Val Tyr Lys Ala Leu Lys Val Ser Glu Thr Arg Pro Gly Gln Phe Met
                165                 170                 175

Val Ile Ser Gly Val Gly Gly Leu Gly His Ile Ala Val Gln Tyr Ala
            180                 185                 190

Ala Ala Met Gly Met Arg Val Ile Ala Val Asp Ile Ala Asp Asp Lys
        195                 200                 205

Leu Glu Leu Ala Arg Lys His Gly Ala Glu Phe Thr Val Asn Ala Arg
    210                 215                 220

Asn Glu Asp Ser Gly Glu Ala Val Gln Lys Tyr Thr Asn Gly Gly Ala
225                 230                 235                 240

His Gly Val Leu Val Thr Ala Val His Glu Ala Ala Phe Gly Gln Ala
                245                 250                 255

Leu Asp Met Ala Arg Arg Ala Gly Thr Ile Val Phe Asn Gly Leu Pro
            260                 265                 270

Pro Gly Glu Phe Pro Ala Ser Val Phe Asn Ile Val Phe Lys Gly Leu
        275                 280                 285

Thr Ile Arg Gly Ser Leu Val Gly Thr Arg Gln Asp Leu Ala Glu Ala
    290                 295                 300

Leu Asp Phe Phe Ala Arg Gly Leu Ile Lys Pro Thr Val Ser Glu Cys
305                 310                 315                 320

Ser Leu Asp Glu Val Asn Gly Val Leu Asp Arg Met Arg Asn Gly Lys
                325                 330                 335

Ile Asp Gly Arg Val Ala Ile Arg Phe
            340                 345

<210>  72
<211>  1038
<212>  DNA
<213>  Corynebacterium glutamicum

<400>  72
atgaccactg ctgcacccca agaatttacc gctgctgttg ttgaaaaatt cggtcatgac      60

gtgaccgtga aggatattga ccttccaaag ccagggccac accaggcatt ggtgaaggta     120

ctcacctccg gcatctgcca caccgacctc cacgccttgg agggcgattg gccagtaaag     180

ccggaaccac cattcgtacc aggacacgaa ggtgtaggtg aagttgttga gctcggacca     240

ggtgaacacg atgtgaaggt cggcgatatt gtcggcaatg cgtggctctg gtcagcgtgt     300

ggcacctgcg aatactgcat caccggcagg gaaactcagt gcaacgaagc tgagtatggt     360

ggctacaccc aaaatggatc cttcggccag tacatgctgg tggatacccg ttacgccgct     420

cgcatcccag acggcgtgga ctacctcgaa gcagcaccaa ttctgtgtgc aggcgtgact     480

gtctacaagg cactcaaagt ctctgaaacc cgcccgggcc aattcatggt gatctccggt 540

gtcggcggac ttggccacat cgcagtccaa tacgcagcgg cgatgggcat gcgtgtcatt 600

gcggtagata ttgccgatga caagctggaa cttgcccgta agcacggtgc ggaatttacc 660

gtgaatgcgc gtaatgaaga ttcaggcgaa gctgtacaga agtacaccaa cggtggcgca 720

cacggcgtgc ttgtgactgc agttcacgag gcagcattcg gccaggcact ggatatggct 780

cgacgtgcag gaacaattgt gttcaacggt ctgccaccgg gagagttccc agcatccgtg 840

ttcaacatcg tattcaaggg cctgaccatc cgtggatccc tcgtgggaac ccgccaagac 900

ttggccgaag cgctcgattt ctttgcacgc ggactaatca agccaaccgt gagtgagtgc 960

tccctcgatg aggtcaatgg tgtgcttgac cgcatgcgaa acggcaagat cgatggtcgt 1020

gtggcgattc gtttctaa 1038

<210> 73
<211> 356
<212> PRT
<213> Corynebacterium glutamicum

<400> 73

Met Ser Leu Gln Phe Asp His Glu Thr Leu Gly Gln Arg Val Leu Phe
1               5                   10                  15

Gly Ser Gly Glu Ala Ala Gln Asn Leu Ala Ala Glu Ile Ser Arg Leu
            20                  25                  30

Asp Ala Lys Asn Val Met Val Val Ala Gly Asp Phe Glu Leu Pro Met
        35                  40                  45

Ala Arg Gln Val Ala Ala Asp Ile Asp Val Lys Val Trp His Ser Asn
    50                  55                  60

Val Val Met His Val Pro Ile Glu Thr Ala Glu Glu Ala Arg Ser Val
65                  70                  75                  80

Ala Lys Glu Asn Asp Ile Asp Val Val Val Cys Val Gly Gly Gly Ser
                85                  90                  95

Thr Thr Gly Leu Ala Lys Ala Ile Ala Met Thr Thr Ala Leu Pro Ile
            100                 105                 110

Ile Ala Val Pro Thr Thr Tyr Ala Gly Ser Glu Ala Thr Asn Val Trp
        115                 120                 125

Gly Leu Thr Glu Ala Ala Arg Lys Thr Thr Gly Val Asp Asn Lys Val
    130                 135                 140

Leu Pro Val Thr Val Ile Tyr Asp Ser Ala Leu Thr Met Ser Leu Pro
145                 150                 155                 160

Val Glu Met Ser Val Ala Ser Gly Leu Asn Gly Leu Ala His Cys Ile
                165                 170                 175

Asp Ser Leu Trp Gly Pro Lys Ala Asp Pro Ile Asn Ala Ala Met Ala
            180                 185                 190

Ala Glu Gly Ile Arg Ala Leu Ser Ala Gly Leu Pro Lys Ile Val Ala
        195                 200                 205

Asp Ala Gln Asp Val Asp Gly Arg Asp Glu Ala Leu Tyr Gly Ala Tyr
    210                 215                 220

Leu Ala Ala Val Ser Phe Ala Ser Ala Gly Ser Gly Leu His His Lys
225                 230                 235                 240

Ile Cys His Val Leu Gly Gly Thr Phe Asn Leu Pro His Ala Gln Thr
                245                 250                 255

His Ala Thr Val Leu Pro Tyr Val Leu Ala Phe Asn Ala Pro Tyr Ala
            260                 265                 270

Pro Gln Ala Glu Gln Arg Ala Ala Ala Ala Phe Gly Ser Ala Thr Ala
        275                 280                 285

Leu Glu Gly Leu Gln Gln Leu Arg Ala Gln Val Gly Ala Pro Gln Arg
    290                 295                 300

Leu Ser Asp Tyr Gly Phe Thr Ala Ala Gly Ile Pro Glu Ala Val Glu
305                 310                 315                 320

Ile Ile Leu Glu Lys Val Pro Ala Asn Asn Pro Arg Thr Val Thr Glu
                325                 330                 335

Glu Asn Leu Thr Ala Leu Leu Thr Thr Ala Leu Asn Gly Asp Asp Pro
            340                 345                 350

Ala Thr Leu Asn
        355

<210>  74
<211>  1071
<212>  DNA
<213>  Corynebacterium glutamicum

<400>  74
atgtctttac agttcgatca tgaaaccctc ggtcaacgag ttctgttcgg ttcaggtgag      60

gcggcgcaaa atctcgccgc tgaaattagc cgactcgatg ccaaaaacgt catggtggtt     120

gccggtgatt tcgagcttcc catggcacgg caagtagcag cagatattga tgtcaaggtg     180

tggcattcaa atgtcgtgat gcatgtgccc atcgaaacag cagaagaagc acgcagtgtt     240

gcgaaagaaa acgacattga tgttgtggtg tgtgtgggcg gtggatccac aacaggtcta     300

gctaaagcga ttgccatgac caccgcattg ccgatcattg cggtacccac tacttatgca     360

ggttctgaag caacaaatgt gtggggattg accgaagccg cgcgcaaaac aactggtgtt     420

gataacaaag tgctgccagt gacagttatc tacgattcag cgttaaccat gtctttgccg     480

gtagaaatgt cggttgcttc tggtctcaat ggtttggctc actgcattga ttctttgtgg     540

ggaccgaagg cggatcccat caatgcggct atggctgctg agggaattcg agcactttct     600

gctggccttc ccaagattgt ggcagatgct caggacgtag atggtcgcga tgaagcgctc     660

tacggtgcct acctggctgc ggtgtctttt gcctctgctg gctctggtct ccaccacaag     720

atctgccacg tgttgggtgg aacttttaac cttccacacg cgcaaaccca tgcaacagta     780

ctgccttatg ttcttgcctt caacgcgcca tatgcgccac aggcagaaca acgcgcagcg     840

gcagctttcg gttctgcgac agcacttgaa ggattgcaac agctgcgtgc ccaagtggga     900

gcaccacagc gactatccga ttacggattc accgcagcag gaatcccaga ggcagtggaa     960

atcatcttgg agaaagtacc ggcgaataat ccacggacgg tcacagaaga aaacctcact    1020

gcgctgctta ccacagcgct caacggcgac gatccagcaa ctttgaatta a             1071


<210>  75
<211>  292
<212>  PRT
<213>  Corynebacterium glutamicum

<400>  75

Met Gln Thr Leu Ala Ala Ile Val Arg Ala Thr Lys Gln Pro Phe Glu
1               5                   10                  15


Ile Thr Thr Ile Asp Leu Asp Ala Pro Arg Pro Asp Glu Val Gln Ile
            20                  25                  30


Arg Val Ile Ala Ala Gly Val Arg His Thr Asp Ala Ile Val Arg Asp
        35                  40                  45


Gln Ile Tyr Pro Thr Phe Leu Pro Ala Val Phe Gly His Glu Gly Ala
    50                  55                  60


Gly Val Val Val Ala Val Gly Ser Ala Val Thr Ser Val Lys Pro Asp
65                  70                  75                  80

Asp Lys Val Val Leu Gly Phe Asn Ser Cys Gly Gln Cys Leu Lys Cys
                85                  90                  95

Leu Gly Gly Lys Pro Ala Tyr Cys Glu Lys Phe Tyr Asp Arg Asn Phe
            100                 105                 110

Ala Cys Thr Arg Asp Ala Gly His Thr Thr Leu Phe Thr Arg Ala Thr
        115                 120                 125

Lys Glu Gln Ala Glu Ala Ile Ile Asp Thr Leu Asp Asp Val Phe Tyr
    130                 135                 140

Asp Ala Asp Ala Gly Phe Leu Ala Tyr Pro Ala Thr Pro Pro Glu Ala
145                 150                 155                 160

Ser Gly Val Ser Val Leu Val Val Ala Ala Gly Thr Ser Asp Leu Pro
                165                 170                 175

Gln Ala Lys Glu Ala Leu His Thr Ala Ser Tyr Leu Gly Arg Ser Thr
            180                 185                 190

Ser Leu Ile Val Asp Phe Gly Val Ala Gly Ile His Arg Leu Leu Ser
        195                 200                 205

Tyr Glu Glu Glu Leu Arg Ala Ala Gly Val Leu Ile Val Ala Ala Gly
    210                 215                 220

Met Asp Gly Ala Leu Pro Gly Val Val Ala Gly Leu Val Ser Ala Pro
225                 230                 235                 240

Val Val Ala Leu Pro Thr Ser Val Gly Tyr Gly Ala Gly Ala Gly Gly
                245                 250                 255

Ile Ala Pro Leu Leu Thr Met Leu Asn Ala Cys Ala Pro Gly Val Gly
            260                 265                 270

Val Val Asn Ile Asp Asn Gly Tyr Gly Ala Gly His Leu Ala Ala Gln
        275                 280                 285

Ile Ala Ala Arg
    290

<210>  76
<211>  879
<212>  DNA
<213>  Corynebacterium glutamicum

<400>  76
atgcaaaccc ttgctgctat tgttcgtgcc acgaagcaac cttttgagat caccaccatt      60

gatctggatg caccacgacc agatgaagtt caaatccgtg ttattgctgc cggagtgcgc     120

cacactgacg caattgttcg tgatcagatt tacccaactt ttcttcccgc agttttcggc     180

cacgaaggcg ccggagtagt tgtcgccgtg ggttctgcag tcacctcggt gaaaccagat     240

gacaaggtag tgctgggatt caactcttgt ggccagtgct tgaagtgttt gggcggtaag     300

cctgcgtact gtgagaaatt ctatgaccgc aacttcgcat gcacccgcga tgccgggcac     360

actactttgt ttacccgtgc aacaaaagag caggcagagg ccatcatcga cacccttgat     420

gatgttttct acgatgcgga tgcgggtttc ctggcatacc cagcaactcc cccagaggct     480

tcgggagtaa gcgtgttggt tgtcgcggct ggtacctctg atctccccca agcaaaggaa     540

gcactacaca ctgcctccta cttggggcgc tccacctcac tgattgttga ttttggagtg     600

gctggcatcc accgcctgct ttcatacgaa gaagaactcc gcgctgcggg cgtgctcatc     660

gttgccgctg gaatggatgg tgcgctaccc ggagttgtcg caggcttagt gtccgcacct     720

gtcgtcgcac tgccaacctc cgtgggatac ggcgcaggtg ctggaggaat cgcaccactt     780

ctgaccatgc ttaacgcctg cgcgccggga gttggagtgg tcaacattga taacggctat     840

ggagcaggac acctggctgc gcagattgcg gcgaggtaa                            879

<210>  77
<211>  253
<212>  PRT
<213>  Corynebacterium glutamicum

<400>  77

Met Leu Asn Ala Val Gly Lys Ala Gln Asn Ile Leu Leu Leu Gly Gly
1               5                   10                  15

Thr Ser Glu Ile Gly Ile Ser Ile Val Ser Arg Phe Leu Lys Gln Gly
            20                  25                  30

Pro Ser His Val Thr Leu Ala Ala Arg Lys Asp Ser Pro Arg Val Asp
        35                  40                  45

Ala Ala Val Ala Glu Ile Lys Ala Ala Gly Ala Ala Ser Val Ala Val
    50                  55                  60

Val Asp Phe Asp Ala Leu Asp Thr Glu Ser His Pro Ala Ala Ile Asp
65                  70                  75                  80

Ala Ala Phe Glu Asn Gly Asp Val Asp Val Ala Ile Val Ala Phe Gly
                85                  90                  95

Ile Leu Gly Asp Asn Glu Ala Gln Trp Arg Asp Gln Ala Leu Ala Val
            100                 105                 110

Glu Ala Thr Thr Val Asn Tyr Thr Ala Gly Val Ser Val Gly Val Leu
        115                 120                 125

Leu Gly Gln Lys Phe Glu Gln Gln Gly His Gly Thr Ile Val Ala Leu
    130                 135                 140

Ser Ser Val Ala Gly Gln Arg Val Arg Arg Ser Asn Phe Val Tyr Gly
145                 150                 155                 160

Ser Ala Lys Ala Gly Phe Asp Gly Phe Tyr Thr Gln Leu Gly Glu Ala
                165                 170                 175

Leu Arg Gly Ser Gly Ala Asn Val Leu Val Val Arg Pro Gly Gln Val
            180                 185                 190

Arg Thr Lys Met Ser Ala Asp Gly Gly Glu Ala Pro Leu Thr Val Asn
        195                 200                 205

Arg Glu Asp Val Ala Asp Ala Val Tyr Asp Ala Val Val Asn Lys Lys
    210                 215                 220

Asp Ile Ile Phe Val His Pro Leu Phe Gln Tyr Val Ser Phe Ala Phe
225                 230                 235                 240

Gln Phe Ile Pro Arg Ala Ile Phe Arg Lys Leu Pro Phe
                245                 250

<210>  78
<211>  762
<212>  DNA
<213>  Corynebacterium glutamicum

<400>  78
atgcttaacg cagtgggcaa agcccaaaac attctccttc ttggtggaac ctctgagatc      60

ggtatttcca ttgtctcccg cttcctcaag cagggtccat cccatgtgac cttggcagcg     120

cgtaaagatt ccccacgcgt ggacgcagca gtcgcagaga tcaaagcagc tggcgctgct     180

tccgttgctg ttgttgattt cgatgcgctc gacaccgaat cccaccctgc agccatcgac     240

gcagcctttg aaaacggcga cgttgacgta gcaatcgtgg ctttcggcat cctcggcgac     300

aacgaagcac agtggcgcga ccaagcacta gcagtggaag caaccaccgt gaactacacc     360

gccggcgttt ccgtaggtgt actgctgggc cagaaatttg agcagcaggg ccacggcacc     420

atcgtggcat tgtcctctgt ggcaggccag cgagtccgcc gctccaactt tgtctacggc     480

tccgccaagg caggtttcga cggtttctac acccagctcg gcgaagccct gcgtggatcc     540

ggtgccaacg tattggtggt tcgcccaggc caggtacgca ccaagatgtc cgcagatggt 600

ggcgaagccc cactgaccgt caaccgcgaa gacgtggcag atgctgttta tgatgcagtg 660

gtgaacaaga aggacatcat ctttgtccac ccactgttcc agtacgtctc ttttgcgttc 720

caattcattc cgcgagcaat cttccgcaag ctgccgttct aa 762

<210> 79
<211> 315
<212> PRT
<213> Corynebacterium glutamicum

<400> 79

Met Glu His Gly Val Thr Val Ile Lys Gly Thr Glu Phe Asp Val Phe
1               5                   10                  15

Pro Leu Asn Leu Gly Gly Asn Thr Phe Gly Trp Thr Ser Asn Arg Glu
            20                  25                  30

Gln Thr Phe Ala Val Leu Asp Ala Phe Val Ala Ala Gly Gly Asn Phe
        35                  40                  45

Val Asp Thr Ala Asp Ser Tyr Ser Ala Trp Val Glu Gly Asn Glu Gly
    50                  55                  60

Gly Glu Ser Glu Arg Glu Leu Gly Ala Trp Ile Lys Glu Arg Gly Ala
65                  70                  75                  80

Asp Lys Leu Ile Ile Ala Thr Lys Ser Gly Ala Leu Glu Pro Val Ala
                85                  90                  95

Gly Arg Ser Arg Glu Ala Thr Phe Lys Ala Val Glu Gly Ser Leu Glu
            100                 105                 110

Arg Leu Gly Val Glu Ser Ile Asp Ile Phe Tyr Tyr His Tyr Asp Asp
        115                 120                 125

Glu Ala Val Ser Ile Asp Glu Gln Val Ala Ile Ala Asn Asp Leu Ile
    130                 135                 140

Ala Gln Gly Lys Ile Lys His Leu Ala Leu Ser Asn Tyr Ser Ala Glu
145                 150                 155                 160

Arg Leu Ala Glu Phe Phe Glu Lys Ser Val Gly Thr Pro Ala Gln Pro
                165                 170                 175

Val Ala Leu Gln Pro His Tyr Asn Leu Val Ser Arg Lys Asp Tyr Glu
            180                 185                 190

Glu Asn Val Gln Pro Leu Ala Glu Lys His Gly Val Ala Val Phe Pro
        195                 200                 205

Tyr Phe Ala Leu Ala Ala Gly Leu Leu Thr Gly Lys Tyr Thr Ser Lys
    210                 215                 220

Glu Asp Ile Ser Gly Lys Ala Arg Ala Gly Gln Leu Asp Arg Tyr Ala
225                 230                 235                 240

Ser Asp Glu Ala Phe Ala Val Val Thr Glu Leu Arg Ala Val Ala Asp
                245                 250                 255

Glu Leu Gly Val Ala Pro Thr Thr Val Ala Leu Ala Trp Leu Val Ala
            260                 265                 270

His Gly Val Thr Ala Pro Ile Ala Ser Val Ser Lys Val Glu Gln Leu
        275                 280                 285

Lys Asp Leu Met Ala Val Lys Asp Val Glu Leu Ser Ala Glu Gln Leu
    290                 295                 300

Ala Arg Leu Asp Lys Val Ser Glu Pro Phe Ala
305                 310                 315

<210>  80
<211>  948
<212>  DNA
<213>  Corynebacterium glutamicum

<400>  80
atggagcacg gcgtgaccgt tattaaaggc actgaatttg atgttttccc actaaacctc      60

ggtggaaata cctttggctg gacctcgaat agggaacaga ccttcgcggt tttggatgca     120

ttcgtggcag cgggaggaaa ctttgttgac accgccgatt cttattctgc atgggttgaa     180

ggcaatgagg gtggcgagtc ggagcgggag ctcggcgcgt ggattaagga acgtggcgca     240

gacaagctga tcattgctac caagtctggt gcgttggagc ctgttgctgg tcgttcccgt     300

gaggcaactt tcaaggctgt cgagggttcc ctggagcgtt tgggcgtgga atcgatcgat     360

attttttact accactacga cgatgaggca gtcagcattg atgagcaggt tgctatcgct     420

aatgatctga ttgcacaggg caagattaag cacctcgcat tgtctaacta cagcgcggag     480

cgtttagctg agttctttga gaagtctgta ggcactccag cgcagccggt tgctctgcaa     540

ccgcactaca acctggtgtc gaggaaggat tatgaggaga acgtgcagcc actcgccgag     600

aagcatggcg ttgcagtctt cccttatttc gcgcttgccg cgggtctttt gaccggaaag     660

tacacctcca aggaggatat ttcgggtaaa gcgcgtgcgg ggcagttgga tcgttacgcc     720

agcgatgagg cgtttgccgt ggtgacagag ttgcgtgctg ttgccgatga gttgggtgtt 780

gcgccaacga ctgtggcgct tgcgtggttg gttgcgcatg gtgtgaccgc accgattgcg 840

tccgtgtcca aggtagagca gttgaaggat ttgatggctg tgaaggatgt ggagctgagc 900

gctgagcagc ttgcacgttt ggataaggtt tcggagcctt tcgcttaa 948

<210> 81
<211> 363
<212> PRT
<213> Corynebacterium glutamicum

<400> 81

Met Asn Asn Ser Leu Ala Phe Asn His Asp Thr Leu Pro Gln Lys Val
1               5                   10                  15

Met Phe Gly Tyr Gly Lys Ser Ser Ala Phe Leu Lys Gln Glu Val Glu
            20                  25                  30

Arg Arg Gly Ser Ala Lys Val Met Val Ile Ala Gly Glu Arg Glu Met
        35                  40                  45

Ser Ile Ala His Lys Val Ala Ser Glu Ile Glu Val Ala Ile Trp His
    50                  55                  60

Asp Glu Val Val Met His Val Pro Ile Glu Val Ala Glu Arg Ala Arg
65                  70                  75                  80

Ala Val Ala Thr Asp Asn Glu Ile Asp Leu Leu Val Cys Val Gly Gly
                85                  90                  95

Gly Ser Thr Ile Gly Leu Ala Lys Ala Ile Ala Met Thr Thr Ala Leu
            100                 105                 110

Pro Ile Val Ala Ile Pro Thr Thr Tyr Ala Gly Ser Glu Ala Thr Asn
        115                 120                 125

Val Trp Gly Leu Thr Glu Ala Ala Arg Lys Thr Thr Gly Val Asp Leu
    130                 135                 140

Lys Val Leu Pro Glu Thr Val Ile Tyr Asp Ser Glu Leu Thr Met Ser
145                 150                 155                 160

Leu Pro Val Glu Met Ser Val Ala Ser Gly Leu Asn Gly Leu Ala His
                165                 170                 175

Cys Ile Asp Ser Leu Trp Gly Pro Asn Ala Asp Pro Ile Asn Ala Val
            180                 185                 190

Leu Ala Ala Glu Gly Ile Arg Ala Leu Asn Gln Gly Leu Pro Lys Ile
        195                 200                 205

Val Ala Asn Pro His Ser Ile Glu Gly Arg Asp Glu Ala Leu Tyr Gly
    210                 215                 220

Ala Tyr Leu Ala Ala Val Ser Phe Ala Ser Ala Gly Ser Gly Leu His
225                 230                 235                 240

His Lys Ile Cys His Thr Leu Gly Gly Thr Phe Asn Leu Pro His Ala
                245                 250                 255

Gln Thr His Ala Thr Val Leu Pro Tyr Val Leu Ala Phe Asn Ala Gly
            260                 265                 270

Asp Ala Pro Glu Ala Glu Arg Arg Ala Ala Ala Ala Phe Gly Thr Asp
        275                 280                 285

Thr Ala Leu Glu Gly Leu Gln Arg Leu Arg Leu Ser Val Asn Ala Pro
    290                 295                 300

Lys Arg Leu Ser Asp Tyr Gly Phe Glu Ala Ser Gly Ile Ala Glu Ala
305                 310                 315                 320

Val Asp Val Thr Leu Glu Lys Val Pro Ala Asn Asn Pro Arg Pro Val
                325                 330                 335

Thr Arg Glu Asn Leu Ser Arg Leu Leu Glu Ala Ala Leu Asn Gly Glu
            340                 345                 350

Asp Pro Ala Val Leu Ser Ala Val Leu Ser Asn
        355                 360

<210>  82
<211>  1092
<212>  DNA
<213>  Corynebacterium glutamicum

<400>  82
gtgaacaact cactcgcatt caaccacgac accctcccac agaaagtcat gtttggatat      60

ggcaagtcca gtgcattctt aaagcaggaa gttgaacgcc gcggctcagc caaggtcatg     120

gtcattgcgg gtgaacgaga aatgtcgatc gcccataagg tggcctcaga aattgaggtg     180

gcgatctggc acgacgaagt tgtcatgcac gtgcccatcg aagtagccga acgtgcgcgt     240

gcagtggcaa ccgacaatga gattgatctg ctggtgtgtg ttggcggcgg atccaccata     300

ggtttggcca aagcaattgc catgaccact gccctgccca tcgtcgcgat ccccaccacc     360

tacgcaggat cggaagcaac caacgtgtgg ggtctgacgg aagcagcgcg caaaacaacc 420

ggtgttgatc tgaaggtgct ccccgaaaca gtcatttacg attccgaact caccatgtcg 480

cttccagtgg agatgtccgt ggcatccgga ctcaacggcc tggcgcactg cattgattct 540

ttgtggggac ccaacgccga tcccatcaac gcagtgcttg cagccgaagg aatccgcgca 600

ctcaaccagg gactgccgaa aattgttgcg aacccgcaca gcatcgaagg acgcgacgaa 660

gccctctacg gcgcctacct cgcagcagta tccttcgcct ccgcaggctc cggactacac 720

cacaaaatct gccacacctt gggaggcacc ttcaacctcc cccacgccca aacccacgca 780

accgtgctgc cgtatgtttt ggcattcaac gcaggcgacg caccagaagc tgaacgccgc 840

gcagccgcag cctttggaac tgacaccgca ctagaaggcc tgcaacgcct ccgcttgtca 900

gtcaacgcac cgaaacgact ttccgactac ggcttcgagg cttcaggaat tgctgaggca 960

gtggacgtca cgttggagaa agttcccgcc aacaatcctc gcccagtgac ccgggaaaac 1020

ctcagcagat tgctcgaagc agcactcaac ggtgaggatc cggcagttct tagcgcagta 1080

ctcagtaact aa 1092

<210> 83
<211> 316
<212> PRT
<213> Corynebacterium glutamicum

<400> 83

Met Lys Gln Arg Met Val Gly Ser Ser Gly Leu Arg Val Ser Arg Leu
1               5                   10                  15

Gly Leu Gly Thr Ser Thr Trp Gly Ser Gly Thr Glu Leu Ala Glu Ala
            20                  25                  30

Gly Asp Ile Phe Lys Ala Phe Ile Asn Ser Gly Gly Thr Leu Ile Asp
        35                  40                  45

Val Ser Pro Asn Tyr Thr Thr Gly Val Ala Glu Glu Met Leu Gly Thr
    50                  55                  60

Met Leu Asp Ala Glu Val Ser Arg Ser Ala Val Val Ile Ser Ser Ser
65                  70                  75                  80

Ala Gly Val Asn Pro Ala Leu Pro Leu Gly Arg Arg Val Asp Cys Ser
                85                  90                  95

Arg Arg Asn Leu Ile Ala Gln Leu Asp Val Thr Leu Arg Ala Leu Asn
            100                 105                 110

Thr Asp Tyr Leu Asp Leu Trp Ser Val Gly Tyr Trp Asp Glu Gly Thr
        115                 120                 125

Pro Pro His Glu Val Ala Asp Thr Leu Asp Tyr Ala Val Arg Thr Gly
    130                 135                 140

Arg Val Arg Tyr Ala Gly Val Arg Gly Tyr Ser Gly Trp Gln Leu Ala
145                 150                 155                 160

Val Thr His Ala Ala Ser Asn His Ala Ala Ala Ser Ala Arg Pro Val
                165                 170                 175

Val Val Ala Gln Asn Glu Tyr Ser Leu Leu Glu Arg Arg Ala Glu Gln
            180                 185                 190

Glu Leu Leu Pro Ala Thr Gln His Leu Gly Val Gly Phe Phe Ala Gly
        195                 200                 205

Ala Pro Leu Gly Gln Gly Val Leu Thr Ala Lys Tyr Arg Ser Glu Ile
    210                 215                 220

Pro His Asp Ser Arg Ala Ala Ser Thr Gly Arg Asp Ala Glu Val Gln
225                 230                 235                 240

Ser Tyr Leu Asp Asn Arg Gly Arg Ile Ile Val Asp Ala Leu Asp Thr
                245                 250                 255

Ala Ala Lys Gly Leu Gly Ile Ser Pro Ala Val Thr Ala Thr Thr Trp
            260                 265                 270

Val Arg Asp Arg Pro Gly Val Thr Ala Val Ile Val Gly Ala Arg Thr
        275                 280                 285

His Glu Gln Leu Ser His Leu Leu Lys Ala Glu Ser Val Thr Leu Pro
    290                 295                 300

Thr Pro Ile Thr Gln Ala Leu Asp Asp Val Ser Leu
305                 310                 315

<210>  84
<211>  951
<212>  DNA
<213>  Corynebacterium glutamicum

<400>  84
gtgaaacagc gaatggtcgg ttcaagtggt ttgcgggtat ccaggctcgg tttgggcacc      60

tcaacatggg gctcgggcac cgagctggct gaggcaggcg atatctttaa ggcgttcatc     120

aattctggtg gcacgcttat cgacgtctcc cccaactaca ccaccggcgt cgcggaagaa     180

atgctcggca cgatgttgga tgcggaagtc tctcgttcgg ctgtcgtcat ttcctccagc 240

gcaggtgtca accccgctct gccgctcggc cgacgtgtgg attgctcccg ccgcaatttg 300

attgcccaat tagatgtcac cctgcgggca ttaaacactg actatttgga tttgtggtct 360

gtgggctatt gggatgaggg caccccaccg catgaggtgg ccgatacttt ggattacgcc 420

gtgcgcaccg gccgagtccg atatgccggt gtccgaggat attccggttg gcagttagcg 480

gtcacccacg ctgcatccaa tcatgcagcg gcctccgccc gccccgtggt cgttgcacaa 540

aatgaataca gcctgctgga acgccgcgca gaacaagaac tcctccctgc cacccaacac 600

ctaggtgtcg gattctttgc tggcgctccg ctggggcaag gcgtgctgac tgctaaatac 660

cgctccgaaa ttccccatga ttccagagct gcatccacag gacgcgacgc agaagtccaa 720

agctacctag ataatcgagg ccgcatcatt gtcgatgctc ttgatactgc agccaaagga 780

ttaggcatta gccccgctgt cacagccacc acctgggtgc gtgatcgtcc cggagtgaca 840

gctgtcatcg tgggcgctcg cacacatgaa cagctgtcac atcttctcaa ggcggaatcg 900

gtgactttgc caacaccaat cacacaagcc cttgatgatg tctccctgtg a 951

<210> 85
<211> 329
<212> PRT
<213> Bacillus subtilis

<400> 85

Met Lys Ala Val Ile His Asn Gly Lys Ala Gly Leu Leu Gly Leu Ser
1               5                   10                  15

Val Gln Asp Val Pro Ser Thr Lys Pro Gly Tyr Gly Glu Val Lys Val
            20                  25                  30

Lys Leu Lys Ser Ala Gly Leu Asn His Arg Asp Leu Phe Leu Met Lys
        35                  40                  45

Asn Lys Ser Glu Gln Asp Pro His Met Ile Leu Gly Ser Asp Gly Ala
    50                  55                  60

Gly Ile Ile Glu Glu Ile Gly Glu Gly Val Lys Asn Val Thr Val Gln
65                  70                  75                  80

Thr Glu Val Val Ile Phe Pro Thr Leu Asn Trp Asp Leu Thr Glu Asn
                85                  90                  95

Val Pro Pro Val Pro Glu Ile Leu Gly Gly Pro Ser Asp Gly Thr Leu
            100                 105                 110

Ala Glu Tyr Val Ile Ile Pro Ser Gln Asn Ala Ile Lys Lys Pro Ala
        115                 120                 125

Tyr Leu Ser Trp Glu Glu Ala Gly Val Leu Pro Leu Ser Ala Leu Thr
    130                 135                 140

Ala Tyr Arg Ala Leu Phe Thr Lys Gly Gln Leu Lys Lys Gly Glu His
145                 150                 155                 160

Leu Leu Ile Pro Gly Ile Gly Ser Gly Val Ala Thr Tyr Ala Leu Phe
                165                 170                 175

Met Ala Lys Ala Ile Gly Ala Thr Val Ser Val Thr Ser Arg Ser Glu
            180                 185                 190

Glu Lys Arg Lys Lys Ala Leu Lys Leu Gly Ala Asp Tyr Ala Phe Asp
        195                 200                 205

Ser Tyr Ser Asn Trp Asp Glu Gln Leu Gln Gly Lys Lys Ile Asp Val
    210                 215                 220

Val Leu Asp Ser Ile Gly Pro Ala Leu Phe Ser Glu Tyr Phe Arg His
225                 230                 235                 240

Val Lys Pro Asn Gly Arg Ile Val Ser Phe Gly Ala Ser Ser Gly Asp
                245                 250                 255

Asn Leu Ser Phe Pro Val Arg Ser Leu Phe Phe Pro Gln Val Asn Val
            260                 265                 270

Leu Gly Thr Ser Met Gly Ser Gly Glu Glu Phe Gln Ala Met Leu Ala
        275                 280                 285

Phe Ile Asp Lys His Lys Leu Arg Pro Val Ile Asp Arg Ile Tyr Pro
    290                 295                 300

Leu Glu Lys Ala Cys Glu Ala Tyr Lys Arg Met Gln Glu Gly Arg Gln
305                 310                 315                 320

Phe Gly Asn Ile Gly Ile Val Met Glu
                325

<210>  86
<211>  990
<212>  DNA
<213>  Bacillus subtilis

<400>  86
atgaaagctg taattcacaa cggaaaagcc ggtcttctgg ggttatcagt tcaggacgtt        60

ccatcaacaa agcctggata cggagaggta aaggttaaat taaaatctgc aggcctgaat 120

catcgtgact tgtttcttat gaaaaacaaa tctgaacaag atcctcacat gatactgggt 180

tctgacggcg cgggtatcat cgaagagatt ggtgaaggcg tgaaaaatgt tactgttcag 240

acagaagtag tcattttccc gacattgaac tgggatttga cagaaaatgt tccacctgta 300

cctgagattc tgggaggtcc ttcggacgga acacttgctg aatatgtgat cattccttca 360

caaaatgcaa tcaaaaaacc tgcttattta tcttgggaag aagcgggcgt tttaccttta 420

tccgctttaa ctgcatatcg ggctctgttt acaaaggggc aattaaaaaa aggcgagcat 480

ctattgatac ccggcatcgg cagcggtgta gcaacctacg ctttatttat ggctaaggcg 540

attggggcaa cagtaagcgt gacctcccgc agtgaggaga aaagaaaaaa ggcgctgaaa 600

ttaggtgctg attacgcatt tgacagctac agcaattggg atgagcagtt gcagggaaaa 660

aagatagatg ttgttcttga cagcatagga cctgccctct tttcggaata cttccgccat 720

gtaaaaccaa atggccgtat tgtcagcttt ggggcaagct caggggataa tctcagtttt 780

ccggtgcgtt ctttattctt tcctcaggtc aatgttttgg gaacctcgat gggaagcggt 840

gaggaatttc aagctatgct cgctttcatt gacaaacata agctgcggcc tgtaattgac 900

cggatatatc ctttagaaaa agcatgtgaa gcatataaaa gaatgcagga aggcagacag 960

tttggaaaca tcgggatcgt aatggaataa 990

<210> 87
<211> 390
<212> PRT
<213> Bacillus subtilis

<400> 87

Met Glu Asn Phe Thr Tyr Tyr Asn Pro Thr Lys Leu Ile Phe Gly Lys
1               5                   10                  15

Gly Gln Leu Glu Gln Leu Arg Lys Glu Phe Lys Arg Tyr Gly Lys Asn
            20                  25                  30

Val Leu Leu Val Tyr Gly Gly Gly Ser Ile Lys Arg Asn Gly Leu Tyr
        35                  40                  45

Asp Gln Val Thr Gly Ile Leu Lys Glu Glu Gly Ala Val Val His Glu
    50                  55                  60

Leu Ser Gly Val Glu Pro Asn Pro Arg Leu Ala Thr Val Glu Lys Gly
65                  70                  75                  80

Ile Gly Leu Cys Arg Glu His Asp Ile Asp Phe Leu Leu Ala Val Gly
                85                  90                  95

Gly Gly Ser Val Ile Asp Cys Thr Lys Ala Ile Ala Ala Gly Val Lys
        100                 105                 110

Tyr Asp Gly Asp Ala Trp Asp Ile Phe Ser Lys Lys Val Thr Ala Glu
    115                 120                 125

Asp Ala Leu Pro Phe Gly Thr Val Leu Thr Leu Ala Ala Thr Gly Ser
    130                 135                 140

Glu Met Asn Pro Asp Ser Val Ile Thr Asn Trp Glu Thr Asn Glu Lys
145                 150                 155                 160

Phe Val Trp Gly Ser Asn Val Thr His Pro Arg Phe Ser Ile Leu Asp
                165                 170                 175

Pro Glu Asn Thr Phe Thr Val Pro Glu Asn Gln Thr Val Tyr Gly Met
        180                 185                 190

Val Asp Met Met Ser His Val Phe Glu Gln Tyr Phe His Asn Val Glu
    195                 200                 205

Asn Thr Pro Leu Gln Asp Arg Met Cys Phe Ala Val Leu Gln Thr Val
    210                 215                 220

Ile Glu Thr Ala Pro Lys Leu Leu Glu Asp Leu Glu Asn Tyr Glu Leu
225                 230                 235                 240

Arg Glu Thr Ile Leu Tyr Ala Gly Thr Ile Ala Leu Asn Gly Thr Leu
                245                 250                 255

Gln Met Gly Tyr Phe Gly Asp Trp Ala Ser His Thr Met Glu His Ala
        260                 265                 270

Val Ser Ala Val Tyr Asp Ile Pro His Ala Gly Gly Leu Ala Ile Leu
    275                 280                 285

Phe Pro Asn Trp Met Arg Tyr Thr Leu Asp Thr Asn Val Gly Arg Phe
    290                 295                 300

Lys Asn Leu Met Leu Asn Met Phe Asp Ile Asp Thr Glu Gly Lys Thr
305                 310                 315                 320

Asp Lys Glu Ile Ala Leu Glu Gly Ile Asp Lys Leu Ser Ala Phe Trp
                325                 330                 335

Thr Ser Leu Gly Ala Pro Ser Arg Leu Ala Asp Tyr Asn Ile Gly Glu

340                 345                 350

Glu Lys Leu Glu Leu Ile Ala Asp Ile Ala Ala Lys Glu Met Glu His
        355                 360                 365

Gly Gly Phe Gly Asn Phe Gln Lys Leu Asn Lys Asp Asp Val Leu Ala
    370                 375                 380

Ile Leu Arg Ala Ser Leu
385                 390

<210>  88
<211>  1173
<212>  DNA
<213>  Bacillus subtilis

<400>  88
atggaaaatt tcacttatta taatccgaca aagctgattt ttggaaaagg tcagcttgaa      60

caattaagaa aagaattcaa acgatacggc aagaatgtac tgcttgttta cgggggcggc     120

agcattaaac gcaacggcct ttatgatcaa gtcacaggca ttttaaaaga agagggcgct     180

gttgttcatg agctgtcagg tgtagagcca aacccgcgtc ttgcgacagt ggaaaaaggc     240

ataggacttt gcagagagca tgacattgat tttctgcttg ctgtcggcgg aggcagtgtg     300

attgactgta caaaggcaat cgcagctggc gtcaaatatg acggtgacgc ttgggatatt     360

ttcagcaaaa aagtaacagc ggaggatgcg ctgccgtttg gcactgtttt aactcttgct     420

gcaacagggt ctgaaatgaa ccctgattcc gtgattacaa actgggaaac aaacgagaaa     480

tttgtatggg gcagcaatgt cactcatccg cgtttctcta ttttagaccc tgaaaatacg     540

ttcaccgttc cagaaaatca aacagtatac ggcatggttg acatgatgag ccacgtattc     600

gaacaatact tccataatgt tgaaaacacg ccgcttcagg atagaatgtg ctttgctgtt     660

ttgcagacgg tcatcgaaac agctcctaag cttcttgaag atctggaaaa ctacgaactt     720

cgtgaaacga ttttgtatgc tggtactatt gctttaaacg gcacgctcca aatgggatac     780

ttcggtgact gggcttctca tacaatggaa cacgctgttt cagctgtata tgatattccg     840

cacgcgggcg gtttggcaat actgttccca aattggatga gatacacgct tgatacaaat     900

gtaggccgtt ttaaaaacct tatgctcaac atgtttgaca ttgatactga aggcaaaaca     960

gataaagaaa ttgcgcttga aggaatcgat aaactgtctg cgttctggac aagcctcggt    1020

gcaccttctc gtcttgctga ttacaatatt ggagaagaaa agcttgagct gattgctgat    1080

atcgcagcca aggaaatgga acacggcggc ttcggcaact tccaaaaact gaacaaagat    1140

gacgtgcttg ccatccttcg cgcgtctcta taa                                 1173

<210>  89

<211>  387
<212>  PRT
<213>  Bacillus subtilis

<400>  89

Met Gln Asn Phe Thr Tyr Trp Asn Pro Thr Lys Leu Ile Phe Gly Arg
1               5                   10                  15

Gly Glu Val Glu Arg Leu Pro Glu Glu Leu Lys Pro Tyr Gly Lys Asn
            20                  25                  30

Val Leu Leu Val Tyr Gly Gly Gly Ser Ile Lys Arg Ser Gly Leu Tyr
        35                  40                  45

Asp Gln Val Ile Glu Gln Leu Asn Lys Ala Gly Ala Thr Val His Glu
    50                  55                  60

Leu Ala Gly Val Glu Pro Asn Pro Arg Val Ser Thr Val Asn Lys Gly
65                  70                  75                  80

Val Ala Ile Cys Lys Glu Gln Asn Ile Asp Phe Leu Leu Ala Val Gly
                85                  90                  95

Gly Gly Ser Val Ile Asp Cys Thr Lys Ala Ile Ala Ala Gly Ala Lys
            100                 105                 110

Tyr Asp Gly Asp Ala Trp Asp Ile Val Thr Lys Lys His Gln Pro Lys
        115                 120                 125

Asp Ala Leu Pro Phe Gly Thr Val Leu Thr Leu Ala Ala Thr Gly Ser
    130                 135                 140

Glu Met Asn Ser Gly Ser Val Ile Thr Asn Trp Glu Thr Lys Glu Lys
145                 150                 155                 160

Tyr Gly Trp Gly Ser Pro Leu Val Phe Pro Lys Phe Ser Ile Leu Asp
                165                 170                 175

Pro Val Asn Thr Phe Thr Val Pro Lys Asn His Thr Ile Tyr Gly Met
            180                 185                 190

Val Asp Met Met Ser His Val Phe Glu Gln Tyr Phe His His Val Ser
        195                 200                 205

Asn Thr Pro Tyr Gln Asp Arg Met Cys Glu Ser Leu Leu Arg Thr Val
    210                 215                 220

Ile Glu Thr Ala Pro Lys Leu Ile Asn Asp Leu Glu Asn Tyr Glu Leu

225                 230                 235                 240

Arg Glu Thr Ile Leu Tyr Thr Gly Thr Ile Ala Leu Asn Gly Met Leu
                245                 250                 255

Ser Met Gly Ala Arg Gly Asp Trp Ala Thr His Asn Ile Glu His Ala
            260                 265                 270

Val Ser Ala Val Tyr Asp Ile Pro His Ala Gly Gly Leu Ala Ile Leu
        275                 280                 285

Phe Pro Asn Trp Met Arg His Thr Leu Ser Glu Asn Pro Ala Arg Met
    290                 295                 300

Lys Gln Leu Ala Val Arg Val Phe Asp Val Glu Glu Ala Gly Lys Thr
305                 310                 315                 320

Asp Glu Glu Ile Ala Leu Glu Gly Ile Asp Lys Leu Ser Ala Phe Trp
                325                 330                 335

Thr Ser Leu Gly Ala Pro Asn Arg Leu Ala Asp Tyr Asp Ile Asn Asp
            340                 345                 350

Glu Gln Leu Asp Thr Ile Ala Asp Lys Ala Met Ala Asn Gly Thr Phe
        355                 360                 365

Gly Gln Phe Lys Ser Leu Asn Lys Glu Asp Val Leu Ser Ile Leu Lys
    370                 375                 380

Ala Ser Leu
385

<210>  90
<211>  1164
<212>  DNA
<213>  Bacillus subtilis

<400>  90
atgcaaaact ttacatactg gaatccgacc aaattaattt tcgggcgcgg cgaagtggaa      60

agacttccgg aggaactcaa accttacgga aaaaacgtat tgcttgtgta cggaggcggc     120

agcattaaac gcagcggcct gtatgatcaa gtgattgaac agctgaataa agccggagcg     180

accgtgcatg aattagcagg tgtggaaccg aatcctcgtg tgtcgactgt taataaaggt     240

gttgccatct gtaaagaaca aaacattgat ttcttgctgg ctgtcggagg cggaagcgta     300

atcgactgta caaaagcgat tgccgcagga gcgaagtatg atggtgatgc gtgggatatc     360

gttacgaaaa agcatcagcc aaaagatgct ttgccattcg gaacggtatt gactctcgct     420

gcaactggtt cagaaatgaa ctcaggatct gttattacaa actgggaaac aaaagaaaaa 480

tacggctggg gcagcccgct cgtattccct aaattctcga ttcttgatcc ggtgaataca 540

ttcaccgtac ctaaaaacca cacgatctac gggatggttg acatgatgag ccacgtattc 600

gaacaatact tccatcatgt atcaaacacg ccgtatcagg accgcatgtg tgaatcactt 660

ttgcgtacag tcattgaaac agcgcctaag ctgatcaatg atctcgaaaa ttacgaattg 720

cgtgagacga tcctgtacac aggaacaatc gcattaaacg gcatgctttc tatgggggca 780

agaggggatt gggctactca taatattgaa catgcagtat cagccgttta tgatattccg 840

catgccggcg gactggcgat tctgttcccg aattggatga gacacacatt gtctgaaaac 900

cctgcccgca tgaaacagct tgcagttcgc gtgtttgatg ttgaagaagc aggtaaaacg 960

gatgaagaaa ttgcccttga aggtatcgat aagctgtccg cattctggac aagtcttggc 1020

gctccgaacc gtcttgctga ttatgatatt aatgatgagc agcttgacac aatcgctgac 1080

aaggcaatgg ctaacggtac attcggccaa tttaaatcac tcaacaaaga agatgtgctg 1140

tcaattttga aagcatcact ataa 1164

<210> 91
<211> 331
<212> PRT
<213> Bacillus subtilis

<400> 91

Met Glu Tyr Thr Ser Ile Ala Asp Thr Gly Ile Glu Ala Ser Arg Ile
1               5                   10                  15

Gly Leu Gly Thr Trp Ala Ile Gly Gly Thr Met Trp Gly Gly Thr Asp
            20                  25                  30

Glu Lys Thr Ser Ile Glu Thr Ile Arg Ala Ala Leu Asp Gln Gly Ile
        35                  40                  45

Thr Leu Ile Asp Thr Ala Pro Ala Tyr Gly Phe Gly Gln Ser Glu Glu
    50                  55                  60

Ile Val Gly Lys Ala Ile Lys Glu Tyr Gly Lys Arg Asp Gln Val Ile
65                  70                  75                  80

Leu Ala Thr Lys Thr Ala Leu Asp Trp Lys Asn Asn Gln Leu Phe Arg
                85                  90                  95

His Ala Asn Arg Ala Arg Ile Val Glu Glu Val Glu Asn Ser Leu Lys
            100                 105                 110

Arg Leu Gln Thr Asp Tyr Ile Asp Leu Tyr Gln Val His Trp Pro Asp

115                 120                 125

Pro Leu Val Pro Ile Glu Glu Thr Ala Glu Val Met Lys Glu Leu Tyr
    130                 135                 140

Asp Ala Gly Lys Ile Arg Ala Ile Gly Val Ser Asn Phe Ser Ile Glu
145                 150                 155                 160

Gln Met Asp Thr Phe Arg Ala Val Ala Pro Leu His Thr Ile Gln Pro
                165                 170                 175

Pro Tyr Asn Leu Phe Glu Arg Glu Met Glu Glu Ser Val Leu Pro Tyr
            180                 185                 190

Ala Lys Asp Asn Lys Ile Thr Thr Leu Leu Tyr Gly Ser Leu Cys Arg
        195                 200                 205

Gly Leu Leu Thr Gly Lys Met Thr Glu Glu Tyr Thr Phe Glu Gly Asp
    210                 215                 220

Asp Leu Arg Asn His Asp Pro Lys Phe Gln Lys Pro Arg Phe Lys Glu
225                 230                 235                 240

Tyr Leu Ser Ala Val Asn Gln Leu Asp Lys Leu Ala Lys Thr Arg Tyr
                245                 250                 255

Gly Lys Ser Val Ile His Leu Ala Val Arg Trp Ile Leu Asp Gln Pro
            260                 265                 270

Gly Ala Asp Ile Ala Leu Trp Gly Ala Arg Lys Pro Gly Gln Leu Glu
        275                 280                 285

Ala Leu Ser Glu Ile Thr Gly Trp Thr Leu Asn Ser Glu Asp Gln Lys
    290                 295                 300

Asp Ile Asn Thr Ile Leu Glu Asn Thr Ile Ser Asp Pro Val Gly Pro
305                 310                 315                 320

Glu Phe Met Ala Pro Pro Thr Arg Glu Glu Ile
                325                 330

<210>  92
<211>  996
<212>  DNA
<213>  Bacillus subtilis

<400>  92
atggaatata ccagtatagc agatacagga atagaagcct ccagaatcgg cctcggcaca        60

tgggccattg gcggaacgat gtggggaggc actgacgaaa aaacatcgat tgaaacaatc 120

cgcgccgctc ttgatcaggg gattacactg attgacaccg caccggctta cggcttcggg 180

cagtccgagg aaattgtcgg aaaggcaatc aaagagtacg gcaaaagaga ccaggtgatt 240

ctcgcaacga aaacggctct ggactggaag aacaaccagc tgttccgcca tgcgaacaga 300

gcgagaattg tagaggaagt tgagaattct ttgaagcggc ttcaaacaga ctatattgat 360

ctttatcagg tgcattggcc cgatccgctt gtgccaattg aagaaacggc tgaagtcatg 420

aaggaattat atgatgcggg aaaaatccgg gcgattggcg tcagcaattt ttcaattgag 480

caaatggata catttcgcgc cgtcgcacct ctccatacga ttcagcctcc atataatctg 540

tttgaaagag agatggaaga gagtgtcctt ccttatgcga aagataacaa gataacaaca 600

ttattatacg gcagtttatg cagagggctg ttaacaggca aaatgactga agaatataca 660

tttgagggcg atgatctgcg taatcacgat ccaaaattcc agaagccccg ctttaaagag 720

tatctttctg ctgtgaatca attggataag ctggcgaaga cacgttatgg aaaatcagtg 780

attcacttgg ctgtcagatg gatcttagat cagccgggag cggatatcgc tctttgggga 840

gcaagaaagc ctgggcagct tgaggcccta tctgagatta caggctggac gctgaacagt 900

gaagatcaga aagatatcaa tactatattg gaaaatacga tatcagaccc tgtcggaccg 960

gagtttatgg ccccgccgac cagagaggaa atataa 996

<210> 93
<211> 306
<212> PRT
<213> Bacillus subtilis

<400> 93

Met Arg Lys Arg Lys Leu Gly Thr Ser Asp Leu Asp Ile Ser Glu Val
1               5                   10                  15

Gly Leu Gly Cys Met Ser Leu Gly Thr Glu Lys Asn Lys Ala Leu Ser
            20                  25                  30

Ile Leu Asp Glu Ala Ile Glu Leu Gly Ile Asn Tyr Leu Asp Thr Ala
        35                  40                  45

Asp Leu Tyr Asp Arg Gly Arg Asn Glu Glu Ile Val Gly Asp Ala Ile
    50                  55                  60

Gln Asn Arg Arg His Asp Ile Ile Leu Ala Thr Lys Ala Gly Asn Arg
65                  70                  75                  80

Trp Asp Asp Gly Ser Glu Gly Trp Tyr Trp Asp Pro Ser Lys Ala Tyr
                85                  90                  95

Ile Lys Glu Ala Val Lys Lys Ser Leu Thr Arg Leu Lys Thr Asp Tyr
        100                 105                 110

Ile Asp Leu Tyr Gln Leu His Gly Gly Thr Ile Glu Asp Asn Ile Asp
    115                 120                 125

Glu Thr Ile Glu Ala Phe Glu Glu Leu Lys Gln Glu Gly Val Ile Arg
    130                 135                 140

Tyr Tyr Gly Ile Ser Ser Ile Arg Pro Asn Val Ile Lys Glu Tyr Val
145                 150                 155                 160

Lys Lys Ser Asn Ile Val Ser Ile Met Met Gln Phe Ser Leu Phe Asp
                165                 170                 175

Arg Arg Pro Glu Glu Trp Leu Pro Leu Leu Glu Glu His Gln Ile Ser
            180                 185                 190

Val Val Ala Arg Gly Pro Val Ala Lys Gly Leu Leu Thr Glu Lys Pro
        195                 200                 205

Leu Asp Gln Ala Ser Glu Ser Met Lys Gln Asn Gly Tyr Leu Ser Tyr
    210                 215                 220

Ser Phe Glu Glu Leu Thr Asn Ala Arg Lys Ala Met Glu Glu Val Ala
225                 230                 235                 240

Pro Asp Leu Ser Met Thr Glu Lys Ser Leu Gln Tyr Leu Leu Ala Gln
                245                 250                 255

Pro Ala Val Ala Ser Val Ile Thr Gly Ala Ser Lys Ile Glu Gln Leu
            260                 265                 270

Arg Glu Asn Ile Gln Ala Ala Asn Ala Arg Arg Leu Thr Glu Glu Glu
        275                 280                 285

Ile Lys Ala Leu Gln Ser His Thr Lys Gln Asp Ile Tyr Lys Ala His
    290                 295                 300

Arg Ser
305

<210>  94
<211>  921
<212>  DNA
<213>  Bacillus subtilis

<400>  94

atgagaaagc gcaaattggg tacatctgat ttagacatta gcgaagtcgg actcggctgt 60

atgtctcttg gaactgaaaa aaacaaagca ttgtccattc tggatgaagc gatcgagctt 120

ggcatcaact atttggacac agcggatttg tatgaccggg gacgcaatga agaaattgtc 180

ggtgatgcga tccaaaacag acgccatgat attattctgg caacaaaagc gggaaaccgt 240

tgggatgacg gaagcgaggg ctggtattgg gacccttcaa aagcttacat aaaagaggcg 300

gtaaaaaaga gccttacacg tctgaaaaca gattatatcg acctttatca gctccacggc 360

ggcacgatag aggacaacat tgatgaaacg attgaagcgt ttgaggaatt aaaacaagaa 420

ggtgtcatcc gctactacgg catttcttcc atccgcccga atgtgattaa agaatatgta 480

aaaaaatcaa acatcgtcag cattatgatg caattcagcc tgtttgacag acgccctgag 540

gaatggctcc cgcttttaga ggaacatcaa atcagcgtag tcgccagagg tcctgttgcc 600

aaagggctct taactgaaaa accgcttgat caagcttcag aaagtatgaa acaaaacggg 660

tacttgtcct attcattcga ggaactgaca aatgcccgta aggcaatgga ggaagtggct 720

cccgatcttt ccatgacaga aaagtcgctg cagtatctgc tagcacagcc ggctgtcgct 780

tcagtgatta caggcgccag taagattgag cagttacggg aaaatattca ggcagcaaat 840

gcacggcgtt taaccgaaga ggaaatcaaa gcgctccaat ctcatacgaa acaagacatt 900

tacaaagctc atcgctcata g 921

<210> 95
<211> 310
<212> PRT
<213> Bacillus subtilis

<400> 95

Met Asp Gln Thr Arg Thr Leu Gly Lys Thr Lys Leu Lys Val Lys Arg
1               5                   10                  15

Ile Gly Phe Gly Ala Asn Ala Val Gly Gly His Asn Leu Phe Pro Asn
            20                  25                  30

Leu Asn Asp Glu Thr Gly Lys Asp Leu Val Arg Thr Ala Leu Asp Gly
        35                  40                  45

Gly Val Asn Phe Ile Asp Thr Ala Phe Ile Tyr Gly Leu Gly Arg Ser
    50                  55                  60

Glu Glu Leu Ile Gly Glu Val Val Gln Glu Arg Gly Val Arg Asn Glu
65                  70                  75                  80

Leu Ile Ile Ala Thr Lys Gly Ala His Lys Glu Val Asp Gly Ser Ile
                85                  90                  95

Glu Leu Asp Asn Ser Arg Glu Phe Leu Arg Ser Glu Val Glu Lys Ser
                100                 105                 110

Leu Lys Arg Leu Lys Thr Asp Tyr Ile Asp Leu Tyr Tyr Val His Phe
            115                 120                 125

Pro Asp Gly Lys Thr Pro Leu Ala Glu Val Ala Gly Thr Leu Lys Glu
    130                 135                 140

Leu Lys Asp Glu Gly Lys Ile Lys Ala Ile Gly Ala Ser Asn Leu Asp
145                 150                 155                 160

Tyr Gln Gln Leu Gln Asp Phe Asn Ala Asp Gly Tyr Leu Glu Val Phe
                165                 170                 175

Gln Ala Glu Tyr Ser Leu Ile Gln Arg Asp Ala Glu Lys Glu Leu Leu
                180                 185                 190

Pro Tyr Cys Glu Lys Gln Gly Ile Ser Phe Ile Pro Tyr Phe Pro Leu
            195                 200                 205

Ala Ser Gly Leu Leu Thr Gly Lys Phe Thr Gln Asp Thr Val Phe Asp
    210                 215                 220

Asp Phe Arg Lys Asp Lys Pro Gln Phe Gln Gly Glu Thr Phe Ile His
225                 230                 235                 240

Asn Leu Lys Lys Val Asp Lys Leu Lys Ala Val Ala Glu Glu Lys Gln
                245                 250                 255

Ala Asp Thr Ala His Val Ala Leu Ala Trp Leu Leu Thr Arg Pro Ala
                260                 265                 270

Ile Asp Ala Ile Ile Pro Gly Ala Lys Arg Pro Glu Gln Leu Gln Asp
            275                 280                 285

Asn Leu Lys Thr Leu Asn Ile Glu Leu Thr Glu Asp Glu Val Asn Phe
    290                 295                 300

Ile Ser Asp Ile Phe Lys
305                 310

<210>  96
<211>  933
<212>  DNA
<213>  Bacillus subtilis

<400>  96

atggatcaaa cacgtacact cggcaaaacg aagctgaagg tgaagcggat cggattcggc 60

gcgaatgcgg tcggcgggca taatctattt ccaaatctaa atgatgaaac agggaaggat 120

ttagtgcgca cggcattgga tggcggcgtc aattttatcg ataccgcctt tatatatgga 180

ttggggcgat ctgaagaatt aatcggcgaa gtcgtacagg aacgcggcgt gcggaatgag 240

ctcatcatcg ccaccaaagg agctcataaa gaagtggacg gcagcattga attagacaat 300

agccgggagt ttcttcgcag cgaggtggag aagagcctga agcggctgaa aacagattac 360

attgatttgt attatgttca ctttccggat ggaaaaacac ctctcgctga agtggcgggc 420

actttgaaag agctgaagga tgaggggaaa atcaaagcga tcggcgcttc gaacctcgat 480

tatcagcaat tgcaggattt taatgctgac ggctatttgg aggtcttcca ggccgaatat 540

tctctcatac agcgtgatgc cgagaaagag cttcttccat actgtgaaaa acaaggcatc 600

tcctttattc cttactttcc gcttgcgtcc ggactgctga caggaaaatt cacgcaagac 660

acagtctttg atgatttcag aaaggataaa cctcaatttc agggtgaaac gtttatccac 720

aatctcaaaa aagtagataa gctgaaagca gtagcggagg aaaaacaagc ggatacggca 780

catgtcgcct tggcgtggct gttaacgaga ccggcgattg atgccattat tccaggagct 840

aaacgaccgg agcaattaca ggataacctg aaaaccttga acattgaact gaccgaagat 900

gaagtgaatt tcatcagcga cattttcaaa taa 933

<210> 97
<211> 310
<212> PRT
<213> Bacillus subtilis

<400> 97

Met Lys Lys Ala Lys Leu Gly Lys Ser Asp Leu Gln Val Phe Pro Ile
1               5                   10                  15

Gly Leu Gly Thr Asn Ala Val Gly Gly His Asn Leu Tyr Pro Asn Leu
            20                  25                  30

Asn Glu Glu Thr Gly Lys Glu Leu Val Arg Glu Ala Ile Arg Asn Gly
        35                  40                  45

Val Thr Met Leu Asp Thr Ala Tyr Ile Tyr Gly Ile Gly Arg Ser Glu
    50                  55                  60

Glu Leu Ile Gly Glu Val Leu Arg Glu Phe Asn Arg Glu Asp Val Val
65                  70                  75                  80

Ile Ala Thr Lys Ala Ala His Arg Lys Gln Gly Asn Asp Phe Val Phe
                85                  90                  95

Asp Asn Ser Pro Asp Phe Leu Lys Lys Ser Val Asp Glu Ser Leu Lys
            100                 105                 110

Arg Leu Asn Thr Asp Tyr Ile Asp Leu Phe Tyr Ile His Phe Pro Asp
        115                 120                 125

Glu His Thr Pro Lys Asp Glu Ala Val Asn Ala Leu Asn Glu Met Lys
    130                 135                 140

Lys Ala Gly Lys Ile Arg Ser Ile Gly Val Ser Asn Phe Ser Leu Glu
145                 150                 155                 160

Gln Leu Lys Glu Ala Asn Lys Asp Gly Leu Val Asp Val Leu Gln Gly
                165                 170                 175

Glu Tyr Asn Leu Leu Asn Arg Glu Ala Glu Lys Thr Phe Phe Pro Tyr
            180                 185                 190

Thr Lys Glu His Asn Ile Ser Phe Ile Pro Tyr Phe Pro Leu Val Ser
        195                 200                 205

Gly Leu Leu Ala Gly Lys Tyr Thr Glu Asp Thr Thr Phe Pro Glu Gly
    210                 215                 220

Asp Leu Arg Asn Glu Gln Glu His Phe Lys Gly Glu Arg Phe Lys Glu
225                 230                 235                 240

Asn Ile Arg Lys Val Asn Lys Leu Ala Pro Ile Ala Glu Lys His Asn
                245                 250                 255

Val Asp Ile Pro His Ile Val Leu Ala Trp Tyr Leu Ala Arg Pro Glu
            260                 265                 270

Ile Asp Ile Leu Ile Pro Gly Ala Lys Arg Ala Asp Gln Leu Ile Asp
        275                 280                 285

Asn Ile Lys Thr Ala Asp Val Thr Leu Ser Gln Glu Asp Ile Ser Phe
    290                 295                 300

Ile Asp Lys Leu Phe Ala
305                 310

<210>  98
<211>  933
<212>  DNA
<213>  Bacillus subtilis

<400>  98

atgaaaaaag cgaagctcgg aaaatcagac ttgcaggtat tccctatcgg attaggaaca 60

aatgctgtcg gaggacataa cctctacccg aacctaaatg aagaaaccgg aaaagaattg 120

gtgcgcgagg cgatccgtaa tggcgtgaca atgttagaca ccgcttacat ttacgggatc 180

ggccgttccg aagaattaat tggtgaagtg ctgcgtgaat tcaaccgtga agatgttgtc 240

atcgcgacaa aagccgctca cagaaaacaa ggcaatgact ttgtctttga taattcacca 300

gatttcttta aaaaatcagt tgatgaaagc ctgaagcgct tgaataccga ttatattgat 360

ttgttctaca ttcacttccc tgacgaacat acgcctaagg atgaagccgt taacgcgctg 420

aatgagatga agaaagccgg aaaaatccgt tccatcggtg tatccaactt ctctttagag 480

caattgaaag aagcaaacaa agacggtttg gtagatgtat tgcaaggcga atacaacctg 540

ttaaaccgtg aagcggaaaa aacattcttc ccgtatacga aggagcataa tatttcattt 600

atcccttact tccctctcgt atcaggttta ttggcaggaa agtatacaga agatacaacg 660

ttcccagaag gcgacctgcg aaacgaacag gaacacttca agggtgagcg tttcaaagaa 720

aatatcagaa aggtcaacaa gcttgcgccg attgccgaaa aacacaacgt ggatatccct 780

cacatcgtat tggcctggta tttagcaaga ccggaaattg atattttaat cccaggagca 840

aaacgtgccg atcagctgat tgataacatc aaaacagctg acgtgacgct ttctcaagag 900

gatatttcat ttattgataa gctgttcgca taa 933

<210> 99
<211> 326
<212> PRT
<213> Bacillus subtilis

<400> 99

Met Glu Tyr Thr Tyr Leu Gly Arg Thr Gly Leu Arg Val Ser Arg Leu
1               5                   10                  15

Cys Leu Gly Thr Met Asn Phe Gly Val Asp Thr Asp Glu Lys Thr Ala
            20                  25                  30

Phe Arg Ile Met Asp Glu Ala Leu Asp Asn Gly Ile Gln Phe Phe Asp
        35                  40                  45

Thr Ala Asn Ile Tyr Gly Trp Gly Lys Asn Ala Gly Leu Thr Glu Ser
    50                  55                  60

Ile Ile Gly Lys Trp Phe Ala Gln Gly Gly Gln Arg Arg Glu Lys Val
65                  70                  75                  80

Val Leu Ala Thr Lys Val Tyr Glu Pro Ile Ser Asp Pro Asn Asp Gly
                85                  90                  95

Pro Asn Asp Met Arg Gly Leu Ser Leu Tyr Lys Ile Arg Arg His Leu
            100                 105                 110

Glu Gly Ser Leu Lys Arg Leu Gln Thr Asp His Ile Glu Leu Tyr Gln
        115                 120                 125

Met His His Ile Asp Arg Arg Thr Pro Trp Asp Glu Ile Trp Glu Ala
    130                 135                 140

Phe Glu Thr Gln Val Arg Ser Gly Lys Val Asp Tyr Ile Gly Ser Ser
145                 150                 155                 160

Asn Phe Ala Gly Trp His Leu Val Lys Ala Gln Ala Glu Ala Glu Lys
                165                 170                 175

Arg Arg Phe Met Gly Leu Val Thr Glu Gln His Lys Tyr Ser Leu Leu
            180                 185                 190

Glu Arg Thr Ala Glu Met Glu Val Leu Pro Ala Ala Arg Asp Leu Gly
        195                 200                 205

Leu Gly Val Val Ala Trp Ser Pro Leu Ala Gly Gly Leu Leu Gly Gly
    210                 215                 220

Lys Ala Leu Lys Ser Asn Ala Gly Thr Arg Thr Ala Lys Arg Ala Asp
225                 230                 235                 240

Leu Ile Glu Lys His Arg Leu Gln Leu Glu Lys Phe Ser Asp Leu Cys
                245                 250                 255

Lys Glu Leu Gly Glu Lys Glu Ala Asn Val Ala Leu Ala Trp Val Leu
            260                 265                 270

Ala Asn Pro Val Leu Thr Ala Pro Ile Ile Gly Pro Arg Thr Val Glu
        275                 280                 285

Gln Leu Arg Asp Thr Ile Lys Ala Val Glu Ile Ser Leu Asp Lys Glu
    290                 295                 300

Ile Leu Arg Met Leu Asn Asp Ile Phe Pro Gly Pro Gly Gly Glu Thr
305                 310                 315                 320

Pro Glu Ala Tyr Ala Trp
                325

<210>  100
<211>  981

<212>  DNA
<213>  Bacillus subtilis

<400>  100
gtggagtata cctatttagg gagaacagga ttgcgggtga gccgtttatg tttaggcacg      60

atgaattttg gagttgatac agacgaaaag actgcgttcc gtatcatgga tgaagcactt     120

gataacggca ttcaattttt tgatactgcc aatatttacg gctggggcaa aaacgcagga     180

ttgacagaga gcatcattgg aaaatggttt gcacaaggag gacagcgccg cgagaaagtt     240

gttctggcga caaaagtata tgaaccgatt tctgatccga atgacggacc aaatgatatg     300

aggggcttgt ctctatacaa aatcagacgt catctggaag gatcactgaa gcggcttcag     360

acagatcata tcgaattgta ccaaatgcat catatcgata ggcggacacc gtgggatgag     420

atatgggaag cttttgagac tcaggttcgc tccggcaaag tagactatat tggatccagt     480

aattttgcag gctggcattt agttaaagcg caagctgaag ctgaaaaacg gcgattcatg     540

ggactcgtca ctgaacagca taagtatagt ttattagaac gaacagctga aatggaagtg     600

ctgccggctg cacgggatct tggtttagga gtagtggcgt ggagtcccct tgcaggaggg     660

cttcttggcg ggaaggcatt gaaaagcaat gccggaactc gtacagcaaa aagagcagat     720

ttaattgaaa aacatcgttt gcaactcgag aaattttcag atttatgcaa agaactagga     780

gaaaaagaag caaatgtggc tttggcatgg gtgctggcaa atccagtttt aactgcgccg     840

atcatcggac cacgaacggt tgagcagctg cgtgatacga taaaagccgt tgaaatcagt     900

ctggataagg agattctccg catgttaaat gatatctttc ccggacctgg aggagagaca     960

cctgaggcat acgcctggtg a                                               981


<210>  101
<211>  1174
<212>  PRT
<213>  Nocardia iowensis

<400>  101

Met Ala Val Asp Ser Pro Asp Glu Arg Leu Gln Arg Arg Ile Ala Gln
1               5                   10                  15


Leu Phe Ala Glu Asp Glu Gln Val Lys Ala Ala Arg Pro Leu Glu Ala
            20                  25                  30


Val Ser Ala Ala Val Ser Ala Pro Gly Met Arg Leu Ala Gln Ile Ala
        35                  40                  45


Ala Thr Val Met Ala Gly Tyr Ala Asp Arg Pro Ala Ala Gly Gln Arg
    50                  55                  60


Ala Phe Glu Leu Asn Thr Asp Asp Ala Thr Gly Arg Thr Ser Leu Arg

65                  70                  75                  80

Leu Leu Pro Arg Phe Glu Thr Ile Thr Tyr Arg Glu Leu Trp Gln Arg
                85                  90                  95

Val Gly Glu Val Ala Ala Ala Trp His His Asp Pro Glu Asn Pro Leu
            100                 105                 110

Arg Ala Gly Asp Phe Val Ala Leu Leu Gly Phe Thr Ser Ile Asp Tyr
        115                 120                 125

Ala Thr Leu Asp Leu Ala Asp Ile His Leu Gly Ala Val Thr Val Pro
    130                 135                 140

Leu Gln Ala Ser Ala Ala Val Ser Gln Leu Ile Ala Ile Leu Thr Glu
145                 150                 155                 160

Thr Ser Pro Arg Leu Leu Ala Ser Thr Pro Glu His Leu Asp Ala Ala
                165                 170                 175

Val Glu Cys Leu Leu Ala Gly Thr Thr Pro Glu Arg Leu Val Val Phe
            180                 185                 190

Asp Tyr His Pro Glu Asp Asp Asp Gln Arg Ala Ala Phe Glu Ser Ala
        195                 200                 205

Arg Arg Arg Leu Ala Asp Ala Gly Ser Ser Val Ile Val Glu Thr Leu
    210                 215                 220

Asp Ala Val Arg Ala Arg Gly Arg Asp Leu Pro Ala Ala Pro Leu Phe
225                 230                 235                 240

Val Pro Asp Thr Asp Asp Asp Pro Leu Ala Leu Leu Ile Tyr Thr Ser
                245                 250                 255

Gly Ser Thr Gly Thr Pro Lys Gly Ala Met Tyr Thr Asn Arg Leu Ala
            260                 265                 270

Ala Thr Met Trp Gln Gly Asn Ser Met Leu Gln Gly Asn Ser Gln Arg
        275                 280                 285

Val Gly Ile Asn Leu Asn Tyr Met Pro Met Ser His Ile Ala Gly Arg
    290                 295                 300

Ile Ser Leu Phe Gly Val Leu Ala Arg Gly Gly Thr Ala Tyr Phe Ala
305                 310                 315                 320

Ala Lys Ser Asp Met Ser Thr Leu Phe Glu Asp Ile Gly Leu Val Arg
                325                 330                 335

Pro Thr Glu Ile Phe Phe Val Pro Arg Val Cys Asp Met Val Phe Gln
            340                 345                 350

Arg Tyr Gln Ser Glu Leu Asp Arg Arg Ser Val Ala Gly Ala Asp Leu
        355                 360                 365

Asp Thr Leu Asp Arg Glu Val Lys Ala Asp Leu Arg Gln Asn Tyr Leu
    370                 375                 380

Gly Gly Arg Phe Leu Val Ala Val Val Gly Ser Ala Pro Leu Ala Ala
385                 390                 395                 400

Glu Met Lys Thr Phe Met Glu Ser Val Leu Asp Leu Pro Leu His Asp
                405                 410                 415

Gly Tyr Gly Ser Thr Glu Ala Gly Ala Ser Val Leu Leu Asp Asn Gln
            420                 425                 430

Ile Gln Arg Pro Pro Val Leu Asp Tyr Lys Leu Val Asp Val Pro Glu
        435                 440                 445

Leu Gly Tyr Phe Arg Thr Asp Arg Pro His Pro Arg Gly Glu Leu Leu
    450                 455                 460

Leu Lys Ala Glu Thr Thr Ile Pro Gly Tyr Tyr Lys Arg Pro Glu Val
465                 470                 475                 480

Thr Ala Glu Ile Phe Asp Glu Asp Gly Phe Tyr Lys Thr Gly Asp Ile
                485                 490                 495

Val Ala Glu Leu Glu His Asp Arg Leu Val Tyr Val Asp Arg Arg Asn
            500                 505                 510

Asn Val Leu Lys Leu Ser Gln Gly Glu Phe Val Thr Val Ala His Leu
        515                 520                 525

Glu Ala Val Phe Ala Ser Ser Pro Leu Ile Arg Gln Ile Phe Ile Tyr
    530                 535                 540

Gly Ser Ser Glu Arg Ser Tyr Leu Leu Ala Val Ile Val Pro Thr Asp
545                 550                 555                 560

Asp Ala Leu Arg Gly Arg Asp Thr Ala Thr Leu Lys Ser Ala Leu Ala
                565                 570                 575

Glu Ser Ile Gln Arg Ile Ala Lys Asp Ala Asn Leu Gln Pro Tyr Glu
        580                 585                 590

Ile Pro Arg Asp Phe Leu Ile Glu Thr Glu Pro Phe Thr Ile Ala Asn
    595                 600                 605

Gly Leu Leu Ser Gly Ile Ala Lys Leu Leu Arg Pro Asn Leu Lys Glu
    610                 615                 620

Arg Tyr Gly Ala Gln Leu Glu Gln Met Tyr Thr Asp Leu Ala Thr Gly
625                 630                 635                 640

Gln Ala Asp Glu Leu Leu Ala Leu Arg Arg Glu Ala Ala Asp Leu Pro
                645                 650                 655

Val Leu Glu Thr Val Ser Arg Ala Ala Lys Ala Met Leu Gly Val Ala
            660                 665                 670

Ser Ala Asp Met Arg Pro Asp Ala His Phe Thr Asp Leu Gly Gly Asp
        675                 680                 685

Ser Leu Ser Ala Leu Ser Phe Ser Asn Leu Leu His Glu Ile Phe Gly
    690                 695                 700

Val Glu Val Pro Val Gly Val Val Val Ser Pro Ala Asn Glu Leu Arg
705                 710                 715                 720

Asp Leu Ala Asn Tyr Ile Glu Ala Glu Arg Asn Ser Gly Ala Lys Arg
                725                 730                 735

Pro Thr Phe Thr Ser Val His Gly Gly Gly Ser Glu Ile Arg Ala Ala
            740                 745                 750

Asp Leu Thr Leu Asp Lys Phe Ile Asp Ala Arg Thr Leu Ala Ala Ala
        755                 760                 765

Asp Ser Ile Pro His Ala Pro Val Pro Ala Gln Thr Val Leu Leu Thr
    770                 775                 780

Gly Ala Asn Gly Tyr Leu Gly Arg Phe Leu Cys Leu Glu Trp Leu Glu
785                 790                 795                 800

Arg Leu Asp Lys Thr Gly Gly Thr Leu Ile Cys Val Val Arg Gly Ser
                805                 810                 815

Asp Ala Ala Ala Ala Arg Lys Arg Leu Asp Ser Ala Phe Asp Ser Gly
            820                 825                 830

Asp Pro Gly Leu Leu Glu His Tyr Gln Gln Leu Ala Ala Arg Thr Leu
        835                 840                 845

Glu Val Leu Ala Gly Asp Ile Gly Asp Pro Asn Leu Gly Leu Asp Asp
    850                 855                 860

Ala Thr Trp Gln Arg Leu Ala Glu Thr Val Asp Leu Ile Val His Pro
865                 870                 875                 880

Ala Ala Leu Val Asn His Val Leu Pro Tyr Thr Gln Leu Phe Gly Pro
                885                 890                 895

Asn Val Val Gly Thr Ala Glu Ile Val Arg Leu Ala Ile Thr Ala Arg
            900                 905                 910

Arg Lys Pro Val Thr Tyr Leu Ser Thr Val Gly Val Ala Asp Gln Val
        915                 920                 925

Asp Pro Ala Glu Tyr Gln Glu Asp Ser Asp Val Arg Glu Met Ser Ala
    930                 935                 940

Val Arg Val Val Arg Glu Ser Tyr Ala Asn Gly Tyr Gly Asn Ser Lys
945                 950                 955                 960

Trp Ala Gly Glu Val Leu Leu Arg Glu Ala His Asp Leu Cys Gly Leu
                965                 970                 975

Pro Val Ala Val Phe Arg Ser Asp Met Ile Leu Ala His Ser Arg Tyr
            980                 985                 990

Ala Gly Gln Leu Asn Val Gln Asp Val Phe Thr Arg Leu Ile Leu Ser
        995                 1000                1005

Leu Val Ala Thr Gly Ile Ala Pro Tyr Ser Phe Tyr Arg Thr Asp
    1010                1015                1020

Ala Asp Gly Asn Arg Gln Arg Ala His Tyr Asp Gly Leu Pro Ala
    1025                1030                1035

Asp Phe Thr Ala Ala Ala Ile Thr Ala Leu Gly Ile Gln Ala Thr
    1040                1045                1050

Glu Gly Phe Arg Thr Tyr Asp Val Leu Asn Pro Tyr Asp Asp Gly
    1055                1060                1065

Ile Ser Leu Asp Glu Phe Val Asp Trp Leu Val Glu Ser Gly His

1070                    1075                    1080

Pro Ile  Gln Arg Ile Thr Asp  Tyr Ser Asp Trp Phe  His Arg Phe
    1085                    1090                    1095

Glu Thr  Ala Ile Arg Ala Leu  Pro Glu Lys Gln Arg  Gln Ala Ser
    1100                    1105                    1110

Val Leu  Pro Leu Leu Asp Ala  Tyr Arg Asn Pro Cys  Pro Ala Val
    1115                    1120                    1125

Arg Gly  Ala Ile Leu Pro Ala  Lys Glu Phe Gln Ala  Ala Val Gln
    1130                    1135                    1140

Thr Ala  Lys Ile Gly Pro Glu  Gln Asp Ile Pro His  Leu Ser Ala
    1145                    1150                    1155

Pro Leu  Ile Asp Lys Tyr Val  Ser Asp Leu Glu Leu  Leu Gln Leu
    1160                    1165                    1170

Leu

<210>  102
<211>  1174
<212>  PRT
<213>  Mycobacterium marinum

<400>  102

Met Ser Pro Ile Thr Arg Glu Glu Arg Leu Glu Arg Arg Ile Gln Asp
1               5                   10                  15

Leu Tyr Ala Asn Asp Pro Gln Phe Ala Ala Ala Lys Pro Ala Thr Ala
            20                  25                  30

Ile Thr Ala Ala Ile Glu Arg Pro Gly Leu Pro Leu Pro Gln Ile Ile
        35                  40                  45

Glu Thr Val Met Thr Gly Tyr Ala Asp Arg Pro Ala Leu Ala Gln Arg
    50                  55                  60

Ser Val Glu Phe Val Thr Asp Ala Gly Thr Gly His Thr Thr Leu Arg
65                  70                  75                  80

Leu Leu Pro His Phe Glu Thr Ile Ser Tyr Gly Glu Leu Trp Asp Arg
                85                  90                  95

Ile Ser Ala Leu Ala Asp Val Leu Ser Thr Glu Gln Thr Val Lys Pro

            100                 105                 110

Gly Asp Arg Val Cys Leu Leu Gly Phe Asn Ser Val Asp Tyr Ala Thr
        115                 120                 125

Ile Asp Met Thr Leu Ala Arg Leu Gly Ala Val Ala Val Pro Leu Gln
    130                 135                 140

Thr Ser Ala Ala Ile Thr Gln Leu Gln Pro Ile Val Ala Glu Thr Gln
145                 150                 155                 160

Pro Thr Met Ile Ala Ala Ser Val Asp Ala Leu Ala Asp Ala Thr Glu
                165                 170                 175

Leu Ala Leu Ser Gly Gln Thr Ala Thr Arg Val Leu Val Phe Asp His
            180                 185                 190

His Arg Gln Val Asp Ala His Arg Ala Ala Val Glu Ser Ala Arg Glu
        195                 200                 205

Arg Leu Ala Gly Ser Ala Val Val Glu Thr Leu Ala Glu Ala Ile Ala
    210                 215                 220

Arg Gly Asp Val Pro Arg Gly Ala Ser Ala Gly Ser Ala Pro Gly Thr
225                 230                 235                 240

Asp Val Ser Asp Asp Ser Leu Ala Leu Leu Ile Tyr Thr Ser Gly Ser
                245                 250                 255

Thr Gly Ala Pro Lys Gly Ala Met Tyr Pro Arg Arg Asn Val Ala Thr
            260                 265                 270

Phe Trp Arg Lys Arg Thr Trp Phe Glu Gly Gly Tyr Glu Pro Ser Ile
        275                 280                 285

Thr Leu Asn Phe Met Pro Met Ser His Val Met Gly Arg Gln Ile Leu
    290                 295                 300

Tyr Gly Thr Leu Cys Asn Gly Gly Thr Ala Tyr Phe Val Ala Lys Ser
305                 310                 315                 320

Asp Leu Ser Thr Leu Phe Glu Asp Leu Ala Leu Val Arg Pro Thr Glu
                325                 330                 335

Leu Thr Phe Val Pro Arg Val Trp Asp Met Val Phe Asp Glu Phe Gln
            340                 345                 350

Ser Glu Val Asp Arg Arg Leu Val Asp Gly Ala Asp Arg Val Ala Leu
        355                 360                 365

Glu Ala Gln Val Lys Ala Glu Ile Arg Asn Asp Val Leu Gly Gly Arg
    370                 375                 380

Tyr Thr Ser Ala Leu Thr Gly Ser Ala Pro Ile Ser Asp Glu Met Lys
385                 390                 395                 400

Ala Trp Val Glu Glu Leu Leu Asp Met His Leu Val Glu Gly Tyr Gly
                405                 410                 415

Ser Thr Glu Ala Gly Met Ile Leu Ile Asp Gly Ala Ile Arg Arg Pro
            420                 425                 430

Ala Val Leu Asp Tyr Lys Leu Val Asp Val Pro Asp Leu Gly Tyr Phe
        435                 440                 445

Leu Thr Asp Arg Pro His Pro Arg Gly Glu Leu Leu Val Lys Thr Asp
    450                 455                 460

Ser Leu Phe Pro Gly Tyr Tyr Gln Arg Ala Glu Val Thr Ala Asp Val
465                 470                 475                 480

Phe Asp Ala Asp Gly Phe Tyr Arg Thr Gly Asp Ile Met Ala Glu Val
                485                 490                 495

Gly Pro Glu Gln Phe Val Tyr Leu Asp Arg Arg Asn Asn Val Leu Lys
            500                 505                 510

Leu Ser Gln Gly Glu Phe Val Thr Val Ser Lys Leu Glu Ala Val Phe
        515                 520                 525

Gly Asp Ser Pro Leu Val Arg Gln Ile Tyr Ile Tyr Gly Asn Ser Ala
    530                 535                 540

Arg Ala Tyr Leu Leu Ala Val Ile Val Pro Thr Gln Glu Ala Leu Asp
545                 550                 555                 560

Ala Val Pro Val Glu Glu Leu Lys Ala Arg Leu Gly Asp Ser Leu Gln
                565                 570                 575

Glu Val Ala Lys Ala Ala Gly Leu Gln Ser Tyr Glu Ile Pro Arg Asp
            580                 585                 590

Phe Ile Ile Glu Thr Thr Pro Trp Thr Leu Glu Asn Gly Leu Leu Thr
        595                 600                 605

Gly Ile Arg Lys Leu Ala Arg Pro Gln Leu Lys Lys His Tyr Gly Glu
    610                 615                 620

Leu Leu Glu Gln Ile Tyr Thr Asp Leu Ala His Gly Gln Ala Asp Glu
625                 630                 635                 640

Leu Arg Ser Leu Arg Gln Ser Gly Ala Asp Ala Pro Val Leu Val Thr
                645                 650                 655

Val Cys Arg Ala Ala Ala Ala Leu Leu Gly Gly Ser Ala Ser Asp Val
            660                 665                 670

Gln Pro Asp Ala His Phe Thr Asp Leu Gly Gly Asp Ser Leu Ser Ala
        675                 680                 685

Leu Ser Phe Thr Asn Leu Leu His Glu Ile Phe Asp Ile Glu Val Pro
    690                 695                 700

Val Gly Val Ile Val Ser Pro Ala Asn Asp Leu Gln Ala Leu Ala Asp
705                 710                 715                 720

Tyr Val Glu Ala Ala Arg Lys Pro Gly Ser Ser Arg Pro Thr Phe Ala
                725                 730                 735

Ser Val His Gly Ala Ser Asn Gly Gln Val Thr Glu Val His Ala Gly
            740                 745                 750

Asp Leu Ser Leu Asp Lys Phe Ile Asp Ala Ala Thr Leu Ala Glu Ala
        755                 760                 765

Pro Arg Leu Pro Ala Ala Asn Thr Gln Val Arg Thr Val Leu Leu Thr
    770                 775                 780

Gly Ala Thr Gly Phe Leu Gly Arg Tyr Leu Ala Leu Glu Trp Leu Glu
785                 790                 795                 800

Arg Met Asp Leu Val Asp Gly Lys Leu Ile Cys Leu Val Arg Ala Lys
                805                 810                 815

Ser Asp Thr Glu Ala Arg Ala Arg Leu Asp Lys Thr Phe Asp Ser Gly
            820                 825                 830

Asp Pro Glu Leu Leu Ala His Tyr Arg Ala Leu Ala Gly Asp His Leu
        835                 840                 845

Glu Val Leu Ala Gly Asp Lys Gly Glu Ala Asp Leu Gly Leu Asp Arg
    850                 855                 860

Gln Thr Trp Gln Arg Leu Ala Asp Thr Val Asp Leu Ile Val Asp Pro
865                 870                 875                 880

Ala Ala Leu Val Asn His Val Leu Pro Tyr Ser Gln Leu Phe Gly Pro
                885                 890                 895

Asn Ala Leu Gly Thr Ala Glu Leu Leu Arg Leu Ala Leu Thr Ser Lys
            900                 905                 910

Ile Lys Pro Tyr Ser Tyr Thr Ser Thr Ile Gly Val Ala Asp Gln Ile
        915                 920                 925

Pro Pro Ser Ala Phe Thr Glu Asp Ala Asp Ile Arg Val Ile Ser Ala
    930                 935                 940

Thr Arg Ala Val Asp Asp Ser Tyr Ala Asn Gly Tyr Ser Asn Ser Lys
945                 950                 955                 960

Trp Ala Gly Glu Val Leu Leu Arg Glu Ala His Asp Leu Cys Gly Leu
                965                 970                 975

Pro Val Ala Val Phe Arg Cys Asp Met Ile Leu Ala Asp Thr Thr Trp
            980                 985                 990

Ala Gly Gln Leu Asn Val Pro Asp  Met Phe Thr Arg Met  Ile Leu Ser
        995                 1000                 1005

Leu Ala  Ala Thr Gly Ile Ala  Pro Gly Ser Phe Tyr  Glu Leu Ala
    1010                 1015                 1020

Ala Asp  Gly Ala Arg Gln Arg  Ala His Tyr Asp Gly  Leu Pro Val
    1025                 1030                 1035

Glu Phe  Ile Ala Glu Ala Ile  Ser Thr Leu Gly Ala  Gln Ser Gln
    1040                 1045                 1050

Asp Gly  Phe His Thr Tyr His  Val Met Asn Pro Tyr  Asp Asp Gly
    1055                 1060                 1065

Ile Gly  Leu Asp Glu Phe Val  Asp Trp Leu Asn Glu  Ser Gly Cys
    1070                 1075                 1080

Pro Ile  Gln Arg Ile Ala Asp  Tyr Gly Asp Trp Leu  Gln Arg Phe
    1085                 1090                 1095

Glu Thr  Ala Leu Arg Ala Leu  Pro Asp Arg Gln Arg  His Ser Ser

1100                    1105                    1110

Leu Leu  Pro Leu Leu His Asn  Tyr Arg Gln Pro Glu  Arg Pro Val
    1115                    1120                    1125

Arg Gly  Ser Ile Ala Pro Thr  Asp Arg Phe Arg Ala  Ala Val Gln
    1130                    1135                    1140

Glu Ala  Lys Ile Gly Pro Asp  Lys Asp Ile Pro His  Val Gly Ala
    1145                    1150                    1155

Pro Ile  Ile Val Lys Tyr Val  Ser Asp Leu Arg Leu  Leu Gly Leu
    1160                    1165                    1170

Leu

<210>  103
<211>  1178
<212>  PRT
<213>  Mycobacterium abcessus

<400>  103

Met Thr Glu Thr Ile Ser Thr Ala Ala Val Pro Thr Thr Asp Leu Glu
1               5                   10                  15

Glu Gln Val Lys Arg Arg Ile Glu Gln Val Val Ser Asn Asp Pro Gln
            20                  25                  30

Leu Ala Ala Leu Leu Pro Glu Asp Ser Val Thr Glu Ala Val Asn Glu
        35                  40                  45

Pro Asp Leu Pro Leu Val Glu Val Ile Arg Arg Leu Leu Glu Gly Tyr
    50                  55                  60

Gly Asp Arg Pro Ala Leu Gly Gln Arg Ala Phe Glu Phe Val Thr Gly
65                  70                  75                  80

Asp Asp Gly Ala Thr Val Ile Ala Leu Lys Pro Glu Tyr Thr Thr Val
                85                  90                  95

Ser Tyr Arg Glu Leu Trp Glu Arg Ala Glu Ala Ile Ala Ala Ala Trp
            100                 105                 110

His Glu Gln Gly Ile Arg Asp Gly Asp Phe Val Ala Gln Leu Gly Phe
        115                 120                 125

Thr Ser Thr Asp Phe Ala Ser Leu Asp Val Ala Gly Leu Arg Leu Gly

130                 135                 140

Thr Val Ser Val Pro Leu Gln Thr Gly Ala Ser Leu Gln Gln Arg Asn
145                 150                 155                 160

Ala Ile Leu Glu Glu Thr Arg Pro Ala Val Phe Ala Ala Ser Ile Glu
                165                 170                 175

Tyr Leu Asp Ala Ala Val Asp Ser Val Leu Ala Thr Pro Ser Val Arg
            180                 185                 190

Leu Leu Ser Val Phe Asp Tyr His Ala Glu Val Asp Ser Gln Arg Glu
        195                 200                 205

Ala Leu Glu Ala Val Arg Ala Arg Leu Glu Ser Ala Gly Arg Thr Ile
    210                 215                 220

Val Val Glu Ala Leu Ala Glu Ala Leu Ala Arg Gly Arg Asp Leu Pro
225                 230                 235                 240

Ala Ala Pro Leu Pro Ser Ala Asp Pro Asp Ala Leu Arg Leu Leu Ile
                245                 250                 255

Tyr Thr Ser Gly Ser Thr Gly Thr Pro Lys Gly Ala Met Tyr Pro Gln
            260                 265                 270

Trp Leu Val Ala Asn Leu Trp Gln Lys Lys Trp Leu Thr Asp Asp Val
        275                 280                 285

Ile Pro Ser Ile Gly Val Asn Phe Met Pro Met Ser His Leu Ala Gly
    290                 295                 300

Arg Leu Thr Leu Met Gly Thr Leu Ser Gly Gly Gly Thr Ala Tyr Tyr
305                 310                 315                 320

Ile Ala Ser Ser Asp Leu Ser Thr Phe Phe Glu Asp Ile Ala Leu Ile
                325                 330                 335

Arg Pro Ser Glu Val Leu Phe Val Pro Arg Val Val Glu Met Val Phe
            340                 345                 350

Gln Arg Phe Gln Ala Glu Leu Asp Arg Ser Leu Ala Pro Gly Glu Ser
        355                 360                 365

Asn Ser Glu Ile Ala Glu Arg Ile Lys Val Arg Ile Arg Glu Gln Asp
    370                 375                 380

Phe Gly Gly Arg Val Leu Ser Ala Gly Ser Gly Ser Ala Pro Leu Ser
385                 390                 395                 400

Pro Glu Met Thr Glu Phe Met Glu Ser Leu Leu Gln Val Pro Leu Arg
                405                 410                 415

Asp Gly Tyr Gly Ser Thr Glu Ala Gly Gly Val Trp Arg Asp Gly Val
            420                 425                 430

Leu Gln Arg Pro Pro Val Thr Asp Tyr Lys Leu Val Asp Val Pro Glu
        435                 440                 445

Leu Gly Tyr Phe Thr Thr Asp Ser Pro His Pro Arg Gly Glu Leu Arg
    450                 455                 460

Leu Lys Ser Glu Thr Met Phe Pro Gly Tyr Tyr Lys Arg Pro Glu Thr
465                 470                 475                 480

Thr Ala Asp Val Phe Asp Asp Glu Gly Tyr Tyr Lys Thr Gly Asp Val
                485                 490                 495

Val Ala Glu Leu Gly Pro Asp His Leu Lys Tyr Leu Asp Arg Val Lys
            500                 505                 510

Asn Val Leu Lys Leu Ala Gln Gly Glu Phe Val Ala Val Ser Lys Leu
        515                 520                 525

Glu Ala Ala Tyr Thr Gly Ser Pro Leu Val Arg Gln Ile Phe Val Tyr
    530                 535                 540

Gly Asn Ser Glu Arg Ser Phe Leu Leu Ala Val Val Val Pro Thr Pro
545                 550                 555                 560

Glu Val Leu Glu Arg Tyr Ala Asp Ser Pro Asp Ala Leu Lys Pro Leu
                565                 570                 575

Ile Gln Asp Ser Leu Gln Gln Val Ala Lys Asp Ala Glu Leu Gln Ser
            580                 585                 590

Tyr Glu Ile Pro Arg Asp Phe Ile Val Glu Thr Val Pro Phe Thr Val
        595                 600                 605

Glu Ser Gly Leu Leu Ser Asp Ala Arg Lys Leu Leu Arg Pro Lys Leu
    610                 615                 620

Lys Asp His Tyr Gly Glu Arg Leu Glu Ala Leu Tyr Ala Glu Leu Ala
625                 630                 635                 640

Glu Ser Gln Asn Glu Arg Leu Arg Gln Leu Ala Arg Glu Ala Ala Thr
                645                 650                 655

Arg Pro Val Leu Glu Thr Val Thr Asp Ala Ala Ala Ala Leu Leu Gly
            660                 665                 670

Ala Ser Ser Ser Asp Leu Ala Pro Asp Val Arg Phe Ile Asp Leu Gly
        675                 680                 685

Gly Asp Ser Leu Ser Ala Leu Ser Tyr Ser Glu Leu Leu Arg Asp Ile
    690                 695                 700

Phe Glu Val Asp Val Pro Val Gly Val Ile Asn Ser Val Ala Asn Asp
705                 710                 715                 720

Leu Ala Ala Ile Ala Arg His Ile Glu Ala Gln Arg Thr Gly Ala Ala
                725                 730                 735

Thr Gln Pro Thr Phe Ala Ser Val His Gly Lys Asp Ala Thr Val Ile
            740                 745                 750

Thr Ala Gly Glu Leu Thr Leu Asp Lys Phe Leu Asp Glu Ser Leu Leu
        755                 760                 765

Lys Ala Ala Lys Asp Val Gln Pro Ala Thr Ala Asp Val Lys Thr Val
    770                 775                 780

Leu Val Thr Gly Gly Asn Gly Trp Leu Gly Arg Trp Leu Val Leu Asp
785                 790                 795                 800

Trp Leu Glu Arg Leu Ala Pro Asn Gly Gly Lys Val Tyr Ala Leu Ile
                805                 810                 815

Arg Gly Ala Asp Ala Glu Ala Ala Arg Ala Arg Leu Asp Ala Val Tyr
            820                 825                 830

Glu Ser Gly Asp Pro Lys Leu Ser Ala His Tyr Arg Gln Leu Ala Gln
        835                 840                 845

Gln Ser Leu Glu Val Ile Ala Gly Asp Phe Gly Asp Gln Asp Leu Gly
    850                 855                 860

Leu Ser Gln Glu Val Trp Gln Lys Leu Ala Lys Asp Val Asp Leu Ile
865                 870                 875                 880

Val His Ser Gly Ala Leu Val Asn His Val Leu Pro Tyr Ser Gln Leu
                885                 890                 895

Phe Gly Pro Asn Val Ala Gly Thr Ala Glu Ile Ile Lys Leu Ala Ile
            900                 905                 910

Ser Glu Arg Leu Lys Pro Val Thr Tyr Leu Ser Thr Val Gly Ile Ala
        915                 920                 925

Asp Gln Ile Pro Val Thr Glu Phe Glu Glu Asp Ser Asp Val Arg Val
    930                 935                 940

Met Ser Ala Glu Arg Gln Ile Asn Asp Gly Tyr Ala Asn Gly Tyr Gly
945                 950                 955                 960

Asn Ser Lys Trp Ala Gly Glu Val Leu Leu Arg Glu Ala His Asp Leu
                965                 970                 975

Ala Gly Leu Pro Val Arg Val Phe Arg Ser Asp Met Ile Leu Ala His
            980                 985                 990

Ser Asp Tyr His Gly Gln Leu Asn Val Thr Asp Val Phe Thr Arg Ser
        995                 1000                1005

Ile Gln Ser Leu Leu Leu Thr Gly Val Ala Pro Ala Ser Phe Tyr
    1010                1015                1020

Glu Leu Asp Ala Asp Gly Asn Arg Gln Arg Ala His Tyr Asp Gly
    1025                1030                1035

Val Pro Gly Asp Phe Thr Ala Ala Ser Ile Thr Ala Ile Gly Gly
    1040                1045                1050

Val Asn Val Val Asp Gly Tyr Arg Ser Phe Asp Val Phe Asn Pro
    1055                1060                1065

His His Asp Gly Val Ser Met Asp Thr Phe Val Asp Trp Leu Ile
    1070                1075                1080

Asp Ala Gly Tyr Lys Ile Ala Arg Ile Asp Asp Tyr Asp Gln Trp
    1085                1090                1095

Leu Ala Arg Phe Glu Leu Ala Leu Lys Gly Leu Pro Glu Gln Gln
    1100                1105                1110

Arg Gln Gln Ser Val Leu Pro Leu Leu Lys Met Tyr Glu Lys Pro
    1115                1120                1125

Gln Pro Ala Ile Asp Gly Ser Ala Leu Pro Thr Ala Glu Phe Ser

1130                1135                1140

Arg Ala  Val His Glu Ala Lys  Val Gly Asp Ser Gly  Glu Ile Pro
    1145                1150                1155

His Val  Thr Lys Glu Leu Ile  Leu Lys Tyr Ala Ser  Asp Ile Gln
    1160                1165                1170

Leu Leu  Gly Leu Val
    1175

<210>  104
<211>  1193
<212>  PRT
<213>  Mycobacterium smegmatis

<400>  104

Met His Gln Leu Thr Val Thr Gly Met Asn Ile Cys Glu Val Gln Arg
1               5                   10                  15

Leu Phe Pro Arg Met Thr Ser Asp Val His Asp Ala Thr Asp Gly Val
            20                  25                  30

Thr Glu Thr Ala Leu Asp Asp Glu Gln Ser Thr Arg Arg Ile Ala Glu
        35                  40                  45

Leu Tyr Ala Thr Asp Pro Glu Phe Ala Ala Ala Ala Pro Leu Pro Ala
    50                  55                  60

Val Val Asp Ala Ala His Lys Pro Gly Leu Arg Leu Ala Glu Ile Leu
65                  70                  75                  80

Gln Thr Leu Phe Thr Gly Tyr Gly Asp Arg Pro Ala Leu Gly Tyr Arg
                85                  90                  95

Ala Arg Glu Leu Ala Thr Asp Glu Gly Gly Arg Thr Val Thr Arg Leu
            100                 105                 110

Leu Pro Arg Phe Asp Thr Leu Thr Tyr Ala Gln Val Trp Ser Arg Val
        115                 120                 125

Gln Ala Val Ala Ala Ala Leu Arg His Asn Phe Ala Gln Pro Ile Tyr
    130                 135                 140

Pro Gly Asp Ala Val Ala Thr Ile Gly Phe Ala Ser Pro Asp Tyr Leu
145                 150                 155                 160

Thr Leu Asp Leu Val Cys Ala Tyr Leu Gly Leu Val Ser Val Pro Leu

                165                 170                 175

Gln His Asn Ala Pro Val Ser Arg Leu Ala Pro Ile Leu Ala Glu Val
            180                 185                 190

Glu Pro Arg Ile Leu Thr Val Ser Ala Glu Tyr Leu Asp Leu Ala Val
        195                 200                 205

Glu Ser Val Arg Asp Val Asn Ser Val Ser Gln Leu Val Val Phe Asp
    210                 215                 220

His His Pro Glu Val Asp Asp His Arg Asp Ala Leu Ala Arg Ala Arg
225                 230                 235                 240

Glu Gln Leu Ala Gly Lys Gly Ile Ala Val Thr Thr Leu Asp Ala Ile
                245                 250                 255

Ala Asp Glu Gly Ala Gly Leu Pro Ala Glu Pro Ile Tyr Thr Ala Asp
            260                 265                 270

His Asp Gln Arg Leu Ala Met Ile Leu Tyr Thr Ser Gly Ser Thr Gly
        275                 280                 285

Ala Pro Lys Gly Ala Met Tyr Thr Glu Ala Met Val Ala Arg Leu Trp
    290                 295                 300

Thr Met Ser Phe Ile Thr Gly Asp Pro Thr Pro Val Ile Asn Val Asn
305                 310                 315                 320

Phe Met Pro Leu Asn His Leu Gly Gly Arg Ile Pro Ile Ser Thr Ala
                325                 330                 335

Val Gln Asn Gly Gly Thr Ser Tyr Phe Val Pro Glu Ser Asp Met Ser
            340                 345                 350

Thr Leu Phe Glu Asp Leu Ala Leu Val Arg Pro Thr Glu Leu Gly Leu
        355                 360                 365

Val Pro Arg Val Ala Asp Met Leu Tyr Gln His His Leu Ala Thr Val
    370                 375                 380

Asp Arg Leu Val Thr Gln Gly Ala Asp Glu Leu Thr Ala Glu Lys Gln
385                 390                 395                 400

Ala Gly Ala Glu Leu Arg Glu Gln Val Leu Gly Gly Arg Val Ile Thr
                405                 410                 415

Gly Phe Val Ser Thr Ala Pro Leu Ala Ala Glu Met Arg Ala Phe Leu
        420                 425                 430

Asp Ile Thr Leu Gly Ala His Ile Val Asp Gly Tyr Gly Leu Thr Glu
    435                 440                 445

Thr Gly Ala Val Thr Arg Asp Gly Val Ile Val Arg Pro Pro Val Ile
    450                 455                 460

Asp Tyr Lys Leu Ile Asp Val Pro Glu Leu Gly Tyr Phe Ser Thr Asp
465                 470                 475                 480

Lys Pro Tyr Pro Arg Gly Glu Leu Leu Val Arg Ser Gln Thr Leu Thr
                485                 490                 495

Pro Gly Tyr Tyr Lys Arg Pro Glu Val Thr Ala Ser Val Phe Asp Arg
            500                 505                 510

Asp Gly Tyr Tyr His Thr Gly Asp Val Met Ala Glu Thr Ala Pro Asp
        515                 520                 525

His Leu Val Tyr Val Asp Arg Arg Asn Asn Val Leu Lys Leu Ala Gln
    530                 535                 540

Gly Glu Phe Val Ala Val Ala Asn Leu Glu Ala Val Phe Ser Gly Ala
545                 550                 555                 560

Ala Leu Val Arg Gln Ile Phe Val Tyr Gly Asn Ser Glu Arg Ser Phe
                565                 570                 575

Leu Leu Ala Val Val Val Pro Thr Pro Glu Ala Leu Glu Gln Tyr Asp
            580                 585                 590

Pro Ala Ala Leu Lys Ala Ala Leu Ala Asp Ser Leu Gln Arg Thr Ala
        595                 600                 605

Arg Asp Ala Glu Leu Gln Ser Tyr Glu Val Pro Ala Asp Phe Ile Val
    610                 615                 620

Glu Thr Glu Pro Phe Ser Ala Ala Asn Gly Leu Leu Ser Gly Val Gly
625                 630                 635                 640

Lys Leu Leu Arg Pro Asn Leu Lys Asp Arg Tyr Gly Gln Arg Leu Glu
                645                 650                 655

Gln Met Tyr Ala Asp Ile Ala Ala Thr Gln Ala Asn Gln Leu Arg Glu
            660                 665                 670

Leu Arg Arg Ala Ala Ala Thr Gln Pro Val Ile Asp Thr Leu Thr Gln
        675                 680                 685

Ala Ala Ala Thr Ile Leu Gly Thr Gly Ser Glu Val Ala Ser Asp Ala
    690                 695                 700

His Phe Thr Asp Leu Gly Gly Asp Ser Leu Ser Ala Leu Thr Leu Ser
705                 710                 715                 720

Asn Leu Leu Ser Asp Phe Phe Gly Phe Glu Val Pro Val Gly Thr Ile
                725                 730                 735

Val Asn Pro Ala Thr Asn Leu Ala Gln Leu Ala Gln His Ile Glu Ala
            740                 745                 750

Gln Arg Thr Ala Gly Asp Arg Arg Pro Ser Phe Thr Thr Val His Gly
        755                 760                 765

Ala Asp Ala Thr Glu Ile Arg Ala Ser Glu Leu Thr Leu Asp Lys Phe
    770                 775                 780

Ile Asp Ala Glu Thr Leu Arg Ala Ala Pro Gly Leu Pro Lys Val Thr
785                 790                 795                 800

Thr Glu Pro Arg Thr Val Leu Leu Ser Gly Ala Asn Gly Trp Leu Gly
                805                 810                 815

Arg Phe Leu Thr Leu Gln Trp Leu Glu Arg Leu Ala Pro Val Gly Gly
            820                 825                 830

Thr Leu Ile Thr Ile Val Arg Gly Arg Asp Asp Ala Ala Ala Arg Ala
        835                 840                 845

Arg Leu Thr Gln Ala Tyr Asp Thr Asp Pro Glu Leu Ser Arg Arg Phe
    850                 855                 860

Ala Glu Leu Ala Asp Arg His Leu Arg Val Val Ala Gly Asp Ile Gly
865                 870                 875                 880

Asp Pro Asn Leu Gly Leu Thr Pro Glu Ile Trp His Arg Leu Ala Ala
                885                 890                 895

Glu Val Asp Leu Val Val His Pro Ala Ala Leu Val Asn His Val Leu
            900                 905                 910

Pro Tyr Arg Gln Leu Phe Gly Pro Asn Val Val Gly Thr Ala Glu Val
        915                 920                 925

Ile Lys Leu Ala Leu Thr Glu Arg Ile Lys Pro Val Thr Tyr Leu Ser
    930                 935                 940

Thr Val Ser Val Ala Met Gly Ile Pro Asp Phe Glu Glu Asp Gly Asp
945                 950                 955                 960

Ile Arg Thr Val Ser Pro Val Arg Pro Leu Asp Gly Gly Tyr Ala Asn
                965                 970                 975

Gly Tyr Gly Asn Ser Lys Trp Ala Gly Glu Val Leu Leu Arg Glu Ala
            980                 985                 990

His Asp Leu Cys Gly Leu Pro Val Ala Thr Phe Arg Ser Asp Met Ile
        995                 1000                1005

Leu Ala His Pro Arg Tyr Arg Gly Gln Val Asn Val Pro Asp Met
    1010                1015                1020

Phe Thr Arg Leu Leu Leu Ser Leu Leu Ile Thr Gly Val Ala Pro
    1025                1030                1035

Arg Ser Phe Tyr Ile Gly Asp Gly Glu Arg Pro Arg Ala His Tyr
    1040                1045                1050

Pro Gly Leu Thr Val Asp Phe Val Ala Glu Ala Val Thr Thr Leu
    1055                1060                1065

Gly Ala Gln Gln Arg Glu Gly Tyr Val Ser Tyr Asp Val Met Asn
    1070                1075                1080

Pro His Asp Asp Gly Ile Ser Leu Asp Val Phe Val Asp Trp Leu
    1085                1090                1095

Ile Arg Ala Gly His Pro Ile Asp Arg Val Asp Asp Tyr Asp Asp
    1100                1105                1110

Trp Val Arg Arg Phe Glu Thr Ala Leu Thr Ala Leu Pro Glu Lys
    1115                1120                1125

Arg Arg Ala Gln Thr Val Leu Pro Leu Leu His Ala Phe Arg Ala
    1130                1135                1140

Pro Gln Ala Pro Leu Arg Gly Ala Pro Glu Pro Thr Glu Val Phe
    1145                1150                1155

His Ala Ala Val Arg Thr Ala Lys Val Gly Pro Gly Asp Ile Pro

1160                    1165                    1170

His Leu Asp Glu Ala Leu Ile Asp Lys Tyr Ile Arg Asp Leu Arg
    1175                    1180                    1185

Glu Phe Gly Leu Ile
    1190

<210>  105
<211>  3537
<212>  DNA
<213>  Mycobacterium abscessus

<400>  105
atgactgaaa cgatctccac agcggctgtc cccactacgg atctcgaaga gcaggtgaag      60

cgacgcatcg agcaggtcgt gtccaacgat ccgcagctgg cggcgcttct cccggaagat      120

tcggtcaccg aggcggtcaa cgagcccgat ctaccgctgg tcgaggtgat caggcgactg      180

ctggagggct acggtgaccg cccggcactc ggccagcgcg ccttcgagtt cgtcaccggg      240

gacgacggtg cgaccgtgat cgcgctgaag cccgaataca ccaccgtctc ctaccgcgag      300

ttgtgggaac gtgccgaggc tatcgctgcc gcgtggcacg agcagggcat ccgtgacggc      360

gacttcgtcg ctcagttggg tttcaccagc acggacttcg cgtcgctcga cgtcgcggga      420

ttgcgtctgg gcaccgtctc ggtgcccctg cagacgggcg cgtcgctgca gcagcgcaac      480

gcgattctcg aagagacccg gcccgcagtc tttgccgcga gtatcgaata ccttgatgcc      540

gccgtcgatt cggtgcttgc gaccccctcg gtgcgactcc tctcggtttt cgactatcac      600

gcggaggtcg acagccagcg cgaagcgctg gaggctgtgc gggcccggct tgagagtgcc      660

ggccggacga tcgtcgtcga ggccctggcg gaggctctcg cgcgggggcg ggacctgccc      720

gccgcgccgc tgcccagtgc agatcccgat gccttgcgtc tgctcatcta cacctccggc      780

agcaccggta cccccaaggg cgccatgtat ccgcaatggc tggtcgccaa cttgtggcag      840

aagaagtggc tcaccgacga tgtgattccg tccataggcg tgaacttcat gcccatgagc      900

cacctggcgg gtcgcctcac tctcatgggc accctttccg gtggcggaac cgcctactac      960

atcgcttcga gcgatctttc gactttcttc gaggacatcg cgctcatccg cccctccgaa      1020

gtgctcttcg tgccgcgtgt ggtggagatg gtgttccagc gttttcaggc agaattggac      1080

cggtcccttg ccccgggtga gagcaactcc gagatcgcgg agcgaatcaa ggtccgcatc      1140

cgggaacagg acttcggcgg gcgtgtgctc agtgctggct ccgggtcggc cccgttgtct      1200

cctgagatga cggagttcat ggagtcgctg ctgcaggtgc cgttgcgcga cgggtatggg      1260

tccaccgagg ccggtggtgt gtggcgtgac ggagtcctgc agcgtccgcc cgtcaccgac      1320

tacaagctgg ttgacgttcc ggaactcgga tacttcacca cagattcgcc gcatccccgt      1380

ggcgagctgc ggttgaagtc ggagacgatg ttccccggct actacaagcg cccggagacc 1440

actgccgatg tcttcgatga cgaggggtac tacaagaccg gtgacgtggt cgccgagctc 1500

gggccggatc acctcaagta cctcgaccgc gtcaagaacg tcctcaagct cgcgcaggga 1560

gagtttgtcg cggtgtcaaa gctggaggcc gcttacaccg gcagcccgct ggtccggcag 1620

atctttgtgt acgggaacag tgaacgctcg ttcctgctgg ctgtcgtggt cccgacaccc 1680

gaagtccttg agcggtacgc agattcgcca gatgcgctca agcccttgat ccaggattcg 1740

ctgcagcagg tcgccaagga cgcggagctg caatcctatg agataccgcg cgacttcatc 1800

gttgagacgg tgccgttcac cgtcgagtcc ggattgctat cggacgcgcg aaagctgctg 1860

cgccccaagc tgaaggatca ctacggagag aggctggagg cgctgtacgc cgaactggcg 1920

gaaagccaga atgagcggct gcgccagttg gccagggagg cagccacgcg cccggtcctg 1980

gagacggtga ccgatgcggc cgccgcgctg ctgggcgcat cgtcctcgga tctggctcct 2040

gatgtgcgat tcatcgacct cggtggcgac tcactgtcgg cgctgtcgta ctccgagctg 2100

ctgcgcgaca tctttgaggt ggacgttccg gtgggcgtca tcaacagcgt cgccaacgac 2160

cttgccgcga tcgcccggca catcgaggcg cagcggaccg gcgccgctac gcagccgacc 2220

tttgcgtcgg tccacggcaa ggacgcgacg gtcatcaccg ccggtgaact caccctcgac 2280

aagttcttgg acgagtcact gttgaaagcg gccaaggacg ttcagccggc aacggccgat 2340

gtcaagaccg ttctagtgac cggcggcaac ggctggttgg gtcgttggct ggtgctcgat 2400

tggctggagc ggttggcacc caatggtggc aaggtctacg ccctcattcg tggcgccgat 2460

gccgaagcag cccgggcacg gttggacgcc gtgtacgaat cgggtgatcc caagctgtcc 2520

gcgcattatc gtcagctggc gcaacagagt ctggaagtta tcgccggcga tttcggcgac 2580

caggatctcg gtctatccca ggaagtttgg cagaagctgg ccaaggacgt ggacctgatc 2640

gtgcactccg gtgccttggt gaaccacgtg ctgccgtaca gccagttgtt cggtccgaat 2700

gtggcgggta ccgccgagat catcaagctg gcaatttcgg agcggctcaa gccggtcacc 2760

tacctgtcga cggtgggcat cgccgaccag attccggtga cggagttcga ggaagactcc 2820

gatgttcgtg tgatgtcggc cgagcgccag atcaatgacg gctacgcgaa cggatacggc 2880

aactcaaaat gggccggcga ggtgctgttg cgggaggctc atgacctagc ggggctgccg 2940

gtgcgtgtgt tccgctccga catgatcctg gcgcacagtg actaccacgg acagctcaac 3000

gtcaccgacg tgttcacccg gagcatccag agtctgctgc tcaccggtgt tgcaccggcc 3060

agcttctatg aattggatgc cgacggcaat cggcagcgcg ctcactatga cggtgtgccc 3120

ggcgatttca ccgccgcatc gatcaccgcc atcggcggtg tgaacgtggt agacggttac 3180

cgcagcttcg acgtgttcaa cccgcaccat gacggtgtct cgatggatac cttcgtcgac 3240

tggctgatcg acgcaggcta caagatcgcg cggatcgacg attacgacca gtggctcgcc 3300

cggttcgagc tggccctcaa gggattgccc gagcagcagc ggcaacagtc ggtgttgcca 3360

cttctcaaga tgtacgagaa gccgcaaccg gcgatcgacg gaagtgcact tccgaccgca 3420

gaattcagtc gcgccgtgca cgaggcgaag gtcggagaca gcggtgagat accgcacgtc 3480

accaaggagc tgatcctcaa gtacgccagc gatattcagc tgttgggcct ggtgtag 3537

<210> 106
<211> 224
<212> PRT
<213> Bacillus subtilis

<400> 106

Met Lys Ile Tyr Gly Ile Tyr Met Asp Arg Pro Leu Ser Gln Glu Glu
1               5                   10                  15

Asn Glu Arg Phe Met Thr Phe Ile Ser Pro Glu Lys Arg Glu Lys Cys
            20                  25                  30

Arg Arg Phe Tyr His Lys Glu Asp Ala His Arg Thr Leu Leu Gly Asp
        35                  40                  45

Val Leu Val Arg Ser Val Ile Ser Arg Gln Tyr Gln Leu Asp Lys Ser
    50                  55                  60

Asp Ile Arg Phe Ser Thr Gln Glu Tyr Gly Lys Pro Cys Ile Pro Asp
65                  70                  75                  80

Leu Pro Asp Ala His Phe Asn Ile Ser His Ser Gly Arg Trp Val Ile
                85                  90                  95

Gly Ala Phe Asp Ser Gln Pro Ile Gly Ile Asp Ile Glu Lys Thr Lys
            100                 105                 110

Pro Ile Ser Leu Glu Ile Ala Lys Arg Phe Phe Ser Lys Thr Glu Tyr
        115                 120                 125

Ser Asp Leu Leu Ala Lys Asp Lys Asp Glu Gln Thr Asp Tyr Phe Tyr
    130                 135                 140

His Leu Trp Ser Met Lys Glu Ser Phe Ile Lys Gln Glu Gly Lys Gly
145                 150                 155                 160

Leu Ser Leu Pro Leu Asp Ser Phe Ser Val Arg Leu His Gln Asp Gly
                165                 170                 175

Gln Val Ser Ile Glu Leu Pro Asp Ser His Ser Pro Cys Tyr Ile Lys
            180                 185                 190

Thr Tyr Glu Val Asp Pro Gly Tyr Lys Met Ala Val Cys Ala Ala His
        195                 200                 205

Pro Asp Phe Pro Glu Asp Ile Thr Met Val Ser Tyr Glu Glu Leu Leu
    210                 215                 220

<210>  107
<211>  222
<212>  PRT
<213>  Nocardia iowensis

<400>  107

Met Ile Glu Thr Ile Leu Pro Ala Gly Val Glu Ser Ala Glu Leu Leu
1               5                   10                  15

Glu Tyr Pro Glu Asp Leu Lys Ala His Pro Ala Glu Glu His Leu Ile
            20                  25                  30

Ala Lys Ser Val Glu Lys Arg Arg Arg Asp Phe Ile Gly Ala Arg His
        35                  40                  45

Cys Ala Arg Leu Ala Leu Ala Glu Leu Gly Glu Pro Pro Val Ala Ile
    50                  55                  60

Gly Lys Gly Glu Arg Gly Ala Pro Ile Trp Pro Arg Gly Val Val Gly
65                  70                  75                  80

Ser Leu Thr His Cys Asp Gly Tyr Arg Ala Ala Ala Val Ala His Lys
                85                  90                  95

Met Arg Phe Arg Ser Ile Gly Ile Asp Ala Glu Pro His Ala Thr Leu
            100                 105                 110

Pro Glu Gly Val Leu Asp Ser Val Ser Leu Pro Pro Glu Arg Glu Trp
        115                 120                 125

Leu Lys Thr Thr Asp Ser Ala Leu His Leu Asp Arg Leu Leu Phe Cys
    130                 135                 140

Ala Lys Glu Ala Thr Tyr Lys Ala Trp Trp Pro Leu Thr Ala Arg Trp
145                 150                 155                 160

Leu Gly Phe Glu Glu Ala His Ile Thr Phe Glu Ile Glu Asp Gly Ser
                165                 170                 175

Ala Asp Ser Gly Asn Gly Thr Phe His Ser Glu Leu Leu Val Pro Gly
            180                 185                 190

Gln Thr Asn Asp Gly Gly Thr Pro Leu Leu Ser Phe Asp Gly Arg Trp
        195                 200                 205

Leu Ile Ala Asp Gly Phe Ile Leu Thr Ala Ile Ala Tyr Ala
    210                 215                 220

<210>  108
<211>  272
<212>  PRT
<213>  Saccharomyces cerevisiae

<400>  108

Met Val Lys Thr Thr Glu Val Val Ser Glu Val Ser Lys Val Ala Gly
1               5                   10                  15

Val Arg Pro Trp Ala Gly Ile Phe Val Val Glu Ile Gln Glu Asp Ile
            20                  25                  30

Leu Ala Asp Glu Phe Thr Phe Glu Ala Leu Met Arg Thr Leu Pro Leu
        35                  40                  45

Ala Ser Gln Ala Arg Ile Leu Asn Lys Lys Ser Phe His Asp Arg Cys
    50                  55                  60

Ser Asn Leu Cys Ser Gln Leu Leu Gln Leu Phe Gly Cys Ser Ile Val
65                  70                  75                  80

Thr Gly Leu Asn Phe Gln Glu Leu Lys Phe Asp Lys Gly Ser Phe Gly
                85                  90                  95

Lys Pro Phe Leu Asp Asn Asn Arg Phe Leu Pro Phe Ser Met Thr Ile
            100                 105                 110

Gly Glu Gln Tyr Val Ala Met Phe Leu Val Lys Cys Val Ser Thr Asp
        115                 120                 125

Glu Tyr Gln Asp Val Gly Ile Asp Ile Ala Ser Pro Cys Asn Tyr Gly
    130                 135                 140

Gly Arg Glu Glu Leu Glu Leu Phe Lys Glu Val Phe Ser Glu Arg Glu
145                 150                 155                 160

Phe Asn Gly Leu Leu Lys Ala Ser Asp Pro Cys Thr Ile Phe Thr Tyr
                165                 170                 175

Leu Trp Ser Leu Lys Glu Ser Tyr Thr Lys Phe Thr Gly Thr Gly Leu
            180                 185                 190

Asn Thr Asp Leu Ser Leu Ile Asp Phe Gly Ala Ile Ser Phe Phe Pro
        195                 200                 205

Ala Glu Gly Ala Ser Met Cys Ile Thr Leu Asp Glu Val Pro Leu Ile
    210                 215                 220

Phe His Ser Gln Trp Phe Asn Asn Glu Ile Val Thr Ile Cys Met Pro
225                 230                 235                 240

Lys Ser Ile Ser Asp Lys Ile Asn Thr Asn Arg Pro Lys Leu Tyr Asn
                245                 250                 255

Ile Ser Leu Ser Thr Leu Ile Asp Tyr Phe Ile Glu Asn Asp Gly Leu
            260                 265                 270

<210>  109
<211>  669
<212>  DNA
<213>  Nocardia iowensis

<400>  109
atgatcgaga caattttgcc tgctggtgtc gagtcggctg agctgctgga gtatccggag      60

gacctgaagg cgcatccggc ggaggagcat ctcatcgcga agtcggtgga gaagcggcgc     120

cgggacttca tcggggccag gcattgtgcc cggctggcgc tggctgagct cggcgagccg     180

ccggtggcga tcggcaaagg ggagcggggt gcgccgatct ggccgcgcgg cgtcgtcggc     240

agcctcaccc attgcgacgg atatcgggcc gcggcggtgg cgcacaagat gcgcttccgt     300

tcgatcggca tcgatgccga gccgcacgcg acgctgcccg aaggcgtgct ggattcggtc     360

agcctgccgc cggagcggga gtggttgaag accaccgatt ccgcactgca cctggaccgt     420

ttactgttct gcgccaagga agccacctac aaggcgtggt ggccgctgac cgcgcgctgg     480

ctcggcttcg aggaagcgca catcaccttc gagatcgaag acggctccgc cgattccggc     540

aacggcacct ttcacagcga gctgctggtg ccgggacaga cgaatgacgg tgggacgccg     600

ctgctttcgt tcgacggccg gtggctgatc gccgacgggt tcatcctcac cgcgatcgcg     660

tacgcctga                                                             669

<210>  110
<211>  468
<212>  PRT
<213>  Escherichia coli

<400>  110

Met Ile Arg Glu Pro Pro Glu His Ile Leu Asn Arg Leu Pro Ser Ser
1               5                   10                  15

Ala Ser Ala Leu Ala Cys Ser Ala His Ala Leu Asn Leu Ile Glu Lys
            20                  25                  30

Arg Thr Leu Asp His Glu Glu Met Lys Ala Leu Asn Arg Glu Val Ile
        35                  40                  45

Glu Tyr Phe Lys Glu His Val Asn Pro Gly Phe Leu Glu Tyr Arg Lys
    50                  55                  60

Ser Val Thr Ala Gly Gly Asp Tyr Gly Ala Val Glu Trp Gln Ala Gly
65                  70                  75                  80

Ser Leu Asn Thr Leu Val Asp Thr Gln Gly Gln Glu Phe Ile Asp Cys
                85                  90                  95

Leu Gly Gly Phe Gly Ile Phe Asn Val Gly His Arg Asn Pro Val Val
            100                 105                 110

Val Ser Ala Val Gln Asn Gln Leu Ala Lys Gln Pro Leu His Ser Gln
        115                 120                 125

Glu Leu Leu Asp Pro Leu Arg Ala Met Leu Ala Lys Thr Leu Ala Ala
    130                 135                 140

Leu Thr Pro Gly Lys Leu Lys Tyr Ser Phe Phe Cys Asn Ser Gly Thr
145                 150                 155                 160

Glu Ser Val Glu Ala Ala Leu Lys Leu Ala Lys Ala Tyr Gln Ser Pro
                165                 170                 175

Arg Gly Lys Phe Thr Phe Ile Ala Thr Ser Gly Ala Phe His Gly Lys
            180                 185                 190

Ser Leu Gly Ala Leu Ser Ala Thr Ala Lys Ser Thr Phe Arg Lys Pro
        195                 200                 205

Phe Met Pro Leu Leu Pro Gly Phe Arg His Val Pro Phe Gly Asn Ile
    210                 215                 220

Glu Ala Met Arg Thr Ala Leu Asn Glu Cys Lys Lys Thr Gly Asp Asp
225                 230                 235                 240

Val Ala Ala Val Ile Leu Glu Pro Ile Gln Gly Glu Gly Gly Val Ile
                245                 250                 255

Leu Pro Pro Pro Gly Tyr Leu Thr Ala Val Arg Lys Leu Cys Asp Glu
            260                 265                 270

Phe Gly Ala Leu Met Ile Leu Asp Glu Val Gln Thr Gly Met Gly Arg
        275                 280                 285

Thr Gly Lys Met Phe Ala Cys Glu His Glu Asn Val Gln Pro Asp Ile
    290                 295                 300

Leu Cys Leu Ala Lys Ala Leu Gly Gly Gly Val Met Pro Ile Gly Ala
305                 310                 315                 320

Thr Ile Ala Thr Glu Glu Val Phe Ser Val Leu Phe Asp Asn Pro Phe
                325                 330                 335

Leu His Thr Thr Thr Phe Gly Gly Asn Pro Leu Ala Cys Ala Ala Ala
            340                 345                 350

Leu Ala Thr Ile Asn Val Leu Leu Glu Gln Asn Leu Pro Ala Gln Ala
        355                 360                 365

Glu Gln Lys Gly Asp Met Leu Leu Asp Gly Phe Arg Gln Leu Ala Arg
    370                 375                 380

Glu Tyr Pro Asp Leu Val Gln Glu Ala Arg Gly Lys Gly Met Leu Met
385                 390                 395                 400

Ala Ile Glu Phe Val Asp Asn Glu Ile Gly Tyr Asn Phe Ala Ser Glu
                405                 410                 415

Met Phe Arg Gln Arg Val Leu Val Ala Gly Thr Leu Asn Asn Ala Lys
            420                 425                 430

Thr Ile Arg Ile Glu Pro Pro Leu Thr Leu Thr Ile Glu Gln Cys Glu
        435                 440                 445

Leu Val Ile Lys Ala Ala Arg Lys Ala Leu Ala Ala Met Arg Val Ser
    450                 455                 460

Val Glu Glu Ala
465

<210>  111
<211>  456
<212>  PRT
<213>  Pseudomonas aeruginosa

<400>  111

Met Asn Ser Gln Ile Thr Asn Ala Lys Thr Arg Glu Trp Gln Ala Leu
1               5                   10                  15

Ser Arg Asp His His Leu Pro Pro Phe Thr Asp Tyr Lys Gln Leu Asn
            20                  25                  30

Glu Lys Gly Ala Arg Ile Ile Thr Lys Ala Glu Gly Val Tyr Ile Trp
        35                  40                  45

Asp Ser Glu Gly Asn Lys Ile Leu Asp Ala Met Ala Gly Leu Trp Cys
    50                  55                  60

Val Asn Val Gly Tyr Gly Arg Glu Glu Leu Val Gln Ala Ala Thr Arg
65                  70                  75                  80

Gln Met Arg Glu Leu Pro Phe Tyr Asn Leu Phe Phe Gln Thr Ala His
                85                  90                  95

Pro Pro Val Val Glu Leu Ala Lys Ala Ile Ala Asp Val Ala Pro Glu
            100                 105                 110

Gly Met Asn His Val Phe Phe Thr Gly Ser Gly Ser Glu Ala Asn Asp
        115                 120                 125

Thr Val Leu Arg Met Val Arg His Tyr Trp Ala Thr Lys Gly Gln Pro
    130                 135                 140

Gln Lys Lys Val Val Ile Gly Arg Trp Asn Gly Tyr His Gly Ser Thr
145                 150                 155                 160

Val Ala Gly Val Ser Leu Gly Gly Met Lys Ala Leu His Glu Gln Gly
                165                 170                 175

Asp Phe Pro Ile Pro Gly Ile Val His Ile Ala Gln Pro Tyr Trp Tyr
            180                 185                 190

Gly Glu Gly Gly Asp Met Ser Pro Asp Glu Phe Gly Val Trp Ala Ala
        195                 200                 205

Glu Gln Leu Glu Lys Lys Ile Leu Glu Val Gly Glu Glu Asn Val Ala
    210                 215                 220

Ala Phe Ile Ala Glu Pro Ile Gln Gly Ala Gly Gly Val Ile Val Pro
225                 230                 235                 240

Pro Asp Thr Tyr Trp Pro Lys Ile Arg Glu Ile Leu Ala Lys Tyr Asp
                245                 250                 255

Ile Leu Phe Ile Ala Asp Glu Val Ile Cys Gly Phe Gly Arg Thr Gly

260                 265                 270

Glu Trp Phe Gly Ser Gln Tyr Tyr Gly Asn Ala Pro Asp Leu Met Pro
        275                 280                 285

Ile Ala Lys Gly Leu Thr Ser Gly Tyr Ile Pro Met Gly Gly Val Val
    290                 295                 300

Val Arg Asp Glu Ile Val Glu Val Leu Asn Gln Gly Gly Glu Phe Tyr
305                 310                 315                 320

His Gly Phe Thr Tyr Ser Gly His Pro Val Ala Ala Ala Val Ala Leu
                325                 330                 335

Glu Asn Ile Arg Ile Leu Arg Glu Glu Lys Ile Ile Glu Lys Val Lys
            340                 345                 350

Ala Glu Thr Ala Pro Tyr Leu Gln Lys Arg Trp Gln Glu Leu Ala Asp
        355                 360                 365

His Pro Leu Val Gly Glu Ala Arg Gly Val Gly Met Val Ala Ala Leu
    370                 375                 380

Glu Leu Val Lys Asn Lys Lys Thr Arg Glu Arg Phe Thr Asp Lys Gly
385                 390                 395                 400

Val Gly Met Leu Cys Arg Glu His Cys Phe Arg Asn Gly Leu Ile Met
                405                 410                 415

Arg Ala Val Gly Asp Thr Met Ile Ile Ser Pro Pro Leu Val Ile Asp
            420                 425                 430

Pro Ser Gln Ile Asp Glu Leu Ile Thr Leu Ala Arg Lys Cys Leu Asp
        435                 440                 445

Gln Thr Ala Ala Ala Val Leu Ala
    450                 455

<210>  112
<211>  426
<212>  PRT
<213>  Escherichia coli

<400>  112

Met Asn Ser Asn Lys Glu Leu Met Gln Arg Arg Ser Gln Ala Ile Pro
1               5                   10                  15

Arg Gly Val Gly Gln Ile His Pro Ile Phe Ala Asp Arg Ala Glu Asn

20                  25                  30

Cys Arg Val Trp Asp Val Glu Gly Arg Glu Tyr Leu Asp Phe Ala Gly
        35                  40                  45

Gly Ile Ala Val Leu Asn Thr Gly His Leu His Pro Lys Val Val Ala
    50                  55                  60

Ala Val Glu Ala Gln Leu Lys Lys Leu Ser His Thr Cys Phe Gln Val
65                  70                  75                  80

Leu Ala Tyr Glu Pro Tyr Leu Glu Leu Cys Glu Ile Met Asn Gln Lys
                85                  90                  95

Val Pro Gly Asp Phe Ala Lys Lys Thr Leu Leu Val Thr Thr Gly Ser
            100                 105                 110

Glu Ala Val Glu Asn Ala Val Lys Ile Ala Arg Ala Ala Thr Lys Arg
        115                 120                 125

Ser Gly Thr Ile Ala Phe Ser Gly Ala Tyr His Gly Arg Thr His Tyr
    130                 135                 140

Thr Leu Ala Leu Thr Gly Lys Val Asn Pro Tyr Ser Ala Gly Met Gly
145                 150                 155                 160

Leu Met Pro Gly His Val Tyr Arg Ala Leu Tyr Pro Cys Pro Leu His
                165                 170                 175

Gly Ile Ser Glu Asp Asp Ala Ile Ala Ser Ile His Arg Ile Phe Lys
            180                 185                 190

Asn Asp Ala Ala Pro Glu Asp Ile Ala Ala Ile Val Ile Glu Pro Val
        195                 200                 205

Gln Gly Glu Gly Gly Phe Tyr Ala Ser Ser Pro Ala Phe Met Gln Arg
    210                 215                 220

Leu Arg Ala Leu Cys Asp Glu His Gly Ile Met Leu Ile Ala Asp Glu
225                 230                 235                 240

Val Gln Ser Gly Ala Gly Arg Thr Gly Thr Leu Phe Ala Met Glu Gln
                245                 250                 255

Met Gly Val Ala Pro Asp Leu Thr Thr Phe Ala Lys Ser Ile Ala Gly
            260                 265                 270

Gly Phe Pro Leu Ala Gly Val Thr Gly Arg Ala Glu Val Met Asp Ala
        275                 280                 285

Val Ala Pro Gly Gly Leu Gly Gly Thr Tyr Ala Gly Asn Pro Ile Ala
    290                 295                 300

Cys Val Ala Ala Leu Glu Val Leu Lys Val Phe Glu Gln Glu Asn Leu
305                 310                 315                 320

Leu Gln Lys Ala Asn Asp Leu Gly Gln Lys Leu Lys Asp Gly Leu Leu
                325                 330                 335

Ala Ile Ala Glu Lys His Pro Glu Ile Gly Asp Val Arg Gly Leu Gly
            340                 345                 350

Ala Met Ile Ala Ile Glu Leu Phe Glu Asp Gly Asp His Asn Lys Pro
        355                 360                 365

Asp Ala Lys Leu Thr Ala Glu Ile Val Ala Arg Ala Arg Asp Lys Gly
    370                 375                 380

Leu Ile Leu Leu Ser Cys Gly Pro Tyr Tyr Asn Val Leu Arg Ile Leu
385                 390                 395                 400

Val Pro Leu Thr Ile Glu Asp Ala Gln Ile Arg Gln Gly Leu Glu Ile
                405                 410                 415

Ile Ser Gln Cys Phe Asp Glu Ala Lys Gln
            420                 425

<210>  113
<211>  421
<212>  PRT
<213>  Escherichia coli

<400>  113

Met Ser Asn Asn Glu Phe His Gln Arg Arg Leu Ser Ala Thr Pro Arg
1               5                   10                  15

Gly Val Gly Val Met Cys Asn Phe Phe Ala Gln Ser Ala Glu Asn Ala
            20                  25                  30

Thr Leu Lys Asp Val Glu Gly Asn Glu Tyr Ile Asp Phe Ala Ala Gly
        35                  40                  45

Ile Ala Val Leu Asn Thr Gly His Arg His Pro Asp Leu Val Ala Ala
    50                  55                  60

Val Glu Gln Gln Leu Gln Gln Phe Thr His Thr Ala Tyr Gln Ile Val
65                  70                  75                  80

Pro Tyr Glu Ser Tyr Val Thr Leu Ala Glu Lys Ile Asn Ala Leu Ala
                85                  90                  95

Pro Val Ser Gly Gln Ala Lys Thr Ala Phe Phe Thr Thr Gly Ala Glu
            100                 105                 110

Ala Val Glu Asn Ala Val Lys Ile Ala Arg Ala His Thr Gly Arg Pro
        115                 120                 125

Gly Val Ile Ala Phe Ser Gly Gly Phe His Gly Arg Thr Tyr Met Thr
    130                 135                 140

Met Ala Leu Thr Gly Lys Val Ala Pro Tyr Lys Ile Gly Phe Gly Pro
145                 150                 155                 160

Phe Pro Gly Ser Val Tyr His Val Pro Tyr Pro Ser Asp Leu His Gly
                165                 170                 175

Ile Ser Thr Gln Asp Ser Leu Asp Ala Ile Glu Arg Leu Phe Lys Ser
            180                 185                 190

Asp Ile Glu Ala Lys Gln Val Ala Ala Ile Ile Phe Glu Pro Val Gln
        195                 200                 205

Gly Glu Gly Gly Phe Asn Val Ala Pro Lys Glu Leu Val Ala Ala Ile
    210                 215                 220

Arg Arg Leu Cys Asp Glu His Gly Ile Val Met Ile Ala Asp Glu Val
225                 230                 235                 240

Gln Ser Gly Phe Ala Arg Thr Gly Lys Leu Phe Ala Met Asp His Tyr
                245                 250                 255

Ala Asp Lys Pro Asp Leu Met Thr Met Ala Lys Ser Leu Ala Gly Gly
            260                 265                 270

Met Pro Leu Ser Gly Val Val Gly Asn Ala Asn Ile Met Asp Ala Pro
        275                 280                 285

Ala Pro Gly Gly Leu Gly Gly Thr Tyr Ala Gly Asn Pro Leu Ala Val
    290                 295                 300

Ala Ala Ala His Ala Val Leu Asn Ile Ile Asp Lys Glu Ser Leu Cys
305                 310                 315                 320

Glu Arg Ala Asn Gln Leu Gly Gln Arg Leu Lys Asn Thr Leu Ile Asp
                325                 330                 335

Ala Lys Glu Ser Val Pro Ala Ile Ala Ala Val Arg Gly Leu Gly Ser
            340                 345                 350

Met Ile Ala Val Glu Phe Asn Asp Pro Gln Thr Gly Glu Pro Ser Ala
        355                 360                 365

Ala Ile Ala Gln Lys Ile Gln Gln Arg Ala Leu Ala Gln Gly Leu Leu
    370                 375                 380

Leu Leu Thr Cys Gly Ala Tyr Gly Asn Val Ile Arg Phe Leu Tyr Pro
385                 390                 395                 400

Leu Thr Ile Pro Asp Ala Gln Phe Asp Ala Ala Met Lys Ile Leu Gln
                405                 410                 415

Asp Ala Leu Ser Asp
            420

<210>  114
<211>  464
<212>  PRT
<213>  Ruegeria pomeroyi

<400>  114

Met Ala Thr Ile Thr Asn His Met Pro Thr Ala Glu Leu Gln Ala Leu
1               5                   10                  15

Asp Ala Ala His His Leu His Pro Phe Ser Ala Asn Asn Ala Leu Gly
            20                  25                  30

Glu Glu Gly Thr Arg Val Ile Thr Arg Ala Arg Gly Val Trp Leu Asn
        35                  40                  45

Asp Ser Glu Gly Glu Glu Ile Leu Asp Ala Met Ala Gly Leu Trp Cys
    50                  55                  60

Val Asn Ile Gly Tyr Gly Arg Asp Glu Leu Ala Glu Val Ala Ala Arg
65                  70                  75                  80

Gln Met Arg Glu Leu Pro Tyr Tyr Asn Thr Phe Phe Lys Thr Thr His
                85                  90                  95

Val Pro Ala Ile Ala Leu Ala Gln Lys Leu Ala Glu Leu Ala Pro Gly
            100                 105                 110

Asp Leu Asn His Val Phe Phe Ala Gly Gly Gly Ser Glu Ala Asn Asp
        115                 120                 125

Thr Asn Ile Arg Met Val Arg Thr Tyr Trp Gln Asn Lys Gly Gln Pro
    130                 135                 140

Glu Lys Thr Val Ile Ile Ser Arg Lys Asn Ala Tyr His Gly Ser Thr
145                 150                 155                 160

Val Ala Ser Ser Ala Leu Gly Gly Met Ala Gly Met His Ala Gln Ser
                165                 170                 175

Gly Leu Ile Pro Asp Val His His Ile Asn Gln Pro Asn Trp Trp Ala
            180                 185                 190

Glu Gly Gly Asp Met Asp Pro Glu Glu Phe Gly Leu Ala Arg Ala Arg
        195                 200                 205

Glu Leu Glu Glu Ala Ile Leu Glu Leu Gly Glu Asn Arg Val Ala Ala
    210                 215                 220

Phe Ile Ala Glu Pro Val Gln Gly Ala Gly Gly Val Ile Val Ala Pro
225                 230                 235                 240

Asp Ser Tyr Trp Pro Glu Ile Gln Arg Ile Cys Asp Lys Tyr Asp Ile
                245                 250                 255

Leu Leu Ile Ala Asp Glu Val Ile Cys Gly Phe Gly Arg Thr Gly Asn
            260                 265                 270

Trp Phe Gly Thr Gln Thr Met Gly Ile Arg Pro His Ile Met Thr Ile
        275                 280                 285

Ala Lys Gly Leu Ser Ser Gly Tyr Ala Pro Ile Gly Gly Ser Ile Val
    290                 295                 300

Cys Asp Glu Val Ala His Val Ile Gly Lys Asp Glu Phe Asn His Gly
305                 310                 315                 320

Tyr Thr Tyr Ser Gly His Pro Val Ala Ala Ala Val Ala Leu Glu Asn
                325                 330                 335

Leu Arg Ile Leu Glu Glu Glu Asn Ile Leu Asp His Val Arg Asn Val
            340                 345                 350

Ala Ala Pro Tyr Leu Lys Glu Lys Trp Glu Ala Leu Thr Asp His Pro
        355                 360                 365

Leu Val Gly Glu Ala Lys Ile Val Gly Met Met Ala Ser Ile Ala Leu
    370                 375                 380

Thr Pro Asn Lys Ala Ser Arg Ala Lys Phe Ala Ser Glu Pro Gly Thr
385                 390                 395                 400

Ile Gly Tyr Ile Cys Arg Glu Arg Cys Phe Ala Asn Asn Leu Ile Met
                405                 410                 415

Arg His Val Gly Asp Arg Met Ile Ile Ser Pro Pro Leu Val Ile Thr
            420                 425                 430

Pro Ala Glu Ile Asp Glu Met Phe Val Arg Ile Arg Lys Ser Leu Asp
        435                 440                 445

Glu Ala Gln Ala Glu Ile Glu Lys Gln Gly Leu Met Lys Ser Ala Ala
    450                 455                 460

<210>  115
<211>  1407
<212>  DNA
<213>  Escherichia coli

<400>  115
atgatacgcg agcctccgga gcatattttg aacaggttac cttcgagcgc atcggcttta      60

gcgtgcagcg cccacgccct gaatctcatt gagaagcgaa cgctggatca tgaggagatg     120

aaagcactta accgagaggt gattgaatac ttcaaagagc atgtcaatcc ggggttttta     180

gagtatcgca aatctgttac cgccggcggg gattacggag ccgtagagtg gcaagcggga     240

agtttaaata cgcttgtcga cacccagggc caggagttta tcgactgcct gggaggtttt     300

ggaattttca acgtggggca ccgtaatcca gttgtggttt ccgccgtaca gaatcaactt     360

gcgaaacaac cgctgcacag ccaggagctg ctcgatccgt tacgggcgat gttggcgaaa     420

acccttgctg cgctaacgcc cggtaaactg aaatacagct tcttctgtaa tagcggcacc     480

gagtccgttg aagcagcgct gaagctggcg aaagcttacc agtcaccgcg cggcaagttt     540

acttttattg ccaccagcgg cgcgttccac ggtaaatcac ttggcgcgct gtcggccacg     600

gcgaaatcga ccttccgcaa accgtttatg ccgttactgc cgggcttccg tcatgtgccg     660

tttggcaata tcgaagccat gcgcacggct cttaacgagt gcaaaaaaac cggtgatgat     720

gtggctgcgg tgatcctcga accgattcag ggtgaaggtg gcgtaattct gccgccgccg     780

ggctatctca ccgccgtacg taagctatgc gatgagttcg gcgcactgat gatcctcgat     840

gaagtacaaa cgggcatggg gcgcacgggc aagatgttcg cctgcgagca tgagaacgta     900

cagccggata tcctctgcct tgccaaagcg ctcggcggcg gcgtgatgcc gattggcgcg     960

accatcgcca ctgaagaggt gttttcagtt ctgttcgaca acccattcct gcataccacc     1020

acctttggcg gcaacccgct ggcctgtgcg gcggcgctgg cgaccatcaa tgtgttgctg     1080

gagcagaact taccggctca ggctgagcaa aaaggcgata tgttgctgga cggtttccgt     1140

caactggcgc gggaatatcc cgatctggta caggaagcgc gtggtaaagg gatgttgatg     1200

gcgattgagt ttgttgataa cgaaatcggc tataactttg ccagcgagat gttccgccag     1260

cgcgtactgg tggccggaac gctcaataac gccaaaacga tccgcattga accgccactg     1320

acactgacca ttgaacagtg tgaactggtg atcaaagcgg cgcgtaaggc gctggcggcc     1380

atgcgagtaa gtgtcgaaga agcgtaa                                         1407

<210>  116
<211>  36
<212>  DNA
<213>  Artificial

<220>
<223>  forward primer

<400>  116
ttgttgaata aatcgtctgt ttgccgagaa tacgcg                               36

<210>  117
<211>  35
<212>  DNA
<213>  Artificial

<220>
<223>  reverse primer

<400>  117
tatcgcatgc agatctgcgc taatgggctc cagga                                35

<210>  118
<211>  35
<212>  DNA
<213>  Artificial

<220>
<223>  forward primer

<400>  118
ttgttgaata aatcgcgcgc cagcgctggc tgttt                                35

<210>  119
<211>  35
<212>  DNA
<213>  Artificial

<220>
<223>  reverse primer

<400>  119

tatcgcatgc agatcccgcc aatgccggaa gagtt                                35

<210>  120
<211>  35
<212>  DNA
<213>  Artificial

<220>
<223>  forward primer

<400>  120
ttgttgaata aatcgcgatc gtgatgccgc tgtct                                35

<210>  121
<211>  35
<212>  DNA
<213>  Artificial

<220>
<223>  reverse primer

<400>  121
tatcgcatgc agatcgacaa cgtaggcttt gttca                                35

<210>  122
<211>  35
<212>  DNA
<213>  Artificial

<220>
<223>  forward primer

<400>  122
ttgttgaata aatcgcgcca tctcgacact cttga                                35

<210>  123
<211>  35
<212>  DNA
<213>  Artificial

<220>
<223>  reverse primer

<400>  123
tatcgcatgc agatcgcgaa catcgatggg ttagc                                35

<210>  124
<211>  35
<212>  DNA
<213>  Artificial

<220>
<223>  forward primer

<400>  124
ttgttgaata aatcgtggca tttgcccttc ctgtt                                35

<210>  125
<211>  35
<212>  DNA
<213>  Artificial

<220>
<223>  reverse primer

<400>  125
tatcgcatgc agatcgtcct gatcctgcaa cggaa                                35


<210>  126
<211>  35
<212>  DNA
<213>  Artificial

<220>
<223>  forward primer

<400>  126
ttgttgaata aatcgctcat aacggtactg caaac                                35


<210>  127
<211>  35
<212>  DNA
<213>  Artificial

<220>
<223>  reverse primer

<400>  127
tatcgcatgc agatctcgca gcaggtaaga tgatt                                35


<210>  128
<211>  35
<212>  DNA
<213>  Artificial

<220>
<223>  forward primer

<400>  128
ttgttgaata aatcgatatt cgtcctaacg aacag                                35


<210>  129
<211>  35
<212>  DNA
<213>  Artificial

<220>
<223>  reverse primer

<400>  129
tatcgcatgc agatcttttt gattttcaag tatgt                                35


<210>  130
<211>  35
<212>  DNA

<213>  Artificial

<220>
<223>  primer

<400>  130
gatctgcatg cgatatctct agaacgcgta agctt                                35


<210>  131
<211>  29
<212>  DNA
<213>  Artificial

<220>
<223>  primer

<400>  131
tctcgagccg atttattcaa caaagccgc                                       29


<210>  132
<211>  25
<212>  DNA
<213>  Artificial

<220>
<223>  forward primer

<400>  132
actttcgttt tcgggcattt cgtcc                                           25


<210>  133
<211>  24
<212>  DNA
<213>  Artificial

<220>
<223>  reverse primer

<400>  133
ccaatatgag ggcagagaac gatc                                            24


<210>  134
<211>  20
<212>  DNA
<213>  Artificial

<220>
<223>  forward primer

<400>  134
atgcgctgat gtgataatgc                                                 20


<210>  135
<211>  19
<212>  DNA
<213>  Artificial

<220>

<223>  reverse primer

<400>  135
ccttctcctt gttgcttta                                                    19


<210>  136
<211>  20
<212>  DNA
<213>  Artificial

<220>
<223>  forward primer

<400>  136
gaggcctttg ctgcgactgc                                                   20


<210>  137
<211>  21
<212>  DNA
<213>  Artificial

<220>
<223>  reverse primer

<400>  137
agttcctcct tttcggatga t                                                 21


<210>  138
<211>  18
<212>  DNA
<213>  Artificial

<220>
<223>  forward primer

<400>  138
atgccggatg cgacgctt                                                     18


<210>  139
<211>  20
<212>  DNA
<213>  Artificial

<220>
<223>  reverse primer

<400>  139
tcattttgca tatagcccct                                                   20


<210>  140
<211>  20
<212>  DNA
<213>  Artificial

<220>
<223>  forward primer

<400>  140

tgacctgggc agtaatggtg                                                    20

<210>  141
<211>  25
<212>  DNA
<213>  Artificial

<220>
<223>  reverse primer

<400>  141
caggatctcc gttgctttat gagtc                                              25

<210>  142
<211>  24
<212>  DNA
<213>  Artificial

<220>
<223>  forward primer

<400>  142
cgtggtgttg aaagccgatt attg                                               24

<210>  143
<211>  23
<212>  DNA
<213>  Artificial

<220>
<223>  reverse primer

<400>  143
cataaacttc cagttctccg ccc                                                23

<210>  144
<211>  20
<212>  DNA
<213>  Artificial

<220>
<223>  forward primer

<400>  144
tcgcacacta acagactgaa                                                    20

<210>  145
<211>  20
<212>  DNA
<213>  Artificial

<220>
<223>  reverse primer

<400>  145
tgtgtttact cctgattagc                                                    20

<210>  146
<211>  25
<212>  DNA
<213>  Artificial

<220>
<223>  forward primer

<400>  146
tattctcaat ccgtttcagc acgcg                                          25


<210>  147
<211>  21
<212>  DNA
<213>  Artificial

<220>
<223>  reverse primer

<400>  147
ttcgggatca ccaccaggcc g                                              21


<210>  148
<211>  20
<212>  DNA
<213>  Artificial

<220>
<223>  forward primer

<400>  148
cggaaatgga cgaacagtgg                                                20


<210>  149
<211>  22
<212>  DNA
<213>  Artificial

<220>
<223>  reverse primer

<400>  149
cgtcatcagc gtttaccaga tt                                             22


<210>  150
<211>  20
<212>  DNA
<213>  Artificial

<220>
<223>  forward primer

<400>  150
gcataaacac tgtccgcgtc                                                20


<210>  151
<211>  24
<212>  DNA

<213>  Artificial

<220>
<223>  reverse primer

<400>  151
gaaatcgaga aggcagaagc gaaa                                              24


<210>  152
<211>  20
<212>  DNA
<213>  Artificial

<220>
<223>  forward primer

<400>  152
ggtgcggtca ccattgttcg                                                   20


<210>  153
<211>  20
<212>  DNA
<213>  Artificial

<220>
<223>  reverse primer

<400>  153
catatcgcac gccagcagtg                                                   20


<210>  154
<211>  21
<212>  DNA
<213>  Artificial

<220>
<223>  forward primer

<400>  154
ggtgaggatg gagagttcat g                                                 21


<210>  155
<211>  21
<212>  DNA
<213>  Artificial

<220>
<223>  reverse primer

<400>  155
cagttcgatt tgcgccacca g                                                 21


<210>  156
<211>  20
<212>  DNA
<213>  Artificial

<220>

<223>  forward primer

<400>  156
tccgctagtg tgatttcagg                                                    20


<210>  157
<211>  23
<212>  DNA
<213>  Artificial

<220>
<223>  reverse primer

<400>  157
gaaattatta tgccgccagg cgt                                                23


<210>  158
<211>  20
<212>  DNA
<213>  Artificial

<220>
<223>  forward primer

<400>  158
ttatggtctg ggcgacatgc                                                    20


<210>  159
<211>  22
<212>  DNA
<213>  Artificial

<220>
<223>  reverse primer

<400>  159
gcatcatcct ggtcatatac cc                                                 22


<210>  160
<211>  35
<212>  DNA
<213>  Artificial

<220>
<223>  primer

<400>  160
ataaggagat ataccatgat acgcgagcct ccgga                                   35


<210>  161
<211>  35
<212>  DNA
<213>  Artificial

<220>
<223>  primer

<400>  161

atgcggccgc aagctttacg cttcttcgac actta                                    35

<210>  162
<211>  35
<212>  DNA
<213>  Artificial

<220>
<223>  primer

<400>  162
taagaaggag atatacatat gactgaaacg atctc                                    35

<210>  163
<211>  35
<212>  DNA
<213>  Artificial

<220>
<223>  primer

<400>  163
gtggcagcag cctagctaca ccaggcccaa cagct                                    35

<210>  164
<211>  35
<212>  DNA
<213>  Artificial

<220>
<223>  primer

<400>  164
tttaaggagt tcgatatgat cgagacaatt ttgcc                                    35

<210>  165
<211>  35
<212>  DNA
<213>  Artificial

<220>
<223>  primer

<400>  165
ggtggcagca gcctagtcag gcgtacgcga tcgcg                                    35

<210>  166
<211>  20
<212>  DNA
<213>  Artificial

<220>
<223>  primer

<400>  166
ctaggctgct gccaccgctg                                                     20

<210> 167
<211> 40
<212> DNA
<213> Artificial

<220>
<223> primer

<400> 167
atcgaactcc ttaaatttat ctacaccagg cccaacagct 40